# EUROPEAN PATENT SPECIFICATION

(11) **EP 3 423 445 B1**
(45) Date of publication and mention of the grant of the patent: **01.04.2020**
(21) Application number: 17706783.2
(22) Date of filing: 23.02.2017
(51) Int. Cl.: C07D 401/14, C07D 403/04, A61P 35/00

(54) **NOVEL COMPOUNDS AND PHARMACEUTICAL COMPOSITIONS THEREOF FOR THE TREATMENT OF FIBROSIS**
NEUARTIGE VERBINDUNGEN UND PHARMAZEUTISCHE ZUSAMMENSETZUNGEN DAVON ZUR BEHANDLUNG VON FIBROSE
NOUVEAUX COMPOSÉS ET COMPOSITIONS PHARMACEUTIQUES ASSOCIÉES POUR LE TRAITEMENT DE LA FIBROSE

(30) Priority: 04.03.2016 GB 201603745
(43) Date of publication of application: 09.01.2019
(73) Proprietor: Galapagos NV, 2800 Mechelen (BE)
(72) Inventor: MAMMOLITI, Oscar, 2800 Mechelen (BE); JANSEN, Koen, Karel, 2800 Mechelen (BE); PALISSE, Adeline, Marie, Elise, 2800 Mechelen (BE); JOANNESSE, Caroline, Martine, Andrée-Marie, 2800 Mechelen (BE); MENET, Christel, Jeanne, Marie, 1050 Brussels (BE); ALLART, Brigitte, 2800 Mechelen (BE); EL BKASSINY, Sandy, 2800 Mechelen (BE)
(74) Representative: Bar, Grégory
(86) International application number: PCT/EP2017/054139
(87) International publication number: WO 2017/148787

(56) References cited:
- WO-A1-00/47207
- WO-A1-2009/042485

## Description

### FIELD OF THE INVENTION

The present invention relates to compounds useful in the prophylaxis and/or treatment of fibrotic diseases, inflammatory diseases, autoimmune diseases, metabolic diseases, cardiovascular diseases, and/or proliferative diseases. In particular, the compounds of the invention may be sphingosine 1-phosphate (S1P) receptor antagonists, a family of sphingosine receptors that are involved in fibrotic diseases, inflammatory diseases, autoimmune diseases, metabolic diseases, cardiovascular diseases, and/or proliferative diseases. The present invention also provides methods for the production of the compounds of the invention, pharmaceutical compositions comprising the compounds of the invention, and methods for the prophylaxis and/or treatment of fibrotic diseases, inflammatory diseases, autoimmune diseases, metabolic diseases, cardiovascular diseases, and/or proliferative diseases by administering the compounds of the invention.

### BACKGROUND OF THE INVENTION

Sphingolipids are structural components of all eukaryotic cell membranes. In the plasma membrane, they are commonly believed to protect the cell surface by forming the mechanically stable and chemically resistant outer leaflet of the lipid bilayer. All sphingolipids contain a sphingoid long-chain base (sphingosine) backbone, linked to a fatty acid molecule through an amide bond. Sphingosine-1-phosphate (SIP) is produced from sphingosine (2-amino-4-octadecene-1,3-diol; an aliphatic 18-carbon amino alcohol with an unsaturated hydrocarbon chain), by sphingosine kinases (Takabe et al., 2008).

SIP is a potent bioactive sphingolipid involved in cell proliferation, angiogenesis, inflammation and malignant transformation among other functions. SIP binds with low nano-molar affinity to five related G protein-coupled receptors, named SIP receptors (S1PR1, S1PR2, S1PR3, S1PR4, and S1PR5) (Adada et al., 2013; Milstien and Spiegel, 2006).

The S1PR1, S1PR2, and S1PR3 subtypes are widely expressed within the human body, whereas S1PR4 and S1PR5 show much more restricted tissue expression (Sobel et al., 2013).

However, whereas S1PR1, S1PR2, S1PR3, S1PR4, and S1PR5 are all involved in human physiology, S1PR2 appears to be particularly critical in the immune, nervous, metabolic, cardiovascular, musculoskeletal, and renal systems (Adada et al., 2013; Blankenbach et al., 2016).

In addition, there appears to be growing evidence that SIP and S1PR signalling generally plays a role in pro-fibrotic responses in various tissues and isolated cells. Indeed, using various SIP receptor agonists in normal lung fibroblasts, pro-fibrotic responses were observed via activation of S1PR2 and S1PR3, which suggests that antagonists of the specific SIP receptors S1P2R and S1P3R may be particularly beneficial in reducing fibrosis (Sobel et al., 2013).

Fibrosis is a process that can be triggered by chronic tissue damage because of toxic substances, viral infection, inflammation, or mechanical stress (Nanthakumar et al., 2015); and may be defined as the abnormal or excessive production and accumulation of extracellular matrix (ECM).

In particular, fibrosis is a key driver of progressive organ dysfunction in many inflammatory and metabolic diseases, including idiopathic pulmonary fibrosis, advanced liver disease (e.g. non-alcoholic steatohepatitis (NASH)) and advanced kidney disease. These conditions remain poorly treated despite advances in the understanding of the disease mechanism and, more recently, an increase in the number of clinical trials reflecting the need to identify new treatments, particularly in IPF (Nanthakumar et al., 2015). In the case of IPF for example, only two drugs have been approved therefore there is clear room for improved therapies (Raghu, 2015).

Therefore, current therapies are not satisfactory and in developing an effective therapeutic arsenal, novel modulators of SIPR, and in particular S1PR2 would be particularly beneficial for the prevention and or treatment of fibrotic diseases, inflammatory diseases, autoimmune diseases, metabolic diseases, cardiovascular diseases, and/or proliferative diseases.

### SUMMARY OF THE INVENTION

The present invention relates to compounds of the invention useful in the prophylaxis and/or treatment of fibrotic diseases, inflammatory diseases, autoimmune diseases, metabolic diseases, cardiovascular diseases, and/or proliferative diseases. The present invention also provides methods for the production of these compounds, pharmaceutical compositions comprising these compounds and methods for the prophylaxis and/or treatment of fibrotic diseases, inflammatory diseases, autoimmune diseases, metabolic diseases, cardiovascular diseases, and/or proliferative diseases by administering the compounds of the invention.

Accordingly, in a first aspect of the invention, the compounds of the invention are provided having a Formula Ia: 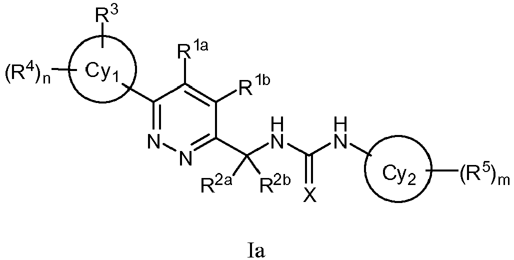 wherein
X is =O, or =N-CN;
R^{1a} is selected from:
   - C₁₋₄ alkyl optionally substituted with one or more groups independently selected from
      ∘ OH,
      ∘ C₁₋₄ alkoxy optionally substituted with one or more independently selected OH, or C₁₋₄ alkoxy,
      ∘ -SO₂-C₁₋₄ alkyl,
      ∘ -O-C₃₋₇ monocyclic cycloalkyl, and
      ∘ -O-heterocycloalkyl wherein said heterocycloalkyl is a 4-7 membered monocyclic heterocycloalkyl comprising one, two, or three heteroatoms independently selected from N, O, and S;
   - -NR^{6a}R^{6b},
   - C₁₋₄ alkoxy,
   - C₃₋₇ monocyclic cycloalkyl,
   - 4-7 membered monocyclic heterocycloalkyl comprising one, two, or three heteroatoms independently selected from N, O, and S, optionally substituted with one or more halo,
   - -O-C₃₋₇ monocyclic cycloalkyl, and
   - -O-heterocycloalkyl wherein said heterocycloalkyl is a 4-7 membered monocyclic heterocycloalkyl comprising one, two, or three heteroatoms independently selected from N, O, and S;
R^{1b} is H, or C₁₋₄ alkyl;
Cy₁ is a 5 membered monocyclic heteroaryl ring, comprising one, two, or three heteroatoms independently selected from N, O, or S, or
Cy₁ is 4-7 membered monocyclic heterocycloalkyl ring comprising one, two, or three heteroatoms independently selected from N, O, or S, or 4-7 membered monocyclic heterocycloalkyl ring comprising one, two, or three heteroatoms independently selected from N, O, or S, fused to a 5-6 membered heteroaryl ring comprising one, two, or three heteroatoms independently selected from N, O, or S, which heteroaryl may optionally substituted with one C₁₋₄ alkyl;
each R^{2a} and R^{2b} is independently selected from H, and C₁₋₄ alkyl optionally substituted with one or more independently selected -OH, or C₁₋₄ alkoxy;
R³ is selected from:
   - C₁₋₄ alkyl optionally substituted with one or more independently selected:
      ∘ halo,
      ∘ -CN,
      ∘ -OH,
      ∘ -C₁₋₄ alkoxy, or
      ∘ -NR^{7a}R^{7b};
   - C₁₋₄ alkoxy substituted with one or more halo,
   - C₃₋₇ monocyclic cycloalkyl,
   - 4-7-membered monocyclic heterocycloalkyl comprising one, two or three heteroatoms independently selected from N, O, and S,
   - -CN,
   - -S(O)₂-C₁₋₄ alkyl,
   - -NR^{8a}R^{8b}, and
   - -C(=O)NR^{8c}R^{8d};
each R⁴ is independently selected from:
   - C₁₋₄ alkyl optionally substituted with one or more independently selected R¹² groups,
   - C₃₋₇ monocyclic cycloalkyl, and
   - 4-7 membered monocyclic heterocycloalkyl comprising one, two, or three heteroatoms independently selected from N, O, and S;
Cy₂ is phenyl or 5-6 membered monocyclic heteroaryl comprising one or two heteroatoms independently selected from N, O, and S;
each R⁵ is independently selected from:
   - halo,
   - -CN,
   - -OH,
   - C₁₋₄ alkyl optionally substituted with one or more independently selected R¹³groups,
   - C₁₋₄ alkoxy optionally substituted with one or more independently selected R¹³groups,
   - C₃₋₇ monocyclic cycloalkyl optionally substituted with one or more independently selected R¹⁴ groups,
   - 4-11 membered monocyclic, or fused or spiro bicyclic heterocycloalkyl comprising one, two, or three heteroatoms independently selected from N, O, and S, optionally substituted with one or more independently selected R¹⁴ groups,
   - -O-C₃₋₇ monocyclic cycloalkyl optionally substituted with one or more independently selected R¹⁴ groups,
   - -O-heterocycloalkyl wherein said heterocycloalkyl is a 4-7 membered monocyclic heterocycloalkyl comprising one, two, or three heteroatoms independently selected from N, O, and S, optionally substituted with one or more independently selected R¹⁴ groups, and wherein if a N heteroatom is present, said N heteroatom, is further substituted with one -C(=O)-C₁₋₄ alkyl, -C(=O)-C₁₋₄ alkoxy, -SO₂-C₁₋₄ alkyl, -C(=O)-NH₂, -C(=O)NHC₁₋₄ alkyl, or -C(=O)N(C₁₋₄ alkyl)₂,
   - -SO₂-C₁₋₄ alkyl,
   - -SO₂NR^{15a}R^{15b},
   - -C(=O)NR^{15c}R^{15d}, and
   - -NR^{17a}R^{17b};
each R¹² is independently selected from:
   - halo,
   - OH,
   - C₁₋₄ alkoxy,
   - -SO₂-C₁₋₄ alkyl,
   - C₃₋₇ monocyclic cycloalkyl optionally substituted with one or more independently selected -OH, halo, -CN, C₁₋₄ alkyl, C₁₋₄ alkoxy or =O,
   - 4-7 membered monocyclic heterocycloalkyl, comprising one, two, or three heteroatoms independently selected from N, O, and S, optionally substituted with one or more independently selected -OH, halo, -CN, C₁₋₄ alkyl, C₁₋₄ alkoxy or =O,
   - -NR^{9a}R^{9b}, and
   - -CN;
each R¹³ is independently selected from:
   - halo,
   - -CN,
   - -OH,
   - C₁₋₄ alkoxy optionally substituted with one or more independently selected OH, C₁₋₄ alkoxy, or halo,
   - -C(=O)NR^{16a}R^{16b},
   - -NR^{16c}C(=O)-C₁₋₄ alkyl,
   - -NR^{16d}C(=O)-C₁₋₄ alkoxy,
   - -SO₂-C₁₋₄ alkyl
   - -SO₂NR^{16e}R^{16f},
   - -NR^{16g}SO₂-C₁₋₄ alkyl,
   - C₃₋₇ monocyclic cycloalkyl optionally substituted with one or more independently selected halo, or C₁₋₄ alkyl optionally substituted with one or more halo, and
   - 4-7 membered monocyclic heterocycloalkyl comprising one, two, or three heteroatoms independently selected from N, O, and S, optionally substituted with one or more independently selected halo, or C₁₋₄ alkyl optionally substituted with one or more halo, and wherein if a N heteroatom is present, said N heteroatom, is further substituted with one -C(=O)-C₁₋₄ alkyl, -C(=O)-C₁₋₄ alkoxy, -SO₂-C₁₋₄ alkyl, -C(=O)-NH₂, -C(=O)NHC₁₋₄ alkyl, or -C(=O)N(C₁₋₄ alkyl)₂;
each R¹⁴ is independently selected from:
   - halo,
   - -CN,
   - -OH,
   - C₁₋₄ alkoxy optionally substituted with one or more halo, and
   - C₁₋₄ alkyl optionally substituted with one or more halo;
each R^{6a}, R^{6b}, R^{7a}, R^{6b}, R^{8a}, R^{8b}, R^{8c}, R^{8d}, R^{9a}, R^{9b}, R^{15a}, R^{15b}, R^{15c}, R^{15d}, R^{16a}, R^{16b}, R^{16c}, R^{16d}, R^{16e}, R^{16f}, and R^{16g} is independently selected from H and C₁₋₄ alkyl;
each R^{17a} and R^{17b} is independently selected from H and C₁₋₄ alkyl optionally substituted with one or more independently selected halo, OH, or C₁₋₄ alkoxy;
the subscript n is 0, 1, 2, or 3; and
the subscript m is 0, 1, 2, 3 or 4;

In a further aspect of the invention, the compounds of the invention are provided having a Formula Ib: 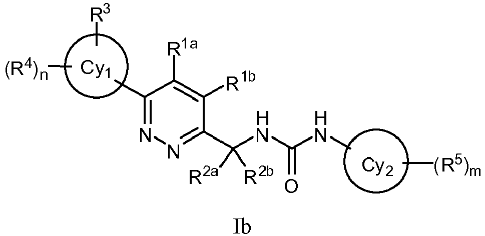 wherein
R^{1a} is selected from:
   - C₁₋₄ alkyl optionally substituted with one or more groups independently selected from:
      ∘ OH,
      ∘ C₁₋₄ alkoxy optionally substituted with one or more independently selected OH, or C₁₋₄ alkoxy,
      ∘ -SO₂-C₁₋₄ alkyl,
      ∘ -O-C₃₋₇ monocyclic cycloalkyl, and
      ∘ -O-heterocycloalkyl wherein said heterocycloalkyl is a 4-7 membered monocyclic heterocycloalkyl comprising one, two, or three heteroatoms independently selected from N, O, and S;
   - -NR^{6a}R^{6b},
   - C₁₋₄ alkoxy,
   - C₃₋₇ monocyclic cycloalkyl,
   - 4-7 membered monocyclic heterocycloalkyl comprising one, two, or three heteroatoms independently selected from N, O, or S, optionally substituted with one or more halo,
   - -O-C₃₋₇ monocyclic cycloalkyl, and
   - -O-heterocycloalkyl wherein said heterocycloalkyl is a 4-7 membered monocyclic heterocycloalkyl comprising one, two, or three heteroatoms independently selected from N, O, and S;
R^{1b} is H, or C₁₋₄ alkyl;
Cy₁ is a 5 membered monocyclic heteroaryl ring, comprising one, two, or three heteroatoms independently selected from N, O, and S;
each R^{2a} and R^{2b} is independently selected from H, and C₁₋₄ alkyl optionally substituted with one or more independently selected -OH, or C₁₋₄ alkoxy;
R³ is selected from:
   - C₁₋₄ alkyl optionally substituted with one or more groups independently selected from:
      ∘ halo,
      ∘ -CN,
      ∘ -C₁₋₄ alkoxy, and
      ∘ -NR^{7a}R^{7b};
   - C₁₋₄ alkoxy substituted with one or more halo,
   - C₃₋₇ monocyclic cycloalkyl,
   - 4-7-membered monocyclic heterocycloalkyl comprising one, two or three heteroatoms independently selected from N, O, and S,
   - -CN,
   - -NR^{8a}R^{8b}, and
   - -CONR^{8c}R^{8d};
each R⁴ is independently selected from:
   - C₁₋₄ alkyl optionally substituted with one or more independently selected R¹² groups,
   - C₃₋₇ monocyclic cycloalkyl, and
   - 4-7 membered monocyclic heterocycloalkyl comprising one, two, or three heteroatoms independently selected from N, O, and S;
Cy₂ is phenyl or 5-6 membered monocyclic heteroaryl comprising one or two heteroatoms independently selected from N, O, and S;
each R⁵ is independently selected from:
   - halo,
   - -CN,
   - -OH,
   - C₁₋₄ alkyl optionally substituted with one or more independently selected R¹³ groups,
   - C₁₋₄ alkoxy optionally substituted with one or more independently selected R¹³groups,
   - C₃₋₇ monocyclic cycloalkyl optionally substituted with one or more independently selected R¹⁴ groups,
   - 4-11 membered monocyclic, or fused or spiro bicyclic heterocycloalkyl comprising one, two, or three heteroatoms independently selected from N, O, and S, optionally substituted with one or more independently selected R¹⁴ groups,
   - -O-C₃₋₇ monocyclic cycloalkyl optionally substituted with one or more independently selected R¹⁴ groups,
   - -O-heterocycloalkyl wherein said heterocycloalkyl is a 4-7 membered monocyclic heterocycloalkyl comprising one, two, or three heteroatoms independently selected from N, O, and S, optionally substituted with one or more independently selected R¹⁴ groups, and wherein if a N heteroatom is present, said N heteroatom, is further substituted with one -C(=O)-C₁₋₄ alkyl, -C(=O)-C₁₋₄ alkoxy, -SO₂-C₁₋₄ alkyl, -C(=O)-NH₂, -C(=O)NHC₁₋₄ alkyl, or -C(=O)N(C₁₋₄ alkyl)₂,
   - -SO₂-C₁₋₄ alkyl,
   - -SO₂NR^{15a}R^{15b},
   - -C(=O)NR^{15e}R^{15d}, and
   - -NR^{17a}R^{17b};
each R¹² is independently selected from:
   - halo,
   - OH,
   - C₁₋₄ alkoxy,
   - -SO₂-C₁₋₄ alkyl,
   - C₃₋₇ monocyclic cycloalkyl optionally substituted with one or more independently selected -OH, halo, -CN, C₁₋₄ alkyl, C₁₋₄ alkoxy or =O,
   - 4-7 membered monocyclic heterocycloalkyl, comprising one, two, or three heteroatoms independently selected from N, O, and S, optionally substituted with one or more independently selected -OH, halo, -CN, C₁₋₄ alkyl, C₁₋₄ alkoxy or =O,
   - -NR^{9a}R^{9b}, and
   - -CN;
each R¹³ is independently selected from:
   - halo,
   - -CN,
   - -OH,
   - C₁₋₄ alkoxy optionally substituted with one or more independently selected OH, C₁₋₄ alkoxy, or halo
   - -C(=O)NR^{16a}R^{16b},
   - -NR^{16c}C(=O)-C₁₋₄ alkyl,
   - -NR^{16d}C(=O)-C₁₋₄ alkoxy,
   - -SO₂-C₁₋₄ alkyl
   - -SO₂NR^{16e}R^{16f},
   - -NR^{16g}SO₂-C₁₋₄ alkyl,
   - C₃₋₇ monocyclic cycloalkyl optionally substituted with one or more independently selected halo, or C₁₋₄ alkyl optionally substituted with one or more halo, and
   - 4-7 membered monocyclic heterocycloalkyl comprising one, two, or three heteroatoms independently selected from N, O, and S, optionally substituted with one or more independently selected halo, or C₁₋₄ alkyl optionally substituted with one or more halo, and wherein if a N heteroatom is present, said N heteroatom, is further substituted with one -C(=O)-C₁₋₄ alkyl, -C(=O)-C₁₋₄ alkoxy, -SO₂-C₁₋₄ alkyl, -C(=O)-NH₂, -C(=O)NHC₁₋₄ alkyl, or -C(=O)N(C₁₋₄ alkyl)₂;
each R¹⁴ is independently selected from:
   - halo,
   - -CN,
   - -OH,
   - C₁₋₄ alkoxy optionally substituted with one or more halo, and
   - C₁₋₄ alkyl optionally substituted with one or more halo;
each R^{6a}, R^{6b}, R^{7a}, R^{6b}, R^{8a}, R^{8b}, R^{8c}, R^{8d}, R^{9a}, R^{9b}, R^{15a}, R^{15b}, R^{15c}, R^{15d}, R^{16a}, R^{16b}, R^{16c}, R^{16d}, R^{16e}, R^{16f}, and R^{16g} is independently selected from H and C₁₋₄ alkyl;
each R^{17a} and R^{17b} is independently selected from H and C₁₋₄ alkyl optionally substituted with one or more independently selected halo, OH, or C₁₋₄ alkoxy;
the subscript n is 0, 1, 2, or 3; and
the subscript m is 0, 1, 2, 3 or 4.

In a particular aspect, the compounds of the invention are provided for use in the prophylaxis and/or treatment of fibrosis.

In a particular aspect the compounds of the invention are sphingosine 1-phosphate receptor (S1PR) modulators. In a more particular embodiment, the compounds of the invention are sphingosine 1-phosphate receptor 2 (S1PR2) antagonists. In a most particular embodiment, the compounds of the invention may show selectivity towards S1PR2, which in turn may be beneficial in reducing off-target related side effects.

In yet another aspect, the compounds of the invention may surprisingly show good ADME properties, in particular good metabolic stability which may result in improved oral bioavailability.

In a further aspect, the present invention provides pharmaceutical compositions comprising a compound of the invention, and a pharmaceutical carrier, excipient or diluent. In a particular aspect, the pharmaceutical composition may additionally comprise further therapeutically active ingredients suitable for use in combination with the compounds of the invention. In a more particular aspect, the further therapeutically active ingredient is an agent for the treatment of fibrotic diseases, inflammatory diseases, autoimmune diseases, metabolic diseases, cardiovascular diseases, and/or proliferative diseases.

Moreover, the compounds of the invention, useful in the pharmaceutical compositions and treatment methods disclosed herein, are pharmaceutically acceptable as prepared and used.

In a further aspect of the invention, this invention provides a method of treating a mammal, in particular humans, afflicted with a condition selected from among those listed herein, and particularly fibrotic diseases, inflammatory diseases, autoimmune diseases, metabolic diseases, cardiovascular diseases, and/or proliferative diseases, which method comprises administering an effective amount of the pharmaceutical composition or compounds of the invention as described herein.

The present invention also provides pharmaceutical compositions comprising a compound of the invention, and a suitable pharmaceutical carrier, excipient or diluent for use in medicine. In a particular aspect, the pharmaceutical composition is for use in the prophylaxis and/or treatment of fibrotic diseases, inflammatory diseases, autoimmune diseases, metabolic diseases, cardiovascular diseases, and/or proliferative diseases.

In additional aspects, this invention provides methods for synthesizing the compounds of the invention, with representative synthetic protocols and pathways disclosed later on herein.

Other objects and advantages will become apparent to those skilled in the art from a consideration of the ensuing detailed description.

It will be appreciated that compounds of the invention may be metabolized to yield biologically active metabolites.

### DETAILED DESCRIPTION OF THE INVENTION

### Definitions

The following terms are intended to have the meanings presented therewith below and are useful in understanding the description and intended scope of the present invention.

When describing the invention, which may include compounds, pharmaceutical compositions containing such compounds and methods of using such compounds and compositions, the following terms, if present, have the following meanings unless otherwise indicated. It should also be understood that when described herein any of the moieties defined forth below may be substituted with a variety of substituents, and that the respective definitions are intended to include such substituted moieties within their scope as set out below. Unless otherwise stated, the term "substituted" is to be defined as set out below. It should be further understood that the terms "groups" and "radicals" can be considered interchangeable when used herein.

The articles 'a' and 'an' may be used herein to refer to one or to more than one (*i.e.* at least one) of the grammatical objects of the article. By way of example 'an analogue' means one analogue or more than one analogue.

'Alkyl' means straight or branched aliphatic hydrocarbon having the specified number of carbon atoms. Particular alkyl groups have 1 to 6 carbon atoms or 1 to 4 carbon atoms. Branched means that one or more alkyl groups such as methyl, ethyl or propyl is attached to a linear alkyl chain. Particular alkyl groups are methyl (-CH₃), ethyl (-CH₂-CH₃), n-propyl (-CH₂-CH₂-CH₃), isopropyl (-CH(CH₃)₂), n-butyl (-CH₂-CH₂-CH₂-CH₃), tert-butyl (-CH₂-C(CH₃)₃), sec-butyl (-CH₂-CH(CH₃)₂), n-pentyl (-CH₂-CH₂-CH₂-CH₂-CH₃), n-hexyl (-CH₂-CH₂-CH₂-CH₂-CH₂-CH₃), and 1,2-dimethylbutyl (-CHCH₃)-C(CH₃)H₂-CH₂-CH₃). Particular alkyl groups have between 1 and 4 carbon atoms.

'Alkoxy' refers to the group O-alkyl, where the alkyl group has the number of carbon atoms specified. In particular the term refers to the group -O-C₁₋₆ alkyl. Particular alkoxy groups are methoxy, ethoxy, n-propoxy, isopropoxy, n-butoxy, tert-butoxy, sec-butoxy, n-pentoxy, n-hexoxy, and 1,2-dimethylbutoxy. Particular alkoxy groups are lower alkoxy, i.e. with between 1 and 6 carbon atoms. Further particular alkoxy groups have between 1 and 4 carbon atoms.

'Aryl' refers to a monovalent aromatic hydrocarbon group derived by the removal of one hydrogen atom from a single carbon atom of a parent aromatic ring system. In particular aryl refers to an aromatic ring structure, monocyclic or fused polycyclic, with the number of ring atoms specified. Specifically, the term includes groups that include from 6 to 10 ring members. Particular aryl groups include phenyl, and naphthyl.

'Cycloalkyl' refers to a non-aromatic hydrocarbyl ring structure, monocyclic, fused polycyclic, bridged polycyclic, or spirocyclic, with the number of ring atoms specified. A cycloalkyl may have from 3 to 12 carbon atoms, in particular from 3 to 10, and more particularly from 3 to 7 carbon atoms. Such cycloalkyl groups include, by way of example, single ring structures such as cyclopropyl, cyclobutyl, cyclopentyl, cyclohexyl, and cycloheptyl.

'Cyano' refers to the radical -CN.

'Halo' or 'halogen' refers to fluoro (F), chloro (CI), bromo (Br) and iodo (I). Particular halo groups are either fluoro or chloro.

'Hetero' when used to describe a compound or a group present on a compound means that one or more carbon atoms in the compound or group have been replaced by a nitrogen, oxygen, or sulfur heteroatom. Hetero may be applied to any of the hydrocarbyl groups described above such as alkyl, *e.g.* heteroalkyl, cycloalkyl, *e.g.* heterocycloalkyl, aryl, *e.g.* heteroaryl, and the like having from 1 to 4, and particularly from 1 to 3 heteroatoms, more typically 1 or 2 heteroatoms, for example a single heteroatom.

'Heteroaryl' means an aromatic ring structure, monocyclic or fused polycyclic, that includes one or more heteroatoms independently selected from O, N and S and the number of ring atoms specified. In particular, the aromatic ring structure may have from 5 to 9 ring members. The heteroaryl group can be, for example, a five membered or six membered monocyclic ring or a fused bicyclic structure formed from fused five and six membered rings or two fused six membered rings or, by way of a further example, two fused five membered rings. Each ring may contain up to four heteroatoms typically selected from nitrogen, sulphur and oxygen. Typically the heteroaryl ring will contain up to 4 heteroatoms, more typically up to 3 heteroatoms, more usually up to 2, for example a single heteroatom. In one embodiment, the heteroaryl ring contains at least one ring nitrogen atom. The nitrogen atoms in the heteroaryl rings can be basic, as in the case of an imidazole or pyridine, or essentially non-basic as in the case of an indole or pyrrole nitrogen. In general the number of basic nitrogen atoms present in the heteroaryl group, including any amino group substituents of the ring, will be less than five.

Examples of five membered monocyclic heteroaryl groups include but are not limited to pyrrolyl, furanyl, thiophenyl, imidazolyl, furazanyl, oxazolyl, oxadiazolyl, oxatriazolyl, isoxazolyl, thiazolyl, isothiazolyl, pyrazolyl, triazolyl and tetrazolyl groups.

Examples of six membered monocyclic heteroaryl groups include but are not limited to pyridinyl, pyrazinyl, pyridazinyl, pyrimidinyl and triazinyl.

Particular examples of bicyclic heteroaryl groups containing a five membered ring fused to another five-membered ring include but are not limited to imidazothiazolyl and imidazoimidazolyl.

Particular examples of bicyclic heteroaryl groups containing a six membered ring fused to a five membered ring include but are not limited to benzofuranyl, benzothiophenyl, benzoimidazolyl, benzoxazolyl, isobenzoxazolyl, benzisoxazolyl, benzothiazolyl, benzoisothiazolyl, isobenzofuranyl, indolyl, isoindolyl, indolizinyl, purinyl (*e.g.* adenine, guanine), indazolyl, pyrazolopyrimidinyl, triazolopyrimidinyl, and pyrazolopyridinyl groups.

Particular examples of bicyclic heteroaryl groups containing two fused six membered rings include but are not limited to quinolinyl, isoquinolinyl, pyridopyridinyl, quinoxalinyl, quinazolinyl, cinnolinyl, phthalazinyl, naphthyridinyl, and pteridinyl groups. Particular heteroaryl groups are those derived from thiophenyl, pyrrolyl, benzothiophenyl, benzofuranyl, indolyl, pyridinyl, quinolinyl, imidazolyl, oxazolyl and pyrazinyl.

Examples of representative heteroaryls include the following: 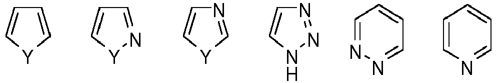 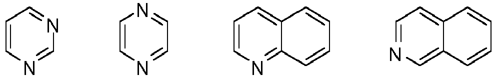 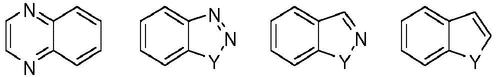 wherein each Y is selected from >C=O, NH, O and S.

'Heterocycloalkyl' means a non-aromatic fully saturated ring structure, monocyclic, fused polycyclic, spirocyclic, or bridged polycyclic, that includes one or more heteroatoms independently selected from O, N and S and the number of ring atoms specified. The heterocycloalkyl ring structure may have from 4 to 12 ring members, in particular from 4 to 10 ring members and more particularly from 4 to 7 ring members. Each ring may contain up to four heteroatoms typically selected from nitrogen, sulphur and oxygen. Typically the heterocycloalkyl ring will contain up to 4 heteroatoms, more typically up to 3 heteroatoms, more usually up to 2, for example a single heteroatom. Examples of heterocyclic rings include, but are not limited to azetidinyl, oxetanyl, thietanyl, pyrrolidinyl (*e.g.* 1-pyrrolidinyl, 2-pyrrolidinyl and 3-pyrrolidinyl), tetrahydrofuranyl (*e.g.* 1-tetrahydrofuranyl, 2-tetrahydrofuranyl and 3-tetrahydrofuranyl), tetrahydrothiophenyl (*e.g.* 1-tetrahydrothiophenyl, 2-tetrahydrothiophenyl and 3-tetrahydrothiophenyl), piperidinyl (*e.g.* 1-piperidinyl, 2-piperidinyl, 3-piperidinyl and 4-piperidinyl), tetrahydropyranyl (*e.g.* 4-tetrahydropyranyl), tetrahydrothiopyranyl (*e.g.* 4-tetrahydrothiopyranyl), morpholinyl, thiomorpholinyl, dioxanyl, or piperazinyl.

Particular examples of monocyclic rings are shown in the following illustrative examples: 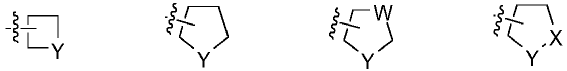 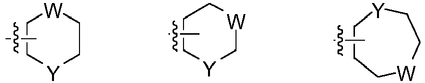 wherein each W and Y is independently selected from -CH₂-, -NH-, -O- and -S-.

Particular examples of fused bicyclic rings are shown in the following illustrative examples: 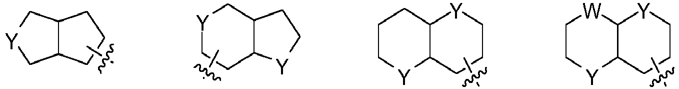 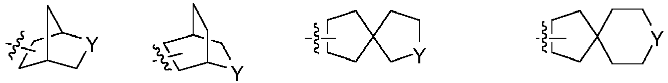 wherein each W and Y is independently selected from -CH₂-, -NH-, -O- and -S-.

Particular examples of bridged bicyclic rings are shown in the following illustrative examples: 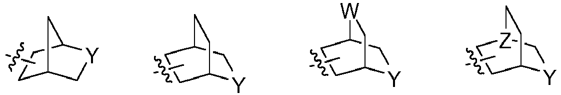 wherein each W and Y is independently selected from -CH₂-, -NH-, -O- and -S-, and Z is selected from N and CH

Particular examples of spirocyclic rings are shown in the following illustrative examples: 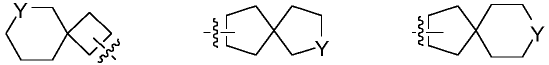 wherein each Y is selected from -CH₂-, -NH-, -O- and -S-.

'Hydroxyl' refers to the radical -OH.

'Oxo' refers to the radical =O.

'Substituted' refers to a group in which one or more hydrogen atoms are each independently replaced with the same or different substituent(s).

'Sulfo' or 'sulfonic acid' refers to a radical such as -SO₃H.

'Thiol' refers to the group -SH.

As used herein, term 'substituted with one or more' refers to one to four substituents. In one embodiment it refers to one to three substituents. In further embodiments it refers to one or two substituents. In a yet further embodiment it refers to one substituent.

'Thioalkoxy' refers to the group -S-alkyl where the alkyl group has the number of carbon atoms specified. In particular the term refers to the group -S-C₁₋₆ alkyl. Particular thioalkoxy groups are thiomethoxy, thioethoxy, n-thiopropoxy, isothiopropoxy, n-thiobutoxy, tert-thiobutoxy, sec-thiobutoxy, n-thiopentoxy, n-thiohexoxy, and 1,2-dimethylthiobutoxy. Particular thioalkoxy groups are lower thioalkoxy, *i.e.* with between 1 and 6 carbon atoms. Further particular alkoxy groups have between 1 and 4 carbon atoms.

One having ordinary skill in the art of organic synthesis will recognize that the maximum number of heteroatoms in a stable, chemically feasible heterocyclic ring, whether it is aromatic or non-aromatic, is determined by the size of the ring, the degree of unsaturation and the valence of the heteroatoms. In general, a heterocyclic ring may have one to four heteroatoms so long as the heteroaromatic ring is chemically feasible and stable.

'Pharmaceutically acceptable' means approved or approvable by a regulatory agency of the Federal or a state government or the corresponding agency in countries other than the United States, or that is listed in the U.S. Pharmacopoeia or other generally recognized pharmacopoeia for use in animals, and more particularly, in humans.

'Pharmaceutically acceptable salt' refers to a salt of a compound of the invention that is pharmaceutically acceptable and that possesses the desired pharmacological activity of the parent compound. In particular, such salts are non-toxic may be inorganic or organic acid addition salts and base addition salts. Specifically, such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, *e.g.* an alkali metal ion, an alkaline earth ion, or an aluminum ion; or coordinates with an organic base such as ethanolamine, diethanolamine, triethanolamine, N-methylglucamine and the like. Salts further include, by way of example only, sodium, potassium, calcium, magnesium, ammonium, tetraalkylammonium, and the like; and when the compound contains a basic functionality, salts of non-toxic organic or inorganic acids, such as hydrochloride, hydrobromide, tartrate, mesylate, acetate, maleate, oxalate and the like. The term 'pharmaceutically acceptable cation' refers to an acceptable cationic counter-ion of an acidic functional group. Such cations are exemplified by sodium, potassium, calcium, magnesium, ammonium, tetraalkylammonium cations, and the like.

'Pharmaceutically acceptable vehicle' refers to a diluent, adjuvant, excipient or carrier with which a compound of the invention is administered.

'Prodrugs' refers to compounds, including derivatives of the compounds of the invention,which have cleavable groups and become by solvolysis or under physiological conditions the compounds of the invention which are pharmaceutically active *in vivo.* Such examples include, but are not limited to, choline ester derivatives and the like, N-alkylmorpholine esters and the like.

'Solvate' refers to forms of the compound that are associated with a solvent, usually by a solvolysis reaction. This physical association includes hydrogen bonding. Conventional solvents include water, EtOH, acetic acid and the like. The compounds of the invention may be prepared *e.g.* in crystalline form and may be solvated or hydrated. Suitable solvates include pharmaceutically acceptable solvates, such as hydrates, and further include both stoichiometric solvates and non-stoichiometric solvates. In certain instances the solvate will be capable of isolation, for example when one or more solvent molecules are incorporated in the crystal lattice of the crystalline solid. 'Solvate' encompasses both solution-phase and isolable solvates. Representative solvates include hydrates, ethanolates and methanolates.

'Subject' includes humans. The terms 'human', 'patient' and 'subject' are used interchangeably herein.

'Effective amount' means the amount of a compound of the invention that, when administered to a subject for treating a disease, is sufficient to effect such treatment for the disease. The "effective amount" can vary depending on the compound, the disease and its severity, and the age, weight, etc., of the subject to be treated.

'Preventing' or 'prevention' refers to a reduction in risk of acquiring or developing a disease or disorder (*i.e.* causing at least one of the clinical symptoms of the disease not to develop in a subject that may be exposed to a disease-causing agent, or predisposed to the disease in advance of disease onset.

The term 'prophylaxis' is related to 'prevention', and refers to a measure or procedure the purpose of which is to prevent, rather than to treat or cure a disease. Non-limiting examples of prophylactic measures may include the administration of vaccines; the administration of low molecular weight heparin to hospital patients at risk for thrombosis due, for example, to immobilization; and the administration of an anti-malarial agent such as chloroquine, in advance of a visit to a geographical region where malaria is endemic or the risk of contracting malaria is high.

'Treating' or 'treatment' of any disease or disorder refers, in one embodiment, to ameliorating the disease or disorder (*i.e.* arresting the disease or reducing the manifestation, extent or severity of at least one of the clinical symptoms thereof). In another embodiment 'treating' or 'treatment' refers to ameliorating at least one physical parameter, which may not be discernible by the subject. In yet another embodiment, 'treating' or 'treatment' refers to modulating the disease or disorder, either physically, (*e.g.* stabilization of a discernible symptom), physiologically, (*e.g.* stabilization of a physical parameter), or both. In a further embodiment, "treating" or "treatment" relates to slowing the progression of the disease.

As used herein the term 'asthma' as used herein refers to any disease of the lungs characterized by variations in pulmonary gas flow associated with airway constriction of whatever cause (intrinsic, extrinsic, or both; allergic or non-allergic). The term asthma may be used with one or more adjectives to indicate the cause.

As used herein the term 'fibrotic diseases' refers to diseases characterized by excessive scarring due to excessive production, deposition, and contraction of extracellular matrix, and are that are associated with the abnormal accumulation of cells and/or fibronectin and/or collagen and/or increased fibroblast recruitment and include but are not limited to fibrosis of individual organs or tissues such as the heart, kidney, liver, joints, lung, pleural tissue, peritoneal tissue, skin, cornea, retina, musculoskeletal and digestive tract. In particular, the term fibrotic diseases refers to idiopathic pulmonary fibrosis (IPF); cystic fibrosis, other diffuse parenchymal lung diseases of different etiologies including iatrogenic drug-induced fibrosis, occupational and/or environmental induced fibrosis, granulomatous diseases (sarcoidosis, hypersensitivity pneumonia), collagen vascular disease, alveolar proteinosis, langerhans cell granulomatosis, lymphangioleiomyomatosis, inherited diseases (Hermansky-Pudlak Syndrome, tuberous sclerosis, neurofibromatosis, metabolic storage diseases, familial interstitial lung disease); radiation induced fibrosis; chronic obstructive pulmonary disease; scleroderma; bleomycin induced pulmonary fibrosis; chronic asthma; silicosis; asbestos induced pulmonary fibrosis; acute respiratory distress syndrome (ARDS); kidney fibrosis; tubulointerstitium fibrosis; glomerular nephritis; diabetic nephropathy, focal segmental glomerular sclerosis; IgA nephropathy; hypertension; Alport; gut fibrosis; liver fibrosis; cirrhosis; alcohol induced liver fibrosis; toxic/drug induced liver fibrosis; hemochromatosis; nonalcoholic steatohepatitis (NASH); cholestasis, biliary duct injury; primary biliary cirrhosis; infection induced liver fibrosis; viral induced liver fibrosis; and autoimmune hepatitis; corneal scarring; hypertrophic scarring; Dupuytren disease, keloids, cutaneous fibrosis; cutaneous scleroderma; systemic sclerosis, spinal cord injury/fibrosis; myelofibrosis; Duchenne muscular dystrophy (DMD) associated musculoskeletal fibrosis, vascular restenosis; atherosclerosis; arteriosclerosis; Wegener's granulomatosis; Peyronie's disease, or chronic lymphocytic. More particularly, the term "fibrotic diseases" refers to idiopathic pulmonary fibrosis (IPF), Dupuytren disease, nonalcoholic steatohepatitis (NASH), portal hypertension, systemic sclerosis, renal fibrosis, and cutaneous fibrosis.

As used herein the term 'inflammatory disease(s)' refers to the group of conditions including, rheumatoid arthritis (RA), osteoarthritis (OA), juvenile idiopathic arthritis, psoriasis, psoriatic arthritis, ankylosing spondylitis, allergic airway disease (*e.g.* asthma, rhinitis), chronic obstructive pulmonary disease (COPD), inflammatory bowel diseases (IBD) (*e.g.* Crohn's disease, ulcerative colitis), endotoxin-driven disease states (*e.g.* complications after bypass surgery or chronic endotoxin states contributing to *e.g.* chronic cardiac failure), and related diseases involving cartilage, such as that of the joints. Particularly the term refers to rheumatoid arthritis, osteoarthritis, allergic airway disease (*e.g.* asthma), chronic obstructive pulmonary disease and inflammatory bowel diseases. More particularly the term refers to rheumatoid arthritis, osteoarthritis, allergic airway disease, chronic obstructive pulmonary disease and inflammatory bowel diseases.

As used herein the term 'autoimmune disease(s)' refers to the group of diseases including obstructive airways disease, including conditions such as chronic obstructive pulmonary disease (COPD), asthma (e.g intrinsic asthma, extrinsic asthma, dust asthma, infantile asthma) particularly chronic or inveterate asthma (for example late asthma and airway hyperreponsiveness), bronchitis, including bronchial asthma, systemic lupus erythematosus (SLE), cutaneous lupus erythematosus, lupus nephritis, dermatomyositis, Sjogren's syndrome, multiple sclerosis, psoriasis, dry eye disease, type I diabetes mellitus and complications associated therewith, atopic eczema (atopic dermatitis), thyroiditis (Hashimoto's and autoimmune thyroiditis), contact dermatitis and further eczematous dermatitis, inflammatory bowel disease (*e.g.* Crohn's disease and ulcerative colitis), atherosclerosis and amyotrophic lateral sclerosis. Particularly the term refers to chronic obstructive pulmonary disease, asthma, systemic lupus erythematosus, type I diabetes mellitus and inflammatory bowel disease. More particularly, the term refers to chronic obstructive pulmonary disease, asthma, systemic lupus erythematosus, type I diabetes mellitus and inflammatory bowel disease.

As used herein the term 'proliferative disease(s)' refers to conditions such as cancer (e.g. uterine leiomyosarcoma or prostate cancer), myeloproliferative diseases (e.g. polycythemia vera, essential thrombocytosis and myelofibrosis), leukemia (e.g. acute myeloid leukaemia, acute and chronic lymphoblastic leukemia), multiple myeloma, psoriasis, restenosis, scleroderma or fibrosis. In particular the term refers to cancer, leukemia, multiple myeloma and psoriasis.

As used herein, the term 'cancer' refers to a malignant or benign growth of cells in skin or in body organs, for example but without limitation, breast, prostate, lung, kidney, pancreas, stomach or bowel. A cancer tends to infiltrate into adjacent tissue and spread (metastasise) to distant organs, for example to bone, liver, lung or the brain. As used herein the term cancer includes both metastatic tumour cell types (such as but not limited to, melanoma, lymphoma, leukaemia, fibrosarcoma, rhabdomyosarcoma, and mastocytoma) and types of tissue carcinoma (such as but not limited to, colorectal cancer, prostate cancer, small cell lung cancer and non-small cell lung cancer, breast cancer, pancreatic cancer, bladder cancer, renal cancer, gastric cancer, glioblastoma, primary liver cancer, ovarian cancer, prostate cancer and uterine leiomyosarcoma). In particular, the term 'cancer' refers to acute lymphoblastic leukemia, acute myeloidleukemia, adrenocortical carcinoma, anal cancer, appendix cancer, astrocytomas, atypical teratoid/rhabdoid tumor, basal cell carcinoma, bile duct cancer, bladder cancer, bone cancer (osteosarcoma and malignant fibrous histiocytoma), brain stem glioma, brain tumors, brain and spinal cord tumors, breast cancer, bronchial tumors, Burkitt lymphoma, cervical cancer, chronic lymphocytic leukemia, chronic myelogenous leukemia, colon cancer, colorectal cancer, craniopharyngioma, cutaneous T -Cell lymphoma, embryonal tumors, endometrial cancer, ependymoblastoma, ependymoma, esophageal cancer, ewing sarcoma family of tumors, eye cancer, retinoblastoma, gallbladder cancer, gastric (stomach) cancer, gastrointestinal carcinoid tumor, gastrointestinal stromal tumor (GIST), gastrointestinal stromal cell tumor, germ cell tumor, glioma, hairy cell leukemia, head and neck cancer, hepatocellular (liver) cancer, hodgkin lymphoma, hypopharyngeal cancer, intraocular melanoma, islet cell tumors (endocrine pancreas), Kaposi sarcoma, kidney cancer, Langerhans cell histiocytosis, laryngeal cancer, leukemia, Acute lymphoblastic leukemia, acute myeloid leukemia, chronic lymphocytic leukemia, chronic myelogenous leukemia, hairy cell leukemia, liver cancer, non-small cell lung cancer, small cell lung cancer, Burkitt lymphoma, cutaneous T-celllymphoma, Hodgkin lymphoma, non-Hodgkin lymphoma, lymphoma, Waldenstrom macroglobulinemia, medulloblastoma, medulloepithelioma, melanoma, mesothelioma, mouth cancer, chronic myelogenous leukemia, myeloid leukemia, multiple myeloma, asopharyngeal cancer, neuroblastoma, non-Hodgkin lymphoma, non-small cell lung cancer, oral cancer, oropharyngeal cancer, osteosarcoma, malignant fibrous histiocytoma of bone, ovarian cancer, ovarian epithelial cancer, ovarian germ cell tumor, ovarian low malignant potential tumor, pancreatic cancer, papillomatosis, parathyroid cancer, penile cancer, pharyngeal cancer, pineal parenchymal tumors of intermediate differentiation, pineoblastoma and supratentorial primitive neuroectodermal tumors, pituitary tumor, plasma cell neoplasm/multiple myeloma, pleuropulmonary blastoma, primary central nervous system lymphoma, prostate cancer, rectal cancer, renal cell (kidney) cancer, retinoblastoma, rhabdomyosarcoma, salivary gland cancer, sarcoma, Ewing sarcoma family of tumors, sarcoma, kaposi, Sezary syndrome, skin cancer, small cell Lung cancer, small intestine cancer, soft tissue sarcoma, squamous cell carcinoma, stomach (gastric) cancer, supratentorial primitive neuroectodermal tumors, T -cell lymphoma, testicular cancer, throat cancer, thymoma and thymic carcinoma, thyroid cancer, urethral cancer, uterine cancer, uterine sarcoma, vaginal cancer, vulvar cancer, Waldenstrom macroglobulinemia, and Wilms tumor.

As used herein the term 'leukemia' refers to neoplastic diseases of the blood and blood forming organs. Such diseases can cause bone marrow and immune system dysfunction, which renders the host highly susceptible to infection and bleeding. In particular the term leukemia refers to acute myeloid leukaemia (AML), and acute lymphoblastic leukemia (ALL) and chronic lymphoblastic leukaemia (CLL).

As used herein the term 'metabolic disease(s)' refers to the group of conditions affecting the body's ability to process certain nutrients and vitamins. Some examples of metabolic disease include cystic fibrosis, phenylketonuria (PKU), diabetes, hyperlipidemia, gout, and rickets. A particular example of metabolic disorders is obesity and/or diabetes.

As used herein the term "cardiovascular diseases" refers to diseases affecting the heart or blood vessels or both. In particular, cardiovascular disease includes arrhythmia (atrial or ventricular or both); atherosclerosis and its sequelae; angina; cardiac rhythm disturbances; myocardial ischemia; myocardial infarction; cardiac or vascular aneurysm; vasculitis, giant cell arteritis, stroke; peripheral obstructive arteriopathy of a limb, an organ, or a tissue; reperfusion injury following ischemia of the brain, heart, kidney or other organ or tissue; endotoxic, surgical, or traumatic shock; hypertension, valvular heart disease, heart failure, abnormal blood pressure; shock; vasoconstriction (including that associated with migraines); vascular abnormality, inflammation, insufficiency limited to a single organ or tissue. In particular the term refers to stroke, vasculitis, angina, atherosclerosis, or peripheral obstructive arteriopathy. More particularly, the term refers to stroke, or vasculitis.

'Compound(s) of the invention', and equivalent expressions, are meant to embrace compounds of the Formula(e) as herein described, which expression includes the pharmaceutically acceptable salts, and the solvates, *e.g*. hydrates, and the solvates of the pharmaceutically acceptable salts where the context so permits. Similarly, reference to intermediates, whether or not they themselves are claimed, is meant to embrace their salts, and solvates, where the context so permits.

When ranges are referred to herein, for example but without limitation, C₁₋₈ alkyl, the citation of a range should be considered a representation of each member of said range.

Other derivatives of the compounds of this invention have activity in both their acid and acid derivative forms, but in the acid sensitive form often offers advantages of solubility, tissue compatibility, or delayed release in the mammalian organism (Bundgard, H, 1985). Prodrugs include acid derivatives well known to practitioners of the art, such as, for example, esters prepared by reaction of the parent acid with a suitable alcohol, or amides prepared by reaction of the parent acid compound with a substituted or unsubstituted amine, or acid anhydrides, or mixed anhydrides. Simple aliphatic or aromatic esters, amides and anhydrides derived from acidic groups pendant on the compounds of this invention are particularly useful prodrugs. In some cases it is desirable to prepare double ester type prodrugs such as (acyloxy)alkyl esters or ((alkoxycarbonyl)oxy)alkylesters. Particular such prodrugs are the C₁₋₈ alkyl, C₂₋₈ alkenyl, C₆₋₁₀ optionally substituted aryl, and (C₆₋₁₀ aryl)-(C₁₋₄ alkyl) esters of the compounds of the invention.

As used herein, the term 'isotopic variant' refers to a compound that contains unnatural proportions of isotopes at one or more of the atoms that constitute such compound. For example, an 'isotopic variant' of a compound can contain one or more non-radioactive isotopes, such as for example, deuterium (²H or D), carbon-13 (¹³C), nitro (¹⁵N), or the like. It will be understood that, in a compound where such isotopic substitution is made, the following atoms, where present, may vary, so that for example, any hydrogen may be ²H/D, any carbon may be ¹³C, or any nitrogen may be ¹⁵N, and that the presence and placement of such atoms may be determined within the skill of the art. Likewise, the invention may include the preparation of isotopic variants with radioisotopes, in the instance for example, where the resulting compounds may be used for drug and/or substrate tissue distribution studies. The radioactive isotopes tritium, i.e. ³H, and carbon-14, i.e. ¹⁴C, are particularly useful for this purpose in view of their ease of incorporation and ready means of detection. Further, compounds may be prepared that are substituted with positron emitting isotopes, such as ¹¹C, ¹⁸F, ¹⁵O and ¹³N, and would be useful in Positron Emission Topography (PET) studies for examining substrate receptor occupancy.

It is also to be understood that compounds that have the same molecular formula but differ in the nature or sequence of bonding of their atoms or the arrangement of their atoms in space are termed 'isomers'. Isomers that differ in the arrangement of their atoms in space are termed 'stereoisomers'.

Stereoisomers that are not mirror images of one another are termed 'diastereomers' and those that are non-superimposable mirror images of each other are termed 'enantiomers'. When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (i.e. as (+) or (-)-isomers respectively). A chiral compound can exist as either individual enantiomer or as a mixture thereof. A mixture containing equal proportions of the enantiomers is called a 'racemic mixture'.

'Tautomers' refer to compounds that are interchangeable forms of a particular compound structure, and that vary in the displacement of hydrogen atoms and electrons. Thus, two structures may be in equilibrium through the movement of π electrons and an atom (usually H). For example, enols and ketones are tautomers because they are rapidly interconverted by treatment with either acid or base. Another example of tautomerism is the aci- and nitro- forms of phenylnitromethane, that are likewise formed by treatment with acid or base.

Tautomeric forms may be relevant to the attainment of the optimal chemical reactivity and biological activity of a compound of interest.

The compounds of the invention may possess one or more asymmetric centers; such compounds can therefore be produced as individual (R)- or (S)- stereoisomers or as mixtures thereof.

Unless indicated otherwise, the description or naming of a particular compound in the specification and claims is intended to include both individual enantiomers and mixtures, racemic or otherwise, thereof. The methods for the determination of stereochemistry and the separation of stereoisomers are well-known in the art.

It will be appreciated that compounds of the invention may be metabolized to yield biologically active metabolites.

### THE INVENTION

The present invention is based on the identification of novel compounds, and their ability to act as sphingosine 1-phosphate (SIP) receptor antagonists, which may be useful in the prophylaxis and/or treatment of fibrotic diseases, inflammatory diseases, autoimmune diseases, metabolic diseases, and/or proliferative diseases.

The present invention also provides methods for the production of these compounds, pharmaceutical compositions comprising these compounds and methods for the prophylaxis and/or treatment of inflammatory diseases, autoimmune diseases, metabolic diseases, and/or proliferative diseases by administering the compounds of the invention.

Accordingly, in a first aspect of the invention, the compounds of the invention are provided having a Formula Ia: 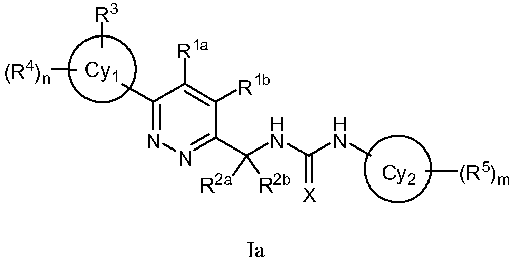 wherein
X is =O, or =N-CN;
R^{1a} is selected from:
   - C₁₋₄ alkyl optionally substituted with one or more groups independently selected from
      ∘ OH,
      ∘ C₁₋₄ alkoxy optionally substituted with one or more independently selected OH, or C₁₋₄ alkoxy,
      ∘ -SO₂-C₁₋₄ alkyl,
      ∘ -O-C₃₋₇ monocyclic cycloalkyl, and
      ∘ -O-heterocycloalkyl wherein said heterocycloalkyl is a 4-7 membered monocyclic heterocycloalkyl comprising one, two, or three heteroatoms independently selected from N, O, and S;
   - -NR^{6a}R^{6b},
   - C₁₋₄ alkoxy,
   - C₃₋₇ monocyclic cycloalkyl,
   - 4-7 membered monocyclic heterocycloalkyl comprising one, two, or three heteroatoms independently selected from N, O, and S, optionally substituted with one or more halo,
   - -O-C₃₋₇ monocyclic cycloalkyl, and
   - -O-heterocycloalkyl wherein said heterocycloalkyl is a 4-7 membered monocyclic heterocycloalkyl comprising one, two, or three heteroatoms independently selected from N, O, and S;
R^{1b} is H, or C₁₋₄ alkyl;
Cy₁ is a 5 membered monocyclic heteroaryl ring, comprising one, two, or three heteroatoms independently selected from N, O, or S, or
Cy₁ is 4-7 membered monocyclic heterocycloalkyl ring comprising one, two, or three heteroatoms independently selected from N, O, or S, or 4-7 membered monocyclic heterocycloalkyl ring comprising one, two, or three heteroatoms independently selected from N, O, or S, fused to a 5-6 membered heteroaryl ring comprising one, two, or three heteroatoms independently selected from N, O, or S, which heteroaryl may optionally substituted with one C₁₋₄ alkyl;
each R^{2a} and R^{2b} is independently selected from H, and C₁₋₄ alkyl optionally substituted with one or more independently selected -OH, or C₁₋₄ alkoxy;
R³ is selected from:
   - C₁₋₄ alkyl optionally substituted with one or more independently selected:
      ∘ halo,
      ∘ -CN,
      ∘ -OH,
      ∘ -C₁₋₄ alkoxy, or
      ∘ -NR^{7a}R^{7b};
   - C₁₋₄ alkoxy substituted with one or more halo,
   - C₃₋₇ monocyclic cycloalkyl,
   - 4-7-membered monocyclic heterocycloalkyl comprising one, two or three heteroatoms independently selected from N, O, and S,
   - -CN,
   - -S(O)₂-C₁₋₄ alkyl,
   - -NR^{8a}R^{8b}, and
   - -C(=O)NR^{8c}R^{8d};
each R⁴ is independently selected from:
   - C₁₋₄ alkyl optionally substituted with one or more independently selected R¹² groups,
   - C₃₋₇ monocyclic cycloalkyl, and
   - 4-7 membered monocyclic heterocycloalkyl comprising one, two, or three heteroatoms independently selected from N, O, and S;
Cy₂ is phenyl or 5-6 membered monocyclic heteroaryl comprising one or two heteroatoms independently selected from N, O, and S;
each R⁵ is independently selected from:
   - halo,
   - -CN,
   - -OH,
   - C₁₋₄ alkyl optionally substituted with one or more independently selected R¹³groups,
   - C₁₋₄ alkoxy optionally substituted with one or more independently selected R¹³groups,
   - C₃₋₇ monocyclic cycloalkyl optionally substituted with one or more independently selected R¹⁴ groups,
   - 4-11 membered monocyclic, or fused or spiro bicyclic heterocycloalkyl comprising one, two, or three heteroatoms independently selected from N, O, and S, optionally substituted with one or more independently selected R¹⁴ groups,
   - -O-C₃₋₇ monocyclic cycloalkyl optionally substituted with one or more independently selected R¹⁴ groups,
   - -O-heterocycloalkyl wherein said heterocycloalkyl is a 4-7 membered monocyclic heterocycloalkyl comprising one, two, or three heteroatoms independently selected from N, O, and S, optionally substituted with one or more independently selected R¹⁴ groups, and wherein if a N heteroatom is present, said N heteroatom, is further substituted with one -C(=O)-C₁₋₄ alkyl, -C(=O)-C₁₋₄ alkoxy, -SO₂-C₁₋₄ alkyl, -C(=O)-NH₂, -C(=O)NHC₁₋₄ alkyl, or -C(=O)N(C₁₋₄ alkyl)₂,
   - -SO₂-C₁₋₄ alkyl,
   - -SO₂NR^{15a}R^{15b},
   - -C(=O)NR^{15c}R^{15d}, and
   - -NR^{17a}R^{17b};
each R¹² is independently selected from:
   - halo,
   - OH,
   - C₁₋₄ alkoxy,
   - -SO₂-C₁₋₄ alkyl,
   - C₃₋₇ monocyclic cycloalkyl optionally substituted with one or more independently selected -OH, halo, -CN, C₁₋₄ alkyl, C₁₋₄ alkoxy or =O,
   - 4-7 membered monocyclic heterocycloalkyl, comprising one, two, or three heteroatoms independently selected from N, O, and S, optionally substituted with one or more independently selected -OH, halo, -CN, C₁₋₄ alkyl, C₁₋₄ alkoxy or =O,
   - -NR^{9a}R^{9b}, and
   - -CN;
each R¹³ is independently selected from:
   - halo,
   - -CN,
   - -OH,
   - C₁₋₄ alkoxy optionally substituted with one or more independently selected OH, C₁₋₄ alkoxy, or halo,
   - -C(=O)NR^{16a}R^{16b},
   - -NR^{16c}C(=O)-C₁₋₄ alkyl,
   - -NR^{16d}C(=O)-C₁₋₄ alkoxy,
   - -SO₂-C₁₋₄ alkyl
   - -SO₂NR^{16e}R^{16f},
   - -NR^{16g}SO₂-C₁₋₄ alkyl,
   - C₃₋₇ monocyclic cycloalkyl optionally substituted with one or more independently selected halo, or C₁₋₄ alkyl optionally substituted with one or more halo, and
   - 4-7 membered monocyclic heterocycloalkyl comprising one, two, or three heteroatoms independently selected from N, O, and S, optionally substituted with one or more independently selected halo, or C₁₋₄ alkyl optionally substituted with one or more halo, and wherein if a N heteroatom is present, said N heteroatom, is further substituted with one -C(=O)-C₁₋₄ alkyl, -C(=O)-C₁₋₄ alkoxy, -SO₂-C₁₋₄ alkyl, -C(=O)-NH₂, -C(=O)NHC₁₋₄ alkyl, or -C(=O)N(C₁₋₄ alkyl)₂;
each R¹⁴ is independently selected from:
   - halo,
   - -CN,
   - -OH,
   - C₁₋₄ alkoxy optionally substituted with one or more halo, and
   - C₁₋₄ alkyl optionally substituted with one or more halo;
each R^{6a}, R^{6b}, R^{7a}, R7^{b}, R^{8a}, R^{8b}, R^{8c}, R^{8d}, R^{9a}, R^{9b}, R^{15a}, R^{15b}, R^{15c}, R^{15d}, R^{16a}, R^{16b}, R^{16c}, R^{16d}, R^{16e}, R^{16f}, and R^{16g} is independently selected from H and C₁₋₄ alkyl;
each R^{17a} and R^{17b} is independently selected from H and C₁₋₄ alkyl optionally substituted with one or more independently selected halo, OH, or C₁₋₄ alkoxy;
the subscript n is 0, 1, 2, or 3; and
the subscript m is 0, 1, 2, 3 or 4.

In one embodiment, the compound of the invention is according to Formula Ia, wherein X is =N-CN.

In one embodiment, the compound of the invention is according to Formula Ia, wherein X is =O.

In one embodiment, the compound of the invention is according to Formula Ia, wherein Cy₁ is 4-7 membered monocyclic heterocycloalkyl ring comprising one, two, or three heteroatoms independently selected from N, O, or S. In a particular embodiment, Cy₁ is azetidinyl, pyrolidinyl, piperidinyl, piperazinyl, or morpholinyl.

In another particular embodiment, the compound of the invention is according to Formula Ia, wherein Cy₁ is 4-7 membered monocyclic heterocycloalkyl ring comprising one, two, or three heteroatoms independently selected from N, O, or S, fused to a 5-6 membered heteroaryl ring comprising one, two, or three heteroatoms independently selected from N, O, or S, which heteroaryl may optionally substituted with one C₁₋₄ alkyl. In a particular embodiment, Cy₁ is 4-7 membered monocyclic heterocycloalkyl ring comprising one, two, or three heteroatoms independently selected from N, O, or S, fused to a 5-6 membered heteroaryl ring comprising one, two, or three heteroatoms independently selected from N, O, or S, which heteroaryl may optionally substituted with one -CH₃. In a more particular embodiment, Cy1 is tetrahydroimidazopyridinyl, or tetrahydropyrazolopyridinyl, each of which may optionally substituted with one -CH₃.

In a further aspect of the invention, the compounds of the invention are provided having a Formula Ib: 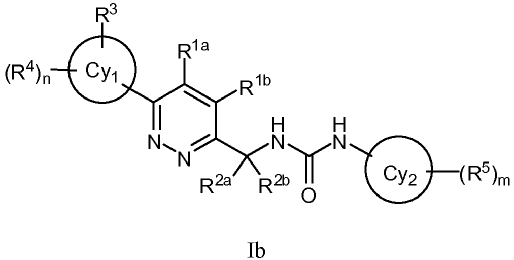 wherein
R^{1a} is selected from:
   - C₁₋₄ alkyl optionally substituted with one or more groups independently selected from:
      ∘ OH,
      ∘ C₁₋₄ alkoxy optionally substituted with one or more independently selected OH, or C₁₋₄ alkoxy,
      ∘ -SO₂-C₁₋₄ alkyl,
      ∘ -O-C₃₋₇ monocyclic cycloalkyl, and
      ∘ -O-heterocycloalkyl wherein said heterocycloalkyl is a 4-7 membered monocyclic heterocycloalkyl comprising one, two, or three heteroatoms independently selected from N, O, and S;
   - -NR^{6a}R^{6b},
   - C₁₋₄ alkoxy,
   - C₃₋₇ monocyclic cycloalkyl,
   - 4-7 membered monocyclic heterocycloalkyl comprising one, two, or three heteroatoms independently selected from N, O, or S, optionally substituted with one or more halo,
   - -O-C₃₋₇ monocyclic cycloalkyl, and
   - -O-heterocycloalkyl wherein said heterocycloalkyl is a 4-7 membered monocyclic heterocycloalkyl comprising one, two, or three heteroatoms independently selected from N, O, and S;
R^{1b} is H, or C₁₋₄ alkyl;
Cy₁ is a 5 membered monocyclic heteroaryl ring, comprising one, two, or three heteroatoms independently selected from N, O, and S;
each R^{2a} and R^{2b} is independently selected from H, and C₁₋₄ alkyl optionally substituted with one or more independently selected -OH, or C₁₋₄ alkoxy;
R³ is selected from:
   - C₁₋₄ alkyl optionally substituted with one or more groups independently selected from:
      ∘ halo,
      ∘ -CN,
      ∘ -C₁₋₄ alkoxy, and
      ∘ -NR^{7a}R^{7b};
   - C₁₋₄ alkoxy substituted with one or more halo,
   - C₃₋₇ monocyclic cycloalkyl,
   - 4-7-membered monocyclic heterocycloalkyl comprising one, two or three heteroatoms independently selected from N, O, and S,
   - -CN,
   - -NR^{8a}R^{8b}, and
   - -C(=O)NR^{8c}R^{8d};
each R⁴ is independently selected from:
   - C₁₋₄ alkyl optionally substituted with one or more independently selected R¹² groups,
   - C₃₋₇ monocyclic cycloalkyl, and
   - 4-7 membered monocyclic heterocycloalkyl comprising one, two, or three heteroatoms independently selected from N, O, and S;
Cy₂ is phenyl or 5-6 membered monocyclic heteroaryl comprising one or two heteroatoms independently selected from N, O, and S;
each R⁵ is independently selected from:
   - halo,
   - -CN,
   - -OH,
   - C₁₋₄ alkyl optionally substituted with one or more independently selected R¹³groups,
   - C₁₋₄ alkoxy optionally substituted with one or more independently selected R¹³groups,
   - C₃₋₇ monocyclic cycloalkyl optionally substituted with one or more independently selected R¹⁴ groups,
   - 4-11 membered monocyclic, or fused or spiro bicyclic heterocycloalkyl comprising one, two, or three heteroatoms independently selected from N, O, and S, optionally substituted with one or more independently selected R¹⁴ groups,
   - -O-C₃₋₇ monocyclic cycloalkyl optionally substituted with one or more independently selected R¹⁴ groups,
   - -O-heterocycloalkyl wherein said heterocycloalkyl is a 4-7 membered monocyclic heterocycloalkyl comprising one, two, or three heteroatoms independently selected from N, O, and S, optionally substituted with one or more independently selected R¹⁴ groups, and wherein if a N heteroatom is present, said N heteroatom, is further substituted with one -C(=O)-C₁₋₄ alkyl, -C(=O)-C₁₋₄ alkoxy, -SO₂-C₁₋₄ alkyl, -C(=O)-NH₂, -C(=O)NHC₁₋₄ alkyl, or -C(=O)N(C₁₋₄ alkyl)₂,
   - -SO₂-C₁₋₄ alkyl,
   - -SO₂NR^{15a}R^{15b},
   - -C(=O)NR^{15c}R^{15d}, and
   - -NR^{17a}R^{17b};
each R¹² is independently selected from:
   - halo,
   - OH,
   - C₁₋₄ alkoxy,
   - -SO₂-C₁₋₄ alkyl,
   - C₃₋₇ monocyclic cycloalkyl optionally substituted with one or more independently selected -OH, halo, -CN, C₁₋₄ alkyl, C₁₋₄ alkoxy or =O,
   - 4-7 membered monocyclic heterocycloalkyl, comprising one, two, or three heteroatoms independently selected from N, O, and S, optionally substituted with one or more independently selected -OH, halo, -CN, C₁₋₄ alkyl, C₁₋₄ alkoxy or =O,
   - -NR^{9a}R^{9b}, and
   - -CN;
Each R¹³ is independently selected from:
   - halo,
   - -CN,
   - -OH,
   - C₁₋₄ alkoxy optionally substituted with one or more independently selected OH, C₁₋₄ alkoxy, or halo,
   - -C(=O)NR^{16a}R^{16b},
   - -NR^{16c}C(=O)-C₁₋₄ alkyl,
   - -NR^{16d}C(=O)-C₁₋₄ alkoxy,
   - -SO₂-C₁₋₄ alkyl
   - -SO₂NR^{16e}R^{16f},
   - -NR^{16g}SO₂-C₁₋₄ alkyl,
   - C₃₋₇ monocyclic cycloalkyl optionally substituted with one or more independently selected halo, or C₁₋₄ alkyl optionally substituted with one or more halo, and
   - 4-7 membered monocyclic heterocycloalkyl comprising one, two, or three heteroatoms independently selected from N, O, and S, optionally substituted with one or more independently selected halo, or C₁₋₄ alkyl optionally substituted with one or more halo, and wherein if a N heteroatom is present, said N heteroatom, is further substituted with one -C(=O)-C₁₋₄ alkyl, -C(=O)-C₁₋₄ alkoxy, -SO₂-C₁₋₄ alkyl, -C(=O)-NH₂, -C(=O)NHC₁₋₄ alkyl, or -C(=O)N(C₁₋₄ alkyl)₂;
each R¹⁴ is independently selected from:
   - halo,
   - -CN,
   - -OH,
   - C₁₋₄ alkoxy optionally substituted with one or more halo, and
   - C₁₋₄ alkyl optionally substituted with one or more halo;
each R^{6a}, R^{6b}, R^{7a}, R^{6b}, R^{8a}, R^{8b}, R^{8c}, R^{8d}, R^{9a}, R^{9b}, R^{15a}, R^{15b}, R^{15c}, R^{15d}, R^{16a}, R^{16b}, R^{16c}, R^{16d}, R^{16e}, R^{16f}, and R^{16g} is independently selected from H and C₁₋₄ alkyl;
each R^{17a} and R^{17b} is independently selected from H and C₁₋₄ alkyl optionally substituted with one or more independently selected halo, OH, or C₁₋₄ alkoxy;
the subscript n is 0, 1, 2, or 3; and
the subscript m is 0, 1, 2, 3 or 4.

In one embodiment, the compound of the invention is according to Formula Ia, wherein R^{1b} is H.

In one embodiment, the compound of the invention is according to Formula Ia, wherein R^{1b} is C₁₋₄ alkyl. In a particular embodiment, R^{1b} is -CH₃.

In one embodiment, the compound of the invention is according to Formula Ia, wherein Cy₁ is a 5-membered monocyclic heteroaryl ring, comprising one, two, or three heteroatoms independently selected from N, O, or S. In a particular embodiment, Cy₁ is imidazolyl, pyrazolyl, oxazolyl, isoxazolyl, thiazolyl, isothiazolyl. In a more particular embodiment, Cy₁ is imidazolyl. In another more particular embodiment, Cy₁ is pyrazolyl.

In one embodiment, the compound of the invention is according to Formula II: 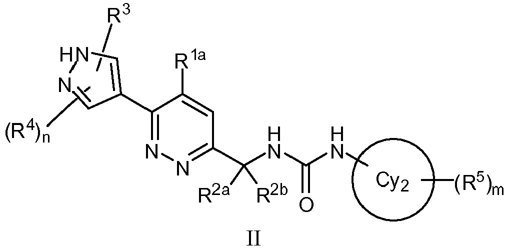 wherein R^{1a}, R^{2a}, R^{2b}, R³, R⁴, R⁵, Cy₂, the subscripts n and m are as previously described.

In one embodiment, the compound of the invention is according to Formula Ia, Ib or II wherein R^{1a} is C₁₋₄ alkyl. In a particular embodiment, R^{1a} is -CH₃.

In one embodiment, the compound of the invention is according to Formula Ia, Ib or II wherein R^{1a} is C₁₋₄ alkyl substituted with one or more independently selected OH; C₁₋₄ alkoxy optionally substituted with one or more independently selected OH, or C₁₋₄ alkoxy; -SO₂-C₁₋₄ alkyl; -O-C₃₋₇ monocyclic cycloalkyl; and -O-heterocycloalkyl wherein said heterocycloalkyl is a 4-7 membered monocyclic heterocycloalkyl comprising one, two, or three heteroatoms independently selected from N, O, and S. In a particular embodiment, R^{1a} is C₁₋₄ alkyl substituted with one OH; C₁₋₄ alkoxy optionally substituted with one or more independently selected OH, or C₁₋₄ alkoxy;-SO₂-C₁₋₄ alkyl; -O-C₃₋₇ monocyclic cycloalkyl; or -O-heterocycloalkyl wherein said heterocycloalkyl is a 4-7 membered monocyclic heterocycloalkyl comprising one, two, or three heteroatoms independently selected from N, O, and S. In a more particular embodiment, R^{1a} is -CH₃ substituted with one OH; C₁₋₄ alkoxy optionally substituted with one or more independently selected OH, or C₁₋₄ alkoxy; -SO₂-C₁₋₄ alkyl; -O-C₃₋₇ monocyclic cycloalkyl; or -O-heterocycloalkyl wherein said heterocycloalkyl is a 4-7 membered monocyclic heterocycloalkyl comprising one, two, or three heteroatoms independently selected from N, O, and S.

In one embodiment, the compound of the invention is according to Formula Ia, Ib or II wherein R^{1a} is -NR^{6a}R^{6b}, and wherein each R^{6a} and R^{6b} is independently selected from H and C₁₋₄ alkyl. In a particular embodiment, each R^{6a} and R^{6b} is independently selected from H, -CH₃, and -CH₂CH₃. In a further particular embodiment, R^{6a} is H and R^{6b} is H, or C₁₋₄ alkyl. In more particular embodiment, R^{6a} is H and R^{6b} is selected from H, -CH₃, and -CH₂CH₃. In a further more particular embodiment, both R^{6a} and R^{6b} are C₁₋₄ alkyl. In a most particular embodiment, R^{1a} is -NHCH₃, or -N(CH₃)₂.

In one embodiment, the compound of the invention is according to Formula Ia, Ib or II wherein R^{1a} is C₁₋₄ alkoxy. In a particular embodiment, R^{1a} is -OCH₃, or -OCH₂CH₃. In a more particular embodiment, R^{1a} is -OCH₃.

In one embodiment, the compound of the invention is according to Formula Ia, Ib or II wherein R^{1a} is C₃₋₇ monocyclic cycloalkyl. In a particular embodiment, R^{1a} is cyclopropyl, cyclobutyl, cyclopentyl, or cyclohexyl. In a more particular embodiment, R^{1a} is cyclopropyl.

In one embodiment, the compound of the invention is according to Formula Ia, Ib or II wherein R^{1a} is 4-7 membered monocyclic heterocycloalkyl comprising one, two, or three heteroatoms independently selected from N, O, or S. In a particular embodiment, R^{1a} is azetidinyl, pyrrolidinyl, piperazinyl, piperidinyl, morpholinyl, tetrahydrofuranyl, or tetrahydropyranyl. In a particular embodiment, R^{1a} is morpholinyl.

In one embodiment, the compound of the invention is according to Formula Ia, Ib or II wherein R^{1a} is 4-7 membered monocyclic heterocycloalkyl comprising one, two, or three heteroatoms independently selected from N, O, or S, substituted with one or more halo. In a particular embodiment, R^{1a} is 4-7 membered monocyclic heterocycloalkyl comprising one, two, or three heteroatoms independently selected from N, O, or S, substituted with one or more fluoro. In a more particular embodiment, R^{1a} is azetidinyl, pyrrolidinyl, piperazinyl, piperidinyl, morpholinyl, tetrahydrofuranyl, or tetrahydropyranyl, each of which is substituted with one or more halo. In a more particular embodiment, R^{1a} is azetidinyl substituted with one or more fluoro.

In one embodiment, the compound of the invention is according to Formula Ia, Ib or II wherein R^{1a} is -O-C₃₋₇ monocyclic cycloalkyl. In a particular embodiment, R^{1a} is -O-cyclopropyl, -O-cyclobutyl, -O-cyclopentyl, or -O-cyclohexyl. In a more particular embodiment, R^{1a} is -O-cyclopropyl.

In one embodiment, the compound of the invention is according to Formula Ia, Ib or II wherein R^{1a} is -O-heterocycloalkyl, wherein said heterocycloalkyl is a 4-7 membered monocyclic heterocycloalkyl comprising one, two, or three heteroatoms independently selected from N, O, and S. In a particular embodiment, R^{1a} is -O-oxetanyl, or -O-tetrahydrofuranyl.

In one embodiment, the compound of the invention is according to Formula III: 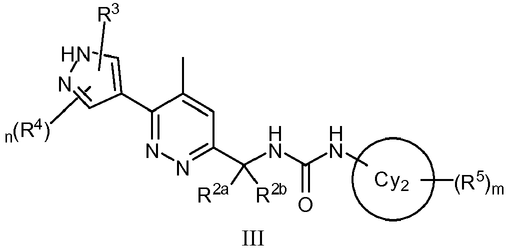 wherein R^{2a}, R^{2b}, R³, R⁴, R⁵, Cy₂, and the subscript m and n are as previously described.

In one embodiment, the compound of the invention is according to any one of Formulae Ia-III wherein each R^{2a} and R^{2b} is independently selected from H, and C₁₋₄ alkyl optionally substituted with one or more independently -OH, or C₁₋₄ alkoxy. In a particular embodiment, R^{2a} is H, and R^{2b} is selected from H, and C₁₋₄ alkyl optionally substituted with one or more groups independently selected from -OH, and C₁₋₄ alkoxy. In a more particular embodiment, R^{2a} is H, and R^{2b} is selected from H, and -CH₃, -CH₂CH₃, each of which is optionally substituted with one or more groups independently selected from -OH, and C₁₋₄ alkoxy. In a further more particular embodiment, R^{2a} is H, and R^{2b} is selected from H, and -CH₃. In a most particular embodiment, R^{2a} and R^{2b} are H.

In one embodiment, the compound of the invention is according to Formula IV: 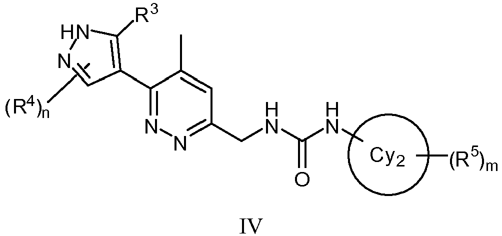 wherein R³, R⁴, R⁵, Cy₂, and the subscript m and n are as previously described.

In one embodiment, the compound of the invention is according to any one of Formulae Ia-IV, wherein R³ is C₁₋₄ alkyl. In a particular embodiment, R³ is -CH₃, -CH₂CH₃, or -(CH₂)₂CH₃. In a more particular embodiment, R³ is -CH₃.

In one embodiment, the compound of the invention is according to any one of Formulae Ia-IV, wherein R³ is C₁₋₄ alkyl substituted with one or more independently selected halo, -CN, -C₁₋₄ alkoxy, or -NR^{7a}R^{7b}. In a particular embodiment, R³ is -CH₃, -CH₂CH₃, or -(CH₂)₂CH₃, each of which is substituted with one or more independently selected halo, -CN, -C₁₋₄ alkoxy, or -NR^{7a}R^{7b}. In another particular embodiment, R³ is C₁₋₄ alkyl substituted with one, two or three independently selected halo, -CN, -C₁₋₄ alkoxy, or -NR^{7a}R^{7b}. In a more particular embodiment, R³ is -CH₃, -CH₂CH₃, or -(CH₂)₂CH₃, substituted with one, two or three independently selected halo, -CN, -C₁₋₄ alkoxy, or -NR^{7a}R^{7b}. In a further more particular embodiment, R³ is C₁₋₄ alkyl substituted with one halo, -CN, -C₁₋₄ alkoxy, or -NR^{7a}R^{7b}. In a more particular embodiment, R³ is -CH₃, -CH₂CH₃, or -(CH₂)₂CH₃, substituted with one halo, -CN, -C₁₋₄ alkoxy, or -NR^{7a}R^{7b}. In a particular embodiment, R^{7a} is H, and R^{7b} is H or C₁₋₄ alkyl. In another particular embodiment, R^{7a} and R^{7b} are C₁₋₄ alkyl. In a more particular embodiment, each R^{7a} and R^{7b} is independently selected from H,-CH₃, and -CH₂CH₃. In a further more particular embodiment, R^{7a} and R^{7b} are -CH₃. In a most particular embodiment, R³ is -CF₃.

In one embodiment, the compound of the invention is according to any one of Formulea Ia-IV wherein R³ is C₁₋₄ alkoxy substituted with one or more halo. In a particular embodiment, R³ is -OCH₃, -OCH₂CH₃, or -OCH₂CH₂CH₃, each of is substituted with one or more halo. In a more particular embodiment, R³ is -OCF₃, -OCH₂CHF₂ or -OCH₂CF₃.

In one embodiment, the compound of the invention is according to any one of Formulae Ia-IV wherein R³ is C₃₋₇ monocyclic cycloalkyl. In a particular embodiment, R³ is cyclopropyl.

In one embodiment, the compound of the invention is according to any one of Formulae Ia-IV wherein R³ is 4-7-membered monocyclic heterocycloalkyl comprising one, two or three heteroatoms independently selected from N, O, and S. In a particular embodiment, R³ is oxetanyl, tetrahydrofuranyl, tetrahydropyranyl, pyrolidinyl, piperidinyl, piperazinyl, morpholinyl, or thiomorpholinyl.

In one embodiment, the compound of the invention is according to any one of Formulae Ia-IV wherein R³ is -NR^{8a}R^{8b} wherein each R^{8a} and R^{8b} is independently selected from H, and C₁₋₄ alkyl. In a particular embodiment, R^{8a} is H, and R^{8b} is H or C₁₋₄ alkyl. In another particular embodiment, R^{8a} and R^{8b} are C₁₋₄ alkyl. In a more particular embodiment, each R^{8a} and R^{8b} is independently selected from H, -CH₃, and -CH₂CH₃. In a further more particular embodiment, R³ is -N(CH₃)₂.

In one embodiment, the compound of the invention is according to any one of Formulae Ia-IV wherein R³ is -C(=O)NR^{8c}R^{8d} wherein each R^{8c} and R^{8d} is independently selected from H, and C₁₋₄ alkyl. In a particular embodiment, R^{8c} is H, and R^{8d} is H or C₁₋₄ alkyl. In another particular embodiment, R^{8c} and R^{8d} are C₁₋₄ alkyl. In a more particular embodiment, each R^{8c} and R^{8d} is independently selected from H, -CH₃, and -CH₂CH₃. In a further more particular embodiment, R³ is -C(=O)N(CH₃)₂.

In one embodiment, the compound of the invention is according to any one of Formulae Ia-IV wherein the subscript n is 0.

In another embodiment, the compound of the invention is according to any one of Formulae Ia-IV wherein the subscript n is 1 or 2. In a particular embodiment, n is 1.

In one embodiment, the compound of the invention is according to Formula Va or Vb 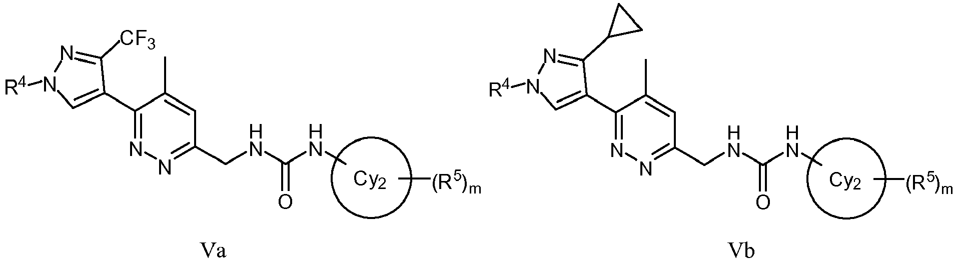 wherein R⁴, R⁵, Cy₂, and the subscript m are as previously described.

In one embodiment, the compound of the invention is according to any one of Formulae Ia-Vb wherein R⁴ is C₁₋₄ alkyl. In a particular embodiment, R⁴ is -CH₃, -CH₂CH₃, -(CH₂)₂CH₃, or -CH(CH₃)₂. In a more particular embodiment, R⁴ is -CH₃.

In another embodiment, the compound of the invention is according to any one of Formulae Ia-Vb wherein R⁴ is C₁₋₄ alkyl substituted with one or more independently selected R¹² groups. In another embodiment, R⁴ is -CH₃, -CH₂CH₃, -(CH₂)₂CH₃, or -CH(CH₃)₂ each of which is substituted with one or more independently selected R¹² groups. In a particular embodiment, R⁴ is C₁₋₄ alkyl substituted with one, two, or three independently selected R¹² groups. In another particular embodiment, R⁴ is -CH₃, -CH₂CH₃, -(CH₂)₂CH₃, or -CH(CH₃)₂ each of which is substituted with one, two or three independently selected R¹² groups. In a more particular embodiment, R⁴ is C₁₋₄ alkyl substituted with one R¹² group. In a more particular embodiment, R⁴ is -CH₃, -CH₂CH₃, -(CH₂)₂CH₃, or -CH(CH₃)₂ each of which is substituted with one independently selected R¹² group. In a most particular embodiment, R⁴ is -CH₂-R¹².

In another embodiment, the compound of the invention is according to any one of Formulae Ia-Vb wherein R¹² is halo. In a particular embodiment R¹² is F, or Cl. In a more particular embodiment, R¹² is F.

In one embodiment, the compound of the invention is according to any one of Formulae Ia-Vb wherein R¹² is OH.

In one embodiment, the compound of the invention is according to any one of Formulae Ia-Vb wherein R¹² is C₁₋₄ alkoxy. In a particular embodiment, R¹² is -OCH₃, or -OCH₂CH₃. In a more particular embodiment, R¹² is -OCH₃.

In one embodiment, the compound of the invention is according to any one of Formulae Ia-Vb wherein R¹² is -SO₂-C₁₋₄ alkyl. In a particular embodiment, R¹² is -SO₂-CH₃.

In one embodiment, the compound of the invention is according to any one of Formulae Ia-Vb wherein R¹² is C₃₋₇ monocyclic cycloalkyl. In a particular embodiment, R¹² is cyclopropyl, cyclobutyl, or cyclopentyl. In a more particular embodiment, R¹² is cyclopropyl.

In one embodiment, the compound of the invention is according to any one of Formulae Ia-Vb wherein R¹² is C₃₋₇ monocyclic cycloalkyl, substituted with one or more independently selected -OH, halo, -CN, C₁₋₄ alkyl, C₁₋₄ alkoxy or =O. In a particular embodiment, R¹² is C₃₋₇ monocyclic cycloalkyl, substituted with one or more independently selected -OH, F, -CN, -CH₃, -OCH₃ or =O.In a more particular embodiment, R¹² is cyclopropyl, cyclobutyl, or cyclopentyl, each of which is substituted with one or more independently selected -OH, halo, -CN, C₁₋₄ alkyl, C₁₋₄ alkoxy or =O. In a further more particular embodiment, R¹² is cyclopropyl, cyclobutyl, or cyclopentyl, each of which is substituted with one or more independently selected -OH, F, -CN, -CH₃, -OCH₃ or =O.

In one embodiment, the compound of the invention is according to any one of Formulae Ia-Vb wherein R¹² is 4-7 membered monocyclic heterocycloalkyl, comprising one, two, or three heteroatoms independently selected from N, O, and S. In a particular embodiment, R¹² is azetidinyl, pyrrolidinyl, or tetrahydrofuranyl.

In one embodiment, the compound of the invention is according to any one of Formulae Ia-Vb wherein R¹² is 4-7 membered monocyclic heterocycloalkyl, comprising one, two, or three heteroatoms independently selected from N, O, and S, substituted with one or more independently selected -OH, halo, -CN, C₁₋₄ alkyl, C₁₋₄ alkoxy or =O. In a particular embodiment, R¹² is 4-7 membered monocyclic heterocycloalkyl, comprising one, two, or three heteroatoms independently selected from N, O, and S, substituted with one or more independently selected -OH, F, -CN, -CH₃, -OCH₃ or =O. In a more particular embodiment, R¹² is azetidinyl, pyrrolidinyl, or tetrahydrofuranyl, each of which is substituted with one or more independently selected -OH, halo, -CN, C₁₋₄ alkyl, C₁₋₄ alkoxy or =O. In a further more particular embodiment, R¹² is azetidinyl, pyrrolidinyl, or tetrahydrofuranyl, each of which is substituted with one or more independently selected -OH, F, -CN, -CH₃, -OCH₃ or =O.

In one embodiment, the compound of the invention is according to any one of Formulae Ia-Vb wherein R¹² is -NR^{9a}R^{9b}, wherein each R^{9a} and R^{9b} is independently selected from H and C₁₋₄ alkyl. In a particular embodiment, one of R^{9a} and R^{9b} is H, and the other is C₁₋₄ alkyl. In another embodiment, R^{9a} and R^{9b} are independently selected C₁₋₄ alkyl. In a particular embodiment, R^{9a} and R^{9b} are independently selected from H, and -CH₃. In a most particular embodiment, R¹² is -N(CH₃)₂.

In one embodiment, the compound of the invention is according to any one of Formulae Ia-Vb wherein R¹² is -CN.

In one embodiment, the compound of the invention is according to any one of Formulae Ia-Vb wherein R⁴ is C₃₋₇ monocyclic cycloalkyl. In a particular embodiment, R⁴ is cyclopropyl, cyclobutyl, cyclopentyl, or cyclohexyl. In a more particular embodiment, R⁴ is cyclopropyl.

In one embodiment, the compound of the invention is according to any one of Formulae Ia-Vb wherein R⁴ is 4-7 membered monocyclic heterocycloalkyl comprising one, two, or three heteroatoms independently selected from N, O, or S. In a particular embodiment, R⁴ is azetidinyl, pyrrolidinyl, piperazinyl, piperidinyl, morpholinyl, tetrahydrofuranyl, or tetrahydropyranyl. In a more particular embodiment, R⁴ is tetrahydrofuranyl.

In one embodiment, the compound of the invention is according to Formula VIa, or VIb: 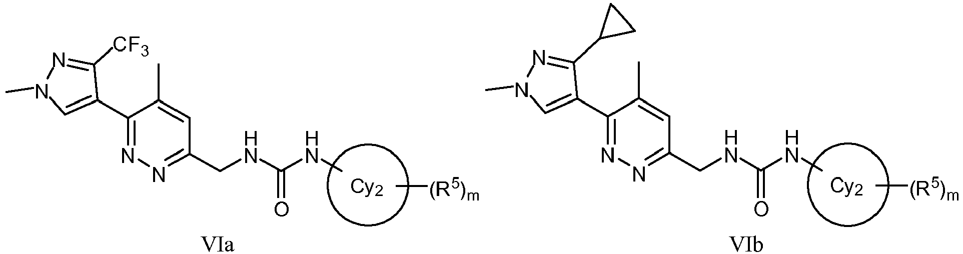 wherein R⁵, Cy₂, and the subscript m are as previously described.

In one embodiment, the compound of the invention is according to any one of Formulae Ia-VIb, wherein Cy₂ is phenyl.

In one embodiment, the compound of the invention is according to any one of Formulae Ia-YIb, wherein Cy₂ is 5-6 membered heteroaryl comprising one or two heteroatoms independently selected from N, O, and S. In a particular embodiment, Cy₂ is pyridinyl.

In one embodiment, the compound of the invention is according to any one of Formulae VIIa, VIIb, VIIc or VIId: 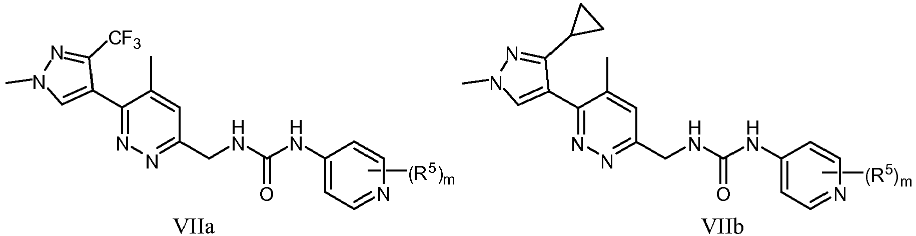 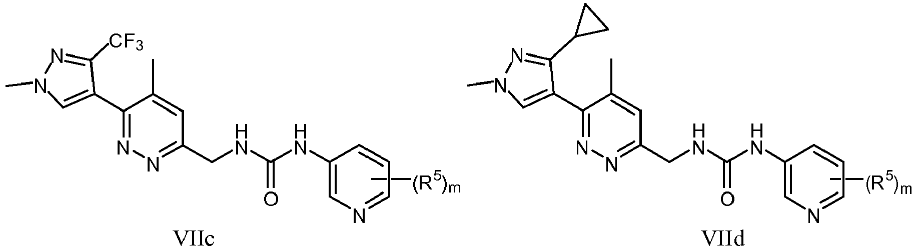 wherein R⁵ and the subscript m are as previously described.

In one embodiment, the compound of the invention is according to any one of Formulae Ia-VIId, wherein the subscript m is 0.

In one embodiment, the compound of the invention is according to any one of Formulae Ia-VIId, wherein the subscript m is 1, 2, 3, or 4. In a particular embodiment, the subscript m is 2, or 3.

In one embodiment, the compound of the invention is according to any one of Formulae Ia-VIId, wherein R⁵ is selected from halo, CN, selected from OH. In a particular embodiment, R⁵ is F, Cl, CN or OH. In a more particular embodiment, R⁵ is F, Cl or CN.

In one embodiment, the compound of the invention is according to any one of Formulae Ia-VIId, wherein R⁵ is C₁₋₄ alkyl. In a particular embodiment, R⁵ is -CH₃, or -CH₂CH₃.

In one embodiment, the compound of the invention is according to any one of Formulae Ia-VIId, wherein R⁵ is C₁₋₄ alkyl substituted with one or more independently selected R¹³. In a particular embodiment, R⁵ is -CH₃, or -CH₂CH₃, each of which is substituted with one or more independently selected R¹³. In a more particular embodiment, R⁵ is C₁₋₄ alkyl substituted with one, two or three independently selected R¹³. In another more particular embodiment, R⁵ is -CH₃, or -CH₂CH₃, each of which is substituted with one, two or three independently selected R¹³ groups. In a most particular embodiment, R⁵ is C₁₋₄ alkyl substituted with one R¹³ group. In another most particular embodiment, R⁵ is -CH₃, or -CH₂CH₃, each of which is substituted with one R¹³ group.

In one embodiment, the compound of the invention is according to any one of Formulae Ia-VIId, wherein R⁵ is C₁₋₄ alkoxy substituted with one or more independently selected R¹³ groups. In a particular embodiment, R⁵ is -OCH₃, or -OCH₂CH₃, each of which is substituted with one or more independently selected R¹³ groups. In a more particular embodiment, R⁵ is C₁₋₄ alkoxy substituted with one, two or three independently selected R¹³ groups. In another more particular embodiment, R⁵ is -OCH₃, or -OCH₂CH₃, each of which is substituted with one, two or three independently selected R¹³ groups. In a most particular embodiment, R⁵ is C₁₋₄ alkoxy substituted with one R¹³ group. In another most particular embodiment, R⁵ is -OCH₃, or -OCH₂CH₃, each of which is substituted with one R¹³ group.

In one embodiment, the compound of the invention is according to any one of Formulae Ia-VIId, wherein R¹³ is halo, -CN, or -OH. In a particular embodiment, R¹³ is F, -CN, or -OH.

In one embodiment, the compound of the invention is according to any one of Formulae Ia-VIId, wherein R¹³ is C₁₋₄ alkoxy optionally substituted with one or more independently selected OH, C₁₋₄ alkoxy, or halo. In a particular embodiment, R¹³ is -OCH₃, or -OCH₂CH₃, each of which is substituted with one or more groups independently selected from OH, C₁₋₄ alkoxy, and halo. In another particular embodiment, R¹³ is C₁₋₄ alkoxy optionally substituted with one or more groups independently selected from -OH, -OCH₃, and F. In a more particular embodiment, R¹³ is -OCH₃, or -OCH₂CH₃, each of which is substituted with one or more groups independently selected from -OH, -OCH₃, and F. In a most particular embodiment, R¹³ is -OCH₃, -OCF₃, -OCH₂CH₃, -OCH₂CF₃, -OCH₂CH₂OH, or -OCH₂CH₂OCH₃.

In one embodiment, the compound of the invention is according to any one of Formulae Ia-VIId, wherein R¹³ is -C(=O)NR^{16a}R^{16b}, wherein each R^{16a} and R^{16b} is independently selected from H, and C₁₋₄ alkyl. In a particular embodiment, each R^{16a} and R^{16b} is independently selected from H, -CH₃, and -CH₂CH₃. In a more particular embodiment, R^{16a} is H and R^{16b} is selected from H, -CH₃, and -CH₂CH₃. In a most particular embodiment, R¹³ is -C(=O)NH₂, -C(=O)NHCH₃, -C(=O)N(CH₃)₂, or -C(=O)N(CH₃)(CH₂CH₃).

In one embodiment, the compound of the invention is according to any one of Formulae Ia-VIId, wherein R¹³ is -NR^{16c}C(=O)-C₁₋₄ alkyl, wherein R^{16c} is independently selected from H, -CH₃, and -CH₂CH₃. In a particular embodiment, R¹³ is -NR^{16c}C(=O)-CH₃, or -NR^{16c}C(=O)-CH₂CH₃, wherein R^{16c} is independently selected from H, -CH₃, and -CH₂CH₃.

In one embodiment, the compound of the invention is according to any one of Formulae Ia-VIId, wherein R¹³ is -NR^{16d}C(=O)-C₁₋₄ alkoxy, wherein R^{16d} is independently selected from H, -CH₃, and -CH₂CH₃. In a particular embodiment, R¹³ is -NR^{16d}C(=O)-OCH₃, or -NR^{16d}C(=O)-OCH₂CH₃, wherein R^{16d} is independently selected from H, -CH₃, and -CH₂CH₃.

In one embodiment, the compound of the invention is according to any one of Formulae Ia-VIId, wherein R¹³ is -SO₂-C₁₋₄ alkyl. In a particular embodiment, R¹³ is -SO₂-CH₃, or -SO₂-CH₂CH₃.

In one embodiment, the compound of the invention is according to any one of Formulae Ia-VIId, wherein R¹³ is -SO₂NR^{16e}R^{16f}, wherein each R^{16e} and R^{16f} is independently selected from H, and C₁₋₄ alkyl. In a particular embodiment, each R^{16e} and R^{16f} is independently selected from H, -CH₃, and -CH₂CH₃. In a more particular embodiment, R^{16e} is H and R^{16f} is selected from H, -CH₃, and -CH₂CH₃. In a most particular embodiment, R¹³ is -SO₂NH₂, -SO₂NHCH₃, -SO₂N(CH₃)₂, or -SO₂N(CH₃)(CH₂CH₃).

In one embodiment, the compound of the invention is according to any one of Formulae Ia-VIId, wherein R¹³ is -NR^{16g}SO₂-C₁₋₄ alkyl, wherein R^{16g} is independently selected from H, -CH₃, and -CH₂CH₃. In a particular embodiment, R¹³ is -NR^{16g}SO₂-CH₃, or -NR^{16g}SO₂-CH₂CH₃, wherein R^{16g} is independently selected from H, -CH₃, and -CH₂CH₃.

In one embodiment, the compound of the invention is according to any one of Formulae Ia-VIId, wherein R¹³ is C₃₋₇ monocyclic cycloalkyl. In a particular embodiment, R¹³ is cyclopropyl, cyclobutyl, cyclopentyl, or cyclohexyl. In a more particular embodiment, R¹³ is cyclopropyl, or cyclobutyl.

In one embodiment, the compound of the invention is according to any one of Formulae Ia-VIId, wherein R¹³ is C₃₋₇ monocyclic cycloalkyl substituted with one or more independently selected halo, or C₁₋₄ alkyl optionally substituted with one or more halo. In a particular embodiment, R¹³ is cyclopropyl, cyclobutyl, cyclopentyl, or cyclohexyl, each of which is substituted with one or more independently selected halo, or C₁₋₄ alkyl optionally substituted with one or more halo.. In another more particular embodiment, R¹³ is C₃₋₇ monocyclic cycloalkyl substituted with one or more independently selected F, -CH₃, -CH₂CH₃. -CF₃, and -CH₂CF₃. In a most particular embodiment, R¹³ is cyclopropyl, cyclobutyl, cyclopentyl, or cyclohexyl, each of which is substituted with one or more independently selected F, -CH₃, -CH₂CH₃. -CF₃, and -CH₂CF₃. In a most particular embodiment, R¹³ is cyclopropyl substituted with one or more independently selected F, -CH₃, -CH₂CH₃. -CF₃, and -CH₂CF₃.

In one embodiment, the compound of the invention is according to any one of Formulae Ia-VIId, wherein R¹³ is 4-7 membered monocyclic heterocycloalkyl comprising one, two, or three heteroatoms independently selected from N, O, and S, optionally substituted with one or more independently selected halo, or C₁₋₄ alkyl optionally substituted with one or more halo, and wherein if a N heteroatom is present, said N heteroatom, is further substituted with one -C(=O)-C₁₋₄ alkyl, -C(=O)-C₁₋₄ alkoxy, -SO₂-C₁₋₄ alkyl, -C(=O)-NH₂, -C(=O)NHC₁₋₄ alkyl, or -C(=O)N(C₁₋₄ alkyl)₂. In a particular embodiment, R¹³ is 4-7 membered monocyclic heterocycloalkyl comprising one, two, or three heteroatoms independently selected from N, O, and S, optionally substituted with one or more independently selected F, -CH₃, -CH₂CH₃. -CF₃, and -CH₂-CF₃, and wherein if a N heteroatom is present, said N heteroatom, is further substituted with one -C(=O)-C₁₋₄ alkyl, -C(=O)-C₁₋₄ alkoxy, -SO₂-C₁₋₄ alkyl, -C(=O)-NH₂, -C(=O)NHC₁₋₄ alkyl, or -C(=O)N(C₁₋₄ alkyl)₂. In a further particular embodiment, R¹³ is 4-7 membered monocyclic heterocycloalkyl comprising one, two, or three heteroatoms independently selected from N, O, and S, optionally substituted with one or more independently selected F, -CH₃, -CH₂CH₃. -CF₃, and -CH₂-CF₃, and wherein if a N heteroatom is present, said N heteroatom, is further substituted with one -C(=O)CH₃, -C(=O)OCH₃, -SO₂CH₃, -C(=O)NH₂, -C(=O)NHCH₃, or -C(=O)N(CH₃)₂. In a further more particular embodiment, R¹³ is 4-7 membered monocyclic heterocycloalkyl comprising one, two, or three heteroatoms independently selected from O, and S, optionally substituted with one or more independently selected F, -CH₃, -CH₂CH₃. -CF₃, and -CH₂-CF₃. In a most particular embodiment, R¹³ is oxetanyl, tetrahydrofuranyl, or tetrahydropyranyl, each of which is optionally substituted with one or more independently selected F, -CH₃, -CH₂CH₃. -CF₃, and -CH₂CF₃. In a further most particular embodiment, R¹³ is oxetanyl, tetrahydrofuranyl, dioxanyl or tetrahydropyranyl.

In one embodiment, the compound of the invention is according to any one of Formulae Ia-VIId, wherein R⁵ is -CF₃.

In one embodiment, the compound of the invention is according to any one of Formulae Ia-VIId, wherein R⁵ is -OCF₃, -OCH2CH2F, -OCH₂CH₂OCH₃, -OCH₂CH₂OH, or -OCH₂CHF₂.

In one embodiment, the compound of the invention is according to any one of Formulae Ia-VIId, wherein R⁵ is

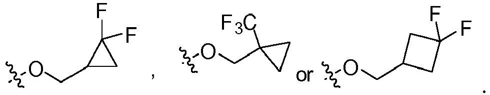

In one embodiment, the compound of the invention is according to any one of Formulae Ia-VIId, wherein R⁵ is C₃₋₇ monocyclic cycloalkyl. In a particular embodiment, R⁵ is cyclopropyl, cyclobutyl, cyclopentyl, or cyclohexyl. In a more particular embodiment, R⁵ is cyclopropyl, or cyclobutyl. In a most particular embodiment, R⁵ is cyclopropyl.

In one embodiment, the compound of the invention is according to any one of Formulae Ia-VIId, wherein R⁵ is C₃₋₇ monocyclic cycloalkyl substituted with one or more independently selected R¹⁴ group. In a particular embodiment, R⁵ is cyclopropyl, cyclobutyl, cyclopentyl, or cyclohexyl, each of which is substituted with one or more independently selected R¹⁴ group. In more particular embodiment, R⁵ is C₃₋₇ monocyclic cycloalkyl substituted with one, two, or three independently selected R¹⁴ group. In another more particular embodiment, R⁵ is cyclopropyl, cyclobutyl, cyclopentyl, or cyclohexyl, each of which is substituted with one, two, or three independently selected R¹⁴ group. In most particular embodiment, R⁵ is C₃₋₇ monocyclic cycloalkyl substituted with one or two independently selected R¹⁴ group. In another more particular embodiment, R⁵ is cyclopropyl, cyclobutyl, cyclopentyl, or cyclohexyl, each of which is substituted with one or two independently selected R¹⁴ group.

In one embodiment, the compound of the invention is according to any one of Formulae Ia-VIId, wherein R⁵ is 4-11 membered monocyclic, or fused or spiro bicyclic heterocycloalkyl comprising one, two, or three heteroatoms independently selected from N, O, and S. In a particular embodiment, R⁵ is azetidinyl, oxetanyl, tetrahydrofuranyl, pyrolidinyl, tetrahydropyranyl, piperidininyl, piperazinyl, morpholinyl, thiomorpholinyl, dioxanyl.

In one embodiment, the compound of the invention is according to any one of Formulae Ia-VIId, wherein R⁵ is 4-11 membered monocyclic, or fused or spiro bicyclic heterocycloalkyl comprising one, two, or three heteroatoms independently selected from N, O, and S, substituted with one or more independently selected R¹⁴ group. In a particular embodiment, R⁵ is azetidinyl, oxetanyl, tetrahydrofuranyl, pyrolidinyl, tetrahydropyranyl, piperidininyl, piperazinyl, morpholinyl, thiomorpholinyl, dioxanyl, each of which is substituted with one or more independently selected R¹⁴ group. In more particular embodiment, R⁵ is 4-11 membered monocyclic, or fused or spiro bicyclic heterocycloalkyl comprising one, two, or three heteroatoms independently selected from N, O, and S substituted with one, two, or three independently selected R¹⁴ group. In another more particular embodiment, R⁵ is azetidinyl, oxetanyl, tetrahydrofuranyl, pyrolidinyl, tetrahydropyranyl, piperidininyl, piperazinyl, morpholinyl, thiomorpholinyl, dioxanyl, each of which is substituted with one, two, or three independently selected R¹⁴ group. In most particular embodiment, R⁵ is 4-11 membered monocyclic, or fused or spiro bicyclic heterocycloalkyl comprising one, two, or three heteroatoms independently selected from N, O, and S, substituted with one or two independently selected R¹⁴ group. In another more particular embodiment, R⁵ is azetidinyl, oxetanyl, tetrahydrofuranyl, pyrolidinyl, tetrahydropyranyl, piperidininyl, piperazinyl, morpholinyl, thiomorpholinyl, dioxanyl, each of which is substituted with one or two independently selected R¹⁴ group.

In one embodiment, the compound of the invention is according to any one of Formulae Ia-VIId, wherein R⁵ is -O-C₃₋₇ monocyclic cycloalkyl. In a particular embodiment, R⁵ is -O-cyclopropyl, -O-cyclobutyl, -O-cyclopentyl, or -O-cyclohexyl. In a more particular embodiment, R⁵ is -O-cyclopropyl, -O-cyclobutyl. In a most particular embodiment, R⁵ is -O-cyclopropyl.

In one embodiment, the compound of the invention is according to any one of Formulae Ia-VIId, wherein R⁵ is -O-C₃₋₇ monocyclic cycloalkyl substituted with one or more independently selected R¹⁴ group. In a particular embodiment, R⁵ is -O-cyclopropyl, -O-cyclobutyl, -O-cyclopentyl, or -O-cyclohexyl, each of which is substituted with one or more independently selected R¹⁴ group. In more particular embodiment, R⁵ is -O-C₃₋₇ monocyclic cycloalkyl substituted with one, two, or three independently selected R¹⁴ groups. In another more particular embodiment, R⁵ is -O-cyclopropyl, -O-cyclobutyl, -O-cyclopentyl, or -O-cyclohexyl, each of which is substituted with one, two, or three independently selected R¹⁴ groups. In most particular embodiment, R⁵ is -O-C₃₋₇ monocyclic cycloalkyl substituted with one or two independently selected R¹⁴ group. In another more particular embodiment, R⁵ is -O-cyclopropyl, -O-cyclobutyl, -O-cyclopentyl, or -O-cyclohexyl, each of which is substituted with one or two independently selected R¹⁴ groups.

In one embodiment, the compound of the invention is according to any one of Formulae Ia-VIId, wherein R⁵ is -O-heterocycloalkyl, wherein said heterocycloalkyl is a 4-7 membered monocyclic heterocycloalkyl comprising one, two, or three heteroatoms independently selected from N, O, and S, and wherein if a N heteroatom is present, said N heteroatom, is further substituted with one -C(=O)-C₁₋₄ alkyl, -C(=O)-C₁₋₄ alkoxy, -SO₂-C₁₋₄ alkyl, -C(=O)-NH₂, -C(=O)NHC₁₋₄ alkyl, or -C(=O)N(C₁₋₄ alkyl)₂. In a particular embodiment, R⁵ is -O-heterocycloalkyl, wherein said heterocycloalkyl is a 4-7 membered monocyclic heterocycloalkyl comprising one, two, or three heteroatoms independently selected from N, O, and S, and wherein if a N heteroatom is present, said N heteroatom, is further substituted with one -C(=O)CH₃, -C(=O)OCH₃, -SO₂CH₃, -C(=O)NH₂, -C(=O)NHCH₃, or -C(=O)N(CH₃)₂. In a more particular embodiment, R⁵ is -O-heterocycloalkyl, wherein said heterocycloalkyl is a 4-7 membered monocyclic heterocycloalkyl comprising one, two, or three heteroatoms independently selected from O, and S. In a more particular embodiment, R⁵ is -O-oxetanyl, -O-tetrahydrofuranyl, -O-tetrahydropyranyl, or -O-dioxanyl. In a most particular embodiment, R⁵ is -O-oxetanyl, -O-tetrahydrofuranyl or -O-dioxanyl.

In one embodiment, the compound of the invention is according to any one of Formulae Ia-VIId, wherein R⁵ is -O-heterocycloalkyl, wherein said heterocycloalkyl is a 4-7 membered monocyclic heterocycloalkyl comprising one, two, or three heteroatoms independently selected from N, O, and S, substituted with one or more independently selected R¹⁴ groups and wherein if a N heteroatom is present, said N heteroatom, is further substituted with one -C(=O)-C₁₋₄ alkyl, -C(=O)-C₁₋₄ alkoxy, -SO₂-C₁₋₄ alkyl, -C(=O)-NH₂, -C(=O)NHC₁₋₄ alkyl, or -C(=O)N(C₁₋₄ alkyl)₂. In a particular embodiment, R⁵ is -O-heterocycloalkyl, wherein said heterocycloalkyl is a 4-7 membered monocyclic heterocycloalkyl comprising one, two, or three heteroatoms independently selected from N, O, and S, substituted with one or more independently selected R¹⁴ groups and wherein if a N heteroatom is present, said N heteroatom, is further substituted with one -C(=O)CH₃, -C(=O)OCH₃, -SO₂CH₃, -C(=O)NH₂, -C(=O)NHCH₃, or -C(=O)N(CH₃)₂. In a more particular embodiment, R⁵ is -O-heterocycloalkyl, wherein said heterocycloalkyl is a 4-7 membered monocyclic heterocycloalkyl comprising one, two, or three heteroatoms independently selected from O, and S, substituted with one or more independently selected R¹⁴ groups. In a further more particular embodiment, R⁵ is -O-oxetanyl, -O-tetrahydrofuranyl, -O-tetrahydropyranyl, or -O-dioxanyl, each of which is substituted with one or more independently selected R¹⁴ group. In a more particular embodiment, R⁵ is -O-heterocycloalkyl, wherein said heterocycloalkyl is a 4-7 membered monocyclic heterocycloalkyl comprising one, two, or three heteroatoms independently selected from O, and S, substituted with one, two, or three independently selected R¹⁴ groups. In a further more particular embodiment, R⁵ is -O-oxetanyl, -O-tetrahydrofuranyl, -O-tetrahydropyranyl, or -O-dioxanyl, each of which is substituted with one, two, or three independently selected R¹⁴ groups. In a most particular embodiment, R⁵ is -O-heterocycloalkyl, wherein said heterocycloalkyl is a 4-7 membered monocyclic heterocycloalkyl comprising one, two, or three heteroatoms independently selected from O, and S, substituted with one R¹⁴ group. In a further most particular embodiment, R⁵ is -O-oxetanyl, -O-tetrahydrofuranyl, -O-tetrahydropyranyl, or -O-dioxanyl, each of which is substituted with one R¹⁴ group.

In one embodiment, the compound of the invention is according to any one of Formulae Ia-VIId, wherein R¹⁴ is selected from halo, CN, OH, C₁₋₄ alkoxy optionally substituted with one or more halo, and C₁₋₄ alkyl optionally substituted with one or more halo. In a particular embodiment, R¹⁴ is F, Cl, -OH, -CN, -CH₃, -CH₂CH₃, -CF₃, -CH₂CHF₂, -OCH₃, -OCH₂CH₃, -OCF₃, or -OCH₂CHF₂.

In one embodiment, the compound of the invention is according to any one of Formulae Ia-VIId, wherein R⁵ is -SO₂-C₁₋₄ alkyl. In a particular embodiment, R⁵ is -SO₂-CH₃.

In one embodiment, the compound of the invention is according to any one of Formulae Ia-VIId, wherein R⁵ is -SO₂-NR^{15a}R^{15b}, wherein each R^{15a} and R^{15b} is independently selected from H and C₁₋₄ alkyl. In a particular embodiment, R⁵ is -SO₂-NR^{15a}R^{15b}, wherein each R^{15a} and R^{15b} is independently selected from H and -CH₃.

In one embodiment, the compound of the invention is according to any one of Formulae Ia-VIId, wherein R⁵ is -C(=O)NR^{15c}R^{15d}, wherein each R^{15c} and R^{15d} is independently selected from H and C₁₋₄ alkyl. In a particular embodiment, R⁵ is -C(=O)NR^{15c}R^{15d}, wherein each R^{15c} and R^{15d} is independently selected from H and -CH₃.

In one embodiment, the compound of the invention is according to any one of Formulae Ia-VIId, wherein R⁵ is -NR^{17a}R^{17b}, wherein each R^{17a} and R^{17b} is independently selected from H and C₁₋₄ alkyl which alkyl is optionally substituted with one or more independently selected halo, OH or C₁₋₄ alkoxy. In a particular embodiment, R⁵ is -NR^{17a}R^{17b}, wherein each R^{17a} and R^{17b} is independently selected from H, -CH₃, -CH₂CH₃, -CH₂CF₃, -CH₂CHF₂, -CH₂CH₂OH and -CH₂CH₂OCH₃.

In one embodiment, the compound of the invention is selected from:
1-(2-Ethoxy-6-trifluoromethyl-pyridin-4-yl)-3-[5-methyl-6-(1-methyl-3-trifluoromethyl-1H-pyrazol-4-yl)-pyridazin-3-ylmethyl]-urea,
1-(2-Chloro-6-ethoxy-pyridin-4-yl)-3-[5-methyl-6-(1-methyl-3-trifluoromethyl-1H-pyrazol-4-yl)-pyridazin-3-ylmethyl]-urea,
1-(2-chloro-6-ethoxy-4-pyridyl)-3-[1-[5-methyl-6-[1-methyl-3-(trifluoromethyl)pyrazol-4-yl]pyridazin-3-yl]ethyl]urea,
1-[2-(2-hydroxyethoxy)-6-(trifluoromethyl)-4-pyridyl]-3-[[5-methyl-6-[1-methyl-3-(trifluoromethyl)pyrazol-4-yl]pyridazin-3-yl]methyl]urea,
1-[2-(2,3-dihydroxypropoxy)-6-(trifluoromethyl)-4-pyridyl]-3-[[5-methyl-6-[1-methyl-3-(trifluoromethyl)pyrazol-4-yl]pyridazin-3-yl]methyl]urea,
1-(3,5-dichlorophenyl)-3-[[5-methyl-6-[1-methyl-3-(trifluoromethyl)pyrazol-4-yl]pyridazin-3-yl]methyl]urea,
1-[6-(2,2-difluoroethoxy)-5-(trifluoromethyl)-3-pyridyl]-3-[[5-methyl-6-[1-methyl-3-(trifluoromethyl)pyrazol-4-yl]pyridazin-3-yl]methyl]urea,
1-(5-chloro-6-ethoxy-3-pyridyl)-3-[[5-methyl-6-[1-methyl-3-(trifluoromethyl)pyrazol-4-yl]pyridazin-3-yl]methyl]urea,
1-[5-chloro-6-(2,2-difluoroethoxy)-3-pyridyl]-3-[[5-methyl-6-[1-methyl-3-(trifluoromethyl)pyrazol-4-yl]pyridazin-3-yl]methyl]urea,
1-[6-ethoxy-5-(trifluoromethyl)-3-pyridyl]-3-[[5-methyl-6-[1-methyl-3-(trifluoromethyl)pyrazol-4-yl]pyridazin-3-yl]methyl]urea,
1-[6-(cyclopropylmethoxy)-5-(trifluoromethyl)-3-pyridyl]-3-[[5-methyl-6-[1-methyl-3-(trifluoromethyl)pyrazol-4-yl]pyridazin-3-yl]methyl]urea,
1-[[5-methyl-6-[1-methyl-3-(trifluoromethyl)pyrazol-4-yl]pyridazin-3-yl]methyl]-3-[6-(2,2,2-trifluoroethoxy)-5-(trifluoromethyl)-3-pyridyl]urea,
1-[5-chloro-6-(cyclopropylmethoxy)-3-pyridyl]-3-[[5-methyl-6-[1-methyl-3-(trifluoromethyl)pyrazol-4-yl]pyridazin-3-yl]methyl]urea,
1-[2-chloro-6-(2,2,2-trifluoroethoxy)-4-pyridyl]-3-[[5-methyl-6-[1-methyl-3-(trifluoromethyl)pyrazol-4-yl]pyridazin-3-yl]methyl]urea,
1-(2-chloro-6-ethoxy-4-pyridyl)-3-[[6-[1-(methoxymethyl)-3-(trifluoromethyl)pyrazol-4-yl]-5-methyl-pyridazin-3-yl]methyl]urea,
1-[2-chloro-6-(2-fluoroethoxy)-4-pyridyl]-3-[[5-methyl-6-[1-methyl-3-(trifluoromethyl)pyrazol-4-yl]pyridazin-3-yl]methyl]urea,
1-[2-chloro-6-(2,2-difluoroethoxy)-4-pyridyl]-3-[[5-methyl-6-[1-methyl-3-(trifluoromethyl)pyrazol-4-yl]pyridazin-3-yl]methyl]urea,
1-(2-cyano-6-ethoxy-4-pyridyl)-3-[[5-methyl-6-[1-methyl-3-(trifluoromethyl)pyrazol-4-yl]pyridazin-3-yl]methyl]urea,
1-[2-(2,2-difluoroethoxy)-6-(trifluoromethyl)-4-pyridyl]-3-[[5-methyl-6-[1-methyl-3-(trifluoromethyl)pyrazol-4-yl]pyridazin-3-yl]methyl]urea,
1-(6-chloro-2-ethoxy-3-methyl-4-pyridyl)-3-[[5-methyl-6-[1-methyl-3-(trifluoromethyl)pyrazol-4-yl]pyridazin-3-yl]methyl]urea,
1-(2,6-dichloro-4-pyridyl)-3-[[5-methyl-6-[1-methyl-3-(trifluoromethyl)pyrazol-4-yl]pyridazin-3-yl]methyl]urea,
1-(2-cyclopropyl-6-ethoxy-4-pyridyl)-3-[[5-methyl-6-[1-methyl-3-(trifluoromethyl)pyrazol-4-yl]pyridazin-3-yl]methyl]urea,
1-[6-chloro-5-(2,2-difluoroethoxy)-3-pyridyl]-3-[[5-methyl-6-[1-methyl-3-(trifluoromethyl)pyrazol-4-yl]pyridazin-3-yl]methyl]urea,
1-(6-chloro-5-ethoxy-3-pyridyl)-3-[[5-methyl-6-[1-methyl-3-(trifluoromethyl)pyrazol-4-yl]pyridazin-3-yl]methyl]urea,
1-[2-(2-methoxyethoxy)-6-(trifluoromethyl)-4-pyridyl]-3-[[5-methyl-6-[1-methyl-3-(trifluoromethyl)pyrazol-4-yl]pyridazin-3-yl]methyl]urea,
1-[[5-methyl-6-[1-methyl-3-(trifluoromethyl)pyrazol-4-yl]pyridazin-3-yl]methyl]-3-[2-tetrahydrofuran-3-yloxy-6-(trifluoromethyl)-4-pyridyl]urea,
1-[2-chloro-6-(2-methoxyethoxy)-4-pyridyl]-3-[[5-methyl-6-[1-methyl-3-(trifluoromethyl)pyrazol-4-yl]pyridazin-3-yl]methyl]urea,
1-[2-cyano-6-(2,2-difluoroethoxy)-4-pyridyl]-3-[[5-methyl-6-[1-methyl-3-(trifluoromethyl)pyrazol-4-yl]pyridazin-3-yl]methyl]urea,
1-[2-cyclopropyl-6-(2,2-difluoroethoxy)-4-pyridyl]-3-[[5-methyl-6-[1-methyl-3-(trifluoromethyl)pyrazol-4-yl]pyridazin-3-yl]methyl]urea,
1-[2-ethoxy-3-methyl-6-(trifluoromethyl)-4-pyridyl]-3-[[5-methyl-6-[1-methyl-3-(trifluoromethyl)pyrazol-4-yl]pyridazin-3-yl]methyl]urea,
1-(2,6-diethoxypyrimidin-4-yl)-3-[[5-methyl-6-[1-methyl-3-(trifluoromethyl)pyrazol-4-yl]pyridazin-3-yl]methyl]urea,
1-(3,5-dichloro-4-fluoro-phenyl)-3-[[5-methyl-6-[1-methyl-3-(trifluoromethyl)pyrazol-4-yl]pyridazin-3-yl]methyl]urea,
1-[2-[(2,2-difluorocyclopropyl)methoxy]-6-(trifluoromethyl)-4-pyridyl]-3-[[5-methyl-6-[1-methyl-3-(trifluoromethyl)pyrazol-4-yl]pyridazin-3-yl]methyl]urea,
1-[2-(2,2-difluoropropoxy)-6-(trifluoromethyl)-4-pyridyl]-3-[[5-methyl-6-[1-methyl-3-(trifluoromethyl)pyrazol-4-yl]pyridazin-3-yl]methyl]urea,
1-(2-ethoxy-6-methyl-4-pyridyl)-3-[[5-methyl-6-[1-methyl-3-(trifluoromethyl)pyrazol-4-yl]pyridazin-3-yl]methyl]urea,
1-[[5-methyl-6-[1-methyl-3-(trifluoromethyl)pyrazol-4-yl]pyridazin-3-yl]methyl]-3-[2-(tetrahydrofuran-3-ylmethoxy)-6-(trifluoromethyl)-4-pyridyl]urea,
1-[2-(1,4-dioxan-2-ylmethoxy)-6-(trifluoromethyl)-4-pyridyl]-3-[[5-methyl-6-[1-methyl-3-(trifluoromethyl)pyrazol-4-yl]pyridazin-3-yl]methyl]urea,
1-(6-chloro-2-ethoxy-pyrimidin-4-yl)-3-[[5-methyl-6-[1-methyl-3-(trifluoromethyl)pyrazol-4-yl]pyridazin-3-yl]methyl]urea,
1-[[5-methyl-6-[1-methyl-3-(trifluoromethyl)pyrazol-4-yl]pyridazin-3-yl]methyl]-3-[2-(trifluoromethyl)-6-[[1-(trifluoromethyl)cyclopropyl]methoxy]-4-pyridyl]urea,
1-[[5-methyl-6-[1-methyl-3-(trifluoromethyl)pyrazol-4-yl]pyridazin-3-yl]methyl]-3-[2-tetrahydropyran-4-yloxy-6-(trifluoromethyl)-4-pyridyl]urea,
1-[2-(2-methoxy-1-methyl-ethoxy)-6-(trifluoromethyl)-4-pyridyl]-3-[[5-methyl-6-[1-methyl-3-(trifluoromethyl)pyrazol-4-yl]pyridazin-3-yl]methyl]urea,
1-[2-[(3,3-difluorocyclobutyl)methoxy]-6-(trifluoromethyl)-4-pyridyl]-3-[[5-methyl-6-[1-methyl-3-(trifluoromethyl)pyrazol-4-yl]pyridazin-3-yl]methyl]urea,
1-[[5-methyl-6-[1-methyl-3-(trifluoromethyl)pyrazol-4-yl]pyridazin-3-yl]methyl]-3-[2-(trifluoromethyl)-4-pyridyl]urea,
1-(2-chloro-4-pyridyl)-3-[[5-methyl-6-[1-methyl-3-(trifluoromethyl)pyrazol-4-yl]pyridazin-3-yl]methyl]urea,
1-[2-ethoxy-3-methyl-6-(trifluoromethyl)-4-pyridyl]-3-[1-[5-methyl-6-[1-methyl-3-(trifluoromethyl)pyrazol-4-yl]pyridazin-3-yl]ethyl]urea,
1-[2-ethoxy-3-ethyl-6-(trifluoromethyl)-4-pyridyl]-3-[[5-methyl-6-[1-methyl-3-(trifluoromethyl)pyrazol-4-yl]pyridazin-3-yl]methyl]urea,
1-[2-(3,3-difluoroazetidin-1-yl)-6-(trifluoromethyl)-4-pyridyl]-3-[[5-methyl-6-[1-methyl-3-(trifluoromethyl)pyrazol-4-yl]pyridazin-3-yl]methyl]urea,
1-[2-(3,3-difluoroazetidin-1-yl)-6-(2,2-difluoroethoxy)-4-pyridyl]-3-[[5-methyl-6-[1-methyl-3-(trifluoromethyl)pyrazol-4-yl]pyridazin-3-yl]methyl]urea
1-[3-bromo-2-ethoxy-6-(trifluoromethyl)-4-pyridyl]-3-[[5-methyl-6-[1-methyl-3-(trifluoromethyl)pyrazol-4-yl]pyridazin-3-yl]methyl]urea,
1-[6-chloro-2-(2,2-difluoroethoxy)-3-methyl-4-pyridyl]-3-[[5-methyl-6-[1-methyl-3-(trifluoromethyl)pyrazol-4-yl]pyridazin-3-yl]methyl]urea,
1-[2-ethoxy-6-(trifluoromethyl)-4-pyridyl]-3-[[5-methyl-6-[3-(trifluoromethyl)-1H-pyrazol-4-yl]pyridazin-3-yl]methyl]urea,
1-[2-ethoxy-6-(trifluoromethyl)-4-pyridyl]-3-[[5-methyl-6-[1-tetrahydrofuran-3-yl-3-(trifluoromethyl)pyrazol-4-yl]pyridazin-3-yl]methyl]urea,
1-[2-ethoxy-6-(trifluoromethyl)-4-pyridyl]-3-[[6-[1-(2-methoxyethyl)-3-(trifluoromethyl)pyrazol-4-yl]-5-methyl-pyridazin-3-yl]methyl]urea,
1-[[6-(3-cyclopropyl-1-methyl-pyrazol-4-yl)-5-methyl-pyridazin-3-yl]methyl]-3-[2-ethoxy-6-(trifluoromethyl)-4-pyridyl]urea,
1-[[6-[1-(1,4-dioxan-2-ylmethyl)-3-(trifluoromethyl)pyrazol-4-yl]-5-methyl-pyridazin-3-yl]methyl]-3-[2-ethoxy-6-(trifluoromethyl)-4-pyridyl]urea,
1-[2-ethoxy-6-(trifluoromethyl)-4-pyridyl]-3-[[6-[1-(2-hydroxypropyl)-3-(trifluoromethyl)pyrazol-4-yl]-5-methyl-pyridazin-3-yl]methyl]urea,
1-(6-cyano-2-ethoxy-3-methyl-4-pyridyl)-3-[[5-methyl-6-[1-methyl-3-(trifluoromethyl)pyrazol-4-yl]pyridazin-3-yl]methyl]urea,
1-(6-cyano-3-cyclopropyl-2-ethoxy-4-pyridyl)-3-[[5-methyl-6-[1-methyl-3-(trifluoromethyl)pyrazol-4-yl]pyridazin-3-yl]methyl]urea,
1-[6-cyano-2-(2,2-difluoroethoxy)-3-methyl-4-pyridyl]-3-[[5-methyl-6-[1-methyl-3-(trifluoromethyl)pyrazol-4-yl]pyridazin-3-yl]methyl]urea,
1-[2-chloro-6-(2,2-difluoroethoxy)-4-pyridyl]-3-[[5-(3,3-difluoroazetidin-1-yl)-6-[1-methyl-3-(trifluoromethyl)pyrazol-4-yl]pyridazin-3-yl]methyl]urea,
1-[2-chloro-6-(2,2-difluoroethoxy)-4-pyridyl]-3-[[5-cyclopropyl-6-[1-methyl-3-(trifluoromethyl)pyrazol-4-yl]pyridazin-3-yl]methyl]urea,
1-[2-chloro-6-(2,2-difluoroethoxy)-4-pyridyl]-3-[[6-[1-methyl-3-(trifluoromethyl)pyrazol-4-yl]-5-morpholino-pyridazin-3-yl]methyl]urea,
1-[2-chloro-6-(2,2-difluoroethoxy)-4-pyridyl]-3-[[5-(dimethylamino)-6-[1-methyl-3-(trifluoromethyl)pyrazol-4-yl]pyridazin-3-yl]methyl]urea,
1-[2-chloro-6-(2,2-difluoroethoxy)-4-pyridyl]-3-[[5-methoxy-6-[1-methyl-3-(trifluoromethyl)pyrazol-4-yl]pyridazin-3-yl]methyl]urea,
1-[2-chloro-6-(2,2-difluoroethoxy)-4-pyridyl]-3-[[5-(dimethylaminomethyl)-6-[1-methyl-3-(trifluoromethyl)pyrazol-4-yl]pyridazin-3-yl]methyl]urea,
1-[[4,5-dimethyl-6-[1-methyl-3-(trifluoromethyl)pyrazol-4-yl]pyridazin-3-yl]methyl]-3-[2-ethoxy-6-(trifluoromethyl)-4-pyridyl]urea
N,N-dimethyl-2-[[4-[[5-methyl-6-[1-methyl-3-(trifluoromethyl)pyrazol-4-yl]pyridazin-3-yl]methylcarbamoylamino]-6-(trifluoromethyl)-2-pyridyl]oxy]acetamide,
1-(2-chloro-6-ethoxy-4-pyridyl)-3-[[6-(1,3-dimethylpyrazol-4-yl)-5-methyl-pyridazin-3-yl]methyl]urea, and
1-[[6-(1,5-dimethylpyrazol-4-yl)-5-methyl-pyridazin-3-yl]methyl]-3-[2-ethoxy-6-(trifluoromethyl)-4-pyridyl]urea.

In another embodiment, the compound of the invention is selected from:
1-(3-chloro-2-ethoxy-6-methyl-4-pyridyl)-3-[[5-methyl-6-[1-methyl-3-(trifluoromethyl)pyrazol-4-yl]pyridazin-3-yl]methyl]urea,
1-(2-ethoxy-3,6-dimethyl-4-pyridyl)-3-[[5-methyl-6-[1-methyl-3-(trifluoromethyl)pyrazol-4-yl]pyridazin-3-yl]methyl]urea,
1-[2-(2-hydroxyethoxy)-3-methyl-6-(trifluoromethyl)-4-pyridyl]-3-[[5-methyl-6-[1-methyl-3-(trifluoromethyl)pyrazol-4-yl]pyridazin-3-yl]methyl]urea,
1-[2-(2,2-difluoroethoxy)-3,6-dimethyl-4-pyridyl]-3-[[5-methyl-6-[1-methyl-3-(trifluoromethyl)pyrazol-4-yl]pyridazin-3-yl]methyl]urea,
1-[2-(2,2-difluoroethoxy)-6-methyl-4-pyridyl]-3-[[5-methyl-6-[1-methyl-3-(trifluoromethyl)pyrazol-4-yl]pyridazin-3-yl]methyl]urea,
2-cyano-1-[2-ethoxy-6-(trifluoromethyl)-4-pyridyl]-3-[[5-methyl-6-[1-methyl-3-(trifluoromethyl)pyrazol-4-yl]pyridazin-3-yl]methyl]guanidine,
1-(3-cyano-2-ethoxy-6-methyl-4-pyridyl)-3-[[5-methyl-6-[1-methyl-3-(trifluoromethyl)pyrazol-4-yl]pyridazin-3-yl]methyl]urea,
2-cyano-1-(2-ethoxy-3,6-dimethyl-4-pyridyl)-3-[[5-methyl-6-[1-methyl-3-(trifluoromethyl)pyrazol-4-yl]pyridazin-3-yl]methyl]guanidine,
2-cyano-1-[2-(2,2-difluoroethoxy)-3,6-dimethyl-4-pyridyl]-3-[[5-methyl-6-[1-methyl-3-(trifluoromethyl)pyrazol-4-yl]pyridazin-3-yl]methyl]guanidine,
2-cyano-1-[2-ethoxy-3-methyl-6-(trifluoromethyl)-4-pyridyl]-3-[[5-methyl-6-[1-methyl-3-(trifluoromethyl)pyrazol-4-yl]pyridazin-3-yl]methyl]guanidine,
1-(2-chloro-6-ethoxy-4-pyridyl)-3-[[6-(3,3-difluoropyrrolidin-1-yl)-5-methyl-pyridazin-3-yl]methyl]urea,
1-(2-chloro-6-ethoxy-4-pyridyl)-3-[[6-(2,6-dimethylmorpholin-4-yl)-5-methyl-pyridazin-3-yl]methyl]urea,
1-(2-chloro-6-ethoxy-4-pyridyl)-3-[[6-(3,3-difluoroazetidin-1-yl)-5-methyl-pyridazin-3-yl]methyl]urea,
1-[5-chloro-6-(2,2-difluoroethoxy)-3-pyridyl]-3-[[6-(2,6-dimethylmorpholin-4-yl)-5-methyl-pyridazin-3-yl]methyl]urea,
1-[[6-(2,6-dimethylmorpholin-4-yl)-5-methyl-pyridazin-3-yl]methyl]-3-[6-ethoxy-5-(trifluoromethyl)-3-pyridyl]urea,
1-(5-chloro-6-ethoxy-3-pyridyl)-3-[[6-(2,6-dimethylmorpholin-4-yl)-5-methyl-pyridazin-3-yl]methyl]urea,
1-[[6-(2,6-dimethylmorpholin-4-yl)-5-methyl-pyridazin-3-yl]methyl]-3-[2-ethoxy-6-(trifluoromethyl)-4-pyridyl]urea,
1-[5-chloro-6-(cyclopropylmethoxy)-3-pyridyl]-3-[[6-(2,6-dimethylmorpholin-4-yl)-5-methyl-pyridazin-3-yl]methyl]urea,
1-[2-chloro-6-(2,2-difluoroethoxy)-4-pyridyl]-3-[[5-methyl-6-[2-(trifluoromethyl)morpholin-4-yl]pyridazin-3-yl]methyl]urea,
1-[2-chloro-6-(2,2-difluoroethoxy)-4-pyridyl]-3-[[6-[3-fluoro-3-(hydroxymethyl)pyrrolidin-1-yl]-5-methyl-pyridazin-3-yl]methyl]urea,
1-[2-chloro-6-(2,2-difluoroethoxy)-4-pyridyl]-3-[[5-methyl-6-(3-methylsulfonylpyrrolidin-1-yl)pyridazin-3-yl]methyl]urea,
1-[2-chloro-6-(2,2-difluoroethoxy)-4-pyridyl]-3-[[5-methyl-6-(3-methylsulfonylazetidin-1-yl)pyridazin-3-yl]methyl]urea,
1-[[6-[(2R,6S)-2,6-dimethylmorpholin-4-yl]-5-methyl-pyridazin-3-yl]methyl]-3-(2-ethoxy-6-methyl-4-pyridyl)urea,
1-(3-chloro-2-ethoxy-6-methyl-4-pyridyl)-3-[[6-[(2R,6S)-2,6-dimethylmorpholin-4-yl]-5-methyl-pyridazin-3-yl]methyl]urea,
1-[[6-[(2R,6S)-2,6-dimethylmorpholin-4-yl]-5-methyl-pyridazin-3-yl]methyl]-3-(2-ethoxy-3,6-dimethyl-4-pyridyl)urea,
1-[2-ethoxy-6-(trifluoromethyl)-4-pyridyl]-3-[[5-methyl-6-(2-methyl-6,7-dihydro-5H-pyrazolo[4,3-b]pyridin-4-yl)pyridazin-3-yl]methyl]urea,
1-[2-chloro-6-(2,2-difluoroethoxy)-4-pyridyl]-3-[[6-[2-(dimethylaminomethyl)morpholin-4-yl]-5-methyl-pyridazin-3-yl]methyl]urea,
1-(2,6-dimethyl-4-pyridyl)-3-[[5-methyl-6-[1-methyl-3-(trifluoromethyl)pyrazol-4-yl]pyridazin-3-yl]methyl]urea, and
1-[[5-methyl-6-[1-methyl-3-(trifluoromethyl)pyrazol-4-yl]pyridazin-3-yl]methyl]-3-[2-(1,1,2,2,2-pentadeuterioethoxy)-6-(trifluoromethyl)-4-pyridyl]urea

In one embodiment, the compound of the invention is 1-(2-Ethoxy-6-trifluoromethyl-pyridin-4-yl)-3-[5-methyl-6-(1-methyl-3-trifluoromethyl-1H-pyrazol-4-yl)-pyridazin-3-ylmethyl]-urea, 1-(2-ethoxy-3,6-dimethyl-4-pyridyl)-3-[[5-methyl-6-[1-methyl-3-(trifluoromethyl)pyrazol-4-yl]pyridazin-3-yl]methyl]urea, or 2-cyano-1-[2-ethoxy-6-(trifluoromethyl)-4-pyridyl]-3-[[5-methyl-6-[1-methyl-3-(trifluoromethyl)pyrazol-4-yl]pyridazin-3-yl]methyl]guanidine.

In another embodiment the compound of the invention is not 1-(2-Ethoxy-6-trifluoromethyl-pyridin-4-yl)-3-[5-methyl-6-(1-methyl-3-trifluoromethyl-1H-pyrazol-4-yl)-pyridazin-3-ylmethyl]-urea 1-(2-ethoxy-3,6-dimethyl-4-pyridyl)-3-[[5-methyl-6-[1-methyl-3-(trifluoromethyl)pyrazol-4-yl]pyridazin-3-yl]methyl]urea, and/or 2-cyano-1-[2-ethoxy-6-(trifluoromethyl)-4-pyridyl]-3-[[5-methyl-6-[1-methyl-3-(trifluoromethyl)pyrazol-4-yl]pyridazin-3-yl]methyl]guanidine.

In one embodiment, a compound of the invention is not an isotopic variant.

In one aspect a compound of the invention according to any one of the embodiments herein described is present as the free base.

In one aspect a compound of the invention according to any one of the embodiments herein described is a pharmaceutically acceptable salt.

In one aspect a compound of the invention according to any one of the embodiments herein described is a solvate of the compound.

In one aspect a compound of the invention according to any one of the embodiments herein described is a solvate of a pharmaceutically acceptable salt of a compound.

While specified groups for each embodiment have generally been listed above separately, a compound of the invention includes one in which several or each embodiment in the above Formula, as well as other formulae presented herein, is selected from one or more of particular members or groups designated respectively, for each variable. Therefore, this invention is intended to include all combinations of such embodiments within its scope.

While specified groups for each embodiment have generally been listed above separately, a compound of the invention may be one for which one or more variables (for example, R groups) is selected from one or more embodiments according to any of the Formula(e) listed above. Therefore, the present invention is intended to include all combinations of variables from any of the disclosed embodiments within its scope.

Alternatively, the exclusion of one or more of the specified variables from a group or an embodiment, or combinations thereof is also contemplated by the present invention.

In certain aspects, the present invention provides prodrugs and derivatives of the compounds according to the formulae above. Prodrugs are derivatives of the compounds of the invention, which have metabolically cleavable groups and become by solvolysis or under physiological conditions the compounds of the invention, which are pharmaceutically active, in vivo. Such examples include, but are not limited to, choline ester derivatives and the like, N-alkylmorpholine esters and the like.

Other derivatives of the compounds of this invention have activity in both their acid and acid derivative forms, but the acid sensitive form often offers advantages of solubility, tissue compatibility, or delayed release in the mammalian organism (Bundgard, H, 1985). Prodrugs include acid derivatives well known to practitioners of the art, such as, for example, esters prepared by reaction of the parent acid with a suitable alcohol, or amides prepared by reaction of the parent acid compound with a substituted or unsubstituted amine, or acid anhydrides, or mixed anhydrides. Simple aliphatic or aromatic esters, amides and anhydrides derived from acidic groups pendant on the compounds of this invention are preferred prodrugs. In some cases it is desirable to prepare double ester type prodrugs such as (acyloxy)alkyl esters or ((alkoxycarbonyl)oxy)alkylesters. Particularly useful are the C₁ to C₈ alkyl, C₂-C₈ alkenyl, aryl, C₇-C₁₂ substituted aryl, and C₇-C₁₂ arylalkyl esters of the compounds of the invention.

In a further aspect, a compound of the invention according to one or more of the embodiments described above may show a good ADME profile. In a particular aspect, a compound of the invention according to one or more of the embodiments described above may show a low plasma protein binding (PPB).

In a further aspect, the ADME profile of a compound of the invention according to any one of the embodiments described above may allow for a lower dose regimen and good compliance with dose regimen.

### CLAUSES

1. A compound according to Formula Ia: 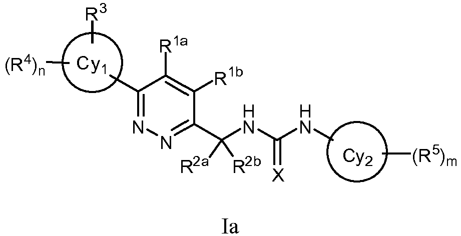 wherein
   X is =O, or =N-CN;
   R^{1a} is selected from:
      - C₁₋₄ alkyl optionally substituted with one or more groups independently selected from
         ∘ OH,
         ∘ C₁₋₄ alkoxy optionally substituted with one or more independently selected OH, or C₁₋₄ alkoxy,
         ∘ -SO₂-C₁₋₄ alkyl,
         ∘ -O-C₃₋₇ monocyclic cycloalkyl, and
         ∘ -O-heterocycloalkyl wherein said heterocycloalkyl is a 4-7 membered monocyclic heterocycloalkyl comprising one, two, or three heteroatoms independently selected from N, O, and S;
      - -NR^{6a}R^{6b},
      - C₁₋₄ alkoxy,
      - C₃₋₇ monocyclic cycloalkyl,
      - 4-7 membered monocyclic heterocycloalkyl comprising one, two, or three heteroatoms independently selected from N, O, and S, optionally substituted with one or more halo,
      - -O-C₃₋₇ monocyclic cycloalkyl, and
      - -O-heterocycloalkyl wherein said heterocycloalkyl is a 4-7 membered monocyclic heterocycloalkyl comprising one, two, or three heteroatoms independently selected from N, O, and S;
   R^{1b} is H, or C₁₋₄ alkyl;
   Cy₁ is a 5 membered monocyclic heteroaryl ring, comprising one, two, or three heteroatoms independently selected from N, O, or S, or
   Cy₁ is 4-7 membered monocyclic heterocycloalkyl ring comprising one, two, or three heteroatoms independently selected from N, O, or S, or 4-7 membered monocyclic heterocycloalkyl ring comprising one, two, or three heteroatoms independently selected from N, O, or S, fused to a 5-6 membered heteroaryl ring comprising one, two, or three heteroatoms independently selected from N, O, or S, which heteroaryl may optionally substituted with one C₁₋₄ alkyl;
   each R^{2a} and R^{2b} is independently selected from H, and C₁₋₄ alkyl optionally substituted with one or more independently selected -OH, or C₁₋₄ alkoxy;
   R³ is selected from:
      - C₁₋₄ alkyl optionally substituted with one or more independently selected:
         ∘ halo,
         ∘ -CN,
         ∘ -OH,
         ∘ -C₁₋₄ alkoxy, or
         ∘ -NR^{7a}R^{7b};
      - C₁₋₄ alkoxy substituted with one or more halo,
      - C₃₋₇ monocyclic cycloalkyl,
      - 4-7-membered monocyclic heterocycloalkyl comprising one, two or three heteroatoms independently selected from N, O, and S,
      - -CN,
      - -S(O)₂-C₁₋₄ alkyl,
      - -NR^{8a}R^{8b}, and
      - -C(=O)NR^{8c}R^{8d};
   each R⁴ is independently selected from:
      - C₁₋₄ alkyl optionally substituted with one or more independently selected R¹² groups,
      - C₃₋₇ monocyclic cycloalkyl, and
      - 4-7 membered monocyclic heterocycloalkyl comprising one, two, or three heteroatoms independently selected from N, O, and S;
   Cy₂ is phenyl or 5-6 membered monocyclic heteroaryl comprising one or two heteroatoms independently selected from N, O, and S;
   each R⁵ is independently selected from:
      - halo,
      - -CN,
      - -OH,
      - C₁₋₄ alkyl optionally substituted with one or more independently selected R¹³ groups,
      - C₁₋₄ alkoxy optionally substituted with one or more independently selected R¹³ groups,
      - C₃₋₇ monocyclic cycloalkyl optionally substituted with one or more independently selected R¹⁴ groups,
      - 4-11 membered monocyclic, or fused or spiro bicyclic heterocycloalkyl comprising one, two, or three heteroatoms independently selected from N, O, and S, optionally substituted with one or more independently selected R¹⁴ groups,
      - -O-C₃₋₇ monocyclic cycloalkyl optionally substituted with one or more independently selected R¹⁴ groups,
      - -O-heterocycloalkyl wherein said heterocycloalkyl is a 4-7 membered monocyclic heterocycloalkyl comprising one, two, or three heteroatoms independently selected from N, O, and S, optionally substituted with one or more independently selected R¹⁴ groups, and wherein if a N heteroatom is present, said N heteroatom, is further substituted with one -C(=O)-C₁₋₄ alkyl, -C(=O)-C₁₋₄ alkoxy, -SO₂-C₁₋₄ alkyl, -C(=O)-NH₂, -C(=O)NHC₁₋₄ alkyl, or -C(=O)N(C₁₋₄ alkyl)₂,
      - -SO₂-C₁₋₄ alkyl,
      - -SO₂NR^{15a}R^{15b},
      - -C(=O)NR^{15c}R^{15d}, and
      - -NR^{17a}R^{17b};
   each R¹² is independently selected from:
      - halo,
      - OH,
      - C₁₋₄ alkoxy,
      - -SO₂-C₁₋₄ alkyl,
      - C₃₋₇ monocyclic cycloalkyl optionally substituted with one or more independently selected -OH, halo, -CN, C₁₋₄ alkyl, C₁₋₄ alkoxy or =O,
      - 4-7 membered monocyclic heterocycloalkyl, comprising one, two, or three heteroatoms independently selected from N, O, and S, optionally substituted with one or more independently selected -OH, halo, -CN, C₁₋₄ alkyl, C₁₋₄ alkoxy or =O,
      - -NR^{9a}R^{9b}, and
      - -CN;
   each R¹³ is independently selected from:
      - halo,
      - -CN,
      - -OH,
      - C₁₋₄ alkoxy optionally substituted with one or more independently selected OH, C₁₋₄ alkoxy, or halo,
      - -C(=O)NR^{16a}R^{16b},
      - -NR^{16c}C(=O)-C₁₋₄ alkyl,
      - -NR^{16d}C(=O)-C₁₋₄ alkoxy,
      - -SO₂-C₁₋₄ alkyl
      - -SO₂NR^{16e}R^{16f},
      - -NR^{16g}SO₂-C₁₋₄ alkyl,
      - C₃₋₇ monocyclic cycloalkyl optionally substituted with one or more independently selected halo, or C₁₋₄ alkyl optionally substituted with one or more halo, and
      - 4-7 membered monocyclic heterocycloalkyl comprising one, two, or three heteroatoms independently selected from N, O, and S, optionally substituted with one or more independently selected halo, or C₁₋₄ alkyl optionally substituted with one or more halo, and wherein if a N heteroatom is present, said N heteroatom, is further substituted with one -C(=O)-C₁₋₄ alkyl, -C(=O)-C₁₋₄ alkoxy, -SO₂-C₁₋₄ alkyl, -C(=O)-NH₂, -C(=O)NHC₁₋₄ alkyl, or -C(=O)N(C₁₋₄ alkyl)₂;
   each R¹⁴ is independently selected from:
      - halo,
      - -CN,
      - -OH,
      - C₁₋₄ alkoxy optionally substituted with one or more halo, and
      - C₁₋₄ alkyl optionally substituted with one or more halo;
   each R^{6a}, R^{6b}, R^{7a}, R^{7b}, R^{8a}, R^{8b}, R^{8c}, R^{8d}, R^{9a}, R^{9b}, R^{15a}, R^{15b}, R^{15c}, R^{15d}, R^{16a}, R^{16b}, R^{16c}, R^{16d}, R^{16e}, R^{16f}, and R^{16g} is independently selected from H and C₁₋₄ alkyl;
   each R^{17a} and R^{17b} is independently selected from H and C₁₋₄ alkyl optionally substituted with one or more independently selected halo, OH, or C₁₋₄ alkoxy;
   the subscript n is 0, 1, 2, or 3; and
   the subscript m is 0, 1, 2, 3 or 4;
   or pharmaceutically acceptable salt thereof, or the solvate or the salt of a solvate thereof.
2. A compound or a pharmaceutically acceptable salt thereof, according to clause 1, wherein the compound is not an isotopic variant.
3. A compound or a pharmaceutically acceptable salt thereof according to clause 1 or 2, wherein X is =N-CN.
4. A compound or a pharmaceutically acceptable salt thereof according to clause 1 or 2, wherein X is =O.
5. A compound or a pharmaceutically acceptable salt thereof according to any one of clause 1, 2, 3 or 4, wherein Cy₁ is 4-7 membered monocyclic heterocycloalkyl ring comprising one, two, or three heteroatoms independently selected from N, O, or S.
6. A compound or a pharmaceutically acceptable salt thereof according to any one of clause 1, 2, 3 or 4, wherein Cy₁ is azetidinyl, pyrolidinyl, piperidinyl, piperazinyl, or morpholinyl.
7. A compound or a pharmaceutically acceptable salt thereof according to any one of clause 1, 2, 3 or 4, wherein Cy₁ is 4-7 membered monocyclic heterocycloalkyl ring comprising one, two, or three heteroatoms independently selected from N, O, or S, fused to a 5-6 memebered heteroaryl ring comprising one, two, or three heteroatoms independently selected from N, O, or S, which heteroaryl may optionally substituted with on C₁₋₄ alkyl.
8. A compound or a pharmaceutically acceptable salt thereof according to any one of clause 1, 2, 3 or 4, wherein Cy₁ is tetrahydroimidazopyridinyl, or tetrahydropyrazolopyridinyl.
9. A compound or a pharmaceutically acceptable salt thereof according to clause 1 or 2, wherein the compound is according to Formula Ib 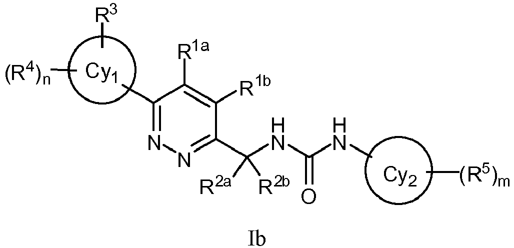 wherein
   R^{1a} is selected from:
      - C₁₋₄ alkyl optionally substituted with one or more groups independently selected from
         ∘ OH,
         ∘ C₁₋₄ alkoxy optionally substituted with one or more independently selected OH, or C₁₋₄ alkoxy,
         ∘ -SO₂-C₁₋₄ alkyl,
         ∘ -O-C₃₋₇ monocyclic cycloalkyl, and
         ∘ -O-heterocycloalkyl wherein said heterocycloalkyl is a 4-7 membered monocyclic heterocycloalkyl comprising one, two, or three heteroatoms independently selected from N, O, and S;
      - -NR^{6a}R^{6b},
      - C₁₋₄ alkoxy,
      - C₃₋₇ monocyclic cycloalkyl,
      - 4-7 membered monocyclic heterocycloalkyl comprising one, two, or three heteroatoms independently selected from N, O, or S, optionally substituted with one or more halo,
      - -O-C₃₋₇ monocyclic cycloalkyl, and
      - -O-heterocycloalkyl wherein said heterocycloalkyl is a 4-7 membered monocyclic heterocycloalkyl comprising one, two, or three heteroatoms independently selected from N, O, and S;
   R^{1b} is H, or C₁₋₄ alkyl;
   Cy₁ is a 5 membered monocyclic heteroaryl ring, comprising one, two, or three heteroatoms independently selected from N, O, or S;
   each R^{2a} and R^{2b} is independently selected from H, and C₁₋₄ alkyl optionally substituted with one or more independently selected -OH, or C₁₋₄ alkoxy;
   R³ is selected from:
      - C₁₋₄ alkyl optionally substituted with one or more independently selected:
         ∘ halo,
         ∘ -CN,
         ∘ -C₁₋₄ alkoxy, or
         ∘ -NR^{7a}R^{7b};
      - C₁₋₄ alkoxy substituted with one or more halo,
      - C₃₋₇ monocyclic cycloalkyl,
      - 4-7-membered monocyclic heterocycloalkyl comprising one, two or three heteroatoms independently selected from N, O, and S,
      - -CN,
      - -NR^{8a}R^{8b}, and
      - -C(=O)NR^{8c}R^{8d};
   each R⁴ is independently selected from:
      - C₁₋₄ alkyl optionally substituted with one or more independently selected R¹² groups,
      - C₃₋₇ monocyclic cycloalkyl, and
      - 4-7 membered monocyclic heterocycloalkyl comprising one, two, or three heteroatoms independently selected from N, O, and S;
   Cy₂ is phenyl or 5-6 membered monocyclic heteroaryl comprising one or two heteroatoms independently selected from N, O, and S;
   each R⁵ is independently selected from:
      - halo,
      - -CN,
      - -OH,
      - C₁₋₄ alkyl optionally substituted with one or more independently selected R¹³ groups,
      - C₁₋₄ alkoxy optionally substituted with one or more independently selected R¹³ groups,
      - C₃₋₇ monocyclic cycloalkyl optionally substituted with one or more independently selected R¹⁴ groups,
      - 4-11 membered monocyclic, or fused or spiro bicyclic heterocycloalkyl comprising one, two, or three heteroatoms independently selected from N, O, and S, optionally substituted with one or more independently selected R¹⁴ groups,
      - -O-C₃₋₇ monocyclic cycloalkyl optionally substituted with one or more independently selected R¹⁴ groups,
      - -O-heterocycloalkyl, wherein said heterocycloalkyl is a 4-7 membered monocyclic heterocycloalkyl comprising one, two, or three heteroatoms independently selected from N, O, and S, optionally substituted with one or more independently selected R¹⁴ groups, and wherein if a N heteroatom is present, said N heteroatom, is further substituted with one -C(=O)-C₁₋₄ alkyl, -C(=O)-C₁₋₄ alkoxy, -SO₂-C₁₋₄ alkyl, -C(=O)-NH₂, -C(=O)NHC₁₋₄ alkyl, or -C(=O)N(C₁₋₄ alkyl)₂,
      - -SO₂-C₁₋₄ alkyl,
      - -SO₂NR^{15a}R^{15b},
      - -C(=O)NR^{15c}R^{15d}, and
      - -NR^{17a}R^{17b};
   each R¹² is independently selected from:
      - halo,
      - OH,
      - C₁₋₄ alkoxy,
      - -SO₂-C₁₋₄ alkyl,
      - C₃₋₇ monocyclic cycloalkyl optionally substituted with one or more independently selected -OH, halo, -CN, C₁₋₄ alkyl, C₁₋₄ alkoxy or =O,
      - 4-7 membered monocyclic heterocycloalkyl comprising one, two, or three heteroatoms independently selected from N, O, and S, optionally substituted with one or more independently selected -OH, halo, -CN, C₁₋₄ alkyl, C₁₋₄ alkoxy or =O,
      - -NR^{9a}R^{9b}, and
      - -CN;
   Each R¹³ is independently selected from:
      - halo,
      - -CN,
      - -OH,
      - C₁₋₄ alkoxy optionally substituted with one or more independently selected OH, C₁₋₄ alkoxy, or halo,
      - -C(=O)NR^{16a}R^{16b},
      - -NR^{16c}C(=O)-C₁₋₄ alkyl,
      - -NR^{16d}C(=O)-C₁₋₄ alkoxy,
      - -SO₂-C₁₋₄ alkyl
      - -SO₂NR^{16e}R^{16f},
      - -NR^{16g}SO₂-C₁₋₄ alkyl,
      - C₃₋₇ monocyclic cycloalkyl optionally substituted with one or more independently selected halo, or C₁₋₄ alkyl optionally substituted with one or more halo, and
      - 4-7 membered monocyclic heterocycloalkyl comprising one, two, or three heteroatoms independently selected from N, O, and S, optionally substituted with one or more independently selected halo, or C₁₋₄ alkyl optionally substituted with one or more halo, and wherein if a N heteroatom is present, said N heteroatom, is further substituted with one -C(=O)-C₁₋₄ alkyl, -C(=O)-C₁₋₄ alkoxy, -SO₂-C₁₋₄ alkyl, -C(=O)-NH₂, -C(=O)NHC₁₋₄ alkyl, or -C(=O)N(C₁₋₄ alkyl)₂;
   each R¹⁴ is independently selected from:
      - halo,
      - -CN,
      - -OH,
      - C₁₋₄ alkoxy optionally substituted with one or more halo, and
      - C₁₋₄ alkyl optionally substituted with one or more halo;
   each R^{6a}, R^{6b}, R^{7a}, R^{7b}, R^{8a}, R^{8b}, R^{8c}, R^{8d}, R^{9a}, R^{9b}, R^{15a}, R^{15b}, R^{15c}, R^{15d}, R^{16a}, R^{16b}, R^{16c}, R^{16d}, R^{16e}, R^{16f}, and R^{16g} is independently selected from H and C₁₋₄ alkyl;
   each R^{17a} and R^{17b} is independently selected from H and C₁₋₄ alkyl optionally substituted with one or more independently selected halo, OH or C₁₋₄ alkoxy;
   the subscript n is 0, 1, 2, or 3; and
   the subscript m is 0, 1, 2, 3 or 4;
   or a pharmaceutically acceptable salt thereof, or the solvate or the salt of a solvate thereof.
10. A compound or a pharmaceutically acceptable salt thereof according to any one of clauses 1-9, wherein R^{1b} is H.
11. A compound or a pharmaceutically acceptable salt thereof according to any one of clauses 1-9, wherein R^{1b} is -CH₃.
12. A compound or a pharmaceutically acceptable salt thereof according to any one of clauses 1-10, wherein Cy₁ is 5-membered monocyclic heteroaryl ring, comprising one, two, or three heteroatoms independently selected from N, O, or S.
13. A compound or a pharmaceutically acceptable salt thereof according to any one of clauses 1-10, wherein Cy₁ is imidazolyl.
14. A compound or a pharmaceutically acceptable salt thereof according to any one of clauses 1-10, wherein Cy₁ is pyrazolyl.
15. A compound or a pharmaceutically acceptable salt thereof according to clause 1 or 2, wherein the compound or pharmaceutically acceptable salt is according to Formula II: 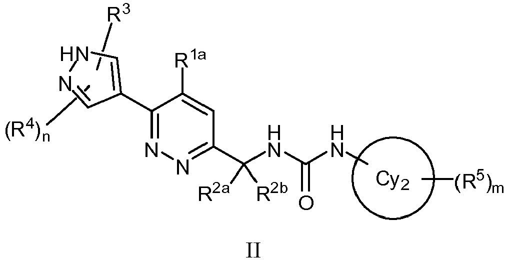
16. A compound or a pharmaceutically acceptable salt thereof according to any one of clauses 1-15, wherein R^{1a} is C₁₋₄ alkyl.
17. A compound or a pharmaceutically acceptable salt thereof according to clause 16, wherein R^{1a} is -CH₃.
18. A compound or a pharmaceutically acceptable salt thereof according to any one of clauses 1-15, wherein R^{1a} is C₁₋₄ alkyl substituted with one OH; C₁₋₄ alkoxy optionally substituted with one or more independently selected OH, or C₁₋₄ alkoxy; -SO₂-C₁₋₄ alkyl; -O-C₃₋₇ monocyclic cycloalkyl; or -O-Heterocycloalkyl wherein said heterocycloalkyl is a 4-7 membered monocyclic heterocycloalkyl comprising one, two, or three heteroatoms independently selected from N, O, and S.
19. A compound or a pharmaceutically acceptable salt thereof according to any one of clauses 1-15, wherein R^{1a} is -CH₃ substituted with one OH; C₁₋₄ alkoxy optionally substituted with one or more independently selected OH, or C₁₋₄ alkoxy; -SO₂-C₁₋₄ alkyl; -O-C₃₋₇ monocyclic cycloalkyl; or -O-Heterocycloalkyl wherein said heterocycloalkyl is a 4-7 membered monocyclic heterocycloalkyl comprising one, two, or three heteroatoms independently selected from N, O, and S.
20. A compound or a pharmaceutically acceptable salt thereof according to any one of clauses 1-15, wherein R^{1a} is -NR^{6a}R^{6b}.
21. A compound or a pharmaceutically acceptable salt thereof according to clause 20, wherein each R^{6a} and R^{6b} is independently selected from H, -CH₃, and -CH₂CH₃.
22. A compound or a pharmaceutically acceptable salt thereof according to any one of clauses 1-15, wherein R^{1a} is -NHCH₃, or -N(CH₃)₂.
23. A compound or a pharmaceutically acceptable salt thereof according to any one of clauses 1-15, wherein R^{1a} is C₁₋₄ alkoxy.
24. A compound or a pharmaceutically acceptable salt thereof according to clause 23, wherein R^{1a} is -OCH₃, or -OCH₂CH₃.
25. A compound or a pharmaceutically acceptable salt thereof according to any one of clauses 1-15, wherein R^{1a} is C₃₋₇ monocyclic cycloalkyl.
26. A compound or a pharmaceutically acceptable salt thereof according to clause 25, wherein R^{1a} is cyclopropyl, cyclobutyl, cyclopentyl, or cyclohexyl.
27. A compound or a pharmaceutically acceptable salt thereof according to clause 25, wherein R^{1a} is cyclopropyl.
28. A compound or a pharmaceutically acceptable salt thereof according to any one of clauses 1-15, wherein R^{1a} is 4-7 membered monocyclic heterocycloalkyl comprising one, two, or three heteroatoms independently selected from N, O, or S, optionally substituted with one or more halo.
29. A compound or a pharmaceutically acceptable salt thereof according to clause 28, wherein R^{1a} is azetidinyl, pyrrolidinyl, piperazinyl, piperidinyl, morpholinyl, tetrahydrofuranyl, or tetrahydropyranyl, each of which is optionally substituted with one or more halo.
30. A compound or a pharmaceutically acceptable salt thereof according to clause 28, wherein R^{1a} is morpholinyl.
31. A compound or a pharmaceutically acceptable salt thereof according to any one of clauses 1-15, wherein R^{1a} is -O-C₃₋₇ monocyclic cycloalkyl.
32. A compound or a pharmaceutically acceptable salt thereof according to clause 31, wherein R^{1a} is -O-cyclopropyl, -O-cyclobutyl, -O-cyclopentyl, or -O-cyclohexyl.
33. A compound or a pharmaceutically acceptable salt thereof according to any one of clauses 1-15, wherein R^{1a} is -O-heterocycloalkyl wherein said heterocycloalkyl is a 4-7 membered monocyclic heterocycloalkyl comprising one, two, or three heteroatoms independently selected from N, O, and S.
34. A compound or a pharmaceutically acceptable salt thereof according to clause 33, wherein R^{1a} is -O-oxetanyl, or -O-tetrahydrofuranyl.
35. A compound or a pharmaceutically acceptable salt thereof according to clause 1 or 2, wherein the compound is according to Formula III: 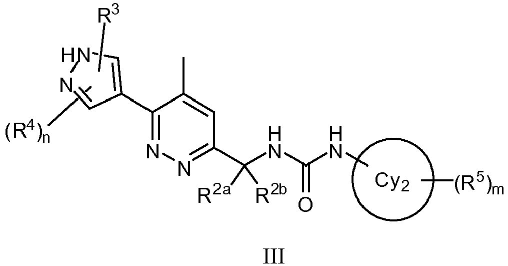
36. A compound or a pharmaceutically acceptable salt thereof according to any one of clauses 1-35, wherein each R^{2a} and R^{2b} is independently selected from H, and C₁₋₄ alkyl optionally substituted with one or more independently selected -OH, or C₁₋₄ alkoxy.
37. A compound or a pharmaceutically acceptable salt thereof according to any one of clauses 1-35, wherein R^{2a} is H and R^{2b} is C₁₋₄ alkyl.
38. A compound or a pharmaceutically acceptable salt thereof according to any one of clauses 1-35, wherein R^{2a} is H and R^{2b} is -CH₃.
39. A compound or a pharmaceutically acceptable salt thereof according to any one of clauses 1-35, wherein R^{2a} and R^{2b} are H.
40. A compound or a pharmaceutically acceptable salt thereof according to clause 1 or 2, wherein the compound is according to Formula IV: 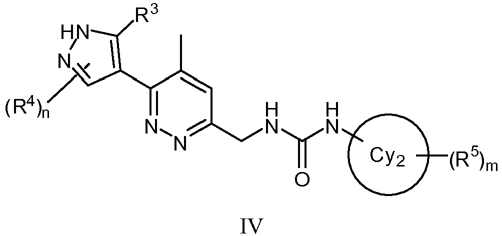
41. A compound or a pharmaceutically acceptable salt thereof according to any one of clauses 1-40, wherein R³ is C₁₋₄ alkyl.
42. A compound or a pharmaceutically acceptable salt thereof according to any one of clauses 1-40, wherein R³ is -CH₃.
43. A compound or a pharmaceutically acceptable salt thereof according to any one of clauses 1-40, wherein R³ is C₁₋₄ alkyl substituted with one or more independently selected halo, -CN, -C₁₋₄ alkoxy, or -NR^{7a}R^{7b}.
44. A compound or a pharmaceutically acceptable salt thereof according to any one of clauses 1-40, wherein R³ is -CH₃, -CH₂CH₃, or -(CH₂)₂CH₃, each of which is substituted with one, two or three independently selected halo, -CN, -C₁₋₄ alkoxy, or -NR^{7a}R^{7b}.
45. A compound or a pharmaceutically acceptable salt thereof according to clause 44, wherein each each R^{7a} and R^{7b} is independently selected from H,-CH₃, and -CH₂CH₃.
46. A compound or a pharmaceutically acceptable salt thereof according to any one of clauses 1-40, wherein R³ is -CF₃.
47. A compound or a pharmaceutically acceptable salt thereof according to any one of clauses 1-40, wherein R³ is C₁₋₄ alkoxy substituted with one or more halo.
48. A compound or a pharmaceutically acceptable salt thereof according to any one of clauses 1-40, wherein R³ is -OCH₃, -OCH₂CH₃, or -OCH₂CH₂CH₃, each of is substituted with one or more halo.
49. A compound or a pharmaceutically acceptable salt thereof according to any one of clauses 1-40, wherein R³ is -OCF₃, -OCH₂CHF₂ or -OCH₂CF₃.
50. A compound or a pharmaceutically acceptable salt thereof according to any one of clauses 1-40, wherein R³ is C₃₋₇ monocyclic cycloalkyl.
51. A compound or a pharmaceutically acceptable salt thereof according to any one of clauses 1-40, wherein R³ is cyclopropyl.
52. A compound or a pharmaceutically acceptable salt thereof according to any one of clauses 1-40, wherein R³ is 4-7-membered monocyclic heterocycloalkyl comprising one, two or three heteroatoms independently selected from N, O, and S.
53. A compound or a pharmaceutically acceptable salt thereof according to any one of clauses 1-40, wherein R³ is oxetanyl, tetrahydrofuranyl, tetrahydropyranyl, pyrolidinyl, piperidinyl, piperazinyl, morpholinyl, or thiomorpholinyl.
54. A compound or a pharmaceutically acceptable salt thereof according to any one of clauses 1-40, wherein R³ is -NR^{8a}R^{8b} wherein each R^{8a} and R^{8b} is independently selected from H, and C₁₋₄ alkyl.
55. A compound or a pharmaceutically acceptable salt thereof according to any one of clauses 1-40, wherein R³ is -NR^{8a}R^{8b} wherein each R^{8a} and R^{8b} is independently selected from H,-CH₃, and -CH₂CH₃.
56. A compound or a pharmaceutically acceptable salt thereof according to any one of clauses 1-40, wherein R³ is -CONR^{8c}R^{8d} wherein each R^{8c} and R^{8d} is independently selected from H, and C₁₋₄ alkyl.
57. A compound or a pharmaceutically acceptable salt thereof according to any one of clauses 1-40, wherein R³ is -CONR^{8c}R^{8d} wherein each R^{8c} and R^{8d} is independently selected from H,-CH₃, and -CH₂CH₃.
58. A compound or a pharmaceutically acceptable salt thereof according to any one of clauses 1-57, wherein the subscript n is 0.
59. A compound or a pharmaceutically acceptable salt thereof according to any one of clauses 1-57, wherein the subscript n is 1.
60. A compound or a pharmaceutically acceptable salt thereof according to clause 1 or 2, wherein the compound or pharmaceutically acceptable salt is according to any one of Formula Va-Vb: 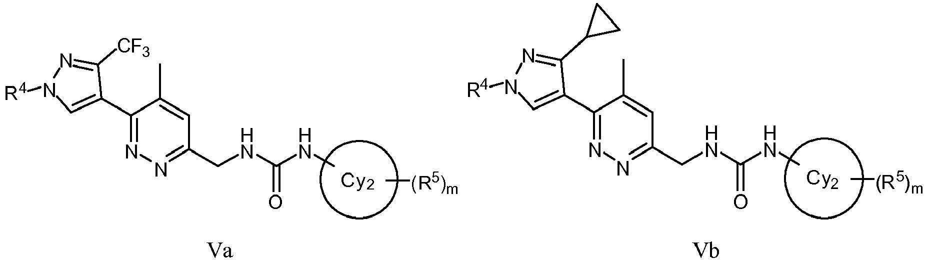
61. A compound or a pharmaceutically acceptable salt thereof according to any one of clauses 1-60, wherein R⁴ is C₁₋₄ alkyl.
62. A compound or a pharmaceutically acceptable salt thereof according to any one of clauses 1-60, wherein R⁴ is -CH₃.
63. A compound or a pharmaceutically acceptable salt thereof according to any one of clauses 1-60, wherein R⁴ is C₁₋₄ alkyl substituted with one or more independently selected R¹² groups.
64. A compound or a pharmaceutically acceptable salt thereof according to any one of clauses 1-60, wherein R⁴ is -CH₃, -CH₂CH₃, -(CH₂)₂CH₃, or -CH(CH₃)₂ each of which is substituted with one or more independently selected R¹² groups.
65. A compound or a pharmaceutically acceptable salt thereof according to any one of clauses 1-60, wherein R⁴ is C₁₋₄ alkyl substituted with one R¹² group.
66. A compound or a pharmaceutically acceptable salt thereof according to any one of clauses 1-60, wherein R⁴ is -CH₃, -CH₂CH₃, -(CH₂)₂CH₃, or -CH(CH₃)₂ each of which is substituted with one R¹² group.
67. A compound or a pharmaceutically acceptable salt thereof according to any one of clauses 1-60, wherein R⁴ is -CH₂-R¹².
68. A compound or a pharmaceutically acceptable salt thereof according to any one of clauses 63-67, wherein R¹² is OH, F, or Cl.
69. A compound or a pharmaceutically acceptable salt thereof according to any one of clauses 63-67, wherein R¹² is C₁₋₄ alkoxy.
70. A compound or a pharmaceutically acceptable salt thereof according to any one of clauses 63-67, wherein R¹² is -OCH₃, or -OCH₂CH₃.
71. A compound or a pharmaceutically acceptable salt thereof according to any one of clauses 63-67, wherein R¹² is -SO₂-C₁₋₄ alkyl.
72. A compound or a pharmaceutically acceptable salt thereof according to any one of clauses 63-67, wherein R¹² is -SO₂-CH₃.
73. A compound or a pharmaceutically acceptable salt thereof according to any one of clauses 63-67, wherein R¹² is C₃₋₇ monocyclic cycloalkyl.
74. A compound or a pharmaceutically acceptable salt thereof according to any one of clauses 63-67, wherein R¹² is cyclopropyl, cyclobutyl, or cyclopentyl.
75. A compound or a pharmaceutically acceptable salt thereof according to any one of clauses 63-67, wherein R¹² is C₃₋₇ monocyclic cycloalkyl substituted with one or more independently selected -OH, halo, -CN, C₁₋₄ alkyl, C₁₋₄ alkoxy or =O.
76. A compound or a pharmaceutically acceptable salt thereof according to any one of clauses 63-67, wherein R¹² is cyclopropyl, cyclobutyl, or cyclopentyl, each of which is substituted with one or more independently selected -OH, halo, -CN, C₁₋₄ alkyl, C₁₋₄ alkoxy or =O.
77. A compound or a pharmaceutically acceptable salt thereof according to any one of clauses 63-67, wherein R¹² is cyclopropyl, cyclobutyl, or cyclopentyl, each of which is substituted with one or more independently selected -OH, F, -CN, -CH₃, -OCH₃ or =O.
78. A compound or a pharmaceutically acceptable salt thereof according to any one of clauses 63-67, wherein R¹² is 4-7 membered monocyclic heterocycloalkyl, comprising one, two, or three heteroatoms independently selected from N, O, and S.
79. A compound or a pharmaceutically acceptable salt thereof according to any one of clauses 63-67, wherein R¹² is azetidinyl, pyrrolidinyl, or tetrahydrofuranyl.
80. A compound or a pharmaceutically acceptable salt thereof according to any one of clauses 63-67, wherein R¹² is 4-7 membered monocyclic heterocycloalkyl, comprising one, two, or three heteroatoms independently selected from N, O, and S, substituted with one or more independently selected -OH, halo, -CN, C₁₋₄ alkyl, C₁₋₄ alkoxy or =O.
81. A compound or a pharmaceutically acceptable salt thereof according to any one of clauses 63-67, wherein R¹² is azetidinyl, pyrrolidinyl, or tetrahydrofuranyl, each of which is substituted with one or more independently selected -OH, halo, -CN, C₁₋₄ alkyl, C₁₋₄ alkoxy or =O.
82. A compound or a pharmaceutically acceptable salt thereof according to any one of clauses 63-67, wherein R¹² is azetidinyl, pyrrolidinyl, or tetrahydrofuranyl, each of which is substituted with one or more independently selected -OH, F, -CN, -CH₃, -OCH₃ or =O.
83. A compound or a pharmaceutically acceptable salt thereof according to any one of clauses 63-67, wherein R¹² is -NR^{9a}R^{9b}, wherein each R^{9a} and R^{9b} is independently selected from H and C₁₋₄ alkyl.
84. A compound or a pharmaceutically acceptable salt thereof according to any one of clauses 63-67, wherein R¹² is -NMe₂.
85. A compound or a pharmaceutically acceptable salt thereof according to any one of clauses 63-67, wherein R¹² is -CN.
86. A compound or a pharmaceutically acceptable salt thereof according to any one of clauses 1-60, wherein R⁴ is C₃₋₇ monocyclic cycloalkyl.
87. A compound or a pharmaceutically acceptable salt thereof according to any one of clauses 1-60, wherein R⁴ is cyclopropyl, cyclobutyl, cyclopentyl, or cyclohexyl.
88. A compound or a pharmaceutically acceptable salt thereof according to any one of clauses 1-60, wherein R⁴ is cyclopropyl.
89. A compound or a pharmaceutically acceptable salt thereof according to any one of clauses 1-60, wherein R⁴ is 4-7 membered monocyclic heterocycloalkyl comprising one, two, or three heteroatoms independently selected from N, O, or S.
90. A compound or a pharmaceutically acceptable salt thereof according to any one of clauses 1-60, wherein R⁴ is azetidinyl, pyrrolidinyl, piperazinyl, piperidinyl, morpholinyl, tetrahydrofuranyl, or tetrahydropyranyl.
91. A compound or a pharmaceutically acceptable salt thereof according to clause 1 or 2, wherein the compound or pharmaceutically acceptable salt is according to any one of Formula VIa, or VIb: 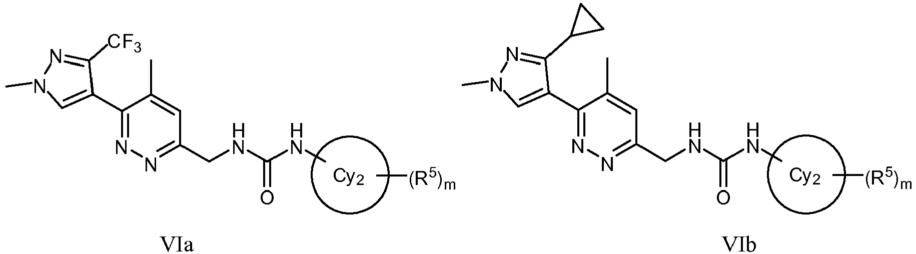
92. A compound or a pharmaceutically acceptable salt thereof according to any one of clauses 1-91, wherein Cy₂ is phenyl.
93. A compound or a pharmaceutically acceptable salt thereof according to any one of clauses 1-91, wherein Cy₂ is 5-6 membered heteroaryl comprising one or two heteroatoms independently selected from N, O, and S.
94. A compound or a pharmaceutically acceptable salt thereof according to any one of clauses 1-91, wherein Cy₂ is pyridinyl.
95. A compound or a pharmaceutically acceptable salt thereof according to clause 1 or 2, wherein the compound or pharmaceutically acceptable salt is according to any one of Formula VIIa, VIIb, VIIc or VIId: 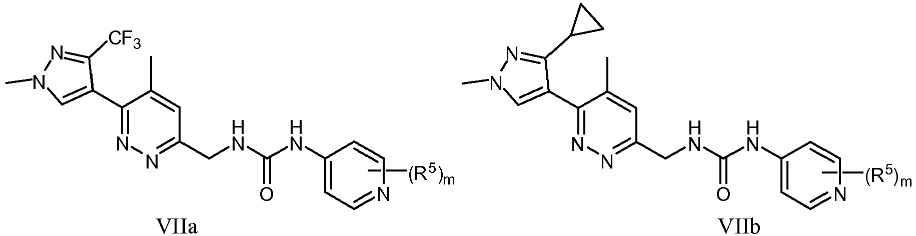 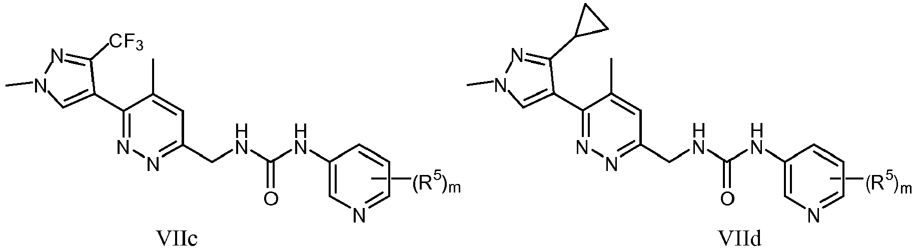
96. A compound or a pharmaceutically acceptable salt thereof according to any one of clauses 1-95, wherein the subscript m is 1, 2, 3, or 4.
97. A compound or a pharmaceutically acceptable salt thereof according to any one of clauses 1-95, wherein the subscript m is 0.
98. A compound or a pharmaceutically acceptable salt thereof according to any one of clauses 1-96, wherein R⁵ is selected from halo, CN, selected from OH.
99. A compound or a pharmaceutically acceptable salt thereof according to any one of clauses 1-96, wherein R⁵ is selected from F, Cl or CN.
100.A compound or a pharmaceutically acceptable salt thereof according to any one of clauses 1-96, wherein R⁵ is C₁₋₄ alkyl.
101. A compound or a pharmaceutically acceptable salt thereof according to any one of clauses 1-96, wherein R⁵ is -CH₃, or -CH₂CH₃.
102.A compound or a pharmaceutically acceptable salt thereof according to any one of clauses 1-96, wherein R⁵ is C₁₋₄ alkyl substituted with one or more independently selected R¹³.
103.A compound or a pharmaceutically acceptable salt thereof according to any one of clauses 1-96, wherein R⁵ is -CH₃, or -CH₂CH₃, each of which is substituted with one or more independently selected R¹³.
104.A compound or a pharmaceutically acceptable salt thereof according to any one of clauses 1-96, wherein R⁵ is C₁₋₄ alkoxy.
105.A compound or a pharmaceutically acceptable salt thereof according to any one of clauses 1-96, wherein R⁵ is -OCH₃, or -OCH₂CH₃.
106.A compound or a pharmaceutically acceptable salt thereof according to any one of clauses 1-96, wherein R⁵ is C₁₋₄ alkoxy substituted with one or more independently selected R¹³.
107.A compound or a pharmaceutically acceptable salt thereof according to any one of clauses 1-96, wherein R⁵ is -OCH₃, or -OCH₂CH₃s, each of which is substituted with one or more independently selected R¹³.
108.A compound or a pharmaceutically acceptable salt thereof according to any one of clauses 102, 103, 106 or 107, wherein R¹³ is F, -CN, -OH, -OCH₃, -OCF₃, -OCH₂CH₃, -OCH₂CF₃, -OCH₂CH₂OH, or -OCH₂CH₂OCH₃.
109.A compound or a pharmaceutically acceptable salt thereof according to any one of clauses 102, 103, 106 or 107, wherein R¹³ is C₃₋₇ monocyclic cycloalkyl substituted with one or more independently selected F, -CH₃, -CH₂CH₃. -CF₃, and -CH₂CF₃.
110.A compound or a pharmaceutically acceptable salt thereof according to any one of clauses 102, 103, 106 or 107, wherein R¹³ is cyclopropyl, cyclobutyl, cyclopentyl, or cyclohexyl, each of which is substituted with one or more independently selected F, -CH₃, -CH₂CH₃. -CF₃, and -CH₂CF₃.
111.A compound or a pharmaceutically acceptable salt thereof according to any one of clauses 1-96, wherein R⁵ is -CF₃.
112.A compound or a pharmaceutically acceptable salt thereof according to any one of clauses 1-96, wherein R⁵ is -OCF₃, -OCH₂CH₂F, -OCH₂CH₂OCH₃, -OCH₂CH₂OH, or -OCH₂CHF₂.
113.A compound or a pharmaceutically acceptable salt thereof according to any one of clauses 1-96, wherein R⁵ is C₃₋₇ monocyclic cycloalkyl.
114.A compound or a pharmaceutically acceptable salt thereof according to any one of clauses 1-96, wherein R⁵ is cyclopropyl, or cyclobutyl.
115.A compound or a pharmaceutically acceptable salt thereof according to any one of clauses 1-96, wherein R⁵ is C₃₋₇ monocyclic cycloalkyl substituted with one or more independently selected R¹⁴ group.
116.A compound or a pharmaceutically acceptable salt thereof according to any one of clauses 1-96, wherein R⁵ is cyclopropyl, cyclobutyl, cyclopentyl, or cyclohexyl, each of which is substituted with one or more independently selected R¹⁴ group.
117.A compound or a pharmaceutically acceptable salt thereof according to any one of clauses 1-96, wherein R⁵ is 4-11 membered monocyclic, or fused or spiro bicyclic heterocycloalkyl comprising one, two, or three heteroatoms independently selected from N, O, and S.
118.A compound or a pharmaceutically acceptable salt thereof according to any one of clauses 1-96, wherein R⁵ is azetidinyl, oxetanyl, tetrahydrofuranyl, pyrolidinyl, tetrahydropyranyl, piperidininyl, piperazinyl, morpholinyl, thiomorpholinyl, dioxanyl.
119.A compound or a pharmaceutically acceptable salt thereof according to any one of clauses 1-96, wherein R⁵ is 4-11 membered monocyclic, or fused or spiro bicyclic heterocycloalkyl comprising one, two, or three heteroatoms independently selected from N, O, and S, substituted with one or more independently selected R¹⁴ group.
120.A compound or a pharmaceutically acceptable salt thereof according to any one of clauses 1-96, wherein R⁵ is azetidinyl, oxetanyl, tetrahydrofuranyl, pyrolidinyl, tetrahydropyranyl, piperidininyl, piperazinyl, morpholinyl, thiomorpholinyl, dioxanyl, each of which is substituted with one or more independently selected R¹⁴ group.
121.A compound or a pharmaceutically acceptable salt thereof according to any one of clauses 1-96, wherein R⁵ is -O-C₃₋₇ monocyclic cycloalkyl.
122.A compound or a pharmaceutically acceptable salt thereof according to any one of clauses 1-96, wherein R⁵ is -O-cyclopropyl, -O-cyclobutyl, -O-cyclopentyl, or -O-cyclohexyl.
123.A compound or a pharmaceutically acceptable salt thereof according to any one of clauses 1-96, wherein R⁵ is -O-C₃₋₇ monocyclic cycloalkyl substituted with one or more independently selected R¹⁴ group.
124.A compound or a pharmaceutically acceptable salt thereof according to any one of clauses 1-96, wherein R⁵ is -O-cyclopropyl, -O-cyclobutyl, -O-cyclopentyl, or -O-cyclohexyl, each of which is substituted with one or more independently selected R¹⁴ group.
125.A compound or a pharmaceutically acceptable salt thereof according to any one of clauses 1-96, wherein R⁵ is -O-heterocycloalkyl, wherein said heterocycloalkyl is a 4-7 membered monocyclic heterocycloalkyl comprising one, two, or three heteroatoms independently selected from O, and S.
126.A compound or a pharmaceutically acceptable salt thereof according to any one of clauses 1-96, wherein R⁵ is -O-oxetanyl, -O-tetrahydrofuranyl, -O-tetrahydropyranyl, or -O-dioxanyl.
127.A compound or a pharmaceutically acceptable salt thereof according to any one of clauses 1-96, wherein R⁵ is -O-heterocycloalkyl, wherein said heterocycloalkyl is a 4-7 membered monocyclic heterocycloalkyl comprising one, two, or three heteroatoms independently selected from O, and S, substituted with one or more independently selected R¹⁴ group.
128.A compound or a pharmaceutically acceptable salt thereof according to any one of clauses 1-96, wherein R⁵ is -O-oxetanyl, -O-tetrahydrofuranyl, -O-tetrahydropyranyl, or -O-dioxanyl, each of which is substituted with one or more independently selected R¹⁴ group.
129.A compound or a pharmaceutically acceptable salt thereof according to any one of clauses 115, 116, 119, 120, 123, 124, 127, or 128, wherein R¹⁴ is F, Cl, -OH, -CN, -CH₃, -CH₂CH₃, -CF₃, -CH₂CHF₂, -OCH₃, -OCH₂CH₃, -OCF₃, -OCH₂CHF₂.
130.A compound or a pharmaceutically acceptable salt thereof according to any one of clauses 1-96, wherein R⁵ is -SO₂-CH₃.
131.A compound or a pharmaceutically acceptable salt thereof according to any one of clauses 1-96, wherein R⁵ is -SO₂-NR^{15a}R^{15b}, wherein each R^{15a} and R^{15b} is independently selected from H and -CH₃.
132.A compound or a pharmaceutically acceptable salt thereof according to any one of clauses 1-96, wherein R⁵ is -C(=O)NR^{15c}R^{15d}, wherein each R^{15c} and R^{15d} is independently selected from H and -CH₃.
133.A compound or a pharmaceutically acceptable salt thereof according to any one of clauses 1-96, wherein R⁵ is -NR^{17a}R^{17b}, wherein each R^{17a} and R^{17b} is independently selected from H, -CH₃, -CH₂CH₃, -CH₂CF₃, -CH₂CHF₂, -CH₂CH₂OH and -CH₂CH₂OCH₃.

### PHARMACEUTICAL COMPOSITIONS

When employed as a pharmaceutical, a compound of the invention is typically administered in the form of a pharmaceutical composition. Such compositions can be prepared in a manner well known in the pharmaceutical art and comprise at least one active compound of the invention according to Formula Ia or Ib. Generally, a compound of the invention is administered in a pharmaceutically effective amount. The amount of compound of the invention actually administered will typically be determined by a physician, in the light of the relevant circumstances, including the condition to be treated, the chosen route of administration, the actual compound of the invention administered, the age, weight, and response of the individual patient, the severity of the patient's symptoms, and the like.

The pharmaceutical compositions of this invention can be administered by a variety of routes including oral, rectal, transdermal, subcutaneous, intra-articular, intravenous, intramuscular, and intranasal. Depending on the intended route of delivery, a compound of the invention is preferably formulated as either injectable or oral compositions or as salves, as lotions or as patches all for transdermal administration.

The compositions for oral administration can take the form of bulk liquid solutions or suspensions, or bulk powders. More commonly, however, the compositions are presented in unit dosage forms to facilitate accurate dosing. The term 'unit dosage forms' refers to physically discrete units suitable as unitary dosages for human subjects and other mammals, each unit containing a predetermined quantity of active material calculated to produce the desired therapeutic effect, in association with a suitable pharmaceutical excipient, vehicle or carrier. Typical unit dosage forms include prefilled, premeasured ampules or syringes of the liquid compositions or pills, tablets, capsules or the like in the case of solid compositions. In such compositions, the compound of the invention according to Formula I is usually a minor component (from about 0.1 to about 50% by weight or preferably from about 1 to about 40% by weight) with the remainder being various vehicles or carriers and processing aids helpful for forming the desired dosing form.

Liquid forms suitable for oral administration may include a suitable aqueous or non-aqueous vehicle with buffers, suspending and dispensing agents, colorants, flavors and the like. Solid forms may include, for example, any of the following ingredients, or compound of the inventions of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint or orange flavoring.

Injectable compositions are typically based upon injectable sterile saline or phosphate-buffered saline or other injectable carriers known in the art. As before, the active compound of the invention according to Formula I in such compositions is typically a minor component, often being from about 0.05 to 10% by weight with the remainder being the injectable carrier and the like.

Transdermal compositions are typically formulated as a topical ointment or cream containing the active ingredient(s), generally in an amount ranging from about 0.01 to about 20% by weight, preferably from about 0.1 to about 20% by weight, preferably from about 0.1 to about 10% by weight, and more preferably from about 0.5 to about 15% by weight. When formulated as an ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or the formulation. All such known transdermal formulations and ingredients are included within the scope of this invention.

A compound of the invention can also be administered by a transdermal device. Accordingly, transdermal administration can be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania, which is incorporated herein by reference.

A compound of the invention can also be administered in sustained release forms or from sustained release drug delivery systems. A description of representative sustained release materials can be found in Remington's Pharmaceutical Sciences.

The following formulation examples illustrate representative pharmaceutical compositions that may be prepared in accordance with this invention. The present invention, however, is not limited to the following pharmaceutical compositions.

### Formulation 1 - Tablets

A compound of the invention according to Formula I may be admixed as a dry powder with a dry gelatin binder in an approximate 1:2 weight ratio. A minor amount of magnesium stearate may be added as a lubricant. The mixture may be formed into 240-270 mg tablets (80-90 mg of active compound of the invention according to Formula I per tablet) in a tablet press.

### Formulation 2 - Capsules

A compound of the invention according to Formula I may be admixed as a dry powder with a starch diluent in an approximate 1:1 weight ratio. The mixture may be filled into 250 mg capsules (125 mg of active compound of the invention according to Formula I per capsule).

### Formulation 3 - Liquid

A compound of the invention according to Formula I (125 mg), may be admixed with sucrose (1.75 g) and xanthan gum (4 mg) and the resultant mixture may be blended, passed through a No. 10 mesh U.S. sieve, and then mixed with a previously made solution of microcrystalline cellulose and sodium carboxymethyl cellulose (11:89, 50 mg) in water. Sodium benzoate (10 mg), flavor, and color may be diluted with water and added with stirring. Sufficient water may then be added with stirring. Further sufficient water may be then added to produce a total volume of 5 mL.

### Formulation 4 - Tablets

A compound of the invention according to Formula I may be admixed as a dry powder with a dry gelatin binder in an approximate 1:2 weight ratio. A minor amount of magnesium stearate may be added as a lubricant. The mixture may be formed into 450-900 mg tablets (150-300 mg of active compound of the invention according to Formula I) in a tablet press.

### Formulation 5 - Injection

A compound of the invention according to Formula I may be dissolved or suspended in a buffered sterile saline injectable aqueous medium to a concentration of approximately 5 mg/mL.

### Formulation 6 - Topical

Stearyl alcohol (250 g) and a white petrolatum (250 g) may be melted at about 75°C and then a mixture of A compound of the invention according to Formula I (50 g) methylparaben (0.25 g), propylparaben (0.15 g), sodium lauryl sulfate (10 g), and propylene glycol (120 g) dissolved in water (about 370 g) may be added and the resulting mixture may be stirred until it congeals.

### METHODS OF TREATMENT

In one embodiment, the present invention provides compounds of the invention, or pharmaceutical compositions comprising a compound of the invention, for use in medicine. In a particular embodiment, the present invention provides compounds of the invention or pharmaceutical compositions comprising a compound of the invention, for use in the prophylaxis and/or treatment of fibrotic diseases, inflammatory diseases, autoimmune diseases, metabolic diseases, cardiovascular diseases, and/or proliferative diseases.

In another embodiment, the present invention provides compounds of the invention, or pharmaceutical compositions comprising a compound of the invention for use in the manufacture of a medicament for use in the prophylaxis and/or treatment of fibrotic diseases, inflammatory diseases, autoimmune diseases, metabolic diseases, cardiovascular diseases, and/or proliferative diseases.

In additional method of treatment aspects, this invention provides methods of prophylaxis and/or treatment of a mammal afflicted with fibrotic diseases, inflammatory diseases, autoimmune diseases, metabolic diseases, cardiovascular diseases, and/or proliferative diseases, which methods comprise the administration of an effective amount of a compound of the invention or one or more of the pharmaceutical compositions herein described for the treatment or prophylaxis of said condition.

In one embodiment, the present invention provides pharmaceutical compositions comprising a compound of the invention, and another therapeutic agent. In a particular embodiment, the other therapeutic agent is a fibrotic diseases, inflammatory diseases, autoimmune diseases, metabolic diseases, cardiovascular diseases, and/or proliferative diseases treatment agent.

In one embodiment, the present invention provides compounds of the invention or pharmaceutical compositions comprising a compound of the invention, for use in the prophylaxis and/or treatment of fibrotic diseases. In a more particular embodiment, the fibrotic disease is selected from idiopathic pulmonary fibrosis, Dupuytren disease, nonalcoholic steatohepatitis, portal hypertension, systemic sclerosis, renal fibrosis, and cutaneous fibrosis. In a most particular embodiment, the fibrotic disease is idiopathic pulmonary fibrosis.

In another embodiment, the present invention provides compounds of the invention, or pharmaceutical compositions comprising a compound of the invention for use in the manufacture of a medicament for use in the prophylaxis and/or treatment of fibrotic diseases. In a more particular embodiment, the fibrotic disease is selected from idiopathic pulmonary fibrosis, Dupuytren disease, nonalcoholic steatohepatitis, portal hypertension, systemic sclerosis, renal fibrosis, and cutaneous fibrosis. In a most particular embodiment, the fibrotic disease is idiopathic pulmonary fibrosis.

In additional method of treatment aspects, this invention provides methods of prophylaxis and/or treatment of a mammal afflicted with fibrotic diseases, which methods comprise the administration of an effective amount of a compound of the invention or one or more of the pharmaceutical compositions herein described for the treatment or prophylaxis of said condition. In a more particular embodiment, the fibrotic disease is selected from idiopathic pulmonary fibrosis, Dupuytren disease, nonalcoholic steatohepatitis, portal hypertension, systemic sclerosis, renal fibrosis, and cutaneous fibrosis. In a most particular embodiment, the fibrotic disease is idiopathic pulmonary fibrosis.

In one embodiment, the present invention provides pharmaceutical compositions comprising a compound of the invention, and another therapeutic agent. In a particular embodiment, the other therapeutic agent is a fibrotic diseases treatment agent. In a more particular embodiment, the fibrotic disease is selected from idiopathic pulmonary fibrosis, Dupuytren disease, nonalcoholic steatohepatitis, portal hypertension, systemic sclerosis, renal fibrosis, and cutaneous fibrosis. In a most particular embodiment, the fibrotic disease is idiopathic pulmonary fibrosis.

In a particular embodiment, the present invention provides compounds of the invention or pharmaceutical compositions comprising a compound of the invention, for use in the prophylaxis and/or treatment of inflammatory diseases. In a particular embodiment, the inflammatory disease is selected from rheumatoid arthritis, osteoarthritis, allergic airway disease, chronic obstructive pulmonary disease and inflammatory bowel diseases.

In another embodiment, the present invention provides compounds of the invention, or pharmaceutical compositions comprising a compound of the invention for use in the manufacture of a medicament for use in the prophylaxis and/or treatment of inflammatory diseases. In a particular embodiment, the inflammatory disease is selected from rheumatoid arthritis, osteoarthritis, allergic airway disease, chronic obstructive pulmonary disease and inflammatory bowel diseases.

In additional method of treatment aspects, this invention provides methods of prophylaxis and/or treatment of a mammal afflicted with inflammatory diseases, which methods comprise the administration of an effective amount of a compound of the invention or one or more of the pharmaceutical compositions herein described for the treatment or prophylaxis of said condition. In a particular embodiment, the inflammatory disease is selected from rheumatoid arthritis, osteoarthritis, allergic airway disease, chronic obstructive pulmonary disease and inflammatory bowel diseases.

In one embodiment, the present invention provides pharmaceutical compositions comprising a compound of the invention, and another therapeutic agent. In a particular embodiment, the other therapeutic agent is an agent for the prophylaxis and/or treatment of inflammatory diseases. In a particular embodiment, the inflammatory disease is selected from rheumatoid arthritis, osteoarthritis, allergic airway disease, chronic obstructive pulmonary disease and inflammatory bowel diseases.

In a particular embodiment, the present invention provides compounds of the invention or pharmaceutical compositions comprising a compound of the invention, for use in the prophylaxis and/or treatment of autoimmune diseases. In a particular embodiment, the autoimmune disease is selected from chronic obstructive pulmonary disease, asthma, systemic lupus erythematosus, type I diabetes mellitus and inflammatory bowel disease.

In another embodiment, the present invention provides compounds of the invention, or pharmaceutical compositions comprising a compound of the invention for use in the manufacture of a medicament for use in the prophylaxis and/or treatment of autoimmune diseases. In a particular embodiment, the autoimmune disease is selected from chronic obstructive pulmonary disease, asthma, systemic lupus erythematosus, type I diabetes mellitus and inflammatory bowel disease.

In additional method of treatment aspects, this invention provides methods of prophylaxis and/or treatment of a mammal afflicted with autoimmune diseases, which methods comprise the administration of an effective amount of a compound of the invention or one or more of the pharmaceutical compositions herein described for the treatment or prophylaxis of said condition. In a particular embodiment, the autoimmune disease is selected from chronic obstructive pulmonary disease, asthma, systemic lupus erythematosus, type I diabetes mellitus and inflammatory bowel disease.

In one embodiment, the present invention provides pharmaceutical compositions comprising a compound of the invention, and another therapeutic agent. In a particular embodiment, the other therapeutic agent is an autoimmune diseases treatment agent. In a particular embodiment, the autoimmune disease is selected from chronic obstructive pulmonary disease, asthma, systemic lupus erythematosus, type I diabetes mellitus and inflammatory bowel disease.

In a particular embodiment, the present invention provides compounds of the invention or pharmaceutical compositions comprising a compound of the invention, for use in the prophylaxis and/or treatment of metabolic diseases. In a particular embodiment, the metabolic disease is selected from cystic fibrosis, phenylketonuria (PKU), diabetes, hyperlipidemia, gout, and rickets. In a more particular embodiment, the cardiovascular disease is diabetes or hyperlidemia.

In another embodiment, the present invention provides compounds of the invention, or pharmaceutical compositions comprising a compound of the invention for use in the manufacture of a medicament for use in the prophylaxis and/or treatment of metabolic diseases. In a particular embodiment, the metabolic disease is selected from cystic fibrosis, phenylketonuria (PKU), diabetes, hyperlipidemia, gout, and rickets. In a more particular embodiment, the cardiovascular disease is diabetes or hyperlidemia.

In additional method of treatment aspects, this invention provides methods of prophylaxis and/or treatment of a mammal afflicted with metabolic diseases, which methods comprise the administration of an effective amount of a compound of the invention or one or more of the pharmaceutical compositions herein described for the treatment or prophylaxis of said condition. In a particular embodiment, the metabolic disease is selected from cystic fibrosis, phenylketonuria (PKU), diabetes, hyperlipidemia, gout, and rickets. In a more particular embodiment, the cardiovascular disease is diabetes or hyperlidemia.

In one embodiment, the present invention provides pharmaceutical compositions comprising a compound of the invention, and another therapeutic agent. In a particular embodiment, the other therapeutic agent is a metabolic diseases treatment agent. In a particular embodiment, the metabolic disease is selected from cystic fibrosis, phenylketonuria (PKU), diabetes, hyperlipidemia, gout, and rickets. In a more particular embodiment, the cardiovascular disease is diabetes or hyperlidemia.

In a particular embodiment, the present invention provides compounds of the invention or pharmaceutical compositions comprising a compound of the invention, for use in the prophylaxis and/or treatment of cardiovascular diseases. In a particular embodiment, the cardiovascular disease is selected from stroke, vasculitis, angina, atherosclerosis, and peripheral obstructive arteriopathy. In a more particular embodiment, the cardiovascular disease is stroke, or vasculitis. In another more particular embodiment, the cardiovascular disease is atherosclerosis or reperfusion injury following ischemia.

In another embodiment, the present invention provides compounds of the invention, or pharmaceutical compositions comprising a compound of the invention for use in the manufacture of a medicament for use in the prophylaxis and/or treatment of cardiovascular diseases. In a particular embodiment, the cardiovascular disease is selected from stroke, vasculitis, angina, atherosclerosis, and peripheral obstructive arteriopathy. In a more particular embodiment, the cardiovascular disease is stroke, or vasculitis. In another more particular embodiment, the cardiovascular disease is atherosclerosis or reperfusion injury following ischemia.

In additional method of treatment aspects, this invention provides methods of prophylaxis and/or treatment of a mammal afflicted with cardiovascular diseases, which methods comprise the administration of an effective amount of a compound of the invention or one or more of the pharmaceutical compositions herein described for the treatment or prophylaxis of said condition. In a particular embodiment, the cardiovascular disease is selected from stroke, vasculitis, angina, atherosclerosis, and peripheral obstructive arteriopathy. In a more particular embodiment, the cardiovascular disease is stroke, or vasculitis. In another more particular embodiment, the cardiovascular disease is atherosclerosis or reperfusion injury following ischemia.

In one embodiment, the present invention provides pharmaceutical compositions comprising a compound of the invention, and another therapeutic agent. In a particular embodiment, the other therapeutic agent is a cardiovascular diseases treatment agent. In a particular embodiment, the cardiovascular disease is selected from stroke, vasculitis, angina, atherosclerosis, and peripheral obstructive arteriopathy. In a more particular embodiment, the cardiovascular disease is stroke, or vasculitis. In another more particular embodiment, the cardiovascular disease is atherosclerosis or reperfusion injury following ischemia.

In a particular embodiment, the present invention provides compounds of the invention or pharmaceutical compositions comprising a compound of the invention, for use in the prophylaxis and/or treatment of proliferative diseases. In a particular embodiment, the proliferative disease is selected from Wilm's tumor, glioblastoma, lung cancer, breast cancer, ovarian cancer, and melanoma.

In another embodiment, the present invention provides compounds of the invention, or pharmaceutical compositions comprising a compound of the invention for use in the manufacture of a medicament for use in the prophylaxis and/or treatment of proliferative diseases. In a particular embodiment, the proliferative disease is selected from Wilm's tumor, glioblastoma, lung cancer, breast cancer, ovarian cancer, and melanoma.

In additional method of treatment aspects, this invention provides methods of prophylaxis and/or treatment of a mammal afflicted with proliferative diseases, which methods comprise the administration of an effective amount of a compound of the invention or one or more of the pharmaceutical compositions herein described for the treatment or prophylaxis of said condition. In a particular embodiment, the proliferative disease is selected from Wilm's tumor, glioblastoma, lung cancer, breast cancer, ovarian cancer, and melanoma.

In one embodiment, the present invention provides pharmaceutical compositions comprising a compound of the invention, and another therapeutic agent. In a particular embodiment, the other therapeutic agent is a proliferative diseases treatment agent. In a particular embodiment, the proliferative disease is selected from Wilm's tumor, glioblastoma, lung cancer, breast cancer, ovarian cancer, and melanoma.

Injection dose levels range from about 0.1 mg/kg/h to at least 10 mg/kg/h, all for from about 1 to about 120 h and especially 24 to 96 h. A preloading bolus of from about 0.1 mg/kg to about 10 mg/kg or more may also be administered to achieve adequate steady state levels. The maximum total dose is not expected to exceed about 1 g/day for a 40 to 80 kg human patient.

For the prophylaxis and/or treatment of long-term conditions, such as degenerative conditions, the regimen for treatment usually stretches over many months or years so oral dosing is preferred for patient convenience and tolerance. With oral dosing, one to four (1-4) regular doses daily, especially one to three (1-3) regular doses daily, typically one to two (1-2) regular doses daily, and most typically one (1) regular dose daily are representative regimens. Alternatively for long lasting effect drugs, with oral dosing, once every other week, once weekly, and once a day are representative regimens. In particular, dosage regimen can be every 1-14 days, more particularly 1-10 days, even more particularly 1-7 days, and most particularly 1-3 days.

Using these dosing patterns, each dose provides from about 1 to about 1000 mg of a compound of the invention, with particular doses each providing from about 10 to about 500 mg and especially about 30 to about 250 mg.

Transdermal doses are generally selected to provide similar or lower blood levels than are achieved using injection doses.

When used to prevent the onset of a condition, a compound of the invention will be administered to a patient at risk for developing the condition, typically on the advice and under the supervision of a physician, at the dosage levels described above. Patients at risk for developing a particular condition generally include those that have a family history of the condition, or those who have been identified by genetic testing or screening to be particularly susceptible to developing the condition.

A compound of the invention can be administered as the sole active agent or it can be administered in combination with other therapeutic agents, including other compound of the inventions that demonstrate the same or a similar therapeutic activity and that are determined to be safe and efficacious for such combined administration. In a specific embodiment, co-administration of two (or more) agents allows for significantly lower doses of each to be used, thereby reducing the side effects seen.

In one embodiment, a compound of the invention or a pharmaceutical composition comprising a compound of the invention is administered as a medicament. In a specific embodiment, said pharmaceutical composition additionally comprises a further active ingredient.

In one embodiment, a compound of the invention is co-administered with one or more further therapeutic agents for the treatment and/or prophylaxis of a fibrotic disease. In a particular embodiment, a compound of the invention is co-administered with one or two further therapeutic agents for the treatment and/or prophylaxis of a fibrotic disease. In a more particular embodiment, a compound of the invention is co-administered with one further therapeutic agent for the treatment and/or prophylaxis of a fibrotic disease.

In one embodiment, the further therapeutic agent for the treatment and/or prophylaxis of a fibrotic disease include, but are not limited to 5-methyl-1-phenyl-2-(1H)-pyridone (Pirfenidone®); Nintedanib (Ofev® or Vargatef®); STX-100 (ClinicalTrials.gov Identifier NCT01371305), FG-3019 (ClinicalTrials.gov Identifier NCT01890265), Lebrikizumab (CAS n# 953400-68-5); Tralokinumab (CAS n# 1044515-88-9). In another particular embodiment, the further therapeutic agent for the treatment and/or prophylaxis of a fibrotic disease is an autotaxin (or ectonucleotide pyrophosphatase/phosphodiesterase 2 or NPP2 or ENPP2) inhibitor.

In one embodiment, a compound of the invention is co-administered with another therapeutic agent for the treatment and/or prophylaxis of a disease involving inflammation, particular agents include, but are not limited to, immunoregulatory agents e.g. azathioprine, corticosteroids (e.g. prednisolone or dexamethasone), cyclophosphamide, cyclosporin A, tacrolimus, mycophenolate, mofetil, muromonab-CD3 (OKT3, e.g. Orthocolone®), ATG, aspirin, acetaminophen, ibuprofen, naproxen, and piroxicam.

In one embodiment, a compound of the invention is co-administered with another therapeutic agent for the treatment and/or prophylaxis of arthritis (e.g. rheumatoid arthritis), particular agents include but are not limited to analgesics, non-steroidal anti-inflammatory drugs (NSAIDS), steroids, synthetic DMARDS (for example but without limitation methotrexate, leflunomide, sulfasalazine, auranofin, sodium aurothiomalate, penicillamine, chloroquine, hydroxychloroquine, azathioprine, tofacitinib, baricitinib, fostamatinib, and cyclosporin), and biological DMARDS (for example but without limitation infliximab, etanercept, adalimumab, rituximab, and abatacept).

In one embodiment, a compound of the invention is co-administered with another therapeutic agent for the treatment and/or prophylaxis of proliferative diseases, particular agents include but are not limited to: methotrexate, leukovorin, adriamycin, prednisone, bleomycin, cyclophosphamide, 5-fluorouracil, paclitaxel, docetaxel, vincristine, vinblastine, vinorelbine, doxorubicin, tamoxifen, toremifene, megestrol acetate, anastrozole, goserelin, anti-HER2 monoclonal antibody (e.g. HerceptinTM), capecitabine, raloxifene hydrochloride, EGFR inhibitors (e.g. lressa®, Tarceva™, Erbitux™), VEGF inhibitors (e.g. Avastin™), proteasome inhibitors (e.g. Velcade™), Glivec® and hsp90 inhibitors (e.g. 17-AAG). Additionally, the compound of the invention according to Formula I may be administered in combination with other therapies including, but not limited to, radiotherapy or surgery. In a specific embodiment the proliferative disease is selected from cancer, myeloproliferative disease or leukaemia.

In one embodiment, a compound of the invention is co-administered with another therapeutic agent for the treatment and/or prophylaxis of autoimmune diseases, particular agents include but are not limited to: glucocorticoids, cytostatic agents (e.g. purine analogs), alkylating agents, (e.g nitrogen mustards (cyclophosphamide), nitrosoureas, platinum compound of the inventions, and others), antimetabolites (e.g. methotrexate, azathioprine and mercaptopurine), cytotoxic antibiotics (e.g. dactinomycin anthracyclines, mitomycin C, bleomycin, and mithramycin), antibodies (e.g. anti-CD20, anti-CD25 or anti-CD3 (OTK3) monoclonal antibodies, Atgam® and Thymoglobuline®), cyclosporin, tacrolimus, rapamycin (sirolimus), interferons (e.g. IFN-β), TNF binding proteins (e.g. infliximab, etanercept, or adalimumab), mycophenolate, fingolimod and myriocin.

In one embodiment, a compound of the invention is co-administered with another therapeutic agent for the treatment and/or prophylaxis of transplant rejection, particular agents include but are not limited to: calcineurin inhibitors (e.g. cyclosporin or tacrolimus (FK506)), mTOR inhibitors (e.g. sirolimus, everolimus), anti-proliferatives (e.g. azathioprine, mycophenolic acid), corticosteroids (e.g. prednisolone, hydrocortisone), antibodies (e.g. monoclonal anti-IL-2Rα receptor antibodies, basiliximab, daclizumab), polyclonal anti-T-cell antibodies (e.g. anti-thymocyte globulin (ATG), anti-lymphocyte globulin (ALG)).

In one embodiment, a compound of the invention is co-administered with another therapeutic agent for the treatment and/or prophylaxis of asthma and/or rhinitis and/or chronic obstructive pulmonary disease, particular agents include but are not limited to: beta2-adrenoceptor agonists (e.g. salbutamol, levalbuterol, terbutaline and bitolterol), epinephrine (inhaled or tablets), anticholinergics (e.g. ipratropium bromide), glucocorticoids (oral or inhaled). Long-acting β2-agonists (e.g. salmeterol, formoterol, bambuterol, and sustained-release oral albuterol), combinations of inhaled steroids and long-acting bronchodilators (e.g. fluticasone/salmeterol, budesonide/formoterol), leukotriene antagonists and synthesis inhibitors (e.g. montelukast, zafirlukast and zileuton), inhibitors of mediator release (e.g. cromoglycate and ketotifen), biological regulators of IgE response (e.g. omalizumab), antihistamines (e.g. ceterizine, cinnarizine, fexofenadine) and vasoconstrictors (e.g. oxymethazoline, xylomethazoline, nafazoline and tramazoline).

Additionally, a compound of the invention may be administered in combination with emergency therapies for asthma and/or chronic obstructive pulmonary disease, such therapies include oxygen or heliox administration, nebulized salbutamol or terbutaline (optionally combined with an anticholinergic (e.g. ipratropium), systemic steroids (oral or intravenous, e.g. prednisone, prednisolone, methylprednisolone, dexamethasone, or hydrocortisone), intravenous salbutamol, non-specific beta-agonists, injected or inhaled (e.g. epinephrine, isoetharine, isoproterenol, metaproterenol), anticholinergics (IV or nebulized, e.g. glycopyrrolate, atropine, ipratropium), methylxanthines (theophylline, aminophylline, bamiphylline), inhalation anesthetics that have a bronchodilatory effect (e.g. isoflurane, halothane, enflurane), ketamine and intravenous magnesium sulfate.

In one embodiment, a compound of the invention is co-administered with another therapeutic agent for the treatment and/or prophylaxis of inflammatory bowel disease (IBD), particular agents include but are not limited to: glucocorticoids (e.g. prednisone, budesonide) synthetic disease modifying, immunomodulatory agents (e.g. methotrexate, leflunomide, sulfasalazine, mesalazine, azathioprine, 6-mercaptopurine and cyclosporin) and biological disease modifying, immunomodulatory agents (infliximab, adalimumab, rituximab, and abatacept).

In one embodiment, a compound of the invention is co-administered with another therapeutic agent for the treatment and/or prophylaxis of SLE, particular agents include but are not limited to: human monoclonal antibodies (belimumab (Benlysta)), Disease-modifying antirheumatic drugs (DMARDs) such as antimalarials (e.g. plaquenil, hydroxychloroquine), immunosuppressants (e.g. methotrexate and azathioprine), cyclophosphamide and mycophenolic acid, immunosuppressive drugs and analgesics, such as nonsteroidal anti-inflammatory drugs, opiates (e.g. dextropropoxyphene and co-codamol), opioids (e.g. hydrocodone, oxycodone, MS Contin, or methadone) and the fentanyl duragesic transdermal patch.

In one embodiment, a compound of the invention is co-administered with another therapeutic agent for the treatment and/or prophylaxis of psoriasis, particular agents include but are not limited to: topical treatments such as bath solutions, moisturizers, medicated creams and ointments containing coal tar, dithranol (anthralin), corticosteroids like desoximetasone (Topicort™), fluocinonide, vitamin D3 analogues (for example, calcipotriol), argan oil and retinoids (etretinate, acitretin, tazarotene), systemic treatments such as methotrexate, cyclosporine, retinoids, tioguanine, hydroxyurea, sulfasalazine, mycophenolate mofetil, azathioprine, tacrolimus, fumaric acid esters or biologics such as Amevive™, Enbrel™, Humira™, Remicade™, Raptiva™ and ustekinumab (a IL-12 and IL-23 blocker). Additionally, a compound of the invention may be administered in combination with other therapies including, but not limited to phototherapy, or photochemotherapy (e.g. psoralen and ultraviolet A phototherapy (PUVA)).

In one embodiment, a compound of the invention is co-administered with another therapeutic agent for the treatment and/or prophylaxis of allergic reaction, particular agents include but are not limited to: antihistamines (e.g. cetirizine, diphenhydramine, fexofenadine, levocetirizine), glucocorticoids (e.g. prednisone, betamethasone, beclomethasone, dexamethasone), epinephrine, theophylline or anti-leukotrienes (e.g. montelukast or zafirlukast), anti-cholinergics and decongestants.

By co-administration is included any means of delivering two or more therapeutic agents to the patient as part of the same treatment regime, as will be apparent to the skilled person. Whilst the two or more agents may be administered simultaneously in a single formulation, i.e. as a single pharmaceutical composition, this is not essential. The agents may be administered in different formulations and at different times.

### CHEMICAL SYNTHETIC PROCEDURES

### General

The compound of the invention can be prepared from readily available starting materials using the following general methods and procedures. It will be appreciated that where typical or preferred process conditions (*i.e.* reaction temperatures, times, mole ratios of reactants, solvents, pressures, etc.) are given, other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. The choice of a suitable protecting group for a particular functional group as well as suitable conditions for protection and deprotection are well known in the art (Greene, T W; Wuts, P G M;, 1991).

The following methods are presented with details as to the preparation of a compound of the invention as defined hereinabove and the comparative examples. A compound of the invention may be prepared from known or commercially available starting materials and reagents by one skilled in the art of organic synthesis.

All reagents were of commercial grade and were used as received without further purification, unless otherwise stated. Commercially available anhydrous solvents were used for reactions conducted under inert atmosphere. Reagent grade solvents were used in all other cases, unless otherwise specified. Column chromatography was performed on silica gel 60 (35-70 µm). Thin layer chromatography was carried out using pre-coated silica gel F-254 plates (thickness 0.25 mm). ¹H NMR spectra were recorded on a Bruker DPX 400 NMR spectrometer (400 MHz or a Bruker Advance 300 NMR spectrometer (300 MHz). Chemical shifts (δ) for 1H NMR spectra are reported in parts per million (ppm) relative to tetramethylsilane (δ 0.00) or the appropriate residual solvent peak, i.e. CHCl₃ (δ 7.27), as internal reference. Multiplicities are given as singlet (s), doublet (d), triplet (t), quartet (q), quintuplet (quin), multiplet (m) and broad (br). Electrospray MS spectra were obtained on a Waters platform LC/MS spectrometer or with Waters Acquity H-Class UPLC coupled to a Waters Mass detector 3100 spectrometer. Columns used: Waters Acquity UPLC BEH C18 1.7µm, 2.1mm ID x 50mm L, Waters Acquity UPLC BEH C18 1.7 µm, 2.1mm ID x 30 mm L, or Waters Xterra MS 5µm C18, 100 x 4.6mm. The methods are using either MeCN/H₂O gradients (H₂O contains either 0.1% TFA or 0.1% NH₃) or MeOH /H₂O gradients (H₂O contains 0.05% TFA). Microwave heating was performed with a Biotage Initiator.

**Table I. List of abbreviations used in the experimental section:**

| **Abbréviation** | **Definition** |
|---|---|
| µL | microliter |
| ALL | acute lymphoblastic leukemia |
| AML | acute myeloid leukaemia |
| aq | aqueous |
| br s | broad singlet |
| BLM | Bleomycin |
| bt | Broad triplet |
| Cat. | Catalytic amount |
| CLL | chronic lymphoblastic leukaemia |
| COPD | chronic obstructive pulmonary disease |
| Cpd | Compound |
| d | doublet |
| DCM | Dichloromethane |
| eq. | Equivalent |
| EtOAc | Ethyl acetate |
| g | gram |
| GTPγS | guanosine 5'-O-[gamma-thio]triphosphate) |
| h | hour |
| Int | Intermediate |
| IPF | idiopathic pulmonary fibrosis |
| iPrOH | Isopropanol |
| L | liter |
| m | multiplet |
| MeCN | Acetonitrile |
| MeOH | Methanol |
| mg | milligram |
| min | minute |
| mL | millilitre |
| MTBE | Methyl tButyl ether |
| MW | Molecular weight |
| NASH | nonalcoholic steatohepatitis |
| PBS | Phosphate buffered saline |
| Pd/C | Palladium on Carbon 10% |
| Pd₂(dba)₃ | Tris(dibenzylideneacetone) dipalladium(0) |
| PdCl₂(dppf).D CM | [1,1'-Bis(diphenylphosphino)ferrocene]dichloropalladium(II), complex with dichloromethane |
| PdCl₂dppf | [1,1'-Bis(diphenylphosphino)ferrocene] dichloropalladium(II) |
| ppm | part-per-million |
| PK | Pharmacokinetic |
| q | quadruplet |
| s | singlet |
| sat | saturated |
| SCX column | ion exchange sulfonic acid cross linked columns |
| SLE | systemic lupus erythematosus |
| SPhos Pd G2 | Chloro(2-dicyclohexylphosphino-2',6'-dimethoxy-1,1'-biphenyl)[2-(2'-amino-1,1'-biphenyl)]palladium(II) |
| t | triplet |
| TFA | Trifluoroacetic acid |
| THF | Tetrahydrofuran |
| XantPhos | 4,5-Bis(diphenylphosphino)-9,9-dimethylxanthene |
| MW (calc) | molecular weight calculated |
| MW (obs) | molecular weight observed |

### SYNTHETIC PREPARATION OF THE COMPOUNDS OF THE INVENTION

### General synthetic methods

### Example 1. Preparation of intermediates towards illustrative compounds of the invention

### 1.1. Intermediate 1: 2-chloro-6-(2,2,2-trifluoroethoxy)pyridin-4-amine

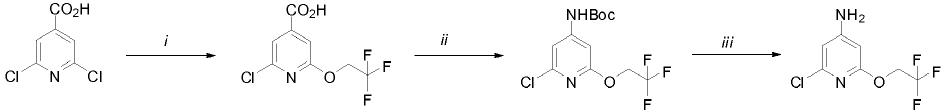

### 1.1.1. Step i: 2-chloro-6-(2,2,2-trifluoroethoxy)pyridine-4-carboxylic acid

To 2,6-dichloropyridine-4-carboxylic acid (200 mg, 1.23 mmol, 1.0 equiv) in dry THF (1.2 mL, 1M) at 0°C is added a solution of *t*BuOK (345 mg, 3.08 mmol, 2.5 equiv) in dry THF (3 mL, 1M). The resulting mixture is stirred at 0°C for 5 min. Trifluoroethanol (185 mg, 1.85 mmol, 1.5 equiv) is added dropwise. The resulting mixture is stirred at room temperature for 24 h. To the reaction mixture acid in dry THF at 0°C is added a solution of *t*BuOK (1.0 equiv) in dry THF. The resulting mixture is stirred at 0°C for 5 min. Trifluoroethanol (1.5 equiv) is added dropwise. The resulting mixture is stirred at room temperature for 24 h. The mixture is diluted with DCM and 1M HCl solution is added. The aqueous phase is extracted with DCM. The combined organic phase is filtered through a phase separator and concentrated under vacuum to afford the desired compound. LCMS: MW (calcd): 256; m/z MW (obsd): 256 (M+H).

### 1.1.2. Step ii: tert-butyl N-[2-chloro-6-(2,2,2-trifluoroethoxy)-4-pyridyl]carbamate

Under N₂, diphenyl phosphoryl azide (0.40 mL, 1.85 mmol, 1.5 equiv) is added to a solution of 2-chloro-6-(2,2,2-trifluoroethoxy)pyridine-4-carboxylic acid (1.23 mmol, 1.0 equiv) in trimethylamine (0.52 mL, 3.69 mmol, 3.0 equiv), dry tert-butanol (5 mL, 0.25 M) and dry toluene (5 mL, 0.25M). The resulting mixture is stirred at 100°C for 2 h. The mixture is diluted with DCM and water. The aqueous phase is extracted with DCM. The combined organic phase is filtered through a phase separator and concentrated under vacuum to afford the desired compound. LCMS: MW (calcd): 327; m/z MW (obsd): 327 (M+H).

### 1.1.3. Step iii: 2-chloro-6-(2,2,2-trifluoroethoxy)pyridin-4-amine

The tert-butyl N-[2-chloro-6-(2,2,2-trifluoroethoxy)-4-pyridyl]carbamate (1.23 mmol, 1.0 equiv) is stirred at room temperature for 6 h in TFA (1 mL, 1M) and DCM (10 mL, 0.1 M). The mixture is diluted with DCM and 1 M HCl solution is added. The two phases are separated and the aqueous phase is basified and extracted with DCM/iPrOH (4/1). The combined organic phase is filtered through a phase separator and concentrated under vacuum to afford the desired compound. LCMS: MW (calcd): 227; m/z MW (obsd): 227 (M+H).

### 1.2. General method A: synthesis of aniline intermediate in two steps. SNAr of alcohol on nitro derivative followed by reduction of nitro group.

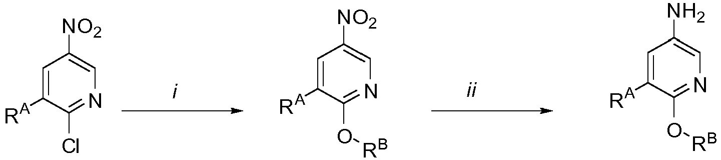

### 1.2.1. Step i:

At 0°C, *t*BuOK (1.2 equiv.) is added to the alcohol (5.0 equiv.), the resulting mixture is stirred at room temperature for 15 min, then the solution of the nitro derivative (1.0 equiv) in dry THF (1 M) is added at 0°C. The resulting mixture is stirred at room temperature for 10 min and heated at 50°C for 3 h. The mixture is diluted with DCM and saturated NaHCO₃ solution. The aqueous phase is extracted with DCM. The combined organic phase is filtered through a phase separator and concentrated under vacuum to afford the desired compound.

### 1.2.2. Step ii:

To the nitro derivative from step i above (1.0 equiv) in methanol (0.15 M) at 0°C, zinc (12.0 equiv), NH₄Cl (12.0 equiv) and formic acid (2 drops) are added. The resulting mixture is stirred at room temperature for 6 h. The mixture is filtered over celite and concentrated. The residue is diluted with DCM and 1 M HCl solution is added. The two phases are separated and the aqueous phase is basified and extracted with DCM/iPrOH (4/1). The combined organic phase is filtered through a phase separator and concentrated under vacuum to afford the desired compound.

### 1.3. Illustrative example of method A: synthesis of intermediate 2, 5-chloro-6-ethoxy-pyridin-3-amine

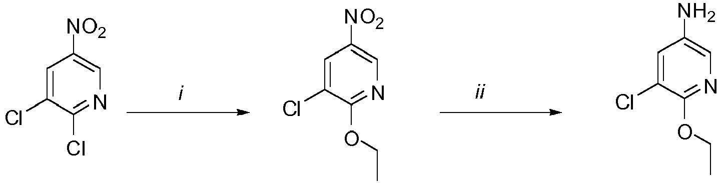

### 1.3.1. Step i: 3-chloro-2-ethoxy-5-nitro-pyridine

At 0°C, *t*BuOK (208.7 mg, 1.86 mmol, 1.2 equiv) is added to ethanol (0.45 mL, 7.77 mmol, 5.0 equiv), the resulting mixture is stirred at room temperature for 15 min, then the solution of 2,3-dichloro-5-nitro-pyridine (300 mg, 1.55 mmol, 1.0 equiv) in dry THF (1.5 mL, 1 M) is added at 0°C. The resulting mixture is stirred at room temperature for 10 min, then heated at 50°C for 3 h. The mixture is diluted with DCM and saturated NaHCO₃ solution. The aqueous phase is extracted with DCM. The combined organic phase is filtered through a phase separator and concentrated under vacuum to afford the desired compound.

### 1.3.2. Step ii: 5-chloro-6-ethoxy-pyridin-3-amine

To 3-chloro-2-ethoxy-5-nitro-pyridine (1.55 mmol, 1.0 equiv) in methanol (10 mL, 0.15 M) at 0°C, are added zinc (1.21 g, 18.6 mmol, 12.0 equiv), NH₄Cl (995 mg, 18.6 mmol, 12.0 equiv) and formic acid (2 drops). The resulting mixture is stirred at room temperature for 6 h. The mixture is filtered over celite and concentrated. The residue is diluted with DCM and 1 M HCl solution is added. The two phases are separated and the aqueous phase is basified and extracted with DCM/iPrOH (4/1). The combined organic phase is filtered through a phase separator and concentrated under vacuum to afford the desired compound. LCMS: MW (calcd): 173; m/z MW (obsd): 173 (M+H).

### 1.4. Intermediate 8: 6-ethoxy-5-(trifluoromethyl)pyridin-3-amine

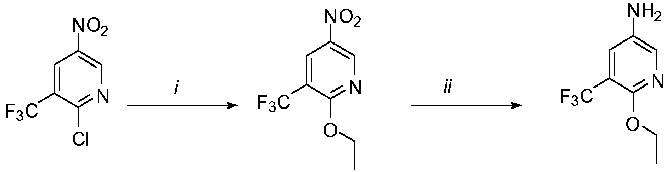

### 1.4.1. Step i: 2-ethoxy-5-nitro-3-(trifluoromethyl)pyridine

At 0°C, NaH (141 mg, 3.53 mmol, 2.0 equiv) is added to 2-chloro-5-nitro-3-(trifluoromethyl)pyridine (400 mg, 1.77 mmol, 1.0 equiv) in ethanol (6 mL, 0.3 M), the resulting mixture is stirred at 85°C for 2 h. The mixture is diluted with DCM and water. The aqueous phase is extracted with DCM. The combined organic phase is filtered through a phase separator and concentrated under vacuum to afford the desired compound.

### 1.4.2. Step ii: 6-ethoxy-5-(trifluoromethyl)pyridin-3-amine

To 2-ethoxy-5-nitro-3-(trifluoromethyl)pyridine (1.77 mmol, 1.0 equiv) in methanol (12 mL, 0.15 M) at 0°C, are added zinc (1.39 g, 21.0 mmol, 12.0 equiv), NH₄Cl (1.12 g, 21.0 mmol, 12.0 equiv) and formic acid (2 drops). The resulting mixture is stirred at room temperature for 6 h. The mixture is filtered over celite and concentrated. The residue is diluted with DCM and 1 M HCl solution is added. The two phases are separated and the aqueous phase is basified and extracted with DCM/iPrOH (4/1). The combined organic phase is filtered through a phase separator and concentrated under vacuum to afford the desired compound. LCMS: MW (calcd): 206; m/z MW (obsd): 207 (M+H).

### 1.5. Intermediate 9: 2-chloro-6-(2-fluoroethoxy)pyridin-4-amine

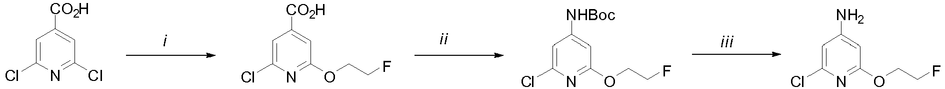

### 1.5.1. Step i: 2-chloro-6-(2-fluoroethoxy)pyridine-4-carboxylic acid

To 2,6-dichloropyridine-4-carboxylic acid (500 mg, 3.07 mmol, 1.0 equiv) in dry THF (3 mL, 1M) at 0°C is added a solution of *t*BuOK (1.72 g, 15.35 mmol, 5.0 equiv) in dry THF (15 mL, 1M). The resulting mixture is stirred at 0°C for 5 min. Fluoroethanol (0.54 mL, 4.61 mmol, 3.0 equiv) is added dropwise. The resulting mixture is stirred at room temperature for 24 h. The mixture is diluted with DCM and 1M HCl solution is added. The aqueous phase is extracted with DCM. The combined organic phase is filtered through a phase separator and concentrated under vacuum to afford the desired compound. LCMS: MW (calcd): 220; m/z MW (obsd): 220 (M+H).

### 1.5.2. Step ii: tert-butyl N-[2-chloro-6-(2-fluoroethoxy)-4-pyridyl]carbamate

Under N₂, diphenyl phosphoryl azide (1.27 g, 4.61 mmol, 1.5 equiv) is added to a solution of 2-chloro-6-(2-fluoroethoxy)pyridine-4-carboxylic acid (3.07 mmol, 1.0 equiv) in trimethylamine (1.29 mL, 9.21 mmol, 3.0 equiv), dry *tert* butanol (12 mL, 0.25 M) and dry toluene (12 mL, 0.25M). The resulting mixture is stirred at 100°C for 2 h. The mixture is diluted with DCM and water. The aqueous phase is extracted with DCM. The combined organic phase is filtered through a phase separator and concentrated under vacuum to afford the desired compound. LCMS: MW (calcd): 291; m/z MW (obsd): 291 (M+H).

### 1.5.3. Step iii: 2-chloro-6-(2-fluoroethoxy)pyridin-4-amine

Tert-butyl N-[2-chloro-6-(2-fluoroethoxy)-4-pyridyl]carbamate (3.07 mmol, 1.0 equiv) is stirred at room temperature for 6 h in TFA (3 mL, 1M) and DCM (30 mL, 0.1 M). The mixture is diluted with DCM and 1 M HCl solution is added. The two phases are separated and the aqueous phase is basified and extracted with DCM/iPrOH (4/1). The combined organic phase is filtered through a phase separator and concentrated under vacuum to afford the desired compound. LCMS: MW (calcd): 191; m/z MW (obsd): 191 (M+H).

### 1.6. Intermediate 10: 2-(2,2-difluoroethoxy)-6-(trifluoromethyl)pyridin-4-amine

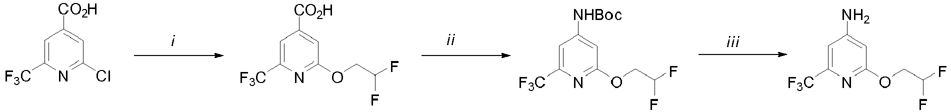

### 1.6.1. Step i: 2-(2,2-difluoroethoxy)-6-(trifluoromethyl)pyridine-4-carboxylic acid

To 2-chloro-6-(trifluoromethyl)pyridine-4-carboxylic acid (300 mg, 1.33 mmol, 1.0 equiv) in dry THF (1.3 mL, 1M) at 0°C is added a solution of *t*BuOK (746 mg, 6.65 mmol, 5.0 equiv) in dry THF (7 mL, 1M). The resulting mixture is stirred at 0°C for 5 min. Fluoroethanol (0.25 mL, 3.99 mmol, 3.0 equiv) is added dropwise. The resulting mixture is stirred at room temperature for 24 h. The mixture is diluted with DCM and 1M HCl solution is added. The aqueous phase is extracted with DCM. The combined organic phase is filtered through a phase separator and concentrated under vacuum to afford the desired compound. LCMS: MW (calcd): 271; m/z MW (obsd): 272 (M+H).

### 1.6.2. Step ii: tert-butyl N-[2-(2,2-difluoroethoxy)-6-(trifluoromethyl)-4-pyridyl]carbamate

Under N₂, diphenyl phosphoryl azide (0.43 mL, 1.99 mmol, 1.5 equiv) is added to a solution of 2-(2,2-difluoroethoxy)-6-(trifluoromethyl)pyridine-4-carboxylic acid (1.33 mmol, 1.0 equiv) in trimethylamine (0.56 mL, 3.99 mmol, 3.0 equiv), dry tertbutanol (5 mL, 0.25 M) and dry toluene (5 mL, 0.25M). The resulting mixture is stirred at 100°C for 2 h. The mixture is diluted with DCM and water. The aqueous phase is extracted with DCM. The combined organic phase is filtered through a phase separator and concentrated under vacuum to afford the desired compound. LCMS: MW (calcd): 342; m/z MW (obsd): 343 (M+H).

### 1.6.3. Step iii: 2-(2,2-difluoroethoxy)-6-(trifluoromethyl)pyridin-4-amine

*tert*-butyl N-[2-(2,2-difluoroethoxy)-6-(trifluoromethyl)-4-pyridyl]carbamate (1.33 mmol, 1.0 equiv) is stirred at room temperature for 6 h in TFA (2 mL, 1M) and DCM (20 mL, 0.1 M). The mixture is diluted with DCM and 1 M HCl solution is added. The two phases are separated and the aqueous phase is basified and extracted with DCM/iPrOH (4/1). The combined organic phase is filtered through a phase separator and concentrated under vacuum to afford the desired compound. LCMS: MW (calcd): 242; m/z MW (obsd): 243 (M+H).

### 1.7. General method B: SNAr of alcohol on aryl halide bearing a NH₂ group

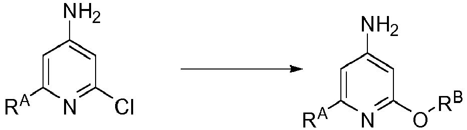

At 0°C, alcohol (3.0 equiv) is slowly added to NaH (3.0 equiv) in suspension in dry THF (0.3 M). The resulting mixture is stirred for 20 min at room temperature. The aryl halide (1.0 equiv) is added, the resulting mixture is stirred at 85°C for approximately 16 h. The mixture is diluted with DCM and water. The aqueous phase is extracted with DCM. The combined organic phase is filtered through a phase separator and concentrated under vacuum to afford the desired compound, which can be used as such or further purified by flash column chromatography.

### 1.8. Illustrative example of method B: synthesis of intermediate 11, 2-chloro-6-(2,2-difluoroethoxy)pyridin-4-amine

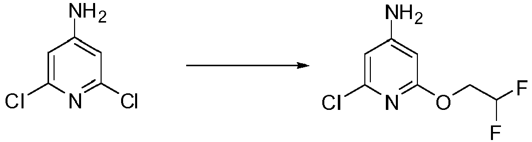

At 0°C, difluoroethanol (11.7 mL, 183.9 mmol, 3.0 equiv) is slowly added to NaH (7.35 g, 183.9 mmol, 3.0 equiv) in suspension in dry THF (200 mL, 0.3 M). The resulting mixture is stirred for 20 min at room temperature. The 2,6-dichloropyridin-4-amine (10 g, 61.3 mmol, 1.0 equiv) is added, the resulting mixture is stirred at 85°C for 15 h. The mixture is diluted with DCM and water. The aqueous phase is extracted with DCM. The combined organic phase is filtered through a phase separator and concentrated under vacuum. The crude is purified by flash chromatography on silica gel (eluting with petroleum ether/EtOAc 95/5) to afford the desired compound. LCMS: MW (calcd): 209; m/z MW (obsd): 209 (M+H).

### 1.9. Intermediate 24: 2,6-diethoxypyrimidin-4-amine

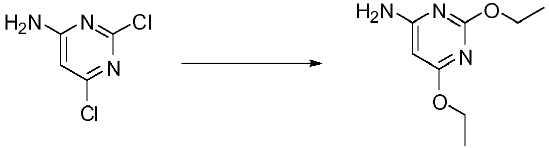

Ethanol (0.27 mL, 4.56 mmol, 3 eq) is added dropwise at 0° C to a suspension of NaH (60% mineral oil, 182 mg, 4.56 mmol, 3 eq) in dry THF (4 mL). 4-Amino-2,6-dichloropyrimidine (250 mg, 1.52 mmol, 1 eq) is added and the reaction is stirred at 85° C for approximately 16 h. The mixture is partitioned between water and DCM. The aqueous layer is extracted with DCM. the organic layers are dried (Na₂SO₄) and concentrated to afford the desired compound. LCMS: MW (calcd): 183; m/z MW (obsd): 184 (M+H).

### 1.10. Intermediate 25: 2-cyclopropyl-6-ethoxy-pyridin-4-amine

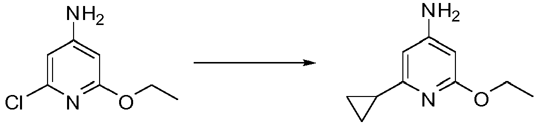

Pd(dppf)Cl₂ (118 mg, 0.145 mmol, 0.1 equiv) is added to a degassed suspension of 2-chloro-6-ethoxy-pyridin-4-amine (250 mg, 1.45 mmol, 1.0 equiv), cyclopropylboronic acid (149 mg, 1.74 mmol, 1.2 equiv) and Cs₂CO₃ (1.18 g, 3.63 mmol, 2.5 equiv) in 1,4-dioxane/water (4/1 mL, 0.30 M). The resulting mixture is stirred at 95°C for 24 h. The mixture is diluted with DCM and water. The aqueous phase is extracted with DCM. The combined organic phase is filtered through a phase separator and concentrated under vacuum. The crude is purified by flash chromatography on silica gel (eluting with petroleum ether/EtOAc 4/1) to afford the desired compound. LCMS: MW (calcd): 178; m/z MW (obsd): 179 (M+H).

### 1.11. Intermediate 26: 4-amino-6-ethoxy-pyridine-2-carbonitrile

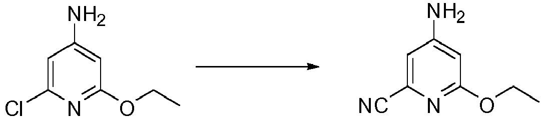

2-chloro-6-ethoxy-pyridin-4-amine (500 mg, 2.90 mmol, 1.0 equiv), Zn(CN)₂ (341 mg, 2.90 mmol, 1.0 equiv) and Pd(PPh₃)₄ (335 mg, 0.290 mmol, 0.1 equiv) are heated in dry DMF (12 mL, 0.25 M) at 150°C for 5 min under microwave conditions. The mixture is diluted with EtOAc and saturated NaHCO₃ solution. The aqueous phase is extracted with EtOAc. The combined organic phase is washed with brine, dried over sodium sulfate and concentrated under vacuum. The crude is purified by flash chromatography on silica gel (eluting with petroleum ether/EtOAc 4/1) to afford the desired compound. LCMS: MW (calcd): 163; m/z MW (obsd): 164 (M+H).

### 1.12. Intermediate 27: 2-ethoxy-6-methyl-pyridin-4-amine

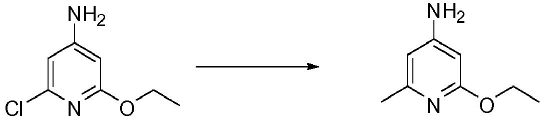

Pd(dppf)Cl₂ (118 mg, 0.145 mmol, 0.1 equiv) is added to a degassed suspension of 2-chloro-6-ethoxy-pyridin-4-amine (250 mg, 1.45 mmol, 1.0 equiv), methylboronic acid (260 mg, 4.35 mmol, 3.0 equiv) and Cs₂CO₃ (2.36 g, 7.25 mmol, 5.0 equiv) in dry 1,4-dioxane (3.6 mL, 0.40 M). The resulting mixture is stirred at 105°C for 24 h. The mixture is diluted with DCM and water. The aqueous phase is extracted with DCM. The combined organic phase is filtered through a phase separator and concentrated under vacuum. The crude is purified by flash chromatography on silica gel (eluting with petroleum ether/EtOAc 4/1) to afford the desired compound. LCMS: MW (calcd): 152; m/z MW (obsd): 153 (M+H).

### 1.13. Intermediate 28: 6-chloro-2-ethoxy-3-methyl-pyridin-4-amine

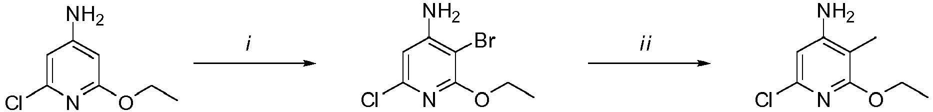

### 1.13.1. Step i: 3-bromo-6-chloro-2-ethoxy-pyridin-4-amine

2-chloro-6-ethoxy-pyridin-4-amine (500 mg, 2.90 mmol, 1.0 equiv) and potassium acetate (284.3 mg, 2.90 mmol, 1.0 equiv) are stirred at room temperature for 1 h in acetic acid (2.90 mL, 1 M). At 0°C, Br₂ (0.15 mL, 2.90 mmol, 1.0 equiv) is slowly added. The reaction mixture is concentrated and the residue is dissolved in EtOAc, washed with saturated NaHCO₃ solution and Na₂S₂O₃ solution, dried over sodium sulfate and concentrated under vacuum. The crude is purified by flash chromatography on silica gel (eluting with petroleum ether) to afford the desired compound. LCMS: MW (calcd): 252; m/z MW (obsd): 253 (M+H).

### 1.13.2. Step ii: 6-chloro-2-ethoxy-3-methyl-pyridin-4-amine

Pd(dppf)Cl₂ (16 mg, 0.020 mmol, 0.1 equiv) is added to a degassed suspension of 3-bromo-6-chloro-2-ethoxy-pyridin-4-amine (50 mg, 0.199 mmol, 1.0 equiv), methylboronic acid (35 mg, 0.596 mmol, 3.0 equiv) and Cs₂CO₃ (324 mg, 0.995 mmol, 5.0 equiv) in dry 1,4-dioxane (0.5 mL, 0.40 M). The resulting mixture is stirred at 105°C for 4 h. The mixture is diluted with DCM and water. The aqueous phase is extracted with DCM. The combined organic phase is filtered through a phase separator and concentrated under vacuum. The crude is purified by flash chromatography on silica gel (eluting with petroleum ether/EtOAc 9/1) to afford the desired compound. LCMS: MW (calcd): 187; m/z MW (obsd): 187 (M+H).

### 1.14. Intermediate 29: 4-amino-6-(2,2-difluoroethoxy)pyridine-2-carbonitrile

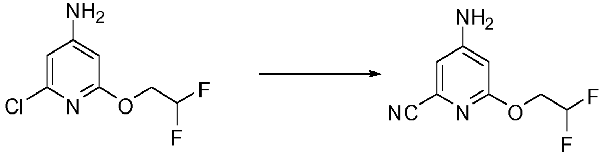

2-chloro-6-(2,2-difluoroethoxy)pyridin-4-amine (250 mg, 1.20 mmol, 1.0 equiv), Zn(CN)₂ (183 mg, 1.56 mmol, 1.3 equiv), Pd₂(dba)₃ (335 mg, 0.290 mmol, 0.05 equiv) and dppf (53 mg, 0.096 mmol, 0.08 equiv) are heated in dry DMF (1.2 mL, 1.0 M) at 135°C for 24 h. The mixture is diluted with EtOAc and saturated NaHCO₃ solution. The aqueous phase is extracted with EtOAc. The combined organic phase is washed with brine, dried over sodium sulfate and concentrated under vacuum. The crude is purified by flash chromatography on silica gel (eluting with petroleum ether/EtOAc 4/1) to afford the desired compound. LCMS: MW (calcd): 199; m/z MW (obsd): 200 (M+H).

### 1.15. Intermediate 30: 2-cyclopropyl-6-(2,2-difluoroethoxy)pyridin-4-amine

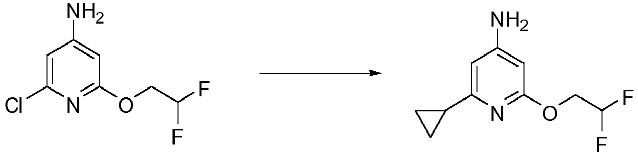

Pd(dppf)Cl₂ (98 mg, 0.120 mmol, 0.1 equiv) is added to a degassed suspension of 2-chloro-6-(2,2-difluoroethoxy)pyridin-4-amine (250 mg, 1.20 mmol, 1.0 equiv), cyclopropylboronic acid MIDA ester (283.7 mg, 1.44 mmol, 1.2 equiv) and Cs₂CO₃ (977 mg, 3.0 mmol, 2.5 equiv) in 1,4-dioxane/water (4/1 mL, 0.30 M). The resulting mixture is stirred at 95°C for 24 h. The mixture is diluted with DCM and water. The aqueous phase is extracted with DCM. The combined organic phase is filtered through a phase separator and concentrated under vacuum. The crude is purified by flash chromatography on silica gel (eluting with petroleum ether/EtOAc 4/1) to afford the desired compound. LCMS: MW (calcd): 214; m/z MW (obsd): 215 (M+H).

### 1.16. General method C: SNAr of alcohol on iodo-derivative followed by Buchwald and Boc deprotection

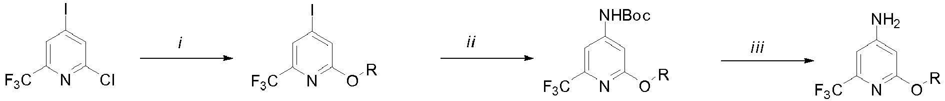

### 1.16.1. Step i:

At 0°C, *t*BuOK (1.2 equiv) is added to the alcohol (5.0 equiv), the resulting mixture is stirred at room temperature for 15 min, then the solution of 2-chloro-4-iodo-6-(trifluoromethyl)pyridine (1.0 equiv) in dry THF (1 M) is added at 0°C. The resulting mixture is stirred at room temperature for 10 min and heated at 50°C for 3 h. The mixture is diluted with EtOAc and water. The aqueous phase is extracted with EtOAc. The combined organic phase is washed with brine, dried over sodium sulfate and concentrated under vacuum. The crude is purified by flash chromatography on silica gel to afford the desired compound.

### 1.16.2. Step ii:

Pd₂(dba)₃ (0.1 equiv) is added to a degassed suspension of the iodo derivative (1.0 equiv), Xantphos (0.3 equiv), tertbutyl carbamate (1.2 equiv) and Cs₂CO₃ (1.5 equiv) in dry 1,4-dioxane (0.25 M). The resulting mixture is stirred at 100°C for 3 h. The mixture is filtered over celite, the filtrate is diluted with EtOAc and water. The aqueous phase is extracted with EtOAc. The combined organic phase is washed with brine, dried over sodium sulfate and concentrated under vacuum to afford the desired compound.

### 1.16.3. Step iii:

*tert*-butyl N-[2-ethoxy-6-(trifluoromethyl)-4-pyridyl]carbamate (1.0 equiv) is stirred at 40°C for 18 h in 1/1 TFA/DCM (0.5 M). The mixture is diluted with DCM and 2 M HCl solution is added. The two phases are separated and the aqueous phase is basified and extracted with DCM/iPrOH (4/1). The combined organic phase is filtered through a phase separator and concentrated under vacuum to afford the desired compound.

### 1.17. Illustrative example of method C: synthesis of intermediate 31, 2-ethoxy-6-(trifluoromethyl)pyridin-4-amine

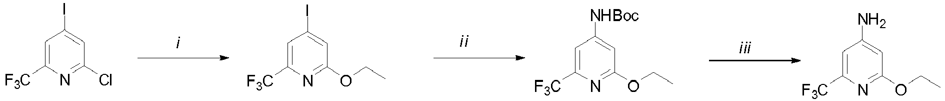

### 1.17.1. Step i: 2-ethoxy-4-iodo-6-(trifluoromethyl)pyridine

At 0°C, *t*BuOK (8.76 g, 78.06 mmol, 1.2 equiv) is added to the ethanol (19 mL, 325.25 mmol, 5.0 equiv), the resulting mixture is stirred at room temperature for 15 min, then the solution of 2-chloro-4-iodo-6-(trifluoromethyl)pyridine (20 g, 65.05 mmol, 1.0 equiv) in dry THF (65 mL, 1 M) is added at 0°C. The resulting mixture is stirred at room temperature for 10 min, then heated at 50°C for 3 h. The mixture is diluted with EtOAc and water. The aqueous phase is extracted with EtOAc. The combined organic phase is washed with brine, dried over sodium sulfate and concentrated under vacuum. The crude is purified by flash chromatography on silica gel (eluting with petroleum ether) to afford the desired compound. LCMS: MW (calcd): 317; m/z MW (obsd): 318 (M+H).

### 1.17.2. Step ii: tert-butyl N-[2-ethoxy-6-(trifluoromethyl)-4-pyridyl]carbamate

Pd₂(dba)₃ (4.10 g, 4.48 mmol, 0.1 equiv) is added to a degassed suspension of 2-ethoxy-4-iodo-6-(trifluoromethyl)pyridine (14.2 g, 44.8 mmol, 1.0 equiv), Xantphos (7.78 g, 13.4 mmol, 0.3 equiv), tertbutyl carbamate (6.30 g, 53.7 mmol, 1.2 equiv) and Cs₂CO₃ (21.9 g, 67.2 mmol, 1.5 equiv) in dry 1,4-dioxane (180 mL, 0.25 M). The resulting mixture is stirred at 100°C for 3 h. The mixture is filtered over celite, the filtrate is diluted with EtOAc and water. The aqueous phase is extracted with EtOAc. The combined organic phase is washed with brine, dried over sodium sulfate and concentrated under vacuum to afford the desired compound. LCMS: MW (calcd): 306; m/z MW (obsd): 307 (M+H).

### 1.17.3. Step iii: 2-ethoxy-6-(trifluoromethyl)pyridin-4-amine

*tert*-butyl N-[2-ethoxy-6-(trifluoromethyl)-4-pyridyl]carbamate (44 mmol, 1.0 equiv) is stirred at 40°C for 18 h in TFA (40 mL) and DCM (40 mL). The mixture is diluted with DCM and 2 M HCl solution is added. The two phases are separated and the aqueous phase is basified and extracted with DCM/iPrOH (4/1). The combined organic phase is filtered through a phase separator and concentrated under vacuum to afford the desired compound. LCMS: MW (calcd): 206; m/z MW (obsd): 207 (M+H).

### 1.18. Intermediate 34: 2-ethoxy-3-methyl-6-(trifluoromethyl)pyridin-4-amine

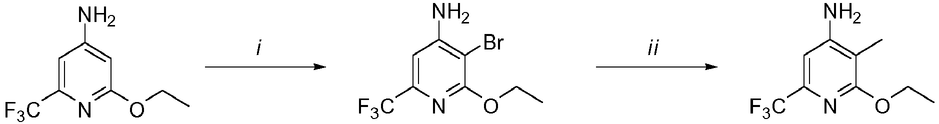

### 1.18.1. Step i: 3-bromo-2-ethoxy-6-(trifluoromethyl)pyridin-4-amine

*N*-bromosuccinimide (216 mg, 1.21 mmol, 1.0 equiv) and 2-ethoxy-6-(trifluoromethyl)pyridin-4-amine (250 mg, 1.21 mmol, 1.0 equiv) are stirred at room temperature in dry DCM for 1 h. The mixture is diluted with DCM and water. The aqueous phase is extracted with DCM. The combined organic phase is filtered through a phase separator and concentrated under vacuum to afford the desired compound. LCMS: MW (calcd): 285; m/z MW (obsd): 287 (M+H).

### 1.18.2. Step ii: 2-ethoxy-3-methyl-6-(trifluoromethyl)pyridin-4-amine

Pd(dppf)Cl₂ (29 mg, 0.035 mmol, 0.1 equiv) is added to a degassed suspension of 3-bromo-2-ethoxy-6-(trifluoromethyl)pyridin-4-amine (100 mg, 0.35 mmol, 1.0 equiv), methylboronic acid (63 mg, 1.05 mmol, 3.0 equiv) and Cs₂CO₃ (570 mg, 1.75 mmol, 5.0 equiv) in dry 1,4-dioxane (1.0 mL, 0.40 M). The resulting mixture is stirred at 105°C for 5 h. The mixture is diluted with DCM and water. The aqueous phase is extracted with DCM. The combined organic phase is filtered through a phase separator and concentrated under vacuum. The crude is purified by flash chromatography on silica gel (eluting with petroleum ether/EtOAc 9/1) to afford the desired compound. LCMS: MW (calcd): 220; m/z MW (obsd): 221 (M+H).

### 1.19. Intermediate 35: 2-ethoxy-3-ethyl-6-(trifluoromethyl)pyridin-4-amine

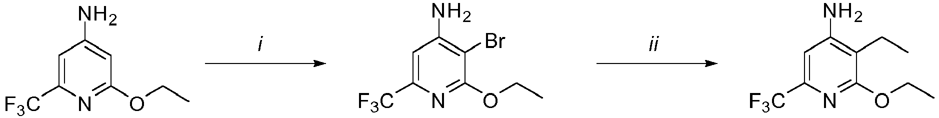

### 1.19.1. Step i: 3-bromo-2-ethoxy-6-(trifluoromethyl)pyridin-4-amine

As described in synthesis of intermediate 34.

### 1.19.2. Step ii: 2-ethoxy-3-ethyl-6-(trifluoromethyl)pyridin-4-amine

Pd(dppf)Cl₂ (29 mg, 0.035 mmol, 0.1 equiv) is added to a degassed suspension of 3-bromo-2-ethoxy-6-(trifluoromethyl)pyridin-4-amine (100 mg, 0.35 mmol, 1.0 equiv), triethyl broane (0.46 mL, 0.46 mmol, 1.3 equiv, 1M in hexane) and Cs₂CO₃ (572 mg, 1.75 mmol, 5.0 equiv) in dry dimethylformamide (2.0 mL, 0.20 M). The resulting mixture is stirred at 80°C for 4 h. The mixture is diluted with DCM and water. The aqueous phase is extracted with DCM. The combined organic phase is filtered through a phase separator and concentrated under vacuum to afford the desired compound. LCMS: MW (calcd): 234; m/z MW (obsd): 235 (M+H).

### 1.20. Intermediate 36: 2-(3,3-difluoroazetidin-1-yl)-6-(trifluoromethyl)pyridin-4-amine

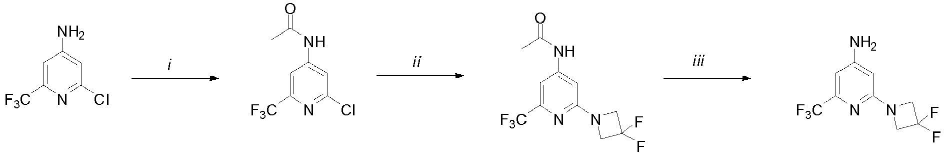

### 1.20.1. Step i: N-[2-chloro-6-(trifluoromethyl)-4-pyridyl]acetamide

Acetyl chloride (0.20 mL, 2.79 mmol, 1.1 equiv) is added to N-[2-chloro-6-(trifluoromethyl)-4-pyridyl]acetamide (500 mg, 2.54 mmol, 1.0 equiv), dimethylaminopyridine (31 mg, 0.254 mmol, 0.1 equiv) and trimethylamine (0.39 mL, 2.79 mmol, 1.1 equiv) at 0°C in dry DCM (6 mL, 0.4 M). The resulting mixture is stirred at room temperature for 20 h. The mixture is diluted with DCM and water. The organic phase is washed with saturated NaHCO₃ solution and filtered through a phase separator and concentrated under vacuum to afford the desired compound. LCMS: MW (calcd): 239; m/z MW (obsd): 239 (M+H).

### 1.20.2. Step ii: N-[2-(3,3-difluoroazetidin-1-yl)-6-(trifluoromethyl)-4-pyridyl]acetamide

Pd₂(dba)₃ (19 mg, 0.051 mmol, 0.05 equiv) and Xantphos (24 mg, 0.042 mmol, 0.1 equiv) are added to a degassed suspension of N-[2-chloro-6-(trifluoromethyl)-4-pyridyl]acetamide (100 mg, 0.419 mmol, 1.0 equiv), 3,3-difluoroazetidine (54 mg, 0.419 mmol, 1.0 equiv) and Cs₂CO₃ (410 mg, 1.26 mmol, 3.0 equiv) in dry 1,4-dioxane (1.7 mL, 0.25 M). The resulting mixture is stirred at 85°C for 4 h. The mixture is diluted with DCM and water. The aqueous phase is extracted with DCM. The combined organic phase is filtered through a phase separator and concentrated under vacuum to afford the desired compound. LCMS: MW (calcd): 295; m/z MW (obsd): 296 (M+H).

### 1.20.3. Step iii: 2-(3,3-difluoroazetidin-1-yl)-6-(trifluoromethyl)pyridin-4-amine

N-[2-(3,3-difluoroazetidin-1-yl)-6-(trifluoromethyl)-4-pyridyl]acetamide (0.419 mmol, 1.0 equiv) is stirred at 50°C in sodium hydroxide (0.8 mL, 2.0 equiv, 1M in H₂O) and ethanol (1.5 mL, 0.25 M) for 24 h. The mixture is diluted with DCM and 1 M HCl solution is added. The two phases are separated, the aqueous phase is basified and extracted with DCM/ iPrOH (4/1). The combined organic phase is filtered through a phase separator and concentrated under vacuum to afford the desired compound. LCMS: MW (calcd): 253; m/z MW (obsd): 254 (M+H).

### 1.21. Intermediate 37: 2-(3,3-difluoroazetidin-1-yl)-6-(2,2-difluoroethoxy)pyridin-4-amine

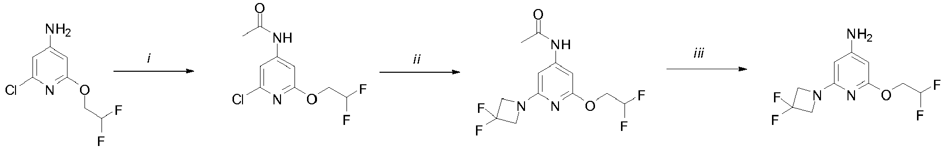

### 1.21.1. Step i: N-[2-chloro-6-(2,2-difluoroethoxy)-4-pyridyl]acetamide

Acetyl chloride (0.19 mL, 2.64 mmol, 1.1 equiv) is added to 2-chloro-6-(2,2-difluoroethoxy)pyridin-4-amine (500 mg, 2.40 mmol, 1.0 equiv), dimethylaminopyridine (29 mg, 0.240 mmol, 0.1 equiv) and trimethylamine (0.37 mL, 2.64 mmol, 1.1 equiv) at 0°C in dry DCM (6 mL, 0.4 M). The resulting mixture is stirred at room temperature for 20 h. The mixture is diluted with DCM and water. The organic phase is washed with saturated NaHCO₃ solution and filtered through a phase separator and concentrated under vacuum to afford the desired compound. LCMS: MW (calcd): 251; m/z MW (obsd): 251 (M+H).

### 1.21.2. Step ii: N-[2-(3,3-difluoroazetidin-1-yl)-6-(2,2-difluoroethoxy)-4-pyridyl]acetamide

Pd₂(dba)₃ (37 mg, 0.010 mmol, 0.05 equiv) and Xantphos (46 mg, 0.080 mmol, 0.1 equiv) are added to a degassed suspension of N-[2-chloro-6-(2,2-difluoroethoxy)-4-pyridyl]acetamide (200 mg, 0.798 mmol, 1.0 equiv), 3,3-difluoroazetidine (103 mg, 0.798 mmol, 1.0 equiv) and Cs₂CO₃ (780 mg, 2.39 mmol, 3.0 equiv) in dry 1,4-dioxane (3.0 mL, 0.25 M). The resulting mixture is stirred at 85°C for 4 h. The mixture is diluted with DCM and water. The aqueous phase is extracted with DCM. The combined organic phase is filtered through a phase separator and concentrated under vacuum to afford the desired compound. LCMS: MW (calcd): 307; m/z MW (obsd): 308 (M+H).

### 1.21.3. Step iii: 2-(3,3-difluoroazetidin-1-yl)-6-(2,2-difluoroethoxy)pyridin-4-amine

N-[2-(3,3-difluoroazetidin-1-yl)-6-(2,2-difluoroethoxy)-4-pyridyl]acetamide (0.798 mmol, 1.0 equiv) is stirred at 50°C in sodium hydroxide (1.60 mL, 2.0 equiv, 1M in H₂O) and ethanol (3.2 mL, 0.25 M) for 24 h. The mixture is diluted with DCM and 1 M HCl solution is added. The two phases are separated, the aqueous phase is basified and extracted with DCM/ iPrOH (4/1). The combined organic phase is filtered through a phase separator and concentrated under vacuum to afford the desired compound. LCMS: MW (calcd): 265; m/z MW (obsd): 266 (M+H).

### 1.22. Intermediate 38: 6-chloro-5-ethoxy-pyridin-3-amine

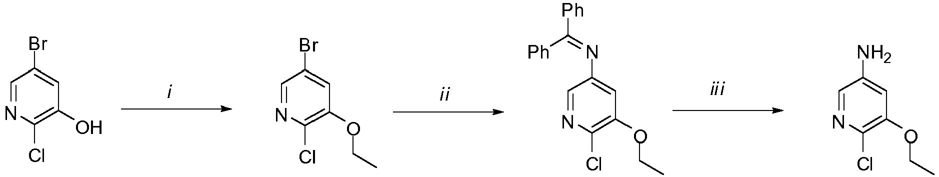

### 1.22.1. Step i: 5-bromo-2-chloro-3-ethoxy-pyridine

To 5-bromo-2-chloro-pyridin-3-ol (500 mg, 2.40 mmol, 1.0 equiv), potassium iodide (319 mg, 1.92 mmol, 0.8 equiv) and potassium carbonate (398 mg, 2.88 mmol, 1.2 equiv) in dry dimethylformamide (12 mL, 0.2 M), ethyl iodide is added (411.8 mg, 2.64 mmol, 1.1 equiv). The resulting mixture is stirred at 50°C for 20 h. The mixture is diluted with DCM and water. The aqueous phase is extracted with DCM. The combined organic phase is filtered through a phase separator and concentrated under vacuum to afford the desired compound. LCMS: MW (calcd): 237; m/z MW (obsd): 238 (M+H).

### 1.22.2. Step ii: N-(6-chloro-5-ethoxy-3-pyridyl)-1,1-diphenyl-methanimine

Palladium acetate (27 mg, 0.12 mmol, 0.05 equiv) is added to a degassed suspension of 5-bromo-2-chloro-3-ethoxy-pyridine (2.40 mmol, 1.0 equiv), benzophenone imine (435 mg, 2.40 mmol, 1.0 equiv), BINAP (2,2'-Bis(diphenylphosphino)-1,1'-binaphthyl) (112 mg, 0.18 mmol, 0.075 equiv) and *t*BuONa (299 mg, 3.12 mmol, 1.3 equiv) in dry toluene (10 mL, 0.25 M). The resulting mixture is stirred at 80°C for 3 h. The mixture is diluted with DCM and water. The aqueous phase is extracted with DCM. The combined organic phase is filtered through a phase separator and concentrated under vacuum to afford the desired compound. LCMS: MW (calcd): 337; m/z MW (obsd): 337 (M+H).

### 1.22.3. Step iii: 6-chloro-5-ethoxy-pyridin-3-amine

N-(6-chloro-5-ethoxy-3-pyridyl)-1,1-diphenyl-methanimine (2.40 mmol, 1.0 equiv) is stirred at room temperature in 2 M HCl solution (5 mL, 0.5M) and THF (5 mL, 0.5 M) for 30 min. The mixture is diluted with DCM and 1 M HCl solution is added. The two phases are separated and the aqueous phase is basified and extracted with DCM/ iPrOH (4/1). The combined organic phase is filtered through a phase separator and concentrated under vacuum. The crude is purified by flash chromatography on silica gel (eluting with petroleum ether/EtOAc 4/1) to afford the desired compound. LCMS: MW (calcd): 173; m/z MW (obsd): 173 (M+H).

### 1.23. Intermediate 39: 6-chloro-5-(2,2-difluoroethoxy)pyridin-3-amine

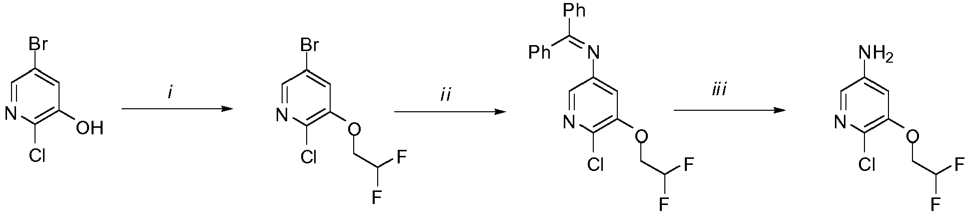

### 1.23.1. Step i: 5-bromo-2-chloro-3-(2,2-difluoroethoxy)pyridine

To 5-bromo-2-chloro-pyridin-3-ol (500 mg, 2.40 mmol, 1.0 equiv), potassium iodide (319 mg, 1.92 mmol, 0.8 equiv) and potassium carbonate (398 mg, 2.88 mmol, 1.2 equiv) in dry dimethylformamide (12 mL, 0.2 M), 2-Iodo-1,1-difluoroethane is added (506.7 mg, 2.64 mmol, 1.1 equiv). The resulting mixture is stirred at 50°C for 20 h. The mixture is diluted with DCM and water. The aqueous phase is extracted with DCM. The combined organic phase is filtered through a phase separator and concentrated under vacuum to afford the desired compound. LCMS: MW (calcd): 273; m/z MW (obsd): 273 (M+H).

### 1.23.2. Step ii: N-[6-chloro-5-(2,2-difluoroethoxy)-3-pyridyl]-1,1-diphenyl-methanimine

Palladium acetate (27 mg, 0.12 mmol, 0.05 equiv) is added to a degassed suspension of 5-bromo-2-chloro-3-(2,2-difluoroethoxy)pyridine (2.40 mmol, 1.0 equiv), benzophenone imine (435 mg, 2.40 mmol, 1.0 equiv), BINAP (2,2'-Bis(diphenylphosphino)-1,1'-binaphthyl) (112 mg, 0.18 mmol, 0.075 equiv) and *t*BuONa (299 mg, 3.12 mmol, 1.3 equiv) in dry toluene (10 mL, 0.25 M). The resulting mixture is stirred at 80°C for 3 h. The mixture is diluted with DCM and water. The aqueous phase is extracted with DCM. The combined organic phase is filtered through a phase separator and concentrated under vacuum to afford the desired compound. LCMS: MW (calcd): 373; m/z MW (obsd): 373 (M+H).

### 1.23.3. Step iii: 6-chloro-5-(2,2-difluoroethoxy)pyridin-3-amine

N-[6-chloro-5-(2,2-difluoroethoxy)-3-pyridyl]-1,1-diphenyl-methanimine (2.40 mmol, 1.0 equiv) is stirred at room temperature in 2 M HCl solution (5 mL, 0.5M) and THF (5 mL, 0.5 M) for 30 min. The mixture is diluted with DCM and 1 M HCl solution is added. The two phases are separated, the aqueous phase is basified and extracted with DCM/ iPrOH (4/1). The combined organic phase is filtered through a phase separator and concentrated under vacuum. The crude is purified by flash chromatography on silica gel (eluting with petroleum ether/EtOAc 4/1) to afford the desired compound. LCMS: MW (calcd): 209; m/z MW (obsd): 209 (M+H).

### 1.24. Intermediate 40: 6-chloro-2-ethoxy-pyrimidin-4-amine

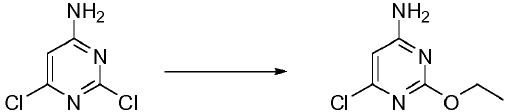

At 0°C, ethanol (0.13 mL, 2.28 mmol, 1.5 equiv) is slowly added to NaH (91 mg, 2.28 mmol, 1.5 equiv) in suspension in dry THF (4 mL, 0.4 M). The resulting mixture is stirred for 20 min at room temperature. 4-Amino-2,6-dichloropyrimidine (250 mg, 1.52 mmol, 1.0 equiv) is added, the resulting mixture is stirred at 50°C for 3 h. The mixture is diluted with DCM and water. The aqueous phase is extracted with DCM. The combined organic phase is filtered through a phase separator and concentrated under vacuum to afford the desired compound. LCMS: MW (calcd): 174; m/z MW (obsd): 174 (M+H).

### 1.25. Intermediate 41: 4-amino-6-ethoxy-5-methyl-pyridine-2-carbonitrile

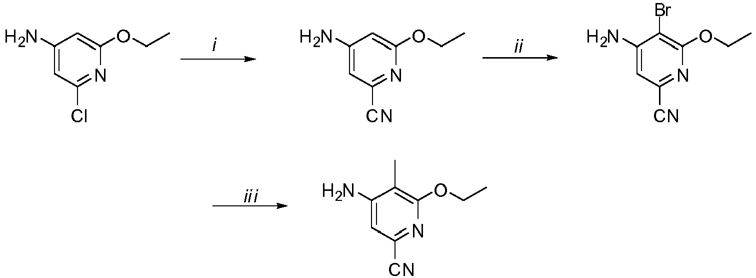

### 1.25.1. Step i: 4-amino-6-ethoxy-pyridine-2-carbonitrile

A degassed mixture of 2-chloro-6-ethoxy-pyridin-4-amine (1.0 eq, 500 mg), Tetrakis(triphenylphosphine)palladium(0) (0.1 eq, 335 mg) and zinc cyanide (1.0 eq, 340 mg) in anhydrous *N,N-*dimethylformamide (12 mL) is heated at 150 °C for 5 min under microwave irradiation. The reaction mixture is diluted with EtOAc and washed with a saturated solution of NaHCO₃. The organic layer is washed with brine, dried over sodium sulfate and concentrated in vacuo. The residue is purified by silica chromatography (petroleum ether/EtOAc: 100/0 to 80/20) to afford the desired compound. LCMS: MW (calcd): 163; m/z MW (obsd): 164 (M+H).

### 1.25.2. Step ii: 4-amino-5-bromo-6-ethoxy-pyridine-2-carbonitrile

A solution of *N*-bromosuccinimide (0.85 eq, 297 mg) in a mixture of anhydrous DCM (3 mL) and anhydrous acetonitrile (1.5 mL) is added dropwise at 0°C and under inert atmosphere to a solution of 4-amino-6-ethoxy-pyridine-2-carbonitrile (1.0 eq, 320 mg) in anhydrous DCM (7 mL). After 10 min at 0°C, the mixture is stirred at room temperature for 3 h. Then, a solution of *N*-bromosuccinimide (0.15 eq, 52 mg) is again added at 0°C to the mixture and the mixture is stirred at room temperature. When the reaction is complete, the mixture diluted with DCM and washed with water. The aqueous layer is backwashed with DCM. The combined organic layers are dried over sodium sulfate and are concentrated in vacuo to afford the desired compound. LCMS: MW (calcd): 242; m/z MW (obsd): 244 (M+H).

### 1.25.3. Step iii: 4-amino-6-ethoxy-5-methyl-pyridine-2-carbonitrile

A degassed mixture of 4-amino-5-bromo-6-ethoxy-pyridine-2-carbonitrile (1.0 eq, 250 mg), methyl boronic acid (3.0 eq, 185 mg), Cs₂CO₃ (3.0 eq, 1.68 g) and Pd(dppf)Cl₂ (0.1 eq, 84 mg) in anhydrous 1,4-dioxane (2.6 mL) is heated at 100 °C for 2 h. The mixture is diluted with DCM and water. The two phases are separated; the organic layer is filtered through a phase separator and concentrated. The residue is purified by silica chromatography (petroleum ether/EtOAc: 90/10 to 80/20) to afford the desired compound. LCMS: MW (calcd): 177; m/z MW (obsd): 178 (M+H).

### 1.26. Intermediate 42: 4-amino-6-ethoxy-5-methyl-pyridine-2-carbonitrile

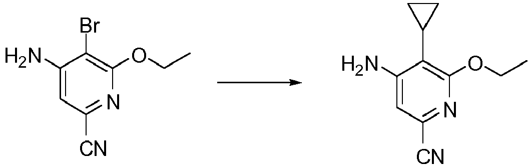

A degassed mixture of 4-amino-5-bromo-6-ethoxy-pyridine-2-carbonitrile (1.0 eq, 210 mg), cyclopropyl boronic acid (2.5 eq, 186 mg), K₃PO₄ (6.0 eq, 1.10 g), tricyclohexylphosphine (0.2 eq, 49 mg), and palladium(II) acetate (0.2 eq, 39 mg) in a mixture of toluene/water (10 mL/0.045 mL) is heated at 100 °C for 3 h. The mixture is diluted with EtOAc and water. The two phases are separated; the organic layer is dried over Na₂SO₄ and concentrated. The residue is purified by silica chromatography (petroleum ether/EtOAc: 65/35 to 50/50) to afford the desired compound. LCMS: MW (calcd): 203; m/z MW (obsd): 204 (M+H).

### 1.27. Intermediate 43: 4-amino-6-(2,2-difluoroethoxy)-5-methyl-pyridine-2-carbonitrile

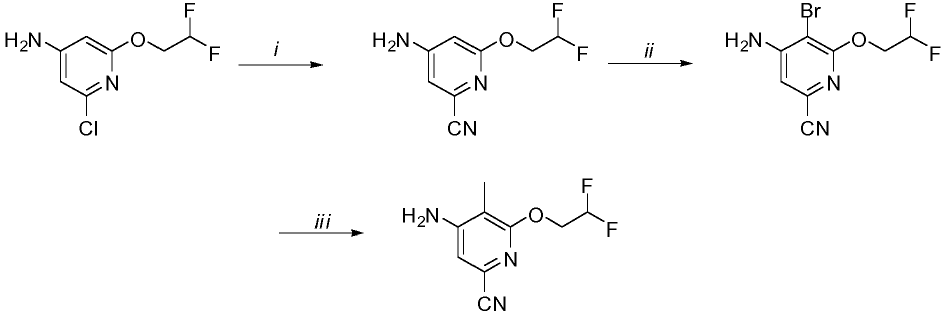

### 1.27.1. Step i: 4-amino-6-(2,2-difluoroethoxy)pyridine-2-carbonitrile

A degassed mixture of 2-chloro-6-(2,2-difluoroethoxy)pyridin-4-amine (1.0 eq, 450 mg), Tetrakis(triphenylphosphine)palladium(0) (0.1 eq, 249 mg) and zinc cyanide (1.0 eq, 253 mg) in anhydrous *N,N*-dimethylformamide (9 mL) is heated at 150 °C for 5 min under microwave irradiation. The reaction mixture is diluted with EtOAc and washed with a saturated solution of NaHCO₃. The organic layer is washed with brine, dried over sodium sulfate and concentrated in vacuo. The residue is purified by silica chromatography (petroleum ether/EtOAc: 100/0 to 80/20) to afford the desired compound. LCMS: MW (calcd): 199; m/z MW (obsd): 200 (M+H).

### 1.27.2. Step ii: 4-amino-5-bromo-6-(2,2-difluoroethoxy)pyridine-2-carbonitrile

A solution of *N*-bromosuccinimide (0.85 eq, 293 mg) in a mixture of anhydrous DCM (2.0 mL) and anhydrous acetonitrile (2.0 mL) is added dropwise at 0°C and under inert atmosphere to a solution of 4-amino-6-(2,2-difluoroethoxy)pyridine-2-carbonitrile (1.0 eq, 385 mg) in anhydrous DCM (6 mL). After 10 min at 0°C, the mixture is stirred at room temperature for 2 h. Then, a solution of *N*-bromosuccinimide (0.15 eq, 52 mg) is again added at 0°C to the mixture and the mixture is stirred at room temperature. When the reaction is complete, the mixture diluted with DCM and washed with water. The aqueous layer is backwashed with DCM. The combined organic layers are dried over sodium sulfate and are concentrated in vacuo to afford the desired compound. LCMS: MW (calcd): 278; m/z MW (obsd): 279 (M+H).

### 1.27.3. Step iii: 4-amino-6-(2,2-difluoroethoxy)-5-methyl-pyridine-2-carbonitrile

A degassed mixture of 4-amino-5-bromo-6-(2,2-difluoroethoxy)pyridine-2-carbonitrile (1.0 eq, 239 mg), methyl boronic acid (3.0 eq, 154 mg), Cs₂CO₃ (3.0 eq, 1.40 g) and Pd(dppf)Cl₂ (0.1 eq, 70 mg) in anhydrous 1,4-dioxane (2.2 mL) is heated at 100 °C for 2 h. The mixture is diluted with DCM and water. The two phases are separated; the organic layer is filtered through a phase separator and concentrated. The residue is purified by silica chromatography (petroleum ether/EtOAc: 90/10 to 80/20) to afford the desired compound. LCMS: MW (calcd): 278; m/z MW (obsd): 279 (M+H).

### 1.28. Intermediate 44: 6-chloro-2-(2,2-difluoroethoxy)-3-methyl-pyridin-4-amine

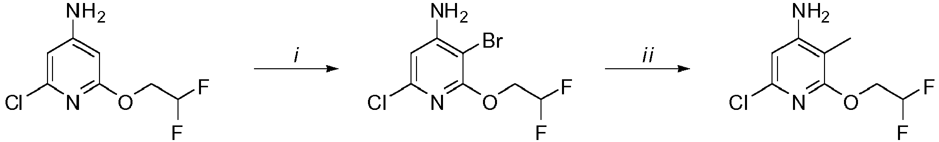

### 1.28.1. Step i: 3-bromo-6-chloro-2-(2,2-difluoroethoxy)pyridin-4-amine

To a solution of 2-chloro-6-(2,2-difluoroethoxy)pyridin-4-amine (100 mg, 0.48 mmol, 1.0 equiv) in dry DCM (2.5 mL) is added N-Bromosuccinimide (68 mg, 0.38 mmol, 0.8 equiv). The reaction is stirred at room temperature for 25 min. The mixture is diluted with DCM and washed with water and brine. The organic layer is filtered through a phase separator and concentrated in vacuo. LCMS: MW (calcd): 288; m/z MW (obsd): 289 (M+H).

### 1.28.2. Step ii: 6-chloro-2-(2,2-difluoroethoxy)-3-methyl-pyridin-4-amine

A degassed mixture of 3-bromo-6-chloro-2-(2,2-difluoroethoxy)pyridin-4-amine (138 mg, 0.48 mmol, 1.0 equiv), methylboronic acid (86 mg, 1.44 mmol, 3.0 equiv), Cs₂CO₃ (782 mg, 2.4 mmol, 5.0 equiv) and Pd(dppf)Cl₂.DCM (39 mg, 0.048 mmol, 0.1 equiv) in dry dioxane (1.2 mL) is heated at 100°C for 1 h. The reaction mixture is diluted with DCM and water. The organic layer is filtered through a phase separator and concentrated in vacuo. The residue is purified by flash chromatography (Petroleum ether/EtOAc 7:3) to afford the desired compound. LCMS: MW (calcd): 223; m/z MW (obsd): 223 (M+H).

### 1.29. Intermediate 45: 6-Chloro-4-methyl-pyridazin-3-ol

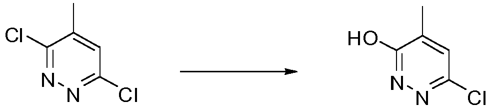

3,6-Dichloro-4-methyl-pyridazine (2.97 g, 18.2 mmol, 1 equiv) in aq. 3.3M NaOH (30 mL) is stirred at reflux temperature for 1 h. Once cooled at room temperature, aq. 50% acetic acid (10 mL) is added to pH∼6 and the resulting solid filtered and washed with water. Purification by silica chromatography (EtOAc/petroleum ether; 25:75 to 100:0) affords the desired compound. LCMS: MW (calcd): 145; m/z MW (obsd): 145 (M+H).

### 1.30. Intermediate 46: 6-Hydroxy-5-methyl-pyridazine-3-carbonitrile

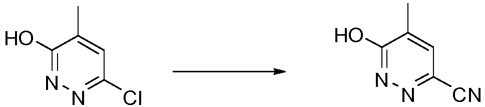

A mixture of 6-chloro-4-methyl-pyridazin-3-ol (21.7 g, 150 mmol, 1 equiv), Zn(CN)₂ (22.9 g, 195 mmol, 1.3 equiv), Pd₂dba₃ (6.87 g, 7.5 mmol, 0.05 equiv) and 1,1'-bis(diphenylphosphino)ferrocene (6.64 g, 12 mmol, 0.08 equiv) in DMF (150 mL) is degassed under nitrogen and heated at 120 °C for 1 h. The resulting mixture is diluted with DCM and aq. sat. NaHCO₃. The aqueous layer is separated and extracted with DCM, DCM/iPrOH (4:1), dried (Na₂SO₄), filtered and concentrated. The residue is triturated with a mixture of petroleum ether/EtOAc (3:1), filtered, washed with petroleum ether and dried to afford the desired compound. LCMS: MW (calcd): 135; m/z MW (obsd): 136 (M+H).

### 1.31. General method D: chlorination of 6-hydroxy-pyridazine derivative

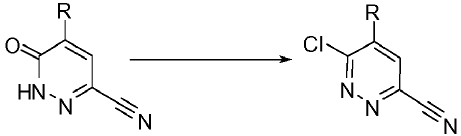

POCl₃ (3 to 6 equiv) is added to 6-hydroxy-pyridazine derivative (1 equiv) in acetonitrile (0.1 to 0.6 M) and the resulting mixture is heated at reflux temperature until completion of the reaction. The cooled mixture is concentrated under reduced pressure and the residue diluted in DCM. This solution is poured in a stirred mixture of ice cold DCM and aqueous NaHCO₃ until pH of approximately 7. Both layers are separated and the aqueous layer extracted with DCM. The combined organic layer is dried (Na₂SO₄), filtered and concentrated to afford the desired compound.

### 1.32. Illustrative example of method D: synthesis of intermediate 47, 6-chloro-5-methyl-pyridazine-3-carbonitrile

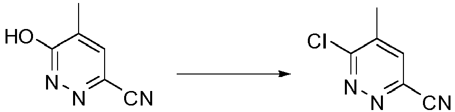

POCl₃ (18 mL, 193 mmol, 3 equiv) is added to 6-hydroxy-5-methyl-pyridazine-3-carbonitrile (8.7 g, 64 mmol, 1 equiv) in acetonitrile (120 mL) and the resulting mixture is heated at reflux temperature until completion of the reaction. The cooled mixture is concentrated under reduced pressure and the residue diluted in DCM. This solution is poured in a stirred mixture of ice cold DCM and aq. NaHCO₃. Solid NaHCO₃ is added in portions to attain a pH of approximately 7. Both layers are separated and the aqueous extracted with DCM. The combined organic layer is dried (Na₂SO₄), filtered and concentrated to afford the desired compound. LCMS: MW (calcd): 154; m/z MW (obsd): 154 (M+H).

### 1.33. General method E: Suzuki reaction on 6-chloro-pyridazine derivative

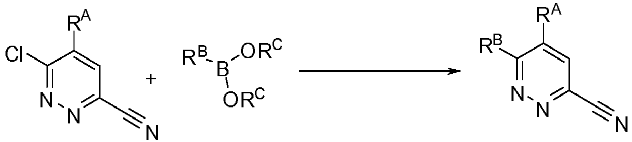

To a degassed solution of heteroaryl chloride (1.0 eq) and the boronic acid or ester (0.5 to 1.5 eq) in dry DMF (0.15 to 0.3 M), which is put under argon atmosphere, tetrakis(triphenylphosphine)palladium(0), Pd(PPh3)4 (0.1 eq) is added and the mixture is stirred at room temperature for 1 to 2 h. Sodium carbonate, Na₂CO₃, 2.5 N (2.5 to 4 eq) is added to the mixture. The mixture is stirred at 80 to 90 °C for approximately 16 h. The mixture is cooled to room temperature, diluted with water and extracted with an organic solvent. After work up, the organic layers are dried over Na₂SO₄ and concentrated. The crude is purified by flash column chromatography to afford the desired compound.

### 1.34. Illustrative example of method E: synthesis of intermediate 48, 6-Chloro-5-methyl-pyridazine-3-carbonitrile

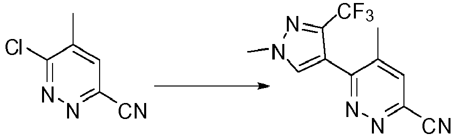

Pd(PPh₃)₄ (7.44 g, 6.64 mmol, 0.1 equiv) is added to a degassed suspension of 6-chloro-5-methylpyridazine-3-carbonitrile (64 mmol, 1.0 equiv), 1-methyl-4-(4,4,5,5-tetramethyl-[1,3,2]dioxaborolan-2-yl)-3-trifluoromethyl-1H-pyrazole (12.4 g, 45 mmol, 0.7 equiv) in DMF (275 mL) and the resulting mixture is stirred at room temperature for 1 h. A 2.5M aq. Na₂CO₃ solution (64 mL) is added and the resulting mixture is stirred at 85°C until completion. The cooled mixture is filtered on Celite and washed with EtOAc. The organic extract is washed 3 times with brine, dried (Na₂SO₄) and concentrated. Purification by silica chromatography (column: 120 g silica 25 µm; EtOAc/DCM; 0:100 to 10:90) affords the desired compound. LCMS: MW (calcd): 267; m/z MW (obsd): 268 (M+H).

### 1.35. General method F1: reduction of nitrile derivative.

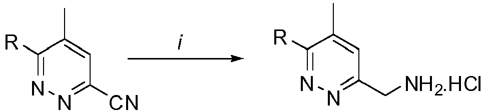

Pd (10% on charcoal, 0.15 equiv) is added to a degassed mixture of the nitrile derivative (1 equiv) and aq. 6M HCl (2.2 equiv) in methanol (0.1-0.3 M).The mixture is stirred at room temperature under hydrogen (1 atm) until completion of the reaction. The resulting mixture is filtered on Celite, washed with methanol, acetonitrile and the filtrate is concentrated under reduced pressure. The residue is triturated with Et₂O to afford the hydrochloride salt of the desired compound.

### 1.36. Illustrative example of method F1: synthesis of intermediate 49, 5-methyl-6-[1-methyl-3-(trifluoromethyl)pyrazol-4-yl]pyridazin-3-yl]methanamine HCl

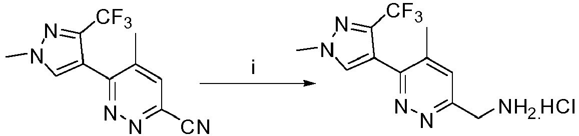

Pd (10% on charcoal, 4.25 g, 4 mmol, 0.15 equiv) is added to a degassed mixture of 5-methyl-6-(1-methyl-3-trifluoromethyl-1H-pyrazol-4-yl)-pyridazine-3-carbonitrile (7.3 g, 27.3 mmol, 1 equiv) and aq. 6M HCl (10 mL, 60 mmol, 2.2 equiv) in methanol (180 mL). The mixture is stirred at room temperature under hydrogen (1 atm) until completion of the reaction. The resulting mixture is filtered on Celite, washed with methanol, acetonitrile and the filtrate is concentrated under reduced pressure. The residue is triturated with Et₂O to afford the hydrochloride salt of the desired compound. LCMS: MW (calcd): 271; m/z MW (obsd): 272 (M+H).

### 1.37. General method F2: reduction of nitrile derivative.

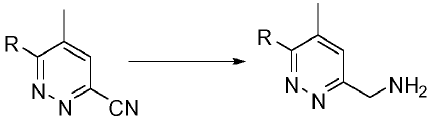

NaBH₄ (5 equiv) is added portionwise to a mixture of the nitrile derivative (1.0 equiv), TFA (5 equiv) and NiCl₂.6H₂O (0.4 equiv) in methanol (0.2-0.3 M) at 0°C. After 3 h, NaBH₄ (2.5 equiv) is added. The resulting mixture is stirred at room temperature for 3 h. The mixture is filtered over celite and concentrated under reduced pressure. Purification by SCX column chromatography affords the desired compound. Alternatively, the reaction mixture is diluted in DCM and compound is purified by extraction into a 1 M HCl solution. The aqueous solution is basified and extracted with 4:1 DCM / iPrOH and filtered through a phase separator. Concentration in *vacuum* gives the desired product.

### 1.38. Illustrative example of method F2: synthesis of intermediate 50, 5-methyl-6-[1-methyl-3-(trifluoromethyl)pyrazol-4-yl]pyridazin-3-yl]methanamine

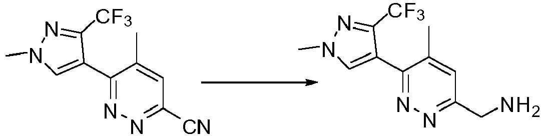

NaBH₄ (462 mg, 12.2 mmol, 5 equiv) is added portionwise to a mixture of 5-methyl-6-(1-methyl-3-trifluoromethyl-1H-pyrazol-4-yl)-pyridazine-3-carbonitrile (2.44 mmol, 1.0 equiv), TFA (0.94 mL, 12.2 mmol, 5 equiv) and NiCl₂.6H₂O (233 mg, 0.98 mmol, 0.4 equiv) in methanol (10 mL) at 0°C. After 3 h, NaBH₄ (230 mg, 6.1 mmol, 2.5 equiv) is added. The resulting mixture is stirred at room temperature for 3 h. The mixture is filtered over celite and concentrated under reduced pressure. Purification by SCX column chromatography affords the desired compound. LCMS: MW (calcd): 271; m/z MW (obsd): 272 (M+H).

### 1.39. General method F3: reduction of nitrile derivative.

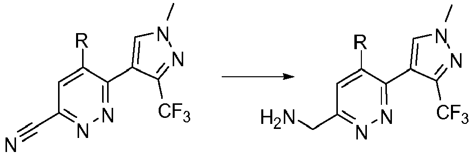

To a solution of nitrile derivative (1 eq) in methanol (0.15 M), HCl 6.0 N (2.5 eq) is added followed by the addition of Pd/C (0.1 eq). Reaction mixture is stirred under hydrogen (1 atm) at room temperature. After 1 h the reaction mixture is filtered through Celite and the pad is further washed with methanol. The filtrate is loaded onto an SCX column. Methanol is passed through the column and the compound is eluted with 7 N NH₃ in methanol /methanol 1:4. The filtrate is concentrated under reduced pressure to give the desired compound.

### 1.40. Illustrative example of method F3: synthesis of intermediate 51, [5-cyclopropyl-6-[1-methyl-3-(trifluoromethyl)pyrazol-4-yl]pyridazin-3-yl]methanamine

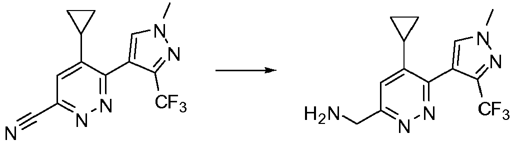

A degassed suspension of 5-cyclopropyl-6-[1-methyl-3-(trifluoromethyl)pyrazol-4-yl]pyridazine-3-carbonitrile (67 mg, 1.0 eq, 0.228 mmol, 100 %), palladium on charcoal, 10 %, Pd/C (25 mg, 0.1 eq, 98 %) and hydrochloric acid, 6N water solution (84 µL, 2.2 eq) in 3 mL of methanol is stirred under hydrogen atmosphere for 90 min. The mixture is filtered over a celite pad and the pad is rinsed with 10 mL of methanol. Methanol solution is applied on a 2 g SCX column and the column is washed with 10 mL of methanol. The compound is washed from the column with IN NH₃ (Methanol) solution. The solvent is evaporated to afford the desired compound. LCMS: MW (calcd): 297; m/z MW (obsd): 299 (M+H).

### 1.41. General method F4: reduction of nitrile derivative.

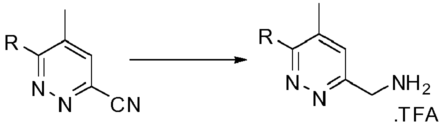

NaBH₄ (5 equiv) is added portionwise to a mixture of the nitrile derivative (1.0 equiv), TFA (5 equiv) and NiCl₂.6H₂O (0.4 equiv) in methanol (0.1 M) at 0°C. After 3 h, NaBH₄ (2.5 equiv) is added. The resulting mixture is stirred at room temperature for 3 h. The mixture is filtered over celite and concentrated under reduced pressure.

### 1.42. Illustrative example of method F4: synthesis of intermediate 135, C-[6-(3,3-Difluoro-pyrrolidin-1-yl)-5-methyl-pyridazin-3-yl]-methylamine hydrochloride salt

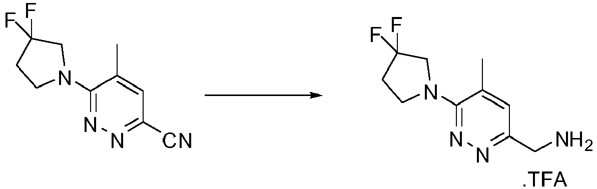

NaBH₄ (462 mg, 12.2 mmol, 5 equiv) is added portionwise to a mixture of 6-(3,3-difluoro-pyrrolidin-1-yl)-5-methyl-pyridazine-3-carbonitrile (0.38 mmol, 1.0 equiv), TFA (0.13 mL, 1.7 mmol, 5 equiv) and NiCl₂.6H₂O (36 mg, 0.15 mmol, 0.4 equiv) in methanol (4 mL) at 0°C. After 3 h, NaBH₄ (36 mg, 0.95 mmol, 2.5 equiv) is added. The resulting mixture is stirred at room temperature for 3 h. The mixture is filtered over celite and concentrated under reduced pressure to afford the desired compound. LCMS: MW (calcd): 228; m/z MW (obsd): 229 (M+H).

### 1.43. Intermediate 52: 1-[4-iodo-3-(trifluoromethyl)pyrazol-1-yl]propan-2-ol

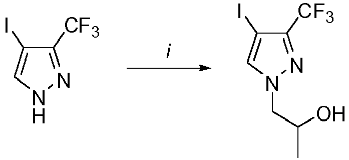

To a solution of 4-iodo-3-trifluoromethyl-1H-pyrazole (1 g, 3.8 mmol, 1.0 equiv) in dry THF (6 mL) at 0°C is added K₂CO₃ (1.6 g, 11.4 mmol, 3.0 equiv). The reaction is stirred at room temperature for 10 min before adding 1-chloropropan-2-ol (1.1 g, 11.4 mmol, 3.0 equiv). The reaction is heated at 80°C overnight. The mixture is diluted with EtOAc and washed with brine. The organic layer is dried over sodium sulfate, filtered and concentrated in vacuo. The residue is purified by automated flash chromatography by using Petroleum ether/EtOAc 80/20 to 0/100) to obtain the desired compound. LCMS: MW (calcd): 320; m/z MW (obsd): 321 (M+H).

### 1.44. General method F4: reduction of nitrile derivative.

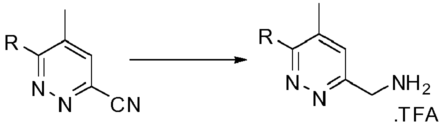

NaBH₄ (5 equiv) is added portionwise to a mixture of the nitrile derivative (1.0 equiv), TFA (5 equiv) and NiCl₂.6H₂O (0.4 equiv) in methanol (0.1 M) at 0°C. After 3 h, NaBH₄ (2.5 equiv) is added. The resulting mixture is stirred at room temperature for 3 h. The mixture is filtered over celite and concentrated under reduced pressure.

### 1.45. Illustrative example of method F4: synthesis of intermediate 135, C-[6-(3,3-Difluoro-pyrrolidin-1-yl)-5-methyl-pyridazin-3-yl]-methylamine hydrochloride salt

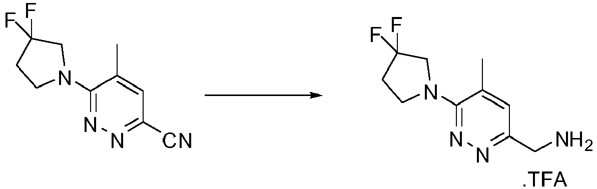

NaBH₄ (462 mg, 12.2 mmol, 5 equiv) is added portionwise to a mixture of 6-(3,3-difluoro-pyrrolidin-1-yl)-5-methyl-pyridazine-3-carbonitrile (0.38 mmol, 1.0 equiv), TFA (0.13 mL, 1.7 mmol, 5 equiv) and NiCl₂.6H₂O (36 mg, 0.15 mmol, 0.4 equiv) in methanol (4 mL) at 0°C. After 3 h, NaBH₄ (36 mg, 0.95 mmol, 2.5 equiv) is added. The resulting mixture is stirred at room temperature for 3 h. The mixture is filtered over celite and concentrated under reduced pressure to afford the desired compound. LCMS: MW (calcd): 228; m/z MW (obsd): 229 (M+H).

### 1.46. Intermediate 53: 1-(1,4-dioxan-2-ylmethyl)-4-iodo-3-(trifluoromethyl)pyrazole

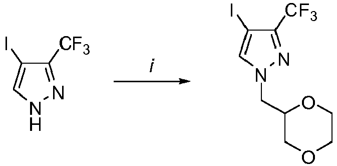

To a solution of 4-iodo-3-trifluoromethyl-1H-pyrazole (350 mg, 1.3 mmol, 1.0 equiv) in dry DMF (2 mL) at 0°C is added K₂CO₃ (359 mg, 2.6 mmol, 2.0 equiv). The reaction is stirred at room temperature for 10 min before adding 2-(chloromethyl)-1,4-dioxane (355 mg, 2.6 mmol, 2.0 equiv). The reaction is heated at 80°C over the weekend. The mixture is diluted with EtOAc and washed with brine. The organic layer is dried over sodium sulfate, filtered and concentrated in vacuo. The residue is purified by automated flash chromatography by using petroleum ether / EtOAc 90/10 to 0/100) to obtain the desired compound. LCMS: MW (calcd): 362; m/z MW (obsd): 363 (M+H).

### 1.47. Intermediate 54: 4-iodo-1-(2-methoxyethyl)-3-(trifluoromethyl)pyrazole

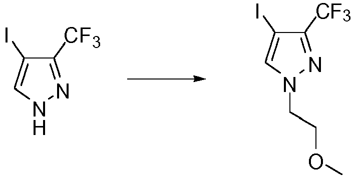

To a solution of 4-iodo-3-trifluoromethyl-1H-pyrazole (1 g, 3.8 mmol, 1.0 equiv) in dry THF (6 mL) at 0°C is added K₂CO₃ (1.3 g, 9.5 mmol, 2.5 equiv). The reaction is stirred at room temperature for 10 min before adding 2-chloroethyl methyl ether (0.87 mL, 9.5 mmol, 2.5 equiv). The reaction is heated at 80°C over the weekend. The mixture is diluted with EtOAc and washed with brine. The organic layer is dried over sodium sulfate, filtered and concentrated in vacuo. The residue is purified by automated flash chromatography by using petroleum ether/EtOAc 90/10 to 0/100) to obtain the desired compound. LCMS: MW (calcd): 320; m/z MW (obsd): 321 (M+H).

### 1.48. Intermediate 55: 4-iodo-1-tetrahydrofuran-3-yl-3-(trifluoromethyl)pyrazole

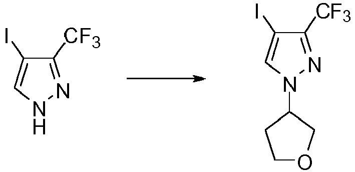

To a solution of 4-iodo-3-trifluoromethyl-1H-pyrazole (1 g, 3.8 mmol, 1.0 equiv) in dry DMF (6 mL) at 0°C is added sodium hydride (228 mg, 5.7 mmol, 1.5 equiv). The reaction is stirred at room temperature for 20 min before adding 3-furanol-tetrahydromethanesulfonate (947 mg, 5.7 mmol, 1.5 equiv). The reaction is heated at 80°C for 2h. The mixture is diluted with EtOAc and washed with brine. The organic layer is dried over sodium sulfate, filtered and concentrated in vacuo. The residue is purified by automated flash chromatography by using petroleum ether/EtOAc 90/10 to 0/100) to obtain the desired compound. LCMS: MW (calcd): 332; m/z MW (obsd): 333 (M+H).

### 1.49. General method G: synthesis of boronic esters from pyrazole derivative

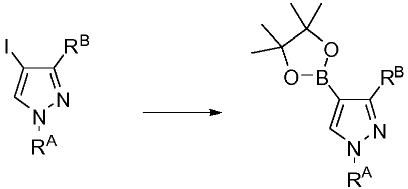

To an oven dried microwave tube charged with lithium chloro(*iso*propyl)magnesium chloride (1.3 M in THF, 1.8 equiv), a solution of the iodo-derivative (1.0 equiv) in dry THF (2 M) is added dropwise at 0°C under nitrogen. The reaction is stirred at room temperature. After 24h, the reaction mixture is cooled to -20°C then 2-*iso*propoxy-4,4,5,5-tetramethyl-1,3,2-dioxa-borolane (2.4 equiv) is added. The cold bath is removed and the reaction is stirred at room temperature for 2 h followed by the addition of a saturated solution of ammonium chloride. The mixture is diluted with EtOAc. The organic layer is dried on sodium sulfate, filtered then concentrated in vacuo. The residue is dissolved in Et₂O and washed with a solution of IN NaOH till pH 10. The organic layer is back-extracted several times with IN NaOH solution. The combined aqueous layers are acidified with 1M HCl till pH 2-3 then extracted with Et₂O (4x50 mL). The resulting organic layer is dried on sodium sulfate, filtered then concentrated in vacuo to yield the desired compound.

### 1.50. Illustrative example of method G: synthesis of intermediate 56, 1-[4-(4,4,5,5-tetramethyl-1,3,2-dioxaborolan-2-yl)-3-(trifluoromethyl)pyrazol-1-yl]propan-2-ol

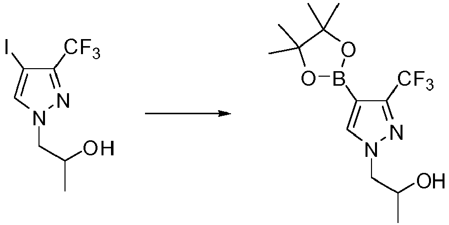

To an oven dried microwave tube charged with lithium chloro(*iso*propyl)magnesium chloride (1.3 M in THF, 3.1 mL, 4.05 mmol, 1.8 equiv), a solution of 1-[4-iodo-3-(trifluoromethyl)pyrazol-1-yl]propan-2-ol (720 mg, 2.25 mmol, 1.0 equiv) in dry THF (1 mL) is added dropwise at 0°C under nitrogen. The reaction is stirred at room temperature. After 24h, the reaction mixture is cooled to -20°C then 2-*iso*propoxy-4,4,5,5-tetramethyl-1,3,2-dioxa-borolane (1 mL, 5.4 mmol, 2.4 equiv) is added. The cold bath is removed and the reaction is stirred at room temperature for 2 h followed by the addition of a saturated solution of ammonium chloride (3 mL). The mixture is diluted with EtOAc (100 mL). The organic layer is dried on sodium sulfate, filtered then concentrated in vacuo. The residue is redissolved in Et₂O (50 mL) and washed with a solution of IN NaOH till pH 10. The organic layer is back-washed several times with IN NaOH solution. The combined aqueous layers are acidified with 1M HCl till pH2-3 then extracted with Et₂O (4x50 mL). The resulting organic layer is dried on sodium sulfate, filtered then concentrated in vacuo to yield the desired compound which is used as such in the next step. LCMS: MW (calcd): 320; m/z MW (obsd): 321 (M+H).

### 1.51. Intermediate 74: 1-[5-methyl-6-[1-methyl-3-(trifluoromethyl)pyrazol-4-yl]pyridazin-3-yl]ethanamine

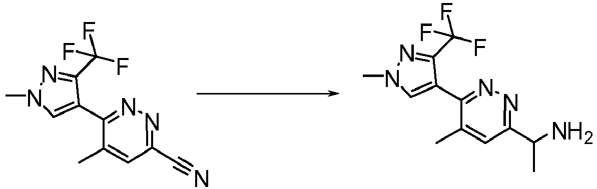

### Step i: 1-[5-methyl-6-[1-methyl-3-(trifluoromethyl)pyrazol-4-yl]pyridazin-3-yl]ethanamine

To a solution of 1 (100.0 mg, 0.374 mmol) in dry THF (1.40 mL), MeMgBr (3.0 M in Et₂O, 137.2 µL, 0.412 mmol) is added dropwise at - 20°C. The resulting mixture is stirred for 30 min at room temperature. The suspension is then treated with methanol (1.40 mL) and NaBH4 (34.0 mg, 0.898 mmol) and left to stir at room temperature. After 90 min the reaction mixture is diluted with 1.0 N NaOH (1.4 mL), and extracted with a mixture of DCM/i-PrOH (2:1) (3×15 mL). The combined organic layers are dried over Na₂SO₄ and concentrated under reduced pressure. The crude is purified by flash column chromatography (SiO₂, DCM / methanol / NH₄OH 100:0:0 to 93:6.5:1) to afford the desired compound. LCMS: MW (calcd): 285; m/z MW (obsd): 286 (M+H).

### 1.52. Intermediate 75: methyl 5-bromo-6-oxo-1H-pyridazine-3-carboxylate

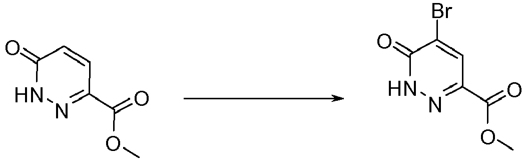

To a solution of methyl 6-oxo-1H-pyridazine-3-carboxylate (2.59 g, 1.0 eq, 16.64 mmol, from Combi-Blocks) and potassium acetate, KOAc (6.60 g, 4.0 eq) in 50 mL of acetic acid, glacial, cooled at 0 °C, bromine, Br2 (2.54 mL, 2.96 eq), is added. After the addition the mixture is stirred at 80 °C for 5 h. The mixture is poured on 200 mL of saturated Na₂S₂O₃ water solution. The mixture is extracted with EtOAc (10 % THF, 3 times, 150 mL in total). The gathered organic layers are washed with 200 mL of 0.01 N HCl water solution and 200 mL of brine and dried over Na₂SO₄. After filtration the solvent is evaporated and the resulting crude is purified by flash column chromatography (SiO₂, DCM / EtOAc 95:5 to 0:100) to afford the desired compound. LCMS: MW (calcd): 233; m/z MW (obsd): 235 (M+H).

### 1.53. Intermediate 76: 5-bromo-6-oxo-1H-pyridazine-3-carboxamide

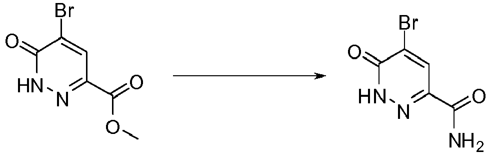

Methyl 5-bromo-6-oxo-1H-pyridazine-3-carboxylate (2.48 g, 1.0 eq, 10.64 mmol, 99 %) is stirred in ammonia 25 % water solution (30 mL) for 16 h (overnight). Volatiles are evaporated to dryness. The residue is suspended in methanol and the solvent is evaporated once again to afford the desired compound. LCMS: MW (calcd): 218; m/z MW (obsd): 220 (M+H).

### 1.54. Intermediate 77: 5-bromo-6-oxo-1H-pyridazine-3-carbonitrile

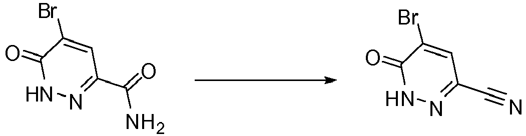

To a solution of 5-bromo-6-oxo-1H-pyridazine-3-carboxamide (2.32 g, 1.0 eq, 10.64 mmol) in 25 mL of dry DMF, cooled at 0 °C, is added phosphorus(V) oxychloride, POCl₃ (2.0 mL, 2.0 eq) and the mixture is stirred at room temperature for 1 h and quenched by pouring it on 200 mL of ice. Using 50 % NaOH water solution pH of the mixture is adjusted to around 2.5. The mixture is extracted with EtOAc (10 % THF, twice, 300 mL use in total). The gathered organic layers are washed with 200 mL of HCl (around pH 3) water solution and 200 mL of brine and dried over Na₂SO₄ and concentrated to afford the desired compound. LCMS: MW (calcd): 200; m/z MW (obsd): 198 (M-H).

### 1.55. Intermediate 78: 5-bromo-6-oxo-1-(2-trimethylsilylethoxymethyl)pyridazine-3-carbonitrile

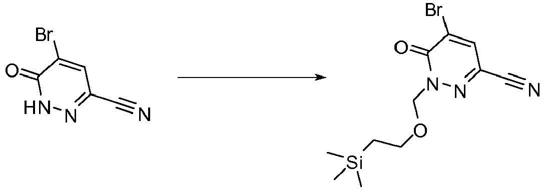

To a stirred solution of 5-bromo-6-oxo-1H-pyridazine-3-carbonitrile (270 mg, 1.0 eq, 1.24 mmol, 91 %) in 4 mL of dry DMF, cooled at 0 °C is added sodium hydride, 60 % mineral oil dispersion (65 mg, 1.1 eq, 60 %) and the mixture is warmed-up to room temperature within 15 min and is stirred for 30 min. The mixture is then cooled to 0 °C and 2-(chloromethoxy)ethyl-trimethyl-silane (245 µL, 1.1 eq) is added. After the addition the mixture is left to be warmed-up to room temperature and it is stirred for approximately 16 h. The reaction mixture is poured into 60 mL of water and extracted with Et₂O (2x40 mL). The gathered organic layers are washed with water (100 mL) and brine (100 mL). The organic layer is dried over Na₂SO₄ and concentrated. The crude is purified by flash column chromatography (SiO₂, cyclohexane / EtOAc 100:0 to 0:100) to afford the desired compound.

### 1.56. Intermediate 79: 5-cyclopropyl-6-oxo-1-(2-trimethylsilylethoxymethyl)pyridazine-3-carbonitrile

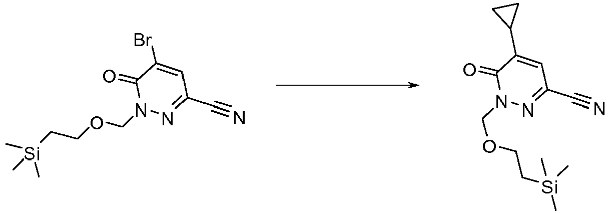

A degassed solution of 5-bromo-6-oxo-1-(2-trimethylsilylethoxymethyl)pyridazine-3-carbonitrile (700 mg, 1.0 eq, 2.01 mmol, 95 %), potassium phosphate, tribasic, K3PO4 (880 mg, 2.0 eq), cyclopropylboronic acid (273 mg, 1.5 eq, 95 %), tricyclohexylphosphine (91 mg, 0.15 eq) and Pd(OAc)₂ (37 mg, 0.08 eq) in a toluene (10 mL)/water (1 mL) solvent mixture is stirred in a sealed vial, under argon atmosphere, at 100 °C for approximately 24 h. The hot reaction is filtered over a celite pad and the pad is rinsed with 50 mL of EtOAc. To obtained organic solution is then washed with 70 mL of water and 70 mL of brine, dried over Na₂SO₄ and concentrated. The crude is purified by flash column chromatography (SiO₂, cyclohexane / EtOAc 100:0 to 85:15) to afford the desired compound.

### 1.57. Intermediate 80: 5-cyclopropyl-6-oxo-1H-pyridazine-3-carbonitrile

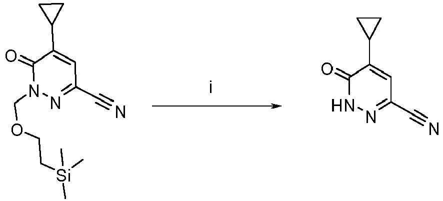

To a solution of 5-cyclopropyl-6-oxo-1-(2-trimethylsilylethoxymethyl)pyridazine-3-carbonitrile (260 mg, 1.0 eq, 0.874 mmol, 98 %) in 10 mL of dry DCM is added TFA, TFA (1.35 mL, 20 eq) and the mixture is stirred at room temperature for approximately 60 h. The reaction is concentrated and the residue taken up in 15 mL of water. The mixture is extracted with 2x15 mL of EtOAc. The gathered organic layers are dried over Na₂SO₄ and concentrated. The residue is dissolved in 5 mL of dry methanol and to the solution is added 1 mL of 7 N NH₃ in methanol solution. The reaction mixture is stirred for 2 h at room temperature. Volatiles are evaporated and the residue is suspended in 10 mL of 0.01 N HCl. The mixture is extracted with DCM (2x10 mL). The gathered organic layers are dried over Na₂SO₄. Filtration and evaporation of the solvent affords the desired compound. LCMS: MW (calcd): 161; m/z MW (obsd): 162 (M+H).

### 1.58. Intermediate 83: 5-(bromomethyl)-6-[1-methyl-3-(trifluoromethyl)pyrazol-4-yl]pyridazine-3-carbonitrile

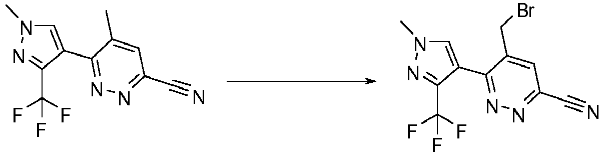

To a solution of 5-methyl-6-[1-methyl-3-(trifluoromethyl)pyrazol-4-yl]pyridazine-3-carbonitrile (300 mg, 1.0 eq, 1.11 mmol) in 6 mL of dry carbon tetrachloride, CCl₄ is added N-bromosuccinimide, NBS (204 mg, 1.0 eq) followed by 2,2'-azobis(2-methylpropionitrile), AIBN (19 mg, 0.1 eq). The mixture is flushed with argon and stirred in a sealed vial at 115 °C for 6 h. The reaction is diluted with 20 mL of DCM and poured in 30 mL of water. Using phase separator cartridge layers are separated. Solvent from the organic layer is evaporated. The resulting crude is purified by flash column chromatography (SiO₂, DCM / EtOAc100:0 to 80:20) to afford the desired compound. LCMS: MW (calcd): 346; m/z MW (obsd): 346 (M+H).

### 1.59. Intermediate 84: 5-(dimethylaminomethyl)-6-[1-methyl-3-(trifluoromethyl)pyrazol-4-yl]pyridazine-3-carbonitrile

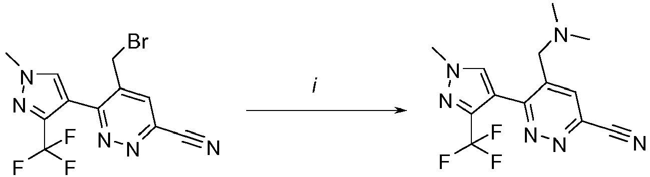

To a solution of 5-(bromomethyl)-6-[1-methyl-3-(trifluoromethyl)pyrazol-4-yl]pyridazine-3-carbonitrile (100 mg, 1.0 eq, 0.286 mmol, 90 %) in 1.0 mL of dry THF dimethylamine, 2N THF solution (429 µL, 3.0 eq, 99 %) is added and the mixture is stirred in a sealed vial at room temperature for 30 min. Volatiles are evaporated and the rest is portioned between DCM (20 mL) and saturated Na₂CO₃ water solution (20 mL). Extraction is performed once more with additional 15 mL of DCM. The gathered organic layers are dried over Na₂SO₄ and concentrated. The organic layers are concentrated and the residue purified by flash column chromatography (SiO₂, DCM / methanol 100:0 to 93.5:6.5) to afford the desired compound. LCMS: MW (calcd): 310; m/z MW (obsd): 311 (M+H).

### 1.60. Intermediate 86: 5-hydroxy-6-oxo-1-(2-trimethylsilylethoxymethyl)pyridazine-3-carbonitrile

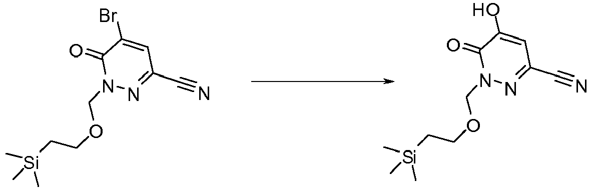

A degassed solution of 5-bromo-6-oxo-1-(2-trimethylsilylethoxymethyl)pyridazine-3-carbonitrile (700 mg, 1.0 eq, 2.01 mmol, 95 %), potassium acetate, KOAc (598 mg, 3.0 eq, 99 %), bis(pinacolatodiboron) (547 mg, 1.05 eq, 98 %) and [1,1'-bis(diphenylphosphino)ferrocene]dichloropalladium(II), complex with DCM, PdCl₂(dppf).DCM (83 mg, 0.05 eq) in 12 mL of dry 1,4-dioxane is put under argon atmosphere and stirred in a sealed vial at 90 °C for 2.0 h. The reaction mixture is cooled to 0 °C. Hydrogen peroxide, 30 % water solution (716 µL, 3.5 eq) is added. The reaction is left to warm up to room temperature and stirred for 90 min and diluted with 80 mL of EtOAc and 100 mL of water. The layers are separated and the water layer is extracted with 35 mL of EtOAc which is gathered with the first organic layer. The resulting organic phase is washed with 80 mL of brine and dried over Na₂SO₄. The solvent is removed to afford the desired compound. LCMS: MW (calcd): 267; m/z MW (obsd): 266 (M-H).

### 1.61. Intermediate 87: 5-methoxy-6-oxo-1-(2-trimethylsilylethoxymethyl)pyridazine-3-carbonitrile

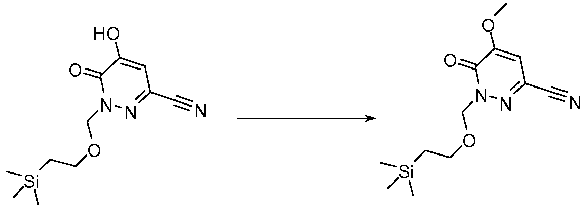

Iodomethane (128 µL, 1.1 eq, 99 %) is added to a suspension of 5-hydroxy-6-oxo-1-(2-trimethylsilylethoxymethyl)pyridazine-3-carbonitrile (440 mg, 1.0 eq, 0.90 mmol) and potassium carbonate, K₂CO₃ (263 mg, 1.3 eq, 99 %) in 5 mL of dry DMF and the suspension is stirred in a sealed vial at 65 °C for 1 h. The reaction mixture is cooled to room temperature and poured into 70 mL of water. The mixture is extracted with EtAOc (2x30 mL). The gathered organic layers are washed with 40 mL of water and 40 mL of brine. After drying over Na₂SO₄ and evaporation of the solvent the resulting crude is purified by flash column chromatography (SiO₂, cyclohexane / EtOAc 100:0 to 50:50) to afford the desired compound.

### 1.62. Intermediate 88: 5-methoxy-6-oxo-1H-pyridazine-3-carbonitrile

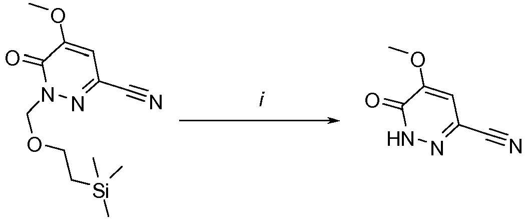

To a solution of 5-methoxy-6-oxo-1-(2-trimethylsilylethoxymethyl)pyridazine-3-carbonitrile (248 mg, 1.0 eq, 0.881 mmol, 100 %) in 10 mL of dry DCM is added TFA (1.02 mL, 15 eq) and the mixture is stirred at room temperature for 4 h. Volatiles are evaporated. Dry methanol (10 mL) and IN NH₃ in methanol (1 mL) are added to the residue. The mixture is stirred at room temperature for 2 h. The volatiles are evaporated. The residue is suspended in 7 mL of Et₂O and after evaporation the desired compound is obtained. LCMS: MW (calcd): 151; m/z MW (obsd): 152 (M+H).

### 1.63. General method G: S_{N}Ar reaction on bromo-hydroxy-pyridazine derivative

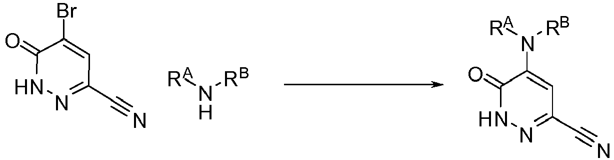

A solution of 5-bromo-6-oxo-1H-pyridazine-3-carbonitrile (1 eq) triethylamine, TEA (3.5 eq) and the appropriate amine (free base or HCl salt, 1.5 to 2 eq) in dry DMF or THF (0.3 M) is stirred in a sealed vial at 80 °C for 2 h. The mixture is cooled to room temperature and poured into water. Using IN HCl water solution pH of the solution is adjusted to around 4.5. The aqueous solution is extracted with an organic solvent. After work up, the organic solution is dried over Na₂SO₄ and the solvent is removed to afford the desired compound.

### 1.64. Illustrative example of method G: synthesis of intermediate 93, 6-chloro-5-(3,3-difluoroazetidin-1-yl)pyridazine-3-carbonitrile

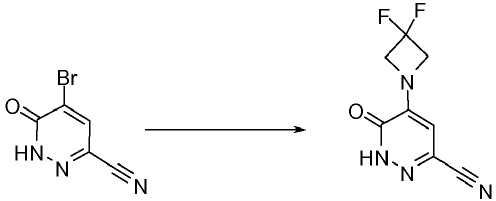

A solution of 5-bromo-6-oxo-1H-pyridazine-3-carbonitrile (300 mg, 1.0 eq, 1.35 mmol, 90 %), triethylamine, TEA (666 µL, 3.5 eq, 99 %) and 3,3-difluoroazetidine hydrochloride (271 mg, 1.5 eq) in 5 mL of dry DMF is stirred in a sealed vial at 80 °C for 90 min. The mixture is cooled to room temperature and poured to 70 mL of water. Using IN HCl water solution pH of the solution is adjusted to around 4.5. The aqueous solution is extracted with EtOAc (3 times, 100 mL is used in total). The gathered organic layer is washed with water (40 mL) and brine (40 mL). The organic solution is dried over Na₂SO₄ and the solvent is removed to afford the desired compound. LCMS: MW (calcd): 344; m/z MW (obsd): 345 (M+H).

### 1.65. Intermediate 104: 6-chloro-4,5-dimethyl-pyridazin-3-ol

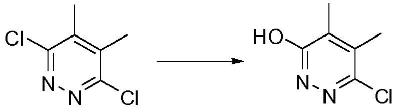

A suspension of 3,6-dichloro-4,5-dimethyl-pyridazine (3.0 g, 17 mmol) in aqueous NaOH (3.3 N, 28 mL) is stirred at reflux for 2 h. The reaction is cooled to room temperature and acetic acid (50% solution in water, 19 mL) is added. The formed precipitate is extracted with 9:1 ethyl acetate:THF (3×100 mL). The combined organic extracts are dried over Na₂SO₄ and concentrated under reduced pressure to afford the desired compound.
LCMS: MW (calcd): 159; m/z MW (obsd): 160 (M+H).

### 1.66. Intermediate 105: 6-hydroxy-4,5-dimethyl-pyridazine-3-carbonitrile

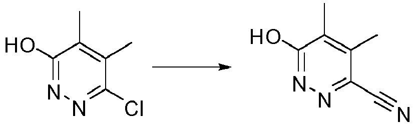

A mixture of 6-chloro-4,5-dimethyl-pyridazin-3-ol (2.7 g, 17 mmol), Zn(CN)₂ (1.23 g, 10.3 mmol), dppf, 1,1'-Bis(diphenylphosphino)ferrocene, (784 mg, 1.37 mmol) in dimethylformamide (38 mL) is flushed three times with argon. Pd₂(dba)₃ (641 mg, 0.686 mmol) is added and the reaction is stirred at 135°C in a sealed vessel for 2 h. The mixture is filtered through a Celite pad. The filtrate is diluted with water (100 mL) and extracted with ethyl acetate (3×50 mL). The combined organic extracts are washed with water (100 mL) and brine (100 mL). The organic layer is dried over Na₂SO₄ and concentrated under reduced pressure to afford the desired compound.
LCMS: MW (calcd): 149; m/z MW (obsd): 150 (M+H).

### 1.67. Intermediate 105: 6-hydroxy-4,5-dimethyl-pyridazine-3-carbonitrile

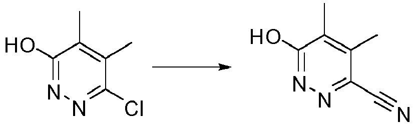

A mixture of 6-chloro-4,5-dimethyl-pyridazin-3-ol (2.7 g, 17 mmol), Zn(CN)₂ (1.23 g, 10.3 mmol), dppf, 1,1'-Bis(diphenylphosphino)ferrocene, (784 mg, 1.37 mmol) in dimethylformamide (38 mL) is flushed three times with argon. Pd₂(dba)₃ (641 mg, 0.686 mmol) is added and the reaction is stirred at 135°C in a sealed vessel for 2 h. The mixture is filtered through a Celite pad. The filtrate is diluted with water (100 mL) and extracted with ethyl acetate (3×50 mL). The combined organic extracts are washed with water (100 mL) and brine (100 mL). The organic layer is dried over Na₂SO₄ and concentrated under reduced pressure to afford the desired compound.
LCMS: MW (calcd): 149; m/z MW (obsd): 150 (M+H).

### 1.68. Intermediate 110: 6-chloro-3-(1,3-dimethylpyrazol-4-yl)-4-methyl-pyridazine

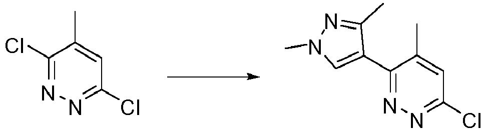

A mixture of 3,6-dichloro-4-methyl-pyridazine (500 mg, 3.0 mmol, 1 equiv), 1,3-dimethyl-4-(4,4,5,5-tetramethyl-1,3,2-dioxaborolan-2-yl)pyrazole (668 mg, 3.0 mmol, 1 equiv), K₂CO₃ (1.04 g, 7.53 mmol, 2.5 equiv) and Pd(dppf)Cl₂ (245 mg, 0.30 mmol, 0.1 equiv) in 1,4-dioxane:H₂O 4:1 is stirred at 80 °C. After 4 h the mixture is partitioned between dichloromethane and saturated NaHCO₃. The mixture is filtered through a phase separator and the organic layer is concentrated under reduced pressure. The residue is purified by silica column chromatography (SiO₂, methanol/dichloromethane 0:100 to 2:98) to afford the desired compound.
LCMS: MW (calcd): 223; m/z MW (obsd): 223 (M+H).

### 1.69. Intermediate 111: 6-(1,3-dimethylpyrazol-4-yl)-5-methyl-pyridazine-3-carbonitrile

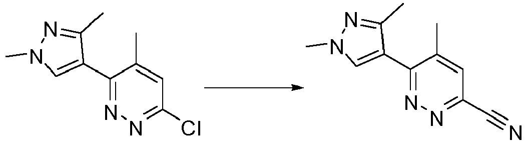

A mixture of 6-chloro-3-(1,3-dimethylpyrazol-4-yl)-4-methyl-pyridazine (100 mg, 0.45 mmol, 1 equiv), Zn(CN)₂ (52 mg, 0.45 mmol, 1 equiv), and Pd(PPh₃)₄ (57 mg, 0.05 mmol, 0.1 equiv) in dimethylformamide (2 mL) is stirred at 150°C in a microwave reactor for 5 min. The mixture is diluted with ethyl acetate, washed with saturated NaHCO₃ and concentrated to afford the desired compound.
LCMS: MW (calcd): 213; m/z MW (obsd): 214 (M+H).

### 1.70. Intermediate 115: 2-(2,2-difluoroethoxy)-6-(trifluoromethyl)pyridin-4-amine

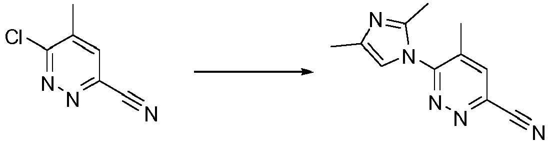

A mixture of 6-chloro-5-methyl-pyridazine-3-carbonitrile (120 mg, 0.78 mmol, 1 equiv), 2,4-dimethyl-1H-imidazole (112 mg, 1.17 mmol, 1.5 equiv) and *N,N-*diisopropylethylamine (0.27 mL, 1.56 mmol, 2 equiv) in THF (0.72 mL) is stirred at 50 °C for 24 h. The mixture was partitioned between DCM and saturated ammonium chloride. The two layers were separated. The organic layer was washed (brine and H₂O), dried (Na₂SO₄) and concentrated to yield the desired compound. LCMS: MW (calcd): 213; m/z MW (obsd): 214 (M+H).

### 1.71. Intermediate 117: 3-chloro-2-ethoxy-6-methyl-pyridin-4-amine

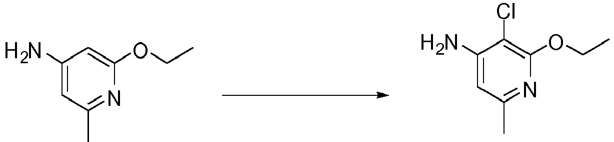

*N*-chlorosuccinimide (88 mg, 0.657 mmol, 1.0 equiv) and 2-ethoxy-6-methylpyridin-4-amine (100 mg, 0.657 mmol, 1.0 equiv) are stirred in DCM (3 ml) at room temperature. After 6 h, the reaction mixture is partitioned between dichloromethane and water. The aqueous phase was extracted with DCM. The combined organic phase was filtered through a phase separator and concentrated under vacuum to afford the desired compound. LCMS: MW (calcd): 187; m/z MW (obsd): 187 (M+H).

### 1.72. Intermediate 118: 3-bromo-2-ethoxy-6-methyl-pyridin-4-amine

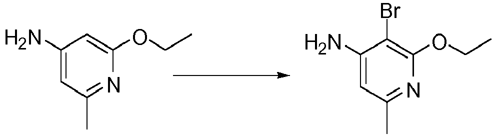

*N*-bromosuccinimide (1.17 g, 6.57 mmol, 1.0 equiv) and 2-ethoxy-6-methyl-pyridin-4-amine (1.0 g, 6.57 mmol, 1.0 equiv) are stirred at room temperature in dry DCM (33 ml) for 1 h. The mixture is diluted with DCM and water. The aqueous phase is extracted with DCM. The combined organic phase is filtered through a phase separator and concentrated under vacuum to afford the desired compound. LCMS: MW (calcd): 231; m/z MW (obsd): 233 (M+H).

### 1.73. Intermediate 119: 2-ethoxy-3,6-dimethyl-pyridin-4-amine

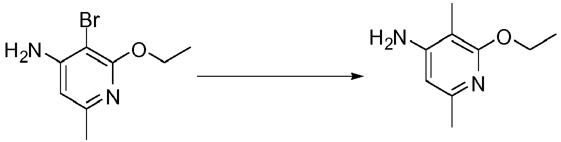

A degassed mixture of 3-bromo-2-ethoxy-6-methyl-pyridin-4-amine (1 g, 4.33 mmol, 1.0 equiv), methylboronic acid (777 mg, 13.0 mmol, 3.0 equiv), Cs2CO3 (7.05 g, 21.7 mmol, 5.0 equiv) and SPhos Pd G2 (155 mg, 0.22 mmol, 0.05 equiv) in dry dioxane (40 mL, 0.1M) is heated at 100°C for 5 h. The reaction mixture is diluted with DCM and water. The organic layer is filtered through a phase separator and concentrated under vacuum. The residue is purified by flash chromatography (Petroleum ether/EtOAc 9/1) to afford the desired compound. LCMS: MW (calcd): 166; m/z MW (obsd): 167 (M+H).

### 1.74. Intermediate 120: 3-bromo-2-[2-[tert-butyl(dimethyl)silyl]oxyethoxy]-6-(trifluoromethyl)pyridin-4-amine

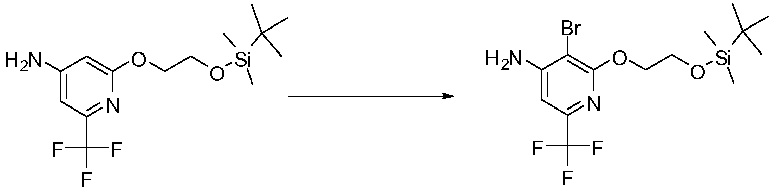

*N*-bromosuccinimide (56 mg, 0.32 mmol, 1.0 equiv) and 2-[2-[tert-butyl(dimethyl)silyl]oxyethoxy]-6-(trifluoromethyl)pyridin-4-amine (106 mg, 0.32 mmol, 1.0 equiv) are stirred at room temperature in dry DCM (1.6 ml) for 30 min. The mixture is diluted with DCM and water. The aqueous phase is extracted with DCM. The combined organic phase is filtered through a phase separator and concentrated under vacuum. The crude is purified by flash chromatography. LCMS: MW (calcd): 415; m/z MW (obsd): 417 (M+H).

### 1.75. Intermediate 121: 2-[2-[tert-butyl(dimethyl)silyl]oxyethoxy]-3-methyl-6-(trifluoromethyl)pyridin-4-amine

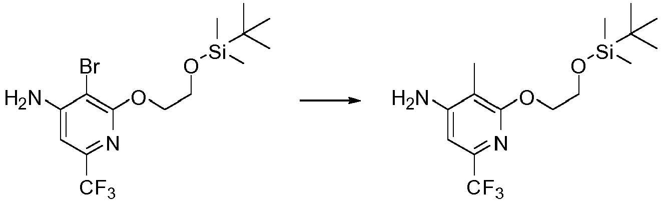

A degassed mixture of 3-bromo-2-[2-[tert-butyl(dimethyl)silyl]oxyethoxy]-6-(trifluoromethyl)pyridin-4-amine (0.315 mmol, 1.0 equiv), methylboronic acid (57 mg, 0.945 mmol, 3.0 equiv), Cs₂CO₃ (513 mg, 1.58 mmol, 5.0 equiv) and SPhos Pd G2 (22 mg, 0.032 mmol, 0.10 equiv) in dry dioxane (3 mL, 0.1M) is heated at 90°C for 24 h. The reaction mixture is diluted with DCM and water. The organic layer is filtered through a phase separator and concentrated in vacuum. The residue is purified by flash chromatography (Petroleum ether/EtOAc 9/1) to afford the desired compound. LCMS: MW (calcd): 350; m/z MW (obsd): 351 (M+H).

### 1.76. Intermediate 122: 3-bromo-6-chloro-2-(2,2-difluoroethoxy)pyridin-4-amine

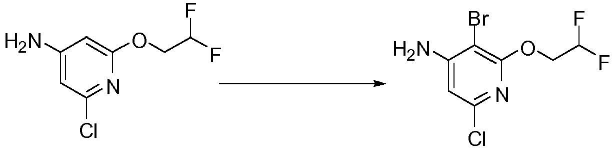

*N*-bromosuccinimide (427 mg, 2.40 mmol, 1.0 equiv) and 2-chloro-6-(2,2-difluoroethoxy)pyridin-4-amine (500 mg, 2.40 mmol, 1.0 equiv) are stirred at room temperature in dry DCM (12 ml) for 2 h. The mixture is diluted with DCM and water. The aqueous phase is extracted with DCM. The combined organic phase is filtered through a phase separator and concentrated under vacuum to afford the desired compound. LCMS: MW (calcd): 288; m/z MW (obsd): 289 (M+H).

### 1.77. Intermediate 123: 2-(2,2-difluoroethoxy)-3,6-dimethyl-pyridin-4-amine

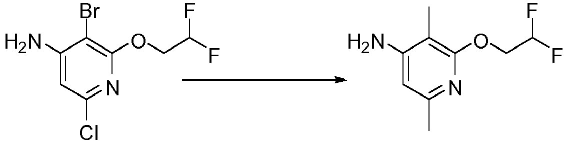

A degassed mixture of 3-bromo-6-chloro-2-(2,2-difluoroethoxy)pyridin-4-amine (2.40 mmol, 1.0 equiv), methylboronic acid (718 mg, 12.0 mmol, 5.0 equiv), Cs2CO3 (3.91 g, 12.0 mmol, 5.0 equiv) and SPhos Pd G2 (173 mg, 0.24 mmol, 0.10 equiv) in dry dioxane (18 mL, 0.15M) is heated at 90°C for 16 h. The reaction mixture is diluted with DCM and water. The aqueous phase was extracted with DCM. The organic layer is filtered through a phase separator and concentrated in vacuum to afford the desired compound. LCMS: MW (calcd): 202; m/z MW (obsd): 203 (M+H).

### 1.78. Intermediate 124: N-[2-chloro-6-(2,2-difluoroethoxy)-4-pyridyl]acetamide

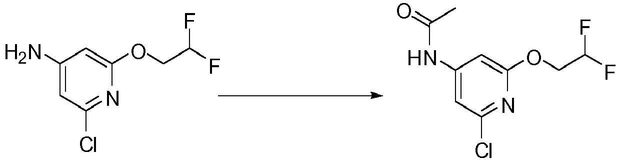

At 0°C, acetyl chloride (0.25 mL, 3.6 mmol, 1.5 equiv) is added to a mixture of 2 chloro 6 (2,2-difluoroethoxy)pyridin-4-amine (500 mg, 2.40 mmol, 1.0 equiv), triethyl amine (1 mL, 7.2 mmol, 3.0 equiv) and DMAP (29 mg, 0.240 mmol, 0.1 equiv) in dichloromethane (6 mL, 0.4 M). After 24 h, starting material is still present. At 0°C, acetyl chloride (0.25 mL, 3.6 mmol, 1.5 equiv) is added. After 2 h, the reaction mixture is diluted with DCM and water. The organic layer is filtered through a phase separator and concentrated in vacuum to afford the desired compound. LCMS: MW (calcd): 251; m/z MW (obsd): 251 (M+H).

### 1.79. Intermediate 125: N-[2-(2,2-difluoroethoxy)-6-methyl-4-pyridyl]acetamide

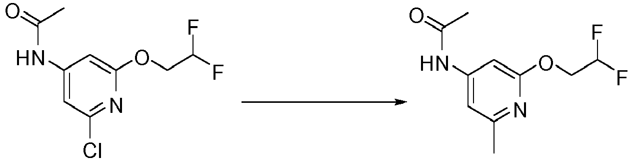

A degassed mixture of N-[2-chloro-6-(2,2-difluoroethoxy)-4-pyridyl]acetamide (1.96 mmol, 1.0 equiv), methylboronic acid (351 mg, 5.87 mmol, 3.0 equiv), Cs2CO3 (3.2 g, 9.8 mmol, 5.0 equiv) and SPhos Pd G2 (141 mg, 0.196 mmol, 0.10 equiv) in dry dioxane (18 mL, 0.1M) is heated at 90°C for 24 h. The reaction mixture is diluted with DCM and water. The organic layer is filtered through a phase separator and concentrated in vacuum to afford the desired compound. LCMS: MW (calcd): 230; m/z MW (obsd): 231 (M+H).

### 1.80. Intermediate 126: 2-(2,2-difluoroethoxy)-6-methyl-pyridin-4-amine

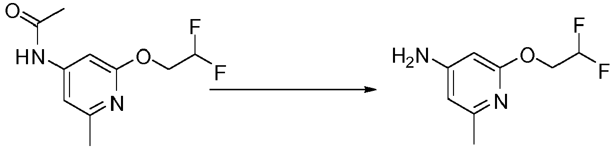

N-[2-(2,2-difluoroethoxy)-6-methyl-4-pyridyl]acetamide (1.96 mmol, 1.0 equiv) is heated at 50°C in a solution of NaOH (4 mL, 3.92 mmol, 2.0 equiv, 1M in water) in ethanol (8 mL, 0.25 M). The reaction mixture is diluted with DCM and water. The aqueous phase was extracted with DCM. The organic layer is filtered through a phase separator and concentrated in vacuum. The residue is purified by silica chromatography (Petroleum ether/EtOAc: 9/1) to afford the desired compound. LCMS: MW (calcd):188; m/z MW (obsd): 189 (M+H).

### 1.81. Intermediate 127: 4-amino-2-ethoxy-6-methyl-pyridine-3-carbonitrile

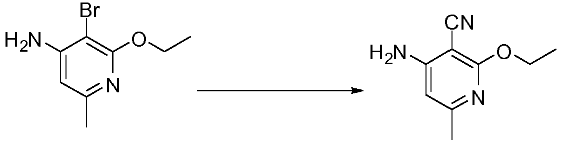

A mixture of 3-bromo-2-ethoxy-6-methyl-pyridin-4-amine (100 mg, 0.433 mmol, 1.0 equiv), tetrakis(triphenylphosphine)palladium(0) (40 mg, 0.034 mmol, 0.08 equiv) and zinc cyanide (50 mg, 0.433 mmol, 1.0 equiv) in anhydrous N,N-dimethylformamide (2 mL, 0.2 M) is heated at 150 °C for 5 min under microwave irradiation. The reaction mixture is diluted with EtOAc and washed with a saturated solution of NaHCO₃. The organic layer is washed with brine, dried over sodium sulfate and concentrated in vacuo. The residue is purified by silica chromatography (petroleum ether/EtOAc: 9/1) to afford the desired compound. LCMS: MW (calcd): 177; m/z MW (obsd): 178 (M+H).

### 1.82. General method I: preparation of cyanoguanidine precursor

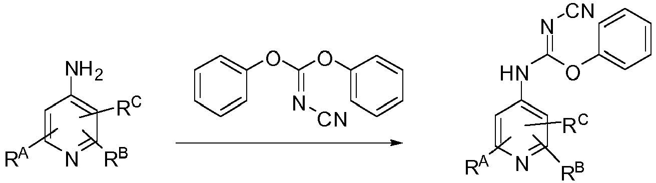

Pyridine (3.0 equiv) and diphenyl n-cyanocarbonimidate (1.0 equiv) are added to aminopyridine (1.0 equiv) in dichloromethane (0.4 M). The resulting mixture is stirred at 70°C. After 16 h the reaction mixture is partitioned between dichloromethane and water. The aqueous phase is extracted with DCM. The combined organic phase was filtered through a phase separator and concentrated under vacuum to afford the desired compound.

### 1.83. Illustrative example of method I: synthesis of intermediate 128: 3-cyano-1-[2-ethoxy-3-methyl-6-(trifluoromethyl)-4-pyridyl]-2-phenyl-isourea

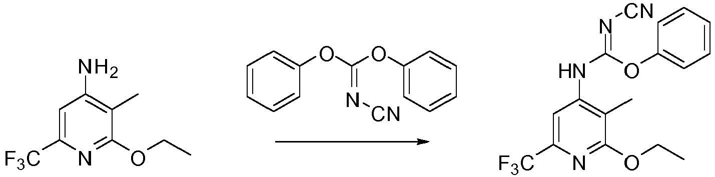

Pyridine (0.2 mL, 2.48 mmol, 3.0 equiv) and diphenyl n-cyanocarbonimidate (197 mg, 0.826 mmol, 1.0 equiv) are added to 2-ethoxy-3-methyl-6-(trifluoromethyl)pyridin-4-amine (200 mg, 0.908 mmol, 1.0 equiv) in dichloromethane (2 mL, 0.4 M). The resulting mixture is stirred at 70°C. After 16 h, the reaction mixture is partitioned between dichloromethane and water. The aqueous phase is extracted with DCM. The combined organic phase was filtered through a phase separator and concentrated under vacuum to afford the desired compound. LCMS: MW (calcd): 364; m/z MW (obsd): 365 (M+H).

### 1.84. Illustrative example of method I: synthesis of intermediate 129: 3-cyano-1-[2-ethoxy-6-(trifluoromethyl)-4-pyridyl]-2-phenyl-isourea

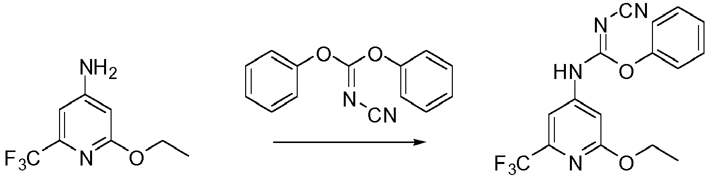

Pyridine (0.12 ml, 1.45 mmol, 3.0 equiv) and diphenyl n-cyanocarbonimidate (105 mg, 0.441mmol, 1.0 equiv) are added to 2-ethoxy-6-(trifluoromethyl)pyridin-4-amine (100 mg, 0.485 mmol, 1.1 equiv) in dichloromethane (1.2 ml). The resulting mixture is stirred at 70°C. After 16 h, the reaction mixture is partitioned between dichloromethane and water. The aqueous phase was extracted with DCM. The combined organic phase was filtered through a phase separator and concentrated under vacuum to afford the desired compound. The solid so obtained was trituared with MTBE. LCMS: MW (calcd): 350; m/z MW (obsd): 351 (M+H).

### 1.85. General method K1: S_{N}Ar reaction on chloro-pyridazine derivative.

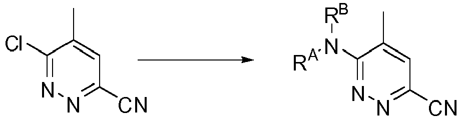

A mixture of the chloro-pyridazine derivative (1.0 equiv), the corresponding amine (1.5 equiv) and DIPEA (1.5 to 2 equiv) in DMSO or DMF (1 to 2 M) is stirred at RT overnight. The resulting mixture is diluted with EtOAc, washed with aq. sat. NaHCO₃, brine, dried and concentrated to afford the desired compound.

### 1.86. Illustrative example of method K1: synthesis of intermediate 132, 6-(3,3-Difluoro-pyrrolidin-1-yl)-5-methyl-pyridazine-3-carbonitrile

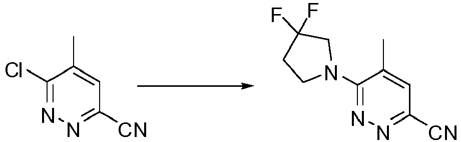

A mixture of 6-chloro-5-methyl-pyridazine-3-carbonitrile (100 mg, 0.65 mmol, 1.0 equiv), 3,3-difluoro-pyrrolidine hydrochloride (141 mg, 0.98 mmol, 1.5 equiv) and DIPEA (0.17 mL, 0.98 mmol, 1.5 equiv) in DMSO (0.5 mL) is stirred at RT overnight. The resulting mixture is diluted with EtOAc, washed with aq. sat. NaHCO₃, brine, dried and concentrated to afford the desired compound. LCMS: MW (calcd): 224; m/z MW (obsd): 225 (M+H).

### 1.87. General method K2: S_{N}Ar reaction on chloro-pyridazine derivative.

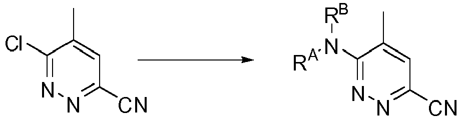

A mixture of the chloro-pyridazine derivative (1.0 equiv), the corresponding amine (1.5 equiv) and DIPEA (3 equiv) in DMSO (0.3 to 2 M) is heated at 40°C. After 4h, pyridine (1 equiv) is added and the resulting mixture heated at 70°C overnight. The resulting mixture is diluted with EtOAc, washed with aq. sat. NH₄Cl, brine, dried and concentrated to afford the desired compound.

### 1.88. Illustrative example of method K2: synthesis of intermediate 134, 6-(3,3-Difluoro-pyrrolidin-1-yl)-5-methyl-pyridazine-3-carbonitrile

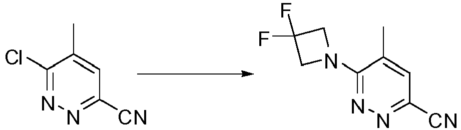

A mixture of 6-chloro-5-methyl-pyridazine-3-carbonitrile (crude, 1.85 mmol, 1.0 equiv), 3,3-difluoro-azetidine hydrochloride (360 mg, 2.78 mmol, 1.5 equiv) and DIPEA (0.97 mL, 5.55 mmol, 3 equiv) in DMSO (2 mL) is stirred at 40°C. After 4h, pyridine (0.15 mL, 1.85 mmol, 1 equiv) is added and the resulting mixture heated at 70°C overnight. The resulting mixture is diluted with EtOAc, washed with aq. sat. NH₄Cl, brine, dried and concentrated to afford the desired compound. LCMS: MW (calcd): 214; m/z MW (obsd): 215 (M+H).

### 1.89. General method K3: S_{N}Ar reaction on chloro-pyridazine derivative.

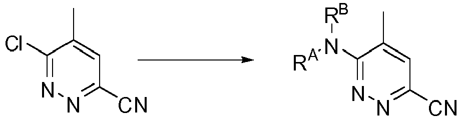

To a solution of 6-chloro-5-methyl-pyridazine-3-carbonitrile (1.0 equiv) in dry THF (0.3 to 1M) is added aminoderivative (1 to 1.5 equiv) and N,N-Diisopropylethylamine (2.0-3.0 equiv). The reaction is stirred at 50°C-80°C for 16 to 24 h. The mixture is diluted with DCM and is washed with NH₄Cl. The organic layer is dried over sodium sulfate, filtered and concentrated in vacuo. The residue is used as such in the next step.

### 1.90. Illustrative example of method K3: synthesis of intermediate 138, 6-[(2S,6S)-2,6-dimethylmorpholin-4-yl]-5-methyl-pyridazine-3-carbonitrile

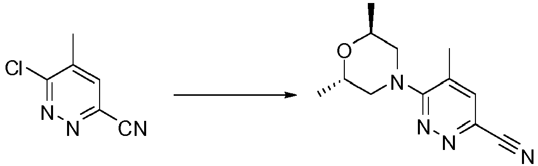

DIPEA (0.5 mL, 2.93 mmol, 3.0 equiv) is added to 6-chloro-5-methyl-pyridazine-3-carbonitrile (150 mg, 0.976 mmol, 1.0 equiv) and (2S,6S)-2,6-dimethyl-morpholine, (135 mg, 1.17 mmol, 1.2 equiv) in THF (3 mL, 0.3 M). The resulting mixture is heated at 55°C. After 24 h, the reaction mixture is diluted with DCM and saturated NH₄Cl solution. The aqueous phase was extracted with DCM. The organic layer is filtered through a phase separator and concentrated in vacuum to afford the desired compound. LCMS: MW (calcd):232; m/z MW (obsd): 233 (M+H).

### 1.91. Intermediate 144: 5-methyl-6-(2-methyl-6,7-dihydro-5H-pyrazolo[4,3-b]pyridin-4-yl)pyridazine-3-carbonitrile

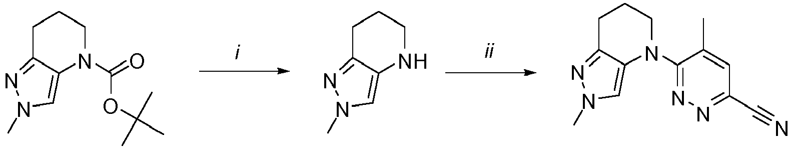

### 1.91.1. Step i: 2-methyl-4,5,6,7-tetrahydropyrazolo[4,3-b]pyridine

To a solution of tert-butyl 2-methyl-6,7-dihydro-5H-pyrazolo[4,3-b]pyridine-4-carboxylate (150 mg, 0.63 mmol, 1.0 equiv) in dry DCM (1.9 mL) is added trifluoroacetic acid (1.2 mL, 10 mmol, 25 equiv). The reaction was stirred at 40°C for 2 h. The reaction was coevaporated with toluene (3x) to give the desired product. LCMS: MW (calcd): 137; m/z MW (obsd): 138 (M+H).

### 1.91.2. Step ii: 5-methyl-6-(2-methyl-6,7-dihydro-5H-pyrazolo[4,3-b]pyridin-4-yl)pyridazine-3-carbonitrile

To a solution of 6-chloro-5-methyl-pyridazine-3-carbonitrile (75 mg, 0.49 mmol, 1.0 equiv) in dry THF (0.49 mL) is added 2-methyl-4,5,6,7-tetrahydropyrazolo[4,3-b]pyridine (82 mg, 0.74 mmol, 1.5 equiv) and N,N-Diisopropylethylamine (0.17 mL, 0.98 mmol, 2.0 equiv). The reaction is stirred at 50°C overnight then at 100°C for 60h. The mixture is diluted with DCM and washed with NH₄Cl. The organic layer is dried over sodium sulfate, filtered and concentrated in vacuum. LCMS: MW (calcd): 254; m/z MW (obsd): 255 (M+H).

### 1.92. Intermediate 156: 2-chloro-4-iodo-6-(trifluoromethyl)pyridine

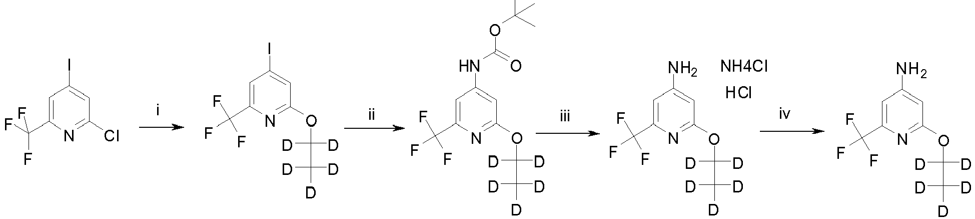

i) Under nitrogen, in a flask at 0°C, CD₃CD₂OH (2eq, 65.05 mmol, 3.32g) is diluted with dioxane (30mL). To the solution is added NaH 60% in oil (2eq, 65.05 mmol, 2.6g) by portion and the resulting mixture is stirred at room temperature for 30 minutes. A solution of 2-Chloro-4-iodo-6-(trifluoromethyl)pyridine (10g, 32.53mmol) in dioxane (40 mL) is added portion wise into the previous mixture. The heterogeneous mixture is stirred at room temperature until complete conversion.

ii) To the reaction mixture are added directly BocNH2 (1.2eq, 39.03 mmol, 4.573g), Pd₂(dba)₃ (0.5%, 0.163mmol, 149mg), Xantphos (1.5%, 0.488mmol, 282mg) and Cs₂CO₃ (1.1eq, 35.78 mmol, 11.658g) and dioxane (30mL). The reaction mixture is stirred at 85°C until complete conversion. The reaction mixture is filtered over a pad of Celite, rinsed with dioxane and concentrated partially.

To the previous mixture is added HCl 4N in dioxane (100mL) at room temperature and the reaction mixture is stirred at 60°C until complete conversion. Then the reaction mixture is cooled down to room temperature and DCM is added (100mL). The suspension is stirred at room temperature for 1 hour and the solid formed is filtered. The precipitate is rinsed with DCM to afford the desired product as hydrochloride salt. The filtrate is evaporated, re-slurried in DCM and the solid formed is filtered to afford a second crop of expected product as hydrochloride salt.

The 2 batches are combined and added by portion into a solution of NaOH 2N under stirring. The product is extracted with DCM and is passed through a phase separator. The organic layer is concentrated to give the expected product. LCMS: MW (calcd): 211; m/z MW (obsd): 212 (M+H).

### Example 2. Preparation of illustrative compounds of the invention

### 2.1. General method H1: urea formation

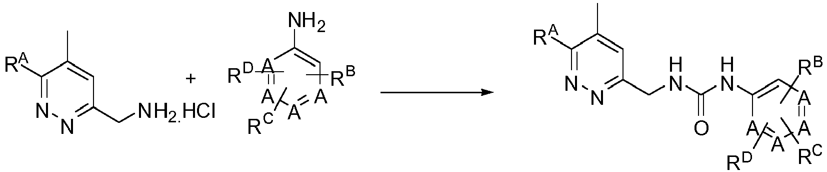

1,1'-carbonyl-di-(1,2,4-triazole) (1 to 2 equiv) is added to the aniline (1 to 2 equiv) in dry pyridine (1.5 to 3 equiv) and dry DCM (0.2 M). The resulting mixture is stirred at 45°C for 30 min and added to a mixture of the benzylamine hydrochloride salt (or trifuloroacetic acid salt, 1.0 equiv) and diisopropylthylamine (4 to 5 equiv) in dry THF (0.1 to 0.3 M). The resulting mixture is stirred at room temperature for 30 min. The mixture is diluted with DCM and water. The organic phase is washed with 1 M HCl solution or sat. NH₄Cl, filtered through a phase separator and concentrated under reduced pressure. The residue is purified by flash column chromatography or preparatory HPLC to afford the desired compound.

### 2.2. Illustrative example of method H1: synthesis of compound 1: 1-(2-Ethoxy-6-trifluoromethyl-pyridin-4-yl)-3-[5-methyl-6-(1-methyl-3-trifluoromethyl-1H-pyrazol-4-yl)-pyridazin-3-ylmethyl]-urea

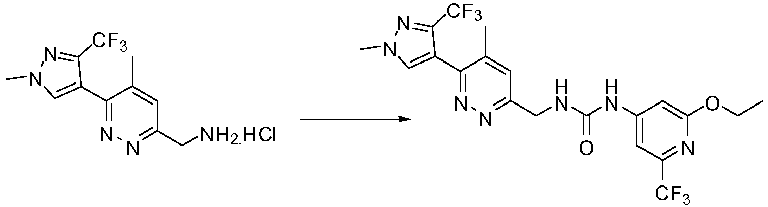

Carbonyltriimidazole (1.14 g, 6.97 mmol, 1.2 equiv) is added to a mixture of 2-ethoxy-6-(trifluoromethyl)pyridin-4-amine (1.44 g, 6.97 mmol, 1.2 equiv) in dry pyridine (1.41 mL, 17.4 mmol, 3.0 equiv) and dry DCM (29.0 mL, 0.2 M).The resulting mixture is stirred at 45°C for 30 min. The above mentioned mixture is added to a suspension of [5-methyl-6-[1-methyl-3-(trifluoromethyl)pyrazol-4-yl]pyridazin-3-yl]methanamine hydrochloride salt (2.0 g, 5.81 mmol, 1.0 equiv) and diisopropylthylamine (4.0 mL, 4.0 equiv) in dry THF (8.0 mL, 0.3 M). The resulting mixture is stirred at room temperature for 30 min. The mixture is diluted with DCM and water. The organic phase is washed with 1 M HCl solution, filtered through a phase separator and concentrated under reduced pressure. These operations are repeated four times. The crudes are gathered and purified by flash chromatography on silica gel (eluting with Et₂O/Acetone 95/5 to 90/10) to give, after trituration in MTBE/MeCN, the desired compound. LCMS: MW (calcd): 503; m/z MW (obsd): 504 (M+H).

### 2.3. General method H2: urea formation

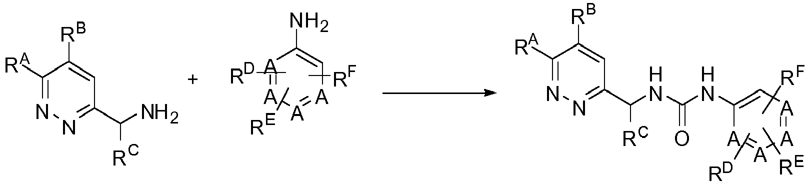

To a solution of aniline (0.7 to 1.5 eq) in dry DCM (0.1 M) and pyridine (2 to 3 eq), 1,1'-carbonyl-di-(1,2,4-triazole) (1 to 1.5 eq) is added. The resulting mixture is stirred at 50 °C. After 30 to 90 min, the reaction mixture is cooled to room temperature and a solution of benzylamine (1 eq) in dry DCM or THF (0.1 to 0.5 M) is added. The resulting mixture is left to stir at room temperature. After 1 to approximately 16 h the reaction mixture is quenched with water or sat. NH₄Cl and extracted with an organic solved. After work up, the organic layers are dried over Na₂SO₄ and concentrated under reduced pressure. The crude is purified by flash column chromatography or preparative HPLC to afford the desired compound.

### 2.4. Illustrative example of method H2: synthesis of compound 2: 1-(2-Chloro-6-ethoxy-pyridin-4-yl)-3-[5-methyl-6-(1-methyl-3-trifluoromethyl-1H-pyrazol-4-yl)-pyridazin-3-ylmethyl]-urea

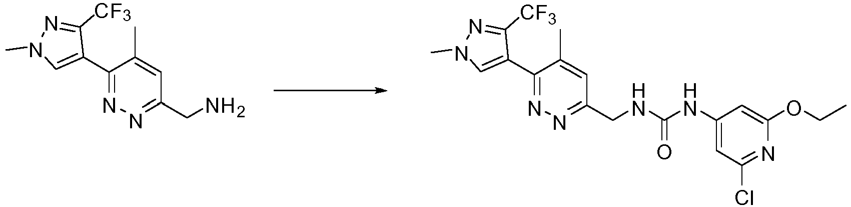

Carbonyltriimidazole (726 mg, 4.42 mmol, 1.5 equiv) is added to 2-chloro-6-ethoxy-pyridin-4-ylamine (356 mg, 2.07 mmol, 0.7 equiv) in pyridine (0.71 mL, 8.85 mmol, 3 equiv) and DCM (15 mL). The reaction mixture is stirred at 45°C for 30 min. Once cooled to room temperature, [5-methyl-6-[1-methyl-3-(trifluoromethyl)pyrazol-4-yl]pyridazin-3-yl]methanamine (800 mg, 2.95 mmol, 1.0 equiv) is added and the resulting mixture is stirred at room temperature for 1 h. The mixture is diluted with DCM, washed with aq. sat. NH₄Cl and aq. sat. NaHCO₃. The organic phase is filtered through a phase separator and concentrated under reduced pressure. The residue is purified by silica chromatography (methanol/DCM; 0:100 to 4:96) followed by triturated in a mixture of methyl tert-butyl ether/petroleum ether to afford the desired compound. LCMS: MW (calcd): 470; m/z MW (obsd): 470 (M+H).

### 2.5. Illustrative example of method H2: synthesis of compound 3: 1-(2-chloro-6-ethoxy-4-pyridyl)-3-[1-[5-methyl-6-[1-methyl-3-(trifluoromethyl)pyrazol-4-yl]pyridazin-3-yl]ethy]urea

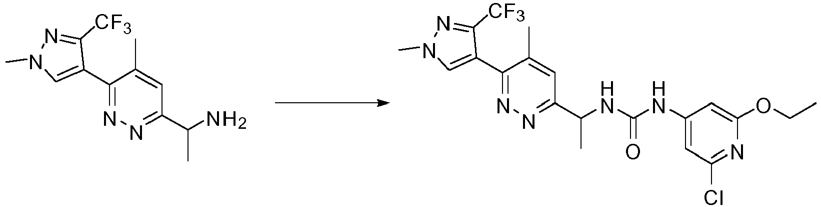

To a solution of 2-chloro-6-ethoxy-pyridin-4-ylamine (23.5 mg, 0.136 mmol) in dry DCM (900 µL) and pyridine (43.1 µL, 0.585 mmol), 1,1'-carbonyl-di-(1,2,4-triazole) (48.0 mg, 0.292 mmol) is added. The resulting mixture is stirred at 50 °C. After 90 min, the reaction mixture is cooled to room temperature and a solution of 1-[5-methyl-6-[1-methyl-3-(trifluoromethyl)pyrazol-4-yl]pyridazin-3-yl]ethanamine (55.6 mg, 0.195 mmol) in dry DCM (250 µL) is added. The resulting mixture is left to stir at room temperature. After 5 h the reaction mixture is quenched with water (30 mL) and extracted with DCM (3 × 10 mL). Combined organic layers are washed with brine (30 mL), dried over Na₂SO₄ and concentrated under reduced pressure. The crude is purified by flash column chromatography (SiO₂, EtOAc / cyclohexane 9:100 to 10:1) to afford the desired compound. LCMS: MW (calcd): 484; m/z MW (obsd): 484 (M+H).

### 2.6. Illustrative example of method H2: synthesis of compound 72: 1-(2-ethoxy-3,6-dimethyl-4-pyridyl)-3-[[5-methyl-6-[1-methyl-3-(trifluoromethyl)pyrazol-4-yl]pyridazin-3-yl]methyl]urea

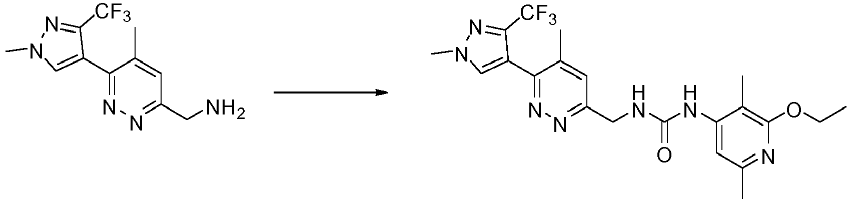

1,1'-carbonyl-di-(1,2,4-triazole) (534 mg, 3.26 mmol, 1.1 equiv) is added to 2-ethoxy-3,6-dimethyl-pyridin-4-amine (492 mg, 2.96 mmol, 1.0 equiv) in pyridine (0.72 mL, 8.88 mmol, 3 equiv) and DCM (15 mL). The reaction mixture is stirred at 45°C for 30 min. Once cooled to room temperature, [5-methyl-6-[1-methyl-3-(trifluoromethyl)pyrazol-4-yl]pyridazin-3-yl]methanamine (802 mg, 2.96 mmol, 1.0 equiv) is added and the resulting mixture is stirred at room temperature for 1 h. The mixture is diluted with DCM and water. The aqueous phase is extracted with DCM. The combined organic phase is filtered through a phase separator and concentrated under vacuum. The precipitate is triturated in MTBE and filtered to afford the desired compound. The solid is divided in two batches. One is dissolved in EtOAc and MeOH and concentrated again under vacuum. The other is dissolved in DCM and MeOH and concentrated again under vacuum. LCMS: MW (calcd): 463; m/z MW (obsd): 464 (M+H).

### 2.7. Compound 4: 1-[2-(2-hydroxyethoxy)-6-(trifluoromethyl)-4-pyridyl]-3-[[5-methyl-6-[1-methyl-3-(trifluoromethyl)pyrazol-4-yl]pyridazin-3-yl]methyl]urea

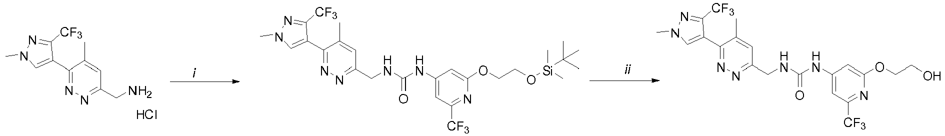

### 2.7.1. Step i: 1-[2-[2-[tert-butyl(dimethyl)silyl]oxyethoxy]-6-(trifluoromethyl)-4-pyridyl]-3-[[5-methyl-6-[1-methyl-3-(trifluoromethyl)pyrazol-4-yl]pyridazin-3-yl]methyl]urea

General method H1 using [5-methyl-6-[1-methyl-3-(trifluoromethyl)pyrazol-4-yl]pyridazin-3-yl]methanamine HCl and 2-[2-[tert-butyl(dimethyl)silyl]oxyethoxy]-6-(trifluoromethyl)pyridin-4-amine. LCMS: MW (calcd): 634; m/z MW (obsd): 634 (M+H).

### 2.7.2. Step ii: 1-[2-(2-hydroxyethoxy)-6-(trifluoromethyl)-4-pyridyl]-3-[[5-methyl-6-[1-methyl-3-(trifluoromethyl)pyrazol-4-yl]pyridazin-3-yl]methyl]urea

1-[2-[2-[tert-butyl(dimethyl)silyl]oxyethoxy]-6-(trifluoromethyl)-4-pyridyl]-3-[[5-methyl-6-[1-methyl-3-(trifluoromethyl)pyrazol-4-yl]pyridazin-3-yl]methyl]urea (0.232 mmol, 1.0 equiv) is stirred in concentrated HCl (0.5 mL, 0.5 M) and methanol (0.5 mL, 0.5 M) at room temperature for 45 min. The mixture is diluted with DCM and water. The aqueous phase is basified and extracted with DCM. The combined organic phase is filtered through a phase separator and concentrated under vacuum. The crude is purified by preparative HPLC to afford the desired compound. LCMS: MW (calcd): 519; m/z MW (obsd): 520 (M+H).

### 2.8. Compound 5: 1-[2-(2,3-dihydroxypropoxy)-6-(trifluoromethyl)-4-pyridyl]-3-[[5-methyl-6-[1-methyl-3-(trifluoromethyl)pyrazol-4-yl]pyridazin-3-yl]methyl]urea

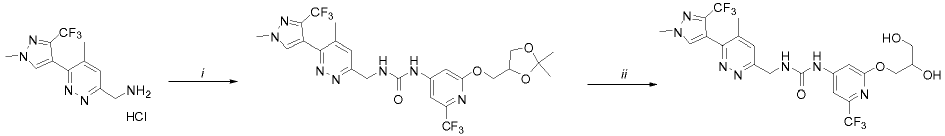

### 2.8.1. Step i: 1-[2-[(2,2-dimethyl-1,3-dioxolan-4-yl)methoxyl-6-(trifluoromethyl)-4-pyridyl]-3-[[5-methyl-6-[1-methyl-3-(trifluoromethyl)pyrazol-4-yl]pyridazin-3-yl]methyl]urea

General method H1 using [5-methyl-6-[1-methyl-3-(trifluoromethyl)pyrazol-4-yl]pyridazin-3-yl]methanamine HCl and 2-[(2,2-dimethyl-1,3-dioxolan-4-yl)methoxy]-6-(trifluoromethyl)pyridin-4-amine. LCMS: MW (calcd): 590; m/z MW (obsd): 590 (M+H).

### 2.8.2. Step ii: 1-[2-(2,3-dihydroxypropoxy)-6-(trifluoromethyl)-4-pyridyl]-3-[[5-methy1-6-[1-methyl-3-(trifluoromethyl)pyrazol-4-yl]pyridazin-3-yl]methyl]urea

1-[2-[(2,2-dimethyl-1,3-dioxolan-4-yl)methoxy]-6-(trifluoromethyl)-4-pyridyl]-3-[[5-methyl-6-[1-methyl-3-(trifluoromethyl)pyrazol-4-yl]pyridazin-3-yl]methyl]urea (0.232 mmol, 1.0 equiv) is stirred in 2 M HCl (0.5 mL, 0.5 M) and THF (0.5 mL, 0.5 M) at room temperature for 2 h. The mixture is diluted with DCM and water. The aqueous phase is basified and extracted with DCM. The combined organic phase is filtered through a phase separator and concentrated under vacuum. The crude is purified by trituration in MeCN/MTBE to afford the desired compound. LCMS: MW (calcd): 549; m/z MW (obsd): 550 (M+H).

### 2.9. Compound 6: 1-(3,5-dichlorophenyl)-3-[[5-methyl-6-[1-methyl-3-(trifluoromethyl)pyrazol-4-yl]pyridazin-3-yl]methyl]urea

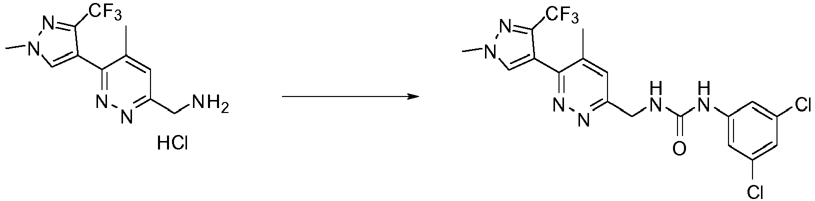

At room temperature, [5-methyl-6-[1-methyl-3-(trifluoromethyl)pyrazol-4-yl]pyridazin-3-yl]methanamine hydrochloride (80 mg, 0.232 mmol, 1.0 equiv) and 1,3-dichloro-5-isocyanato-benzene (44 mg, 0.232 mmol, 1.0 equiv) are stirred in DIPEA (0.16 mL, 0.928 mmol, 4.0 equiv) and THF (1.2 mL, 0.2 M). The mixture is diluted with DCM and water. The aqueous phase is extracted with DCM. The combined organic phase is filtered through a phase separator and concentrated under vacuum. The crude is purified by trituration in MeCN/MTBE to afford the desired compound. LCMS: MW (calcd): 459; m/z MW (obsd): 459 (M+H).

### 2.10. Compound 73: 1-[2-(2-hydroxyethoxy)-3-methyl-6-(trifluoromethyl)-4-pyridyl]-3-[[5-methyl-6-[1-methyl-3-(trifluoromethyl)pyrazol-4-yl]pyridazin-3-yl]methyl]urea

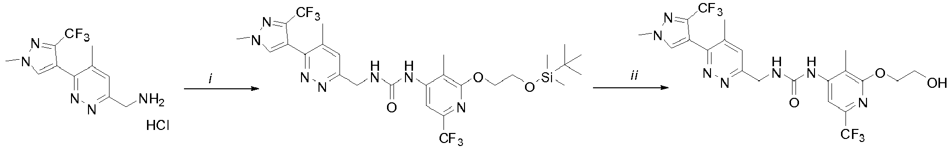

### 2.10.1. Step i: 1-[2-[2-[tert-butyl(dimethyl)silyl]oxyethoxy]-3-methyl-6-(trifluoromethyl)-4-pyridyl]-3-[[5-methyl-6-[1-methyl-3-(trifluoromethyl)pyrazol-4-yl]pyridazin-3-yl]methyl]urea

General method H1 using [5-methyl-6-[1-methyl-3-(trifluoromethyl)pyrazol-4-yl]pyridazin-3-yl]methanamine HCl and 2-[2-[tert-butyl(dimethyl)silyl]oxyethoxy]-3-methyl-6-(trifluoromethyl)pyridin-4-amine. LCMS: MW (calcd): 648; m/z MW (obsd): 648 (M+H).

### 2.10.2. Step ii: 1-[2-(2-hydroxyethoxy)-3-methyl-6-(trifluoromethyl)-4-pyridyl]-3-[[5-methyl-6-[1-methyl-3-(trifluoromethyl)pyrazol-4-yl]pyridazin-3-yl]methyl]urea

1-[2-(2-hydroxyethoxy)-3-methyl-6-(trifluoromethyl)-4-pyridyl]-3-[[5-methyl-6-[1-methyl-3-(trifluoromethyl)pyrazol-4-yl]pyridazin-3-yl]methyl]urea (0.114 mmol, 1.0 equiv) is stirred in concentrated HCl (0.25 mL, 0.5 M) and methanol (0.25 mL, 0.5 M) at room temperature for 45 min. The mixture is diluted with DCM and water. The aqueous phase is basified and extracted with DCM. The combined organic phase is filtered through a phase separator and concentrated under vacuum. The crude is purified by preparative HPLC to afford the desired compound. LCMS: MW (calcd): 533; m/z MW (obsd): 534 (M+H).

### 2.11. General method J: cyanoguanidine formation

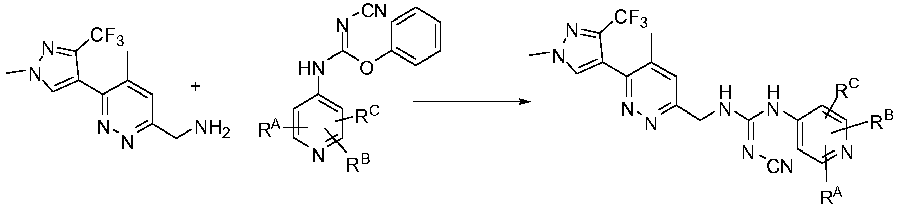

To [5-methyl-6-[1-methyl-3-(trifluoromethyl)pyrazol-4-yl]pyridazin-3-yl]methanamine (free base or HCl salt) (2.2 equiv) is added to cynaguanidine (1.0 equiv), trimethylamine (3.0 equiv) in acetonitrile (0.5 M). The resulting mixture is stirred at 85°C. After 16 h the reaction mixture is partitioned between dichloromethane and water. The aqueous phase was extracted with DCM. The combined organic phase was filtered through a phase separator and concentrated under vacuum. The residue is purified by preparatory HPLC or by silica gel chromatography to afford the desired compound.

### 2.12. Illustrative example of method J: synthesis of compound 80: 2-cyano-1-[2-ethoxy-3-methyl-6-(trifluoromethyl)-4-pyridyl]-3-[[5-methyl-6-[1-methyl-3-(trifluoromethyl)pyrazol-4-yl]pyridazin-3-yl]methyl]guanidine

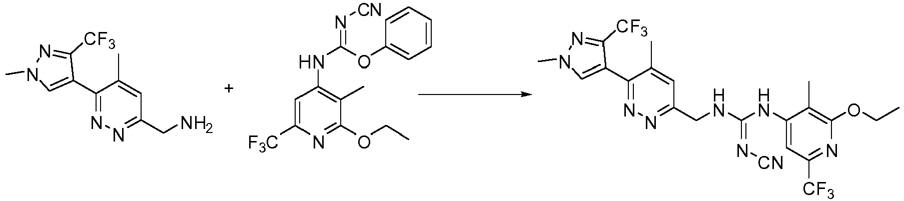

[5-methyl-6-[1-methyl-3-(trifluoromethyl)pyrazol-4-yl]pyridazin-3-yl]methanamine (117.0 mg, 0.431 mmol, 2.2 equiv) is added to 3-cyano-1-[2-ethoxy-3-methyl-6-(trifluoromethyl)-4-pyridyl]-2-phenyl-isourea (67.5 mg, 0.194 mmol, 1.0 equiv), trimethylamine (0.08 mL, 0.588 mmol, 3.0 equiv) in acetonitrile (0.4 mL, 0.5M). The resulting mixture is stirred at 85°C. After 16 h the reaction mixture is partitioned between dichloromethane and water. The aqueous phase was extracted with DCM. The combined organic phase was filtered through a phase separator and concentrated under vacuum. The residue is purified by preparatory HPLC to afford the desired compound. LCMS: MW (calcd): 541; m/z MW (obsd): 542 (M+H).

### 2.13. Illustrative example of method J: synthesis of compound 76: 2-cyano-1-[2-ethoxy-6-(trifluoromethyl)-4-pyridyl]-3-[[5-methyl-6-[1-methyl-3-(trifluoromethyl)pyrazol-4-yl]pyridazin-3-yl]methyl]guanidine

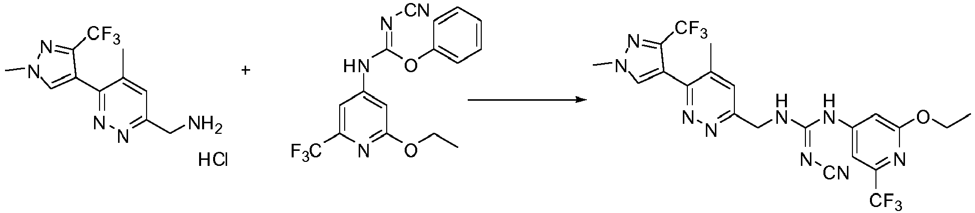

[5-methyl-6-[1-methyl-3-(trifluoromethyl)pyrazol-4-yl]pyridazin-3-yl]methanamine HCl salt (77 mg, 0.25 mmol, 2.2 equiv) is added to 3-cyano-1-[2-ethoxy-6-(trifluoromethyl)-4-pyridyl]-2-phenyl-isourea (40 mg, 0.114 mmol, 1.0 equiv), trimethylamine (0.05 mL, 0.342 mmol, 3.0 equiv) in acetonitrile (0.2 mL, 0.5M). The resulting mixture is stirred at 85°C. After 16 h the reaction mixture is partitioned between dichloromethane and water. The aqueous phase was extracted with DCM. The combined organic phase was filtered through a phase separator and concentrated under vacuum. The residue is purified by flash column chromatography (SiO₂, DCM / MeOH 98:2 to 95:5) and recrystallization (MTBE / petroleum ether) to afford the desired compound. LCMS: MW (calcd): 527; m/z MW (obsd): 528 (M+H).

### 2.14. Compound 99: 1-[[5-methyl-6-[1-methyl-3-(trifluoromethyl)pyrazol-4-yl]pyridazin-3-yl]methyl]-3-[2-(1,1,2,2,2-pentadeuterioethoxy)-6-(trifluoromethyl)-4-pyridyl]urea

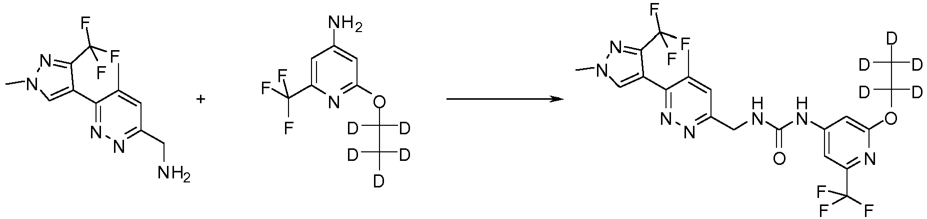

2-(1,1,2,2,2-pentadeuterioethoxy)-6-(trifluoromethyl)pyridin-4-amine (1.0g, 4.85 mmol) is dissolved in dry DCM (5 mL) and pyridine (2eq, 9.70 mmol, 0.78 mL) is added. To the clear solution is added 1,1'-Carbonyl-di-(1,2,4-triazole) (1.25eq, 6.06 mmol, 1g) and the reaction mixture is stirred at room temperature for 15 minutes. Then a solution of [5-methyl-6-[1-methyl-3-(trifluoromethyl)pyrazol-4-yl]pyridazin-3-yl]methanamine (1.2 eq, 5.82 mmol, 1.58g) in anhydrous DCM (9mL) is added slowly. The reaction mixture is stirred at room temperature until complete conversion. At room temperature and under stirring, the reaction mixture is added to HCl 1N solution (14 mL). DCM is evaporated under reduced pressure and the aqueous layer is extracted with EtOAc. The organic layer is washed with brine, dried over Na₂SO₄, filtered and concentrated under reduced pressure. The crude is purified by chromatography on SiO₂ (eluted with DCM/ Ethyl acetate from 100/0 to 30/70) to afford the desired product. LCMS: MW (calcd): 508; m/z MW (obsd): 509 (M+H).

**Table II. Intermediates towards illustrative compounds of the invention**

| **Int** # | **Structure** | **Starting material** | **Mtd** | **MW (calc)** | **MW (obs)** |
|---|---|---|---|---|---|
| Int. 1 | 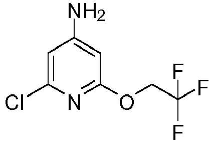 | 2,6-dichloropyridine-4-carboxylic acid | NA | 256 | 256 |
| Int. 2 | 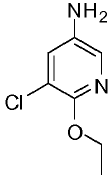 | 2,3-dichloro-5-nitropyridine | A | 173 | 173 |
| Int. 3 | 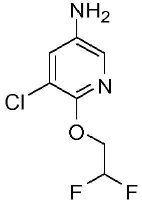 | 2,3-dichloro-5-nitropyridine | A | 209 | 209 |
| Int. 4 | 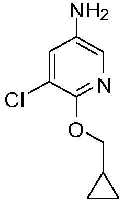 | 2,3-dichloro-5-nitropyridine | A | 199 | 199 |
| Int. 5 | 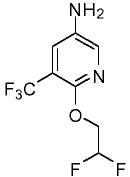 | 2-chloro-5-nitro-3-(trifluoromethyl)pyridine | A | 242 | 243 |
| Int. 6 | 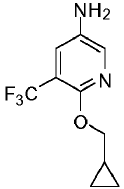 | 2-chloro-5-nitro-3-(trifluoromethyl)pyridine | A | 232 | 233 |
| Int. 7 | 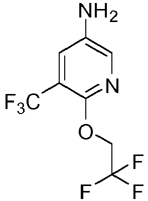 | 2-chloro-5-nitro-3-(trifluoromethyl)pyridine | A | 260 | 261 |
| Int. 8 | 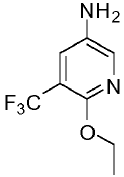 | 2-chloro-5-nitro-3-(trifluoromethyl)pyridine | NA | 206 | 207 |
| Int. 9 | 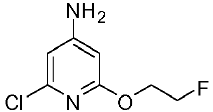 | 2,6-dichloropyridine-4-carboxylic acid | NA | 191 | 191 |
| Int. 10 | 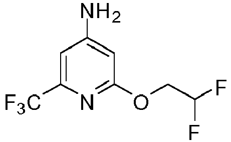 | 2-chloro-6-(trifluoromethyl)pyridine-4-carboxylic acid | NA | 242 | 243 |
| Int. 11 | 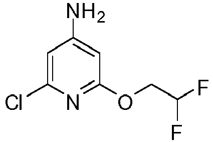 | 2,6-Dichloropyridin-4-amine | B | 209 | 209 |
| Int. 12 | 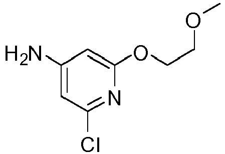 | 2,6-Dichloropyridin-4-amine | B | 203 | 203 |
| Int. 13 | 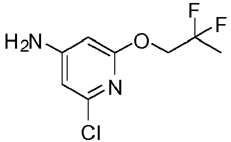 | 2,6-Dichloropyridin-4-amine | B | 223 | 223 |
| Int. 14 | 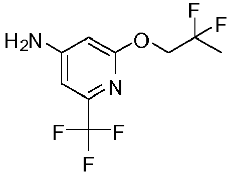 | 2-chloro-6-(trifluoromethyl)pyridin-4-amine | B | 256 | 257 |
| Int. 15 | 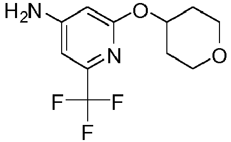 | 2-chloro-6-(trifluoromethyl)pyridin-4-amine | B | 262 | 263 |
| Int. 16 | 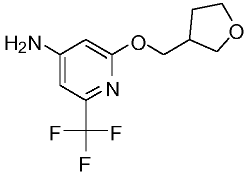 | 2-chloro-6-(trifluoromethyl)pyridin-4-amine | B | 262 | 263 |
| Int. 17 | 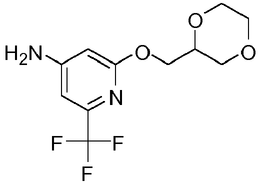 | 2-chloro-6-(trifluoromethyl)pyridin-4-amine | B | 278 | 279 |
| Int. 18 | 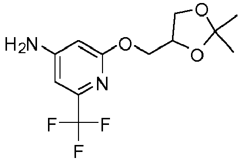 | 2-chloro-6-(trifluoromethyl)pyridin-4-amine | B | 292 | 293 |
| Int. 19 | 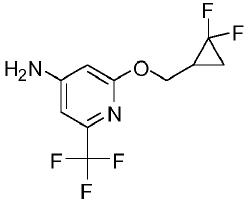 | 2-chloro-6-(trifluoromethyl)pyridin-4-amine | B | 268 | 269 |
| Int. 20 | 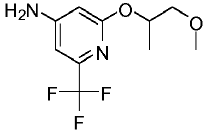 | 2-chloro-6-(trifluoromethyl)pyridin-4-amine | B | 250 | 251 |
| Int. 21 | 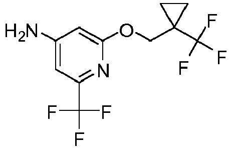 | 2-chloro-6-(trifluoromethyl)pyridin-4-amine | B | 300 | 301 |
| Int. 22 | 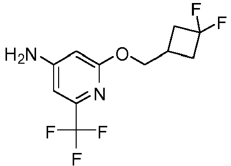 | 2-chloro-6-(trifluoromethyl)pyridin-4-amine | B | 282 | 283 |
| Int. 23 | 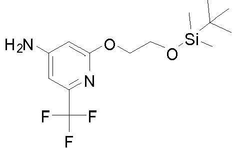 | 2-chloro-6-(trifluoromethyl)pyridin-4-amine | B | 336 | 337 |
| Int. 24 | 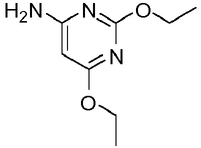 | 4-Amino-2,6-dichloropyrimidine | NA | 183 | 184 |
| Int. 25 | 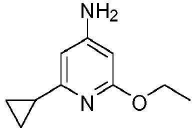 | 2-chloro-6-ethoxy-pyridin-4-amine | NA | 178 | 179 |
| Int. 26 | 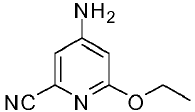 | 2-chloro-6-ethoxy-pyridin-4-amine | NA | 163 | 164 |
| Int. 27 | 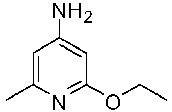 | 2-chloro-6-ethoxy-pyridin-4-amine | NA | 152 | 153 |
| Int. 28 | 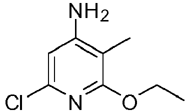 | 2-chloro-6-ethoxy-pyridin-4-amine | NA | 187 | 187 |
| Int. 29 | 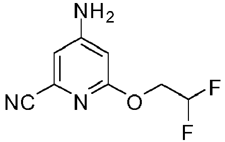 | Int. 11 | NA | 199 | 200 |
| Int. 30 | 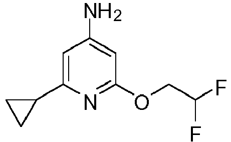 | Int. 11 | NA | 214 | 215 |
| Int. 31 | 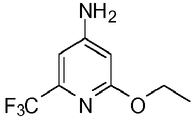 | 2-chloro-4-iodo-6-(trifluoromethyl)pyridine | C | 206 | 207 |
| Int. 32 | 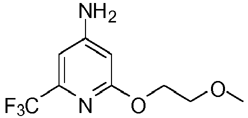 | 2-chloro-4-iodo-6-(trifluoromethyl)pyridine | C | 236 | 237 |
| Int. 33 | 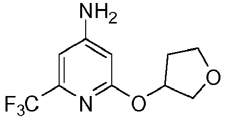 | 2-chloro-4-iodo-6-(trifluoromethyl)pyridine | C | 248 | 249 |
| Int. 34 | 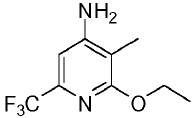 | Int. 31 | NA | 220 | 221 |
| Int. 35 | 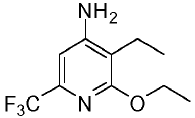 | Int. 31 | NA | 234 | 235 |
| Int. 36 | 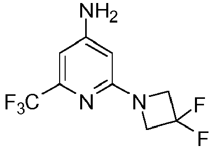 | 2-chloro-6-(trifluoromethyl)pyridin-4-amine | NA | 253 | 254 |
| Int. 37 | 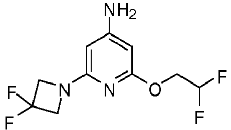 | Int. 11 | NA | 265 | 266 |
| Int. 38 | 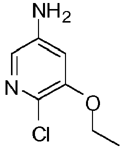 | 5-bromo-2-chloro-pyridin-3-ol | NA | 173 | 173 |
| Int. 39 | 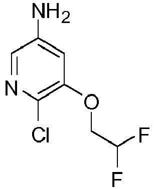 | 5-bromo-2-chloro-pyridin-3-ol | NA | 209 | 209 |
| Int. 40 | 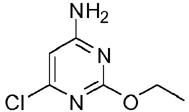 | 4-Amino-2,6-dichloropyrimidine | NA | 174 | 174 |
| Int. 41 | 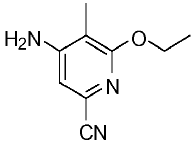 | 2-chloro-6-ethoxy-pyridin-4-amine | NA | 177 | 178 |
| Int. 42 | 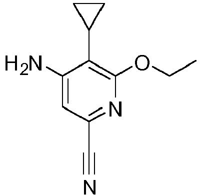 | 4-amino-5-bromo-6-ethoxy-pyridine-2-carbonitrile | NA | 203 | 204 |
| Int. 43 | 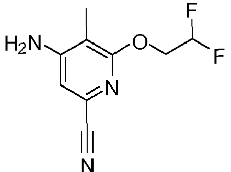 | Int. 11 | NA | 177 | 178 |
| Int. 44 | 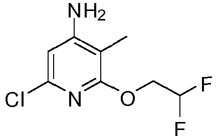 | Int. 11 | NA | 223 | 223 |
| Int. 45 | 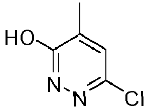 | 3,6-Dichloro-4-methylpyridazine | NA | 145 | 145 |
| Int. 46 | 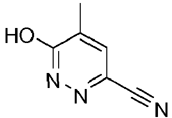 | Int. 45 | NA | 135 | 136 |
| Int. 47 | 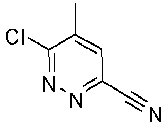 | Int. 46 | D | 154 | 154 |
| Int. 48 | 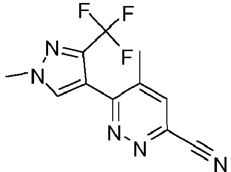 | Int. 47 | E | 267 | 268 |
| Int. 49 | 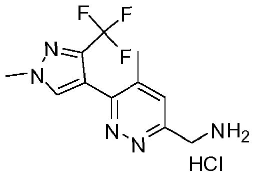 | Int. 48 | F1 | 271 | 272 |
| Int. 50 | 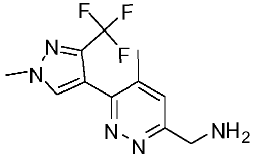 | Int. 48 | F2 | 271 | 272 |
| Int. 51 | 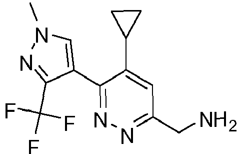 | Int. 82 | F3 | 297 | 299 |
| Int. 52 | 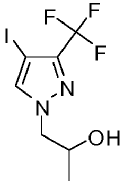 | 4-iodo-3-trifluoromethyl-1H-pyrazole | NA | 320 | 321 |
| Int. 53 | 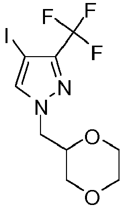 | 4-iodo-3-trifluoromethyl-1H-pyrazole | NA | 362 | 363 |
| Int. 54 | 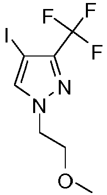 | 4-iodo-3-trifluoromethyl-1H-pyrazole | NA | 320 | 321 |
| Int. 55 | 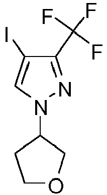 | 4-iodo-3-trifluoromethyl-1H-pyrazole | NA | 332 | 333 |
| Int. 56 | 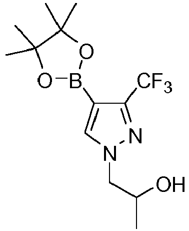 | Int. 52 | G | 320 | 321 |
| Int. 57 | 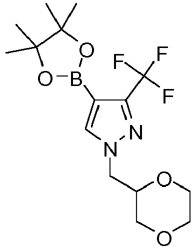 | Int. 53 | G | 362 | 363 |
| Int. 58 | 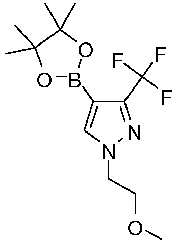 | Int. 54 | G | 320 | 321 |
| Int. 59 | 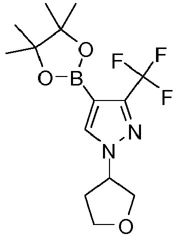 | Int. 55 | G | 332 | 333 |
| Int. 60 | 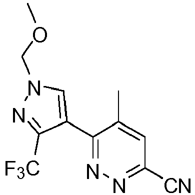 | Int. 47 & 1-(methoxymethyl)-4-(4,4,5,5-tetramethyl-1,3,2-dioxaborolan-2-yl)-3-(trifluoromethyl)pyrazole | E | 297 | 298 |
| Int. 61 | 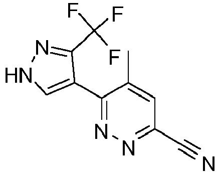 | Int. 47 & 4-(4,4,5,5-tetramethyl-1,3,2-dioxaborolan-2-yl)-3-(trifluoromethyl)-1H-pyrazole | E | 253 | 254 |
| Int. 62 | 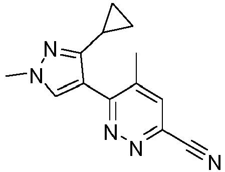 | Int. 47 & 3-cyclopropyl-1-methyl-4-(4,4,5,5-tetramethyl-1,3,2-dioxaborolan-2-yl)pyrazole | E | 239 | 240 |
| Int. 63 | 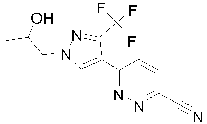 | Int. 47 & Int. 56 | E | 311 | 312 |
| Int. 64 | 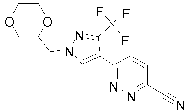 | Int. 47 & Int. 57 | E | 353 | 354 |
| Int. 65 | 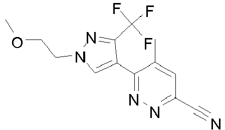 | Int. 47 & Int. 58 | E | 311 | 312 |
| Int. 66 | 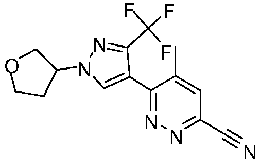 | Int. 47 & Int. 59 | E | 323 | 324 |
| Int. 67 | 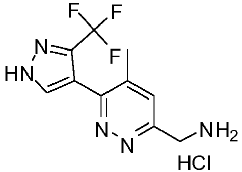 | Int. 61 | F1 | 257 | 258 |
| Int. 68 | 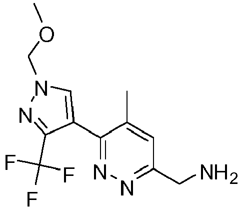 | Int. 60 | F2 | 301 | 302 |
| Int. 69 | 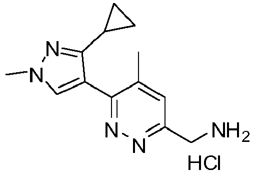 | Int. 62 | F1 | 243 | 244 |
| Int. 70 | 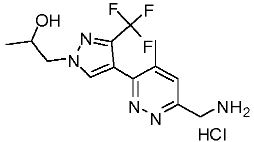 | Int. 63 | F1 | 315 | 316 |
| Int. 71 | 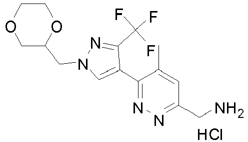 | Int. 64 | F1 | 357 | 358 |
| Int. 72 | 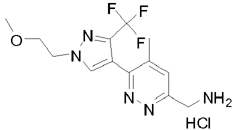 | Int. 65 | F1 | 315 | 316 |
| Int. 73 | 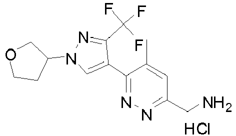 | Int. 66 | F1 | 332 | 333 |
| Int. 74 | 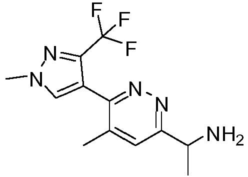 | Int. 48 | NA | 285 | 286 |
| Int. 75 | 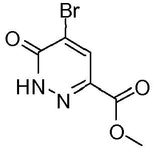 | 6-oxo-1H-pyridazine-3-carboxylate | NA | 233 | 235 |
| Int. 76 | 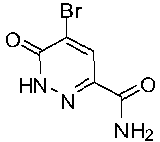 | Int. 75 | NA | 218 | 220 |
| Int. 77 | 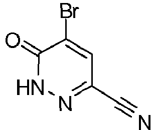 | Int. 76 | NA | 200 | 197.86 [M-1] |
| Int. 78 | 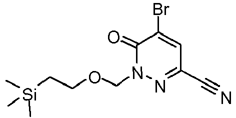 | Int. 77 | NA | 330 | NA |
| Int. 79 | 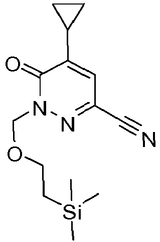 | Int. 78 | NA | 291 | NA |
| Int. 80 | 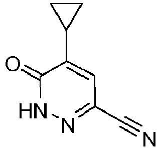 | Int. 79 | NA | 161 | 162 |
| Int. 81 | 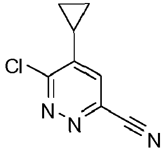 | Int. 80 | D | 180 | 180 |
| Int. 82 | 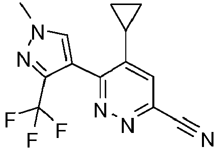 | Int. 81 | E | 293 | 294 |
| Int. 83 | 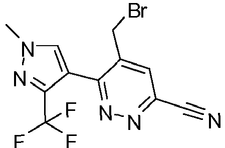 | Int. 48 | NA | 346 | 346 |
| Int. 84 | 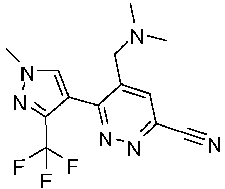 | Int. 83 | NA | 310 | 311 |
| Int. 85 | 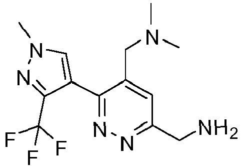 | Int. 84 | F3 | 314 | 316 |
| Int. 86 | 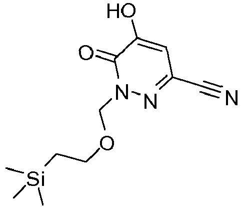 | Int. 78 | NA | 267 | 266.09 (negative mode) |
| Int. 87 | 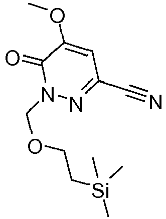 | Int. 86 | NA | 281 | NA |
| Int. 88 | 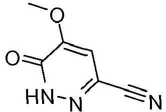 | Int. 87 | NA | 151 | 152 |
| Int. 89 | 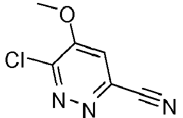 | Int. 88 | D | 170 | 170 |
| Int. 90 | 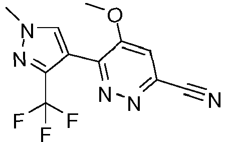 | Int. 89 | E | 283 | 284 |
| Int. 91 | 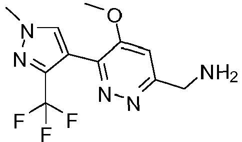 | Int. 90 | F3 | 287 | 288 |
| Int. 92 | 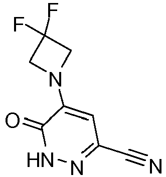 | Int. 77 | G | 212 | 213 |
| Int. 93 | 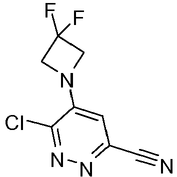 | Int. 92 | D | 231 | 231 |
| Int. 94 | 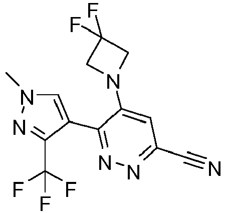 | Int. 93 | E | 344 | 345 |
| Int. 95 | 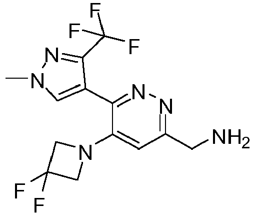 | Int. 94 | F3 | 348 | 349 |
| Int. 96 | 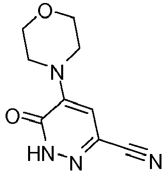 | Int. 77 | G | 206 | 207 |
| Int. 97 | 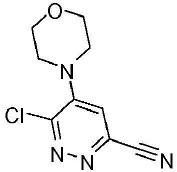 | Int. 96 | D | 225 | 225 |
| Int. 98 | 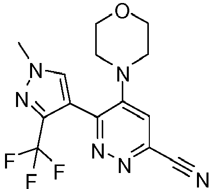 | Int. 97 | E | 338 | 340 |
| Int. 99 | 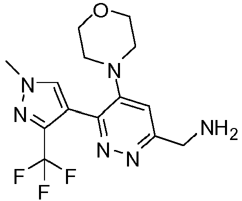 | Int. 98 | F3 | 342 | 344 |
| Int. 100 | 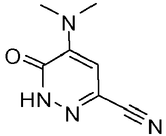 | Int. 77 | G | 164 | 165 |
| Int. 101 | 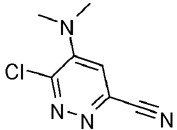 | Int. 100 | D | 183 | 183 |
| Int. 102 | 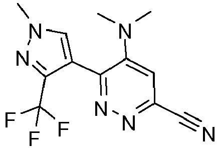 | Int. 101 | E | 296 | 297 |
| Int. 103 | 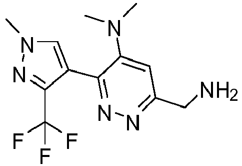 | Int. 102 | F3 | 300 | 302 |
| Int. 104 | 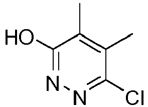 | 3,6-dichloro-4,5-dimethyl-pyridazine | NA | 159 | 160 |
| Int. 105 | 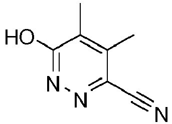 | Int. 104 | NA | 149 | 150 |
| Int. 106 | 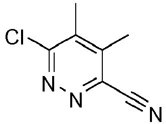 | Int. 105 | D | 168 | 168 |
| Int. 107 | 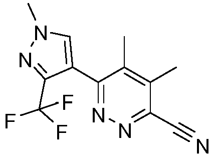 | Int. 106 & 1-methyl-4-(4,4,5,5-tetramethyl-1,3,2-dioxaborolan-2-yl)-3-(trifluoromethyl)pyrazole | E | 281 | 282 |
| Int. 108 | 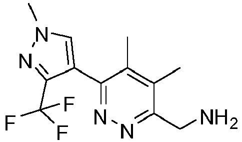 | Int. 107 | F3 | 285 | 286 |
| Int. 109 | 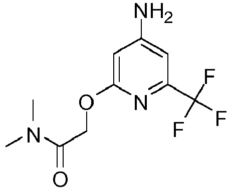 | 2-chloro-6-(trifluoromethyl)pyridin-4-amine | B | 263 | 264 |
| Int. 110 | 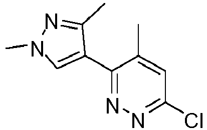 | 3,6-dichloro-4-methylpyridazine & 1,3-dimethyl-4-(4,4,5,5-tetramethyl-1,3,2-dioxaborolan-2-yl)pyrazole | NA | 223 | 223 |
| Int. 111 | 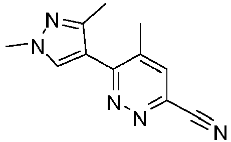 | Int. 110 & 2-chloro-6-ethoxy-pyridin-4-ylamine | NA | 213 | 214 |
| Int. 112 | 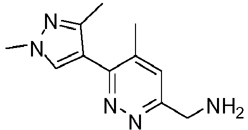 | Int. 111 | F2 | 217 | 218 |
| Int. 113 | 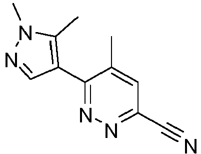 | Int. 47 & (1,5-dimethylpyrazol-4-yl)boronic acid | E | 213 | 214 |
| Int. 114 | 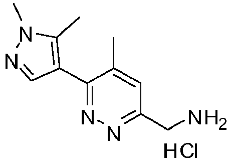 | Int. 113 | F1 | 217 | 218 |
| Int. 115 | 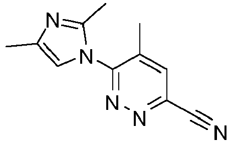 | Int. 47 & 2,4-dimethyl-1H-imidazole | NA | 213 | 214 |
| Int. 116 | 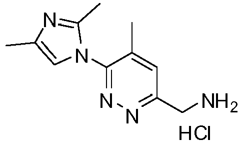 | Int. 115 | F1 | 217 | 218 |
| Int. 117 | 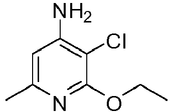 | Int. 27 | NA | 187 | 187 |
| Int. 118 | 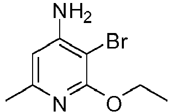 | Int. 27 | NA | 231 | 233 |
| Int. 119 | 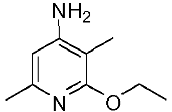 | Int. 118 | NA | 166 | 167 |
| Int. 120 | 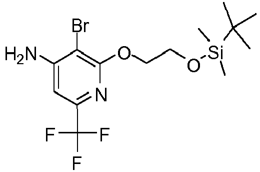 | Int. 23 | NA | 415 | 417 |
| Int. 121 | 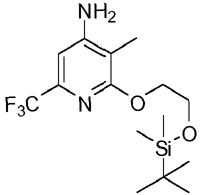 | Int. 120 | NA | 350 | 351 |
| Int. 122 | 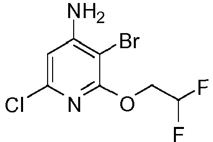 | Int. 11 | NA | 288 | 289 |
| Int. 123 | 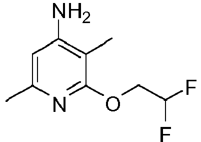 | Int. 122 | NA | 202 | 203 |
| Int. 124 | 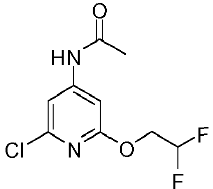 | Int. 11 | NA | 251 | 251 |
| Int. 125 | 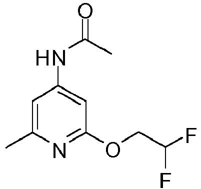 | Int. 124 | NA | 230 | 231 |
| Int. 126 | 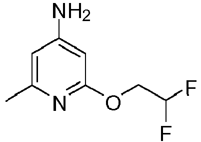 | Int. 125 | NA | 188 | 189 |
| Int. 127 | 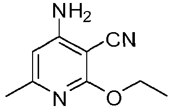 | Int. 118 | NA | 177 | 178 |
| Int. 128 | 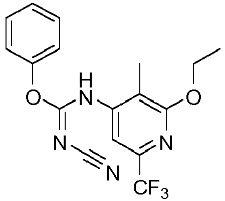 | Int. 34 | I | 364 | 365 |
| Int. 129 | 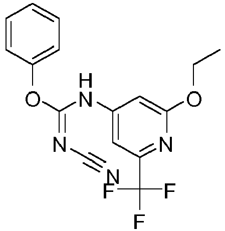 | Int. 31 | I | 350 | 351 |
| Int. 130 | 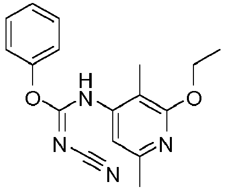 | Int. 119 | I | 310 | 331 |
| Int. 131 | 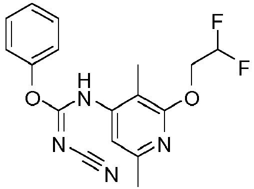 | Int. 123 | I | 346 | 347 |
| Int. 132 | 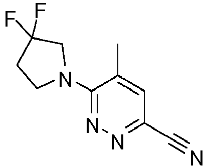 | Int. 47 | K1 | 224 | 225 |
| Int. 133 | 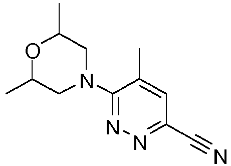 | Int. 47 | K2 | 232 | 233 |
| Int. 134 | 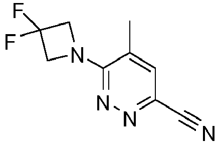 | Int. 47 | K2 | 210 | 211 |
| Int. 135 | 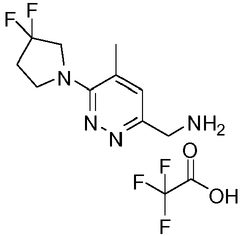 | Int. 132 | F4 | 228 | 229 |
| Int. 136 | 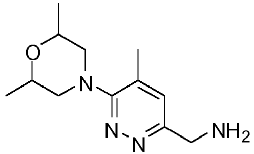 | Int. 133 | F2 | 236 | 237 |
| Int. 137 | 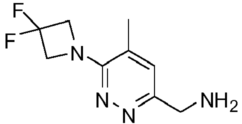 | Int. 134 | F2 | 214 | 215 |
| Int. 138 | 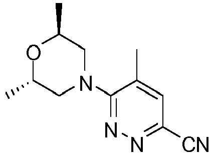 | Int. 47 | K3 | 232 | 233 |
| Int. 139 | 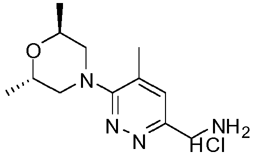 | Int. 138 | F1 | 236 | 237 |
| Int. 140 | 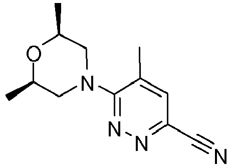 | Int. 47 | K3 | 232 | 233 |
| Int. 141 | 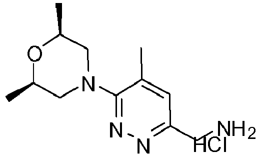 | Int. 140 | F1 | 236 | 237 |
| Int. 142 | 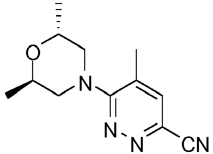 | Int. 47 | K3 | 232 | 233 |
| Int. 143 | 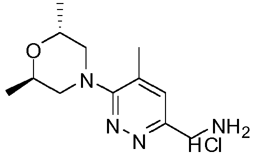 | Int. 142 | F1 | 236 | 237 |
| Int. 144 | 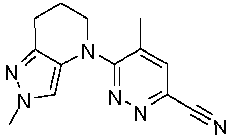 | Int. 47 | NA | 254 | 255 |
| Int. 145 | 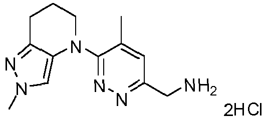 | Int. 144 | F1 | 258 | 259 |
| Int. 146 | 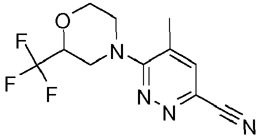 | Int. 47 | K3 | 272 | 273 |
| Int. 147 | 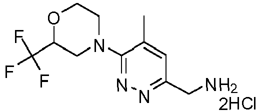 | Int. 146 | F1 | 276 | 277 |
| Int. 148 | 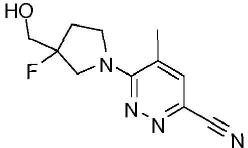 | Int. 47 | K3 | 236 | 237 |
| Int. 149 | 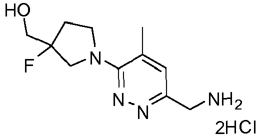 | Int. 148 | F1 | 240 | 241 |
| Int. 150 | 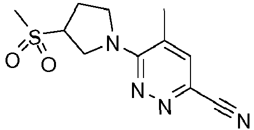 | Int. 47 | K3 | 266 | 267 |
| Int. 151 | 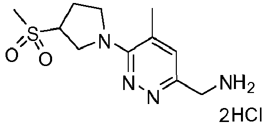 | Int. 150 | F1 | 270 | 271 |
| Int. 152 | 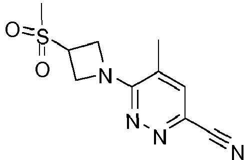 | Int. 47 | K3 | 252 | 253 |
| Int. 153 | 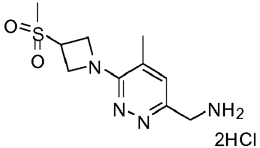 | Int. 152 | F1 | 256 | 258 |
| Int. 154 | 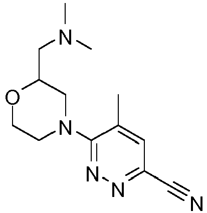 | Int. 47 | K3 | 261 | 262 |
| Int. 155 | 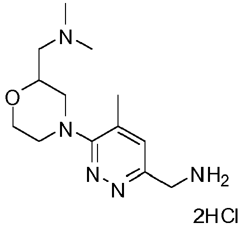 | Int. 154 | K3 | 265 | 266 |
| Int. 156 | 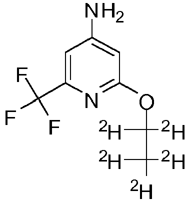 | 2-chloro-4-iodo-6-(trifluoromethyl)pyridine | NA | 211 | 212 |

**Table III. Illustrative compounds of the invention**

| **Cpd** # | **Structure** | **Name** | **Int** | **Mtd** | **MW (calc)** | **MW (obs)** |
|---|---|---|---|---|---|---|
| 1 | 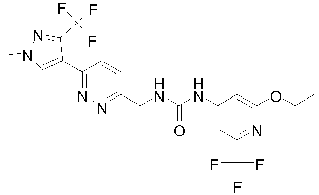 | 1 -(2-Ethoxy-6-trifluoromethyl-pyridin-4-yl)-3-[5-methyl-6-(1-methyl-3-trifluoromethyl-1H-pyrazol-4-yl)-pyridazin-3-ylmethyl]-urea | Int. 49 & Int. 31 | H1 | 503 | 504 |
| 2 | 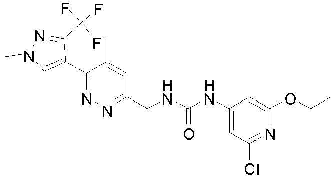 | 1-(2-Chloro-6-ethoxy-pyridin-4-yl)-3-[5-methyl-6-(1-methyl-3-trifluoromethyl-1H-pyrazol-4-yl)-pyridazin-3-ylmethyl]-urea | Int. 49 & 2-chloro-6-ethoxy-pyridin-4-ylamine | H2 | 470 | 470 |
| 3 | 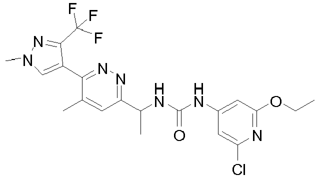 | 1-(2-chloro-6-ethoxy-4-pyridyl)-3-[1-[5-methyl-6-[1-methyl-3-(trifluoromethyl)pyrazol-4-yl]pyridazin-3-yl]ethyl]urea | Int. 74 & 2-chloro-6-ethoxy-pyridin-4-ylamine | H2 | 484 | 484 |
| 4 | 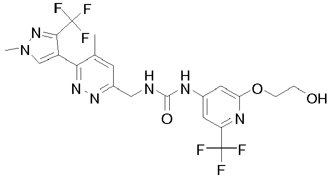 | 1-[2-(2-hydroxyethoxy)-6-(trifluoromethyl)-4-pyridyl]-3-[[5-methyl-6-[1-methyl-3-(trifluoromethyl)pyrazol-4-yl]pyridazin-3-yl]methyl]urea | Int. 49 & Int. 23 | NA | 519 | 520 |
| 5 | 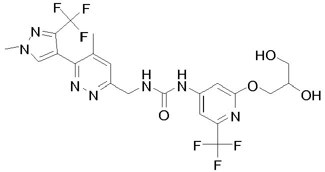 | 1-[2-(2,3-dihydroxypropoxy)-6-(trifluoromethyl)-4-pyridyl]-3-[[5-methyl-6-[1-methyl-3-(trifluoromethyl)pyrazol-4-yl]pyridazin-3-yl]methyl]urea | Int. 49 & Int. 18 | NA | 549 | 550 |
| 6 | 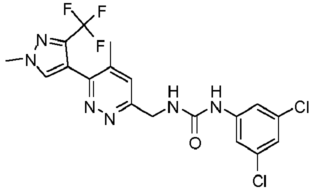 | 1-(3,5-dichlorophenyl)-3-[[5-methyl-6-[1-methyl-3-(trifluoromethyl)pyrazol-4-yl]pyridazin-3-yl]methyl]urea | Int. 49 & 1,3-dichloro-5-isocyanato-benzene, | NA | 459 | 459 |
| 7 | 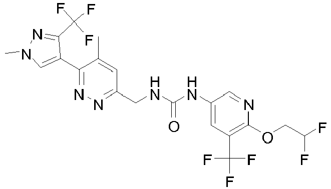 | 1-[6-(2,2-difluoroethoxy)-5-(trifluoromethyl)-3-pyridyl]-3-[[5-methyl-6-[1-methyl-3-(trifluoromethyl) pyrazol-4-yl]pyridazin-3-yl]methyl]urea | Int. 50 & Int. 5 | H2 | 539 | 540 |
| 8 | 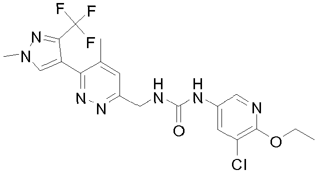 | 1-(5-chloro-6-ethoxy-3-pyridyl)-3-[[5-methyl-6-[1-methyl-3-(trifluoromethyl)pyrazol-4-yl]pyridazin-3-yl]methyl]urea | Int. 50 & Int. 2 | H2 | 470 | 470 |
| 9 | 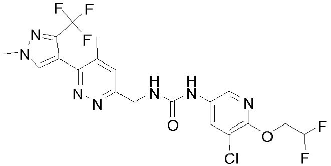 | 1-[5-chloro-6-(2,2-difluoroethoxy)-3-pyridyl]-3-[[5-methyl-6-[1-methyl-3-(trifluoromethyl) pyrazol-4-yl]pyridazin-3-yl]methyl]urea | Int. 50 & Int. 3 | H2 | 506 | 506 |
| 10 | 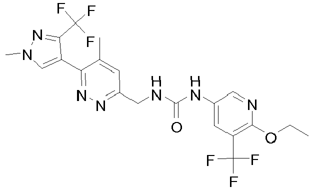 | 1-[6-ethoxy-5-(trifluoromethyl)-3-pyridyl]-3-[[5-methyl-6-[1-methyl-3-(trifluoromethyl)pyrazol-4-yl]pyridazin-3-yl]methyl]urea | Int. 50 & Int. 8 | H2 | 503 | 504 |
| 11 | 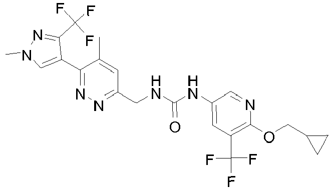 | 1-[6-(cyclopropylmethoxy)-5-(trifluoromethyl)-3-pyridyl]-3-[[5-methyl-6-[1-methyl-3-(trifluoromethyl)pyrazol-4-yl]pyridazin-3-yl]methyl]urea | Int. 50 & Int. 6 | H2 | 529 | 530 |
| 12 | 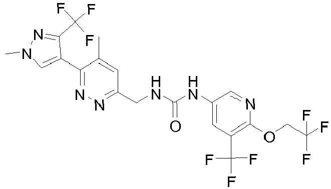 | 1-[[5-methyl-6-[1-methyl-3-(trifluoromethyl)pyrazol-4-yl]pyridazin-3-yl]methyl]-3-[6-(2,2,2-trifluoroethoxy)-5-(trifluoromethyl)-3-pyridyl]urea | Int. 50 & Int. 7 | H2 | 557 | 558 |
| 13 | 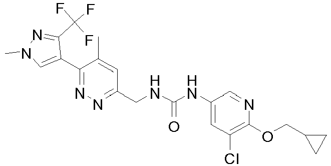 | 1-[5-chloro-6-(cyclopropylmethoxy)-3-pyridyl]-3-[[5-methyl-6-[1-methyl-3-(trifluoromethyl)pyrazol-4-yl]pyridazin-3-yl]methyl]urea | Int. 50 & Int. 4 | H2 | 496 | 496 |
| 14 | 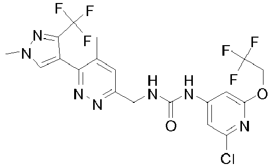 | 1-[2-chloro-6-(2,2,2-trifluoroethoxy)-4-pyridyl]-3-[[5-methyl-6-[1-methyl-3-(trifluoromethyl)pyrazol-4-yl]pyridazin-3-yl]methyl]urea | Int. 50 & Int. 1 | H2 | 524 | 524 |
| 15 | 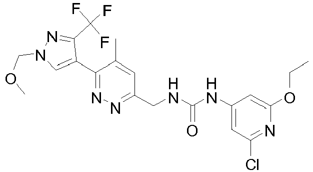 | 1-(2-chloro-6-ethoxy-4-pyridyl)-3-[[6-[1-(methoxymethyl)-3-(trifluoromethyl)pyrazol-4-yl]-5-methyl-pyridazin-3-yl]methyl]urea | Int. 68 & 2-chloro-6-ethoxy-pyridin-4-amine | H2 | 500 | 500 |
| 16 | 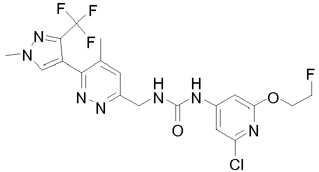 | 1-[2-chloro-6-(2-fluoroethoxy)-4-pyridyl]-3-[[5-methyl-6-[1-methyl-3-(trifluoromethyl)pyrazol-4-yl]pyridazin-3-yl]methyl]urea | Int. 49 & Int. 9 | H1 | 488 | 488 |
| 17 | 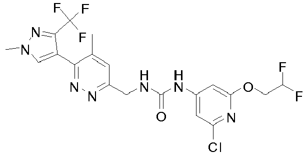 | 1-[2-chloro-6-(2,2-difluoroethoxy)-4-pyridyl]-3-[[5-methyl-6-[1-methyl-3-(trifluoromethyl)pyrazol-4-yl]pyridazin-3-yl]methyl]urea | Int. 49 & Int. 11 | H1 | 506 | 506 |
| 18 | 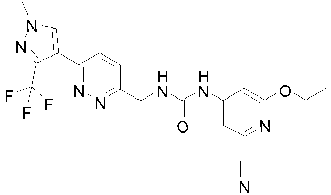 | 1-(2-cyano-6-ethoxy-4-pyridyl)-3-[[5-methyl-6-[1-methyl-3-(trifluoromethyl)pyrazol-4-yl]pyridazin-3-yl]methyl]urea | Int. 49 & Int. 26 | H1 | 460 | 461 |
| 19 | 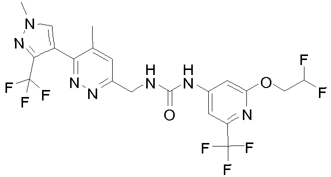 | 1-[2-(2,2-difluoroethoxy)-6-(trifluoromethyl)-4-pyridyl]-3-[[5-methyl-6-[1-methyl-3-(trifluoromethyl)pyrazol-4-yl]pyridazin-3-yl]methyl]urea | Int. 49 & Int. 10 | H1 | 539 | 540 |
| 20 | 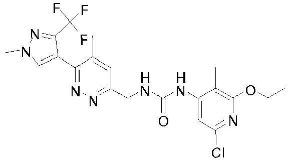 | 1-(6-chloro-2-ethoxy-3-methyl-4-pyridyl)-3-[[5-methyl-6-[1-methyl-3-(trifluoromethyl)pyrazol-4-yl]pyridazin-3-yl]methyl]urea | Int. 49 & Int. 28 | H1 | 484 | 484 |
| 21 | 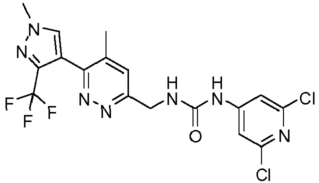 | 1-(2,6-dichloro-4-pyridyl)-3-[[5-methyl-6-[1-methyl-3-(trifluoromethyl)pyrazol-4-yl]pyridazin-3-yl]methyl]urea | Int. 49 & 4-amino-2,6-dichloropyridine | H1 | 460 | 460 |
| 22 | 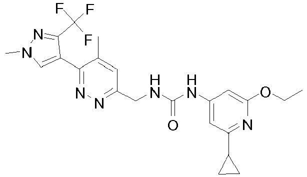 | 1-(2-cyclopropyl-6-ethoxy-4-pyridyl)-3-[[5-methyl-6-[1-methyl-3-(trifluoromethyl)pyrazol-4-yl]pyridazin-3-yl]methyl]urea | Int. 49 & Int. 25 | H1 | 475 | 476 |
| 23 | 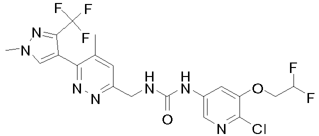 | 1-[6-chloro-5-(2,2-difluoroethoxy)-3-pyridyl]-3-[[5-methyl-6-[1-methyl-3-(trifluoromethyl)pyrazol-4-yl]pyridazin-3-yl]methyl]urea | Int. 49 & Int. 39 | H1 | 506 | 506 |
| 24 | 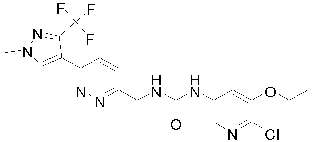 | 1-(6-chloro-5-ethoxy-3-pyridyl)-3-[[5-methyl-6-[1-methyl-3-(trifluoromethyl)pyrazol-4-yl]pyridazin-3-yl]methyl]urea | Int. 49 & Int. 38 | H1 | 470 | 470 |
| 25 | 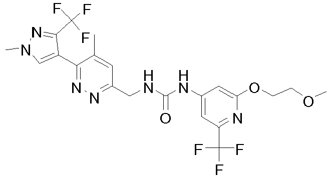 | 1-[2-(2-methoxyethoxy)-6-(trifluoromethyl)-4-pyridyl]-3-[[5-methyl-6-[1-methyl-3-(trifluoromethyl)pyrazol-4-yl]pyridazin-3-yl]methyl]urea | Int. 49 & Int. 32 | H1 | 533 | 534 |
| 26 | 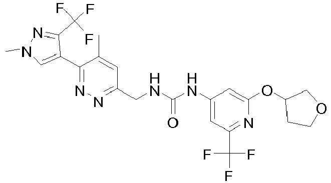 | 1-[[5-methyl-6-[1-methyl-3-(trifluoromethyl)pyrazol-4-yl]pyridazin-3-yl]methyl]-3-[2-tetrahydrofuran-3-yloxy-6-(trifluoromethyl)-4-pyridyl]urea | Int. 49 & Int. 33 | H1 | 545 | 546 |
| 27 | 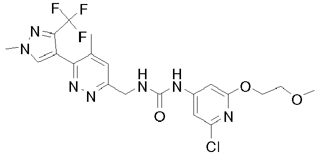 | 1-[2-chloro-6-(2-methoxyethoxy)-4-pyridyl]-3-[[5-methyl-6-[1-methyl-3-(trifluoromethyl)pyrazol-4-yl]pyridazin-3-yl]methyl]urea | Int. 50 & Int. 12 | H2 | 500 | 500 |
| 28 | 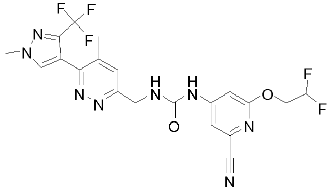 | 1-[2-cyano-6-(2,2-difluoroethoxy)-4-pyridyl]-3-[[5-methyl-6-[1-methyl-3-(trifluoromethyl)pyrazol-4-yl]pyridazin-3-yl]methyl]urea | Int. 50 & Int. 29 | H2 | 496 | 497 |
| 29 | 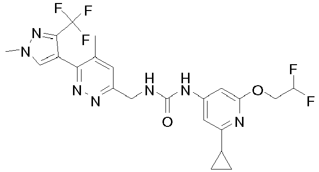 | 1-[2-cyclopropyl-6-(2,2-difluoroethoxy)-4-pyridyl]-3-[[5-methyl-6-[1-methyl-3-(trifluoromethyl)pyrazol-4-yl]pyridazin-3-yl]methyl]urea | Int. 50 & Int. 30 | H2 | 511 | 512 |
| 30 | 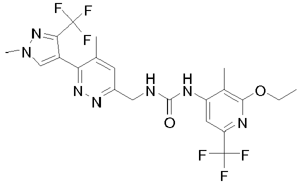 | 1-[2-ethoxy-3-methyl-6-(trifluoromethyl)-4-pyridyl]-3-[[5-methyl-6-[1-methyl-3-(trifluoromethyl)pyrazol-4-yl]pyridazin-3-yl]methyl]urea | Int. 49 & Int. 34 | H1 | 517 | 518 |
| 31 | 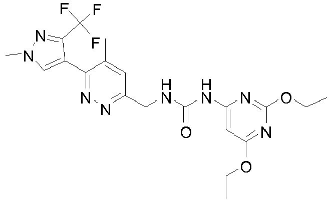 | 1-(2,6-diethoxypyrimidi n-4-yl)-3-[[5-methyl-6-[1-methyl-3-(trifluoromethyl)pyrazol-4-yl]pyridazin-3-yl]methyl]urea | Int. 49 & Int. 24 | H1 | 480 | 481 |
| 32 | 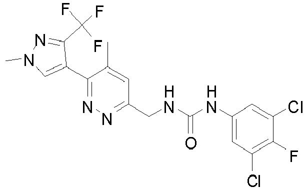 | 1-(3,5-dichloro-4-fluoro-phenyl)-3-[[5-methyl-6-[1-methyl-3-(trifluoromethyl)pyrazol-4-yl]pyridazin-3-yl]methyl]urea | Int. 49 & 3,5-dichloro-4-fluoro aniline | H1 | 477 | 477 |
| 33 | 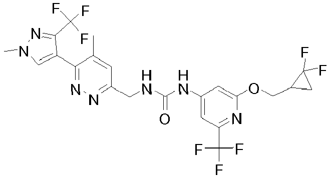 | 1-[2-[(2,2-difluorocyclopropyl)methoxy]-6-(trifluoromethyl)-4-pyridyl]-3-[[5-methyl-6-[1-methyl-3-(trifluoromethyl)pyrazol-4-yl]pyridazin-3-yl]methyl]urea | Int. 49 & Int. 19 | H1 | 565 | 566 |
| 34 | 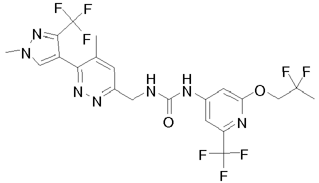 | 1-[2-(2,2-difluoropropoxy)-6-(trifluoromethyl)-4-pyridyl]-3-[[5-methyl-6-[1-methyl-3-(trifluoromethyl)pyrazol-4-yl]pyridazin-3-yl]methyl]urea | Int. 49 & Int. 14 | H1 | 553 | 554 |
| 35 | 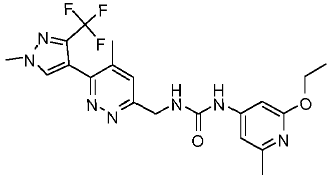 | 1-(2-ethoxy-6-methyl-4-pyridyl)-3-[[5-methyl-6-[1-methyl-3-(trifluoromethyl)pyrazol-4-yl]pyridazin-3-yl]methyl]urea | Int. 49 & Int. 27 | H1 | 449 | 450 |
| 36 | 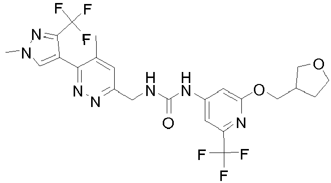 | 1-[[5-methyl-6-[1-methyl-3-(trifluoromethyl)pyrazol-4-yl]pyridazin-3-yl]methyl]-3-[2-(tetrahydrofuran-3-ylmethoxy)-6-(trifluoromethyl)-4-pyridyl]urea | Int. 49 & Int. 16 | H1 | 559 | 560 |
| 37 | 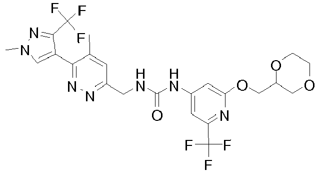 | 1-[2-(1,4-dioxan-2-ylmethoxy)-6-(trifluoromethyl)-4-pyridyl]-3-[[5-methyl-6-[1-methyl-3-(trifluoromethyl)pyrazol-4-yl]pyridazin-3-yl]methyl]urea | Int. 49 & Int. 17 | H1 | 575 | 576 |
| 38 | 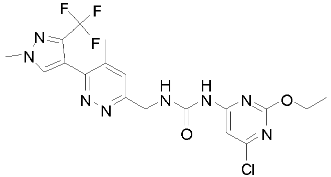 | 1-(6-chloro-2-ethoxy-pyrimidin-4-yl)-3-[[5-methyl-6-[1-methyl-3-(trifluoromethyl)pyrazol-4-yl]pyridazin-3-yl]methyl]urea | Int. 49 & Int. 40 | H1 | 471 | 471 |
| 39 | 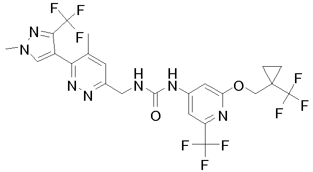 | 1-[[5-methyl-6-[1-methyl-3-(trifluoromethyl)pyrazol-4-yl]pyridazin-3-yl]methyl]-3-[2-(trifluoromethyl)-6-[[1-(trifluoromethyl)c yclopropyl]methoxy]-4-pyridyl]urea | Int. 49 & Int. 21 | H1 | 597 | 598 |
| 40 | 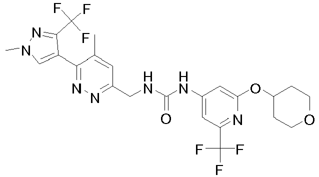 | 1-[[5-methyl-6-[1-methyl-3-(trifluoromethyl)pyrazol-4-yl]pyridazin-3-yl]methyl]-3-[2-tetrahydropyran-4-yloxy-6-(trifluoromethyl)-4-pyridyl]urea | Int. 49 & Int. 15 | H1 | 559 | 560 |
| 41 | 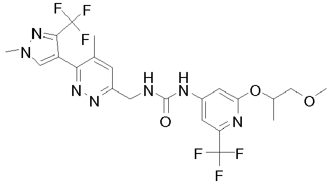 | 1-[2-(2-methoxy-1-methyl-ethoxy)-6-(trifluoromethyl)-4-pyridyl]-3-[[5-methyl-6-[1-methyl-3-(trifluoromethyl)pyrazol-4-yl]pyridazin-3-yl]methyl]urea | Int. 49 & Int. 20 | H1 | 547 | 548 |
| 42 | 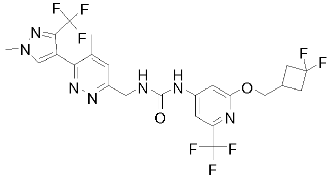 | 1-[2-[(3,3-difluorocyclobutyl)methoxy]-6-(trifluoromethyl)-4-pyridyl]-3-[[5-methyl-6-[1-methyl-3-(trifluoromethyl)pyrazol-4-yl]pyridazin-3-yl]methyl]urea | Int. 49 & Int. 22 | H1 | 579 | 580 |
| 43 | 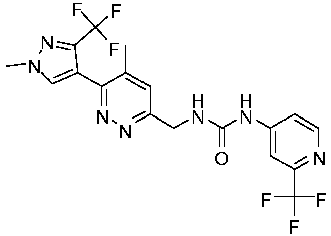 | 1-[[5-methyl-6-[1-methyl-3-(trifluoromethyl)pyrazol-4-yl]pyridazin-3-yl]methyl]-3-[2-(trifluoromethyl)-4-pyridyl]urea | Int. 49 & 4-amino-2-trifluoromethylpyridine | H1 | 459 | 460 |
| 44 | 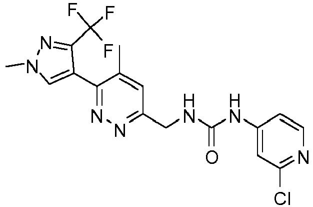 | 1-(2-chloro-4-pyridyl)-3-[[5-methyl-6-[1-methyl-3-(trifluoromethyl)pyrazol-4-yl]pyridazin-3-yl]methyl]urea | Int. 49 & 2-chloro-4-aminopyridine | H1 | 426 | 426 |
| 45 | 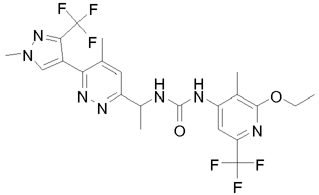 | 1-[2-ethoxy-3-methyl-6-(trifluoromethyl)-4-pyridyl]-3-[1-[5-methyl-6-[1-methyl-3-(trifluoromethyl)pyrazol-4-yl]pyridazin-3-yl]ethyl]urea | Int. 74 & Int.34 | H2 | 531 | 532 |
| 46 | 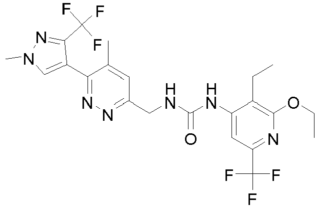 | 1-[2-ethoxy-3-ethyl-6-(trifluoromethyl)-4-pyridyl]-3-[[5-methyl-6-[1-methyl-3-(trifluoromethyl)pyrazol-4-yl]pyridazin-3-yl]methyl]urea | Int. 49 & Int. 35 | H1 | 531 | 532 |
| 47 | 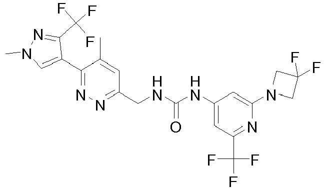 | 1-[2-(3,3-difluoroazetidin-1-yl)-6-(trifluoromethyl)-4-pyridyl]-3-[[5-methyl-6-[1-methyl-3-(trifluoromethyl)pyrazol-4-yl]pyridazin-3-yl]methyl]urea | Int. 49 & Int. 36 | H1 | 550 | 551 |
| 48 | 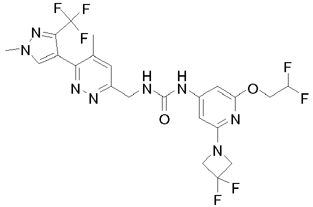 | 1-[2-(3,3-difluoroazetidin-1-yl)-6-(2,2-difluoroethoxy)-4-pyridyl]-3-[[5-methyl-6-[1-methyl-3-(trifluoromethyl)pyrazol-4-yl]pyridazin-3-yl]methyl]urea | Int. 49 & Int. 37 | H1 | 562 | 563 |
| 49 | 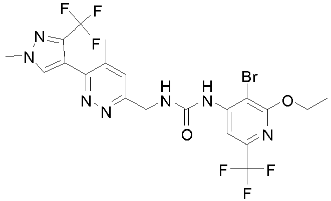 | 1-[3-bromo-2-ethoxy-6-(trifluoromethyl)-4-pyridyl]-3-[[5-methyl-6-[1-methyl-3-(trifluoromethyl)pyrazol-4-yl]pyridazin-3-yl]methyl]urea | Int. 49 & 3-bromo-2-ethoxy-6-(trifluoro methyl)py ndm-4-amine (described in synthesis in Int. 34) | H1 | 582 | 584 |
| 50 | 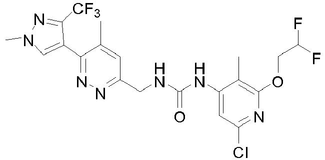 | 1-[6-chloro-2-(2,2-difluoroethoxy)-3-methyl-4-pyridyl]-3-[[5-methyl-6-[1-methyl-3-(trifluoromethyl)pyrazol-4-yl]pyridazin-3-yl]methyl]urea | Int. 49 & Int. 44 | H1 | 520 | 520 |
| 51 | 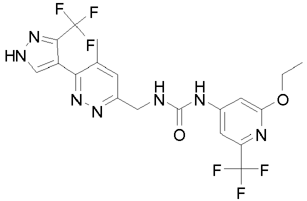 | 1-[2-ethoxy-6-(trifluoromethyl)-4-pyridyl]-3-[[5-methyl-6-[3-(trifluoromethyl)-1H-pyrazol-4-yl]pyridazin-3-yl]methyl]urea | Int. 67 & Int. 31 | H1 | 489 | 490 |
| 52 | 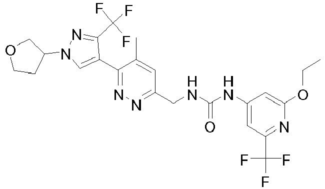 | 1-[2-ethoxy-6-(trifluoromethyl)-4-pyridyl]-3-[[5-methyl-6-[1-tetrahydrofuran-3-(trifluoromethyl)pyrazol-4-yl]pyridazin-3-yl]methyl]urea | Int. 73 & Int. 31 | H1 | 560 | 560 |
| 53 | 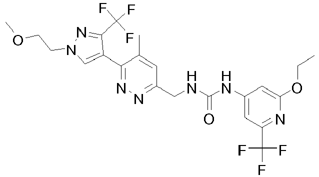 | 1-[2-ethoxy-6-(trifluoromethyl)-4-pyridyl]-3-[[6-[1-(2-methoxyethyl)-3-(trifluoromethyl)pyrazol-4-yl]-5-methyl-pyridazin-3-yl]methyl]urea | Int. 72 & Int. 31 | H1 | 548 | 548 |
| 54 | 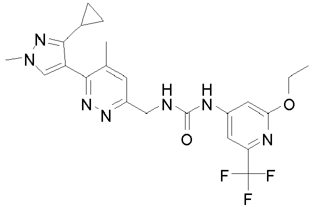 | 1-[[6-(3-cyclopropyl-1-methyl-pyrazol-4-yl)-5-methyl-pyridazin-3-yl]methyl]-3-[2-ethoxy-6-(trifluoromethyl)-4-pyridyl]urea | Int. 69 & Int. 31 | H1 | 476 | 476 |
| 55 | 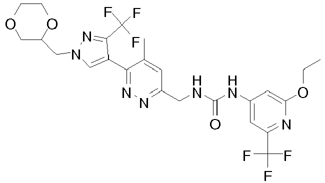 | 1-[[6-[1-(1,4-dioxan-2-ylmethyl)-3-(trifluoromethyl)pyrazol-4-yl]-5-methyl-pyndazm-3-yl]methyl]-3-[2-ethoxy-6-(trifluoromethyl)-4-pyridyl]urea | Int. 71 & Int. 31 | H1 | 590 | 590 |
| 56 | 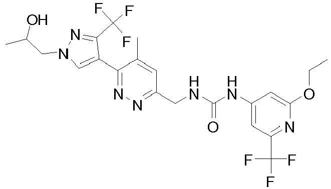 | 1-[2-ethoxy-6-(trifluoromethyl)-4-pyridyl]-3-[[6-[1-(2-hydroxypropyl)-(trifluoromethyl)pyrazol-4-yl]-5-methyl-pyridazin-3-yl]methyl]urea | Int. 70 & Int.31 | H1 | 548 | 548 |
| 57 | 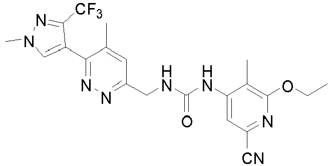 | 1-(6-cyano-2-ethoxy-3-methyl-4-pyridyl)-3-[[5-methyl-6-[1-methyl-3-(trifluoromethyl)pyrazol-4-yl]pyridazin-3-yl]methyl]urea | Int. 49 & Int. 41 | H1 | 474 | 475 |
| 58 | 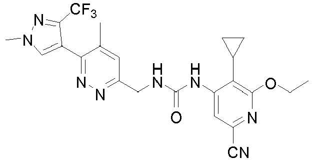 | 1-(6-cyano-3-cyclopropyl-2-ethoxy-4-pyridyl)-3-[[5-methyl-6-[1-methyl-3-(trifluoromethyl)pyrazol-4-yl]pyridazin-3-yl]methyl]urea | Int. 49 & Int. 42 | H1 | 501 | 501 |
| 59 | 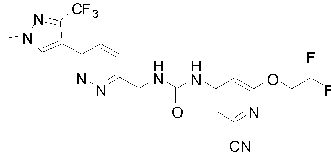 | 1-[6-cyano-2-(2,2-difluoroethoxy)-3-methyl-4-pyridyl]-3-[[5-methyl-6-[1-methyl-3-(trifluoromethyl)pyrazol-4-yl]pyridazin-3-yl]methyl]urea | Int. 49 & Int. 43 | H1 | 510 | 511 |
| 60 | 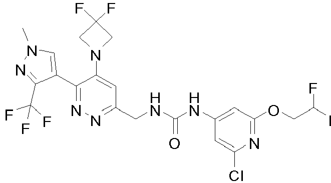 | 1-[2-chloro-6-(2,2-difluoroethoxy)-4-pyridyl]-3-[[5-(3,3-difluoroazetidin-1-yl)-6-[1-methyl-3-(trifluoromethyl)pyrazol-4-yl]pyridazin-3-yl]methyl]urea | Int. 95 & Int. 11 | H2 | 583 | 583 |
| 61 | 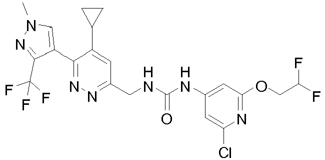 | 1-[2-chloro-6-(2,2-difluoroethoxy)-4-pyridyl]-3-[[5-cyclopropyl-6-[1-methyl-3-(trifluoromethyl)pyrazol-4-yl]pyridazin-3-yl]methyl]urea | Int. 51 & Int. 11 | H2 | 532 | 533 |
| 62 | 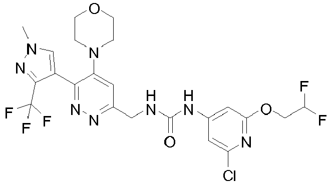 | 1-[2-chloro-6-(2,2-difluoroethoxy)-4-pyridyl]-3-[[6-[1-methyl-3-(trifluoromethyl)pyrazol-4-yl]-5-morpholino-pyridazin-3-yl]methyl]urea | Int. 99 & Int. 11 | H2 | 577 | 578 |
| 63 | 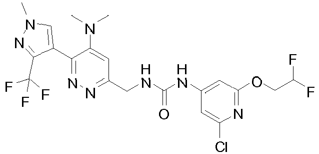 | 1-[2-chloro-6-(2,2-difluoroethoxy)-4-pyridyl]-3-[[5-(dimethylamino)-6-[1-methyl-3-(trifluoromethyl)pyrazol-4-yl]pyridazin-3-yl]methyl]urea | Int. 103 & Int. 11 | H2 | 535 | 536 |
| 64 | 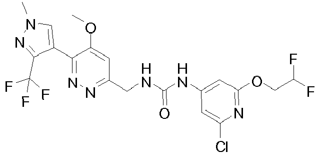 | 1-[2-chloro-6-(2,2-difluoroethoxy)-4-pyridyl]-3-[[5-methoxy-6-[1-methyl-3-(trifluoromethyl)pyrazol-4-yl]pyridazin-3-yl]methyl]urea | Int. 91 & Int. 11 | H2 | 522 | 522 |
| 65 | 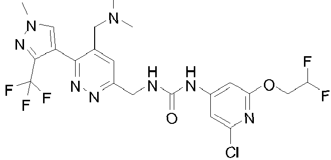 | 1-[2-chloro-6-(2,2-difluoroethoxy)-4-pyridyl]-3-[[5-(dimethylaminomethyl)-6-[1-methyl-3-(trifluoromethyl)pyrazol-4-yl]pyridazin-3-yl]methyl]urea | Int. 85 & Int. 11 | H2 | 549 | 550 |
| 66 | 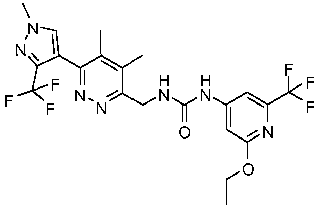 | 1-[[4,5-dimethyl-6-[1-methyl-3-(trifluoromethyl)pyrazol-4-yl]pyridazin-3-yl]methyl]-3-[2-ethoxy-6-(trifluoromethyl)-4-pyridyl]urea | Int. 108 & Int. 31 | H2 | 517 | 518 |
| 67 | 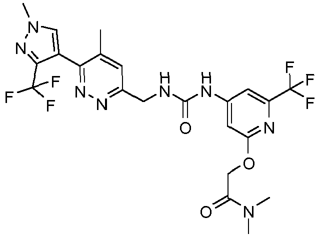 | N,N-dimethyl-2-[[4-[[5-methyl-6-[1-methyl-3-(trifluoromethyl)pyrazol-4-yl]pyridazin-3-yl]methylcarbamo ylamino]-6-(trifluoromethyl)-2-pyridyl] oxy] aceta mide | Int. 49 & Int. 109 | H1 | 560 | 561 |
| 68 | 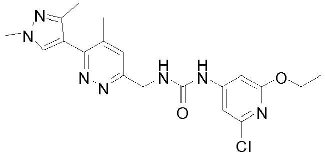 | 1-(2-chloro-6-ethoxy-4-pyridyl)-3-[[6-(1,3-dimethylpyrazol-4-yl)-5-methyl-pyridazin-3-yl]methyl]urea | Int. 112 & 2-chloro-6-ethoxy-pyridin-4-ylamine | H2 | 416 | 416 |
| 69 | 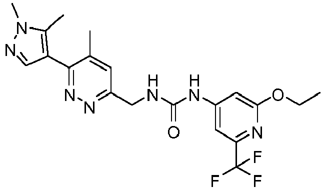 | 1-[[6-(1,5-dimethylpyrazol-4-yl)-5-methyl-pyridazin-3-yl]methyl]-3-[2-ethoxy-6-(trifluoromethyl)-4-pyridyl]urea | Int. 114 & Int. 31 | H1 | 449 | 450 |
| 70 | 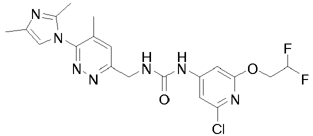 | 1-[2-chloro-6-(2,2-difluoroethoxy)-4-pyridyl]-3-[[6-(2,4-dimethylimidazol-1-yl)-5-methyl-pyridazin-3-yl]methyl]urea | Int. 116 & Int. 11 | HI | 452 | 452 |
| 71 | 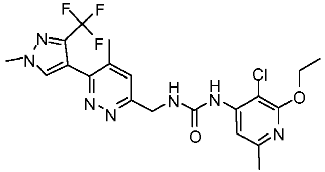 | 1-(3-chloro-2-ethoxy-6-methyl-4-pyridyl)-3-[[5-methyl-6-[1-methyl-3-(trifluoromethyl)pyrazol-4-yl]pyridazin-3-yl]methyl]urea | Int. 49 & Int. 117 | H1 | 484 | 484 |
| 72 | 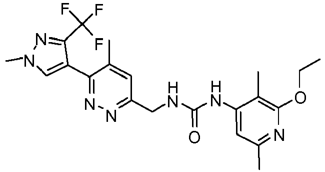 | 1-(2-ethoxy-3,6-dimethyl-4-pyridyl)-3-[[5-methyl-6-[1-methyl-3-(trifluoromethyl)pyrazol-4-yl]pyridazin-3-yl]methyl]urea | Int. 50 & Int. 119 | H2 | 463 | 464 |
| 73 | 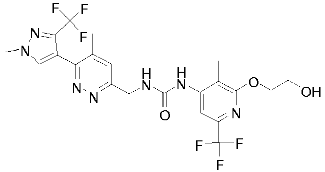 | 1-[2-(2-hydroxyethoxy)-3-methyl-6-(trifluoromethyl)-4-pyridyl]-3-[[5-methyl-6-[1-methyl-3-(trifluoromethyl)pyrazol-4-yl]pyridazin-3-yl]methyl]urea | Int. 49 & Int. 121 | H1 | 533 | 534 |
| 74 | 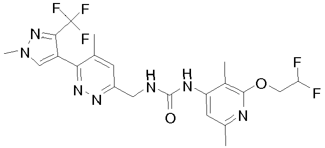 | 1-[2-(2,2-difluoroethoxy)-3,6-dimethyl-4-pyridyl]-3-[[5-methyl-6-[1-methyl-3-(trifluoromethyl)pyrazol-4-yl]pyridazin-3-yl]methyl]urea | Int. 49 & Int. 123 | H1 | 499 | 500 |
| 75 | 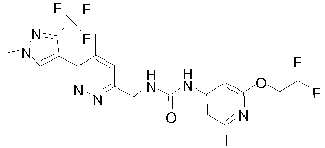 | 1-[2-(2,2-difluoroethoxy)-6-methyl-4-pyridyl]-3-[[5-methyl-6-[1-methyl-3-(trifluoromethyl)pyrazol-4-yl]pyridazin-3-yl]methyl]urea | Int. 49 & Int. 126 | H1 | 485 | 486 |
| 76 | 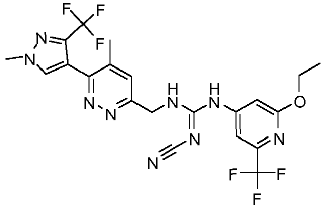 | 2-cyano-1-[2-ethoxy-6-(trifluoromethyl)-4-pyridyl]-3-[[5-methyl-6-[1-methyl-3-(trifluoromethyl)pyrazol-4-yl]pyridazin-3-yl]methyl]guanidine | Int. 49 & Int. 129 | J | 527 | 528 |
| 77 | 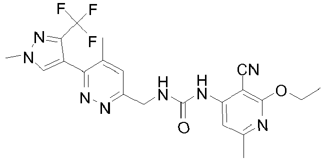 | 1-(3-cyano-2-ethoxy-6-methyl-4-pyridyl)-3-[[5-methyl-6-[1-methyl-3-(trifluoromethyl)pyrazol-4-yl]pyridazin-3-yl]methyl]urea | Int. 49 & Int. 127 | H1 | 474 | 475 |
| 78 | 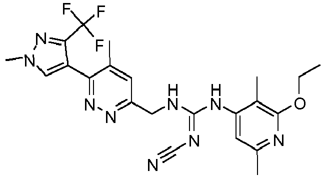 | 2-cyano-1-(2-ethoxy-3,6-dimethyl-4-pyridyl)-3-[[5-methyl-6-[1-methyl-3-(trifluoromethyl)pyrazol-4-yl]pyridazin-3-yl]methyl]guanidine | Int. 50 & Int. 130 | J | 487 | 488 |
| 79 | 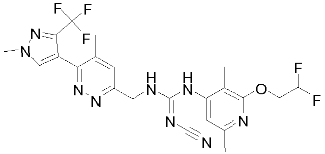 | 2-cyano-1-[2-(2,2-difluoroethoxy)-3,6-dimethyl-4-pyridyl]-3-[[5-methyl-6-[1-methyl-3-(trifluoromethyl)pyrazol-4-yl]pyridazin-3-yl]methyl]guanidine | Int. 50 & Int. 131 | J | 523 | 524 |
| 80 | 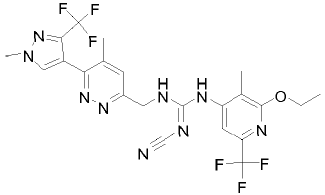 | 2-cyano-1-[2-ethoxy-3-methyl-6-(trifluoromethyl)-4-pyridyl]-3-[[5-methyl-6-[1-methyl-3-(trifluoromethyl)pyrazol-4-yl]pyridazin-3-yl]methyl]guanidine | Int. 50 & Int. 128 | J | 541 | 542 |
| 81 | 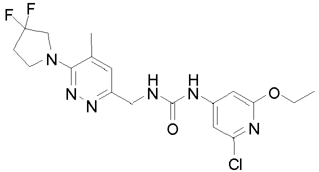 | 1-(2-Chloro-6-ethoxy-pyridin-4-yl)-3-[6-(3,3-difluoro-pyrrolidin-1-yl)-5-methyl-pyridazin-3-ylmethyl]-urea | Int. 135 & 2-chloro-6-ethoxy-pyridin-4-ylamine | H1 | 427 | 427 |
| 82 | 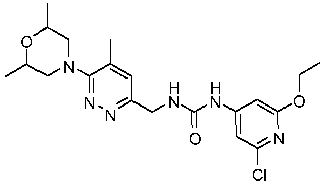 | 1-(2-Chloro-6-ethoxy-pyridin-4-yl)-3-[6-(2,6-dimethyl-morpholin-4-yl)-5-methyl-pyridazin-3-ylmethyl]-urea | Int. 136 & 2-chloro-6-ethoxy-pyridin-4-ylamine | H2 | 435 | 435 |
| 83 | 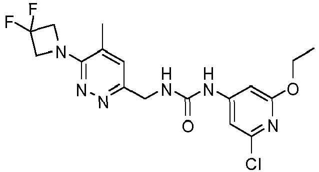 | 1-(2-Chloro-6-ethoxy-pyridin-4-yl)-3-[6-(3,3-difluoro-azetidin-1-yl)-5-methyl-pyridazin-3-ylmethyl]-urea | Int. 137 & 2-chloro-6-ethoxy-pyridin-4-ylamine | H2 | 413 | 413 |
| 84 | 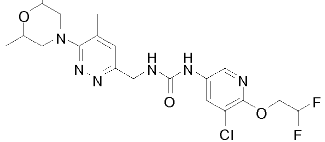 | 1-[5-chloro-6-(2,2-difluoroethoxy)-3-pyridyl]-3-[[6-(2,6-dimethylmorpholin-4-yl)-5-methyl-pyridazin-3-yl]methyl]urea | Int. 133 & Int. 3 | H2 | 471 | 471 |
| 85 | 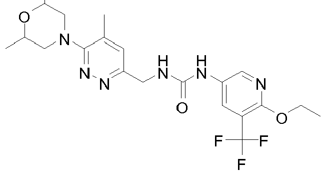 | 1-[[6-(2,6-dimethylmorpholin-4-yl)-5-methyl-pyridazin-3-yl]methyl]-3-[6-ethoxy-5-(trifluoromethyl)-3-pyridyl]urea | Int. 133 & Int. 8 | H2 | 468 | 469 |
| 86 | 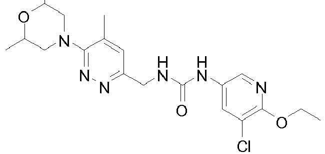 | 1-(5-chloro-6-ethoxy-3-pyridyl)-3-[[6-(2,6-dimethylmorpholin-4-yl)-5-methyl-pyridazin-3-yl]methyl]urea | Int. 133 & Int. 2 | H2 | 435 | 435 |
| 87 | 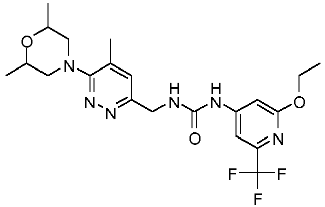 | 1-[[6-(2,6-dimethylmorpholi n-4-yl)-5-methyl-pyridazin-3-yl]methyl]-3-[2-ethoxy-6-(trifluoromethyl)-4-pyridyl]urea | Int. 133 & Int. 31 | H2 | 468 | 469 |
| 88 | 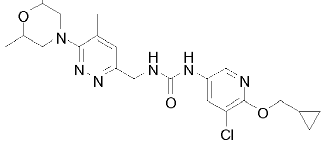 | 1-[5-chloro-6-(cyclopropylmethoxy)-3-pyridyl]-3-[[6-(2,6-dimethylmorpholin-4-yl)-5-methyl-pyridazin-3-yl]methyl]urea | Int. 133 & Int. 4 | H2 | 461 | 461 |
| 89 | 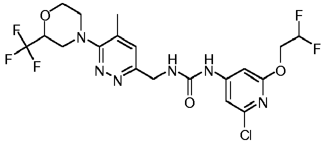 | 1-[2-chloro-6-(2,2-difluoroethoxy)-4-pyridyl]-3-[[5-methyl-6-[2-(trifluoromethyl)morpholin-4-yl]pyridazin-3-yl]methyl]urea | Int. 147 & Int. 11 | H1 | 511 | 511 |
| 90 | 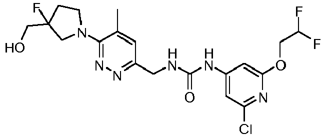 | 1-[2-chloro-6-(2,2-difluoroethoxy)-4-pyridyl]-3-[[6-[3-fluoro-3-(hydroxymethyl)pyrrolidin-1-yl]-5-methyl-pyridazin-3-yl]methyl]urea | Int. 149 & Int. 11 | H1 | 475 | 475 |
| 91 | 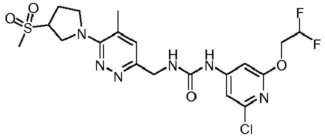 | 1-[2-chloro-6-(2,2-difluoroethoxy)-4-pyridyl]-3-[[5-methyl-6-(3-methylsulfonylpyrrolidin-1-yl)pyridazin-3-yl]methyl]urea | Int. 151 & Int. 11 | H1 | 505 | 505 |
| 92 | 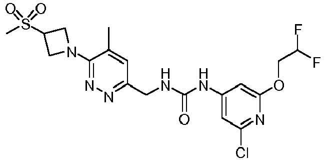 | 1-[2-chloro-6-(2,2-difluoroethoxy)-4-pyridyl]-3-[[5-methyl-6-(3-methylsulfonylazetidin-1-yl)pyridazin-3-yl]methyl]urea | Int. 153 & Int. 11 | H1 | 491 | 491 |
| 93 | 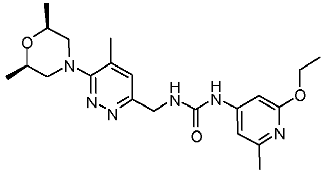 | 1-[[6-[(2R,6S)-2,6-dimethylmorpholin-4-yl]-5-methyl-pyridazin-3-yl]methyl]-3-(2-ethoxy-6-methyl-4-pyridyl)urea | Int. 141 & Int. 27 | H1 | 415 | 415 |
| 94 | 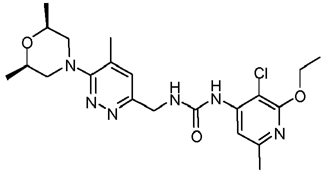 | 1-(3-chloro-2-ethoxy-6-methyl-4-pyridyl)-3-[[6-[(2R,6S)-2,6-dimethylmorpholin-4-yl]-5-methyl-pyridazin-3-yl]methyl]urea | Int. 141 & Int. 117 | H1 | 449 | 449 |
| 95 | 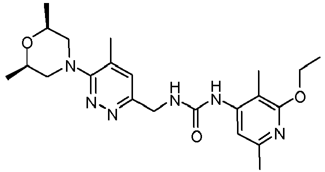 | 1-[[6-[(2R,6S)-2,6-dimethylmorpholin-4-yl]-5-methyl-pyridazin-3-yl]methyl]-3-(2-ethoxy-3,6-dimethyl-4-pyridyl)urea | Int. 141 & Int. 119 | H1 | 429 | 429 |
| 96 | 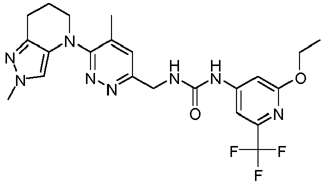 | 1-[2-ethoxy-6-(trifluoromethyl)-4-pyridyl]-3-[[5-methyl-6-(2-methyl-6,7-dihydro-5H-pyrazolo[4,3-b]pyridin-4-yl)pyridazin-3-yl]methyl]urea | Int. 144 & Int. 31 | H1 | 490 | 491 |
| 97 | 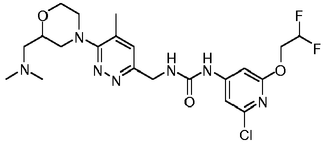 | 1-[2-chloro-6-(2,2-difluoroethoxy)-4-pyridyl]-3-[[6-[2-[(dimethylamino)methyl]morpholin-4-yl]-5-methyl-pyridazin-3-yl]methyl]urea | Int. 155 & Int. 11 | H1 | 500 | 500 |
| 98 | 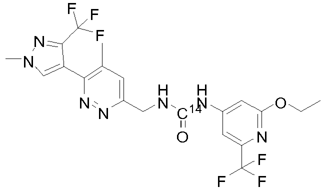 | 1-(2-Ethoxy-6-trifluoromethyl-pyridin-4-yl)-3-[5-methyl-6-(1-methyl-3-trifluoromethyl-1H-pyrazol-4-yl)-pyridazin-3-ylmethyl]-C14 urea | Int. 49 & Int. 31 | H1 | 505 | 506 |
| 99 | 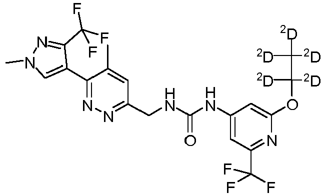 | 1-[[5-methyl-6-[1-methyl-3-(trifluoromethyl)pyrazol-4-yl]pyridazin-3-yl]methyl]-3-[2-(1,1,2,2,2-pentadeuterioethoxy)-6-(trifluoromethyl)-4-pyridyl]urea | Int. 50 & Int. 156 | NA | 508 | 509 |
| 100 | 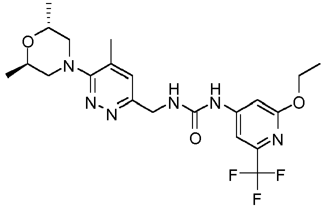 | 1-[[6-[(2R,6R)-2,6-dimethylmorpholin-4-yl]-5-methyl-pyridazin-3-yl]methyl]-3-[2-ethoxy-6-(trifluoromethyl)-4-pyridyl]urea | Int. 143 & Int. 31 | H1 | 468 | 469 |
| 101 | 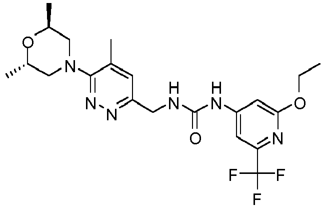 | 1-[[6-[(2S,6S)-2,6-dimethylmorpholin-4-yl]-5-methyl-pyridazin-3-yl]methyl]-3-[2-ethoxy-6-(trifluoromethyl)-4-pyridyl]urea | Int. 139 & Int. 31 | H2 | 468 | 469 |

**Table IV. NMR data of illustrative compounds of the invention**

| **Cpd#** | **NMR** |
|---|---|
| 1 | ¹H NMR (400MHz, DMSO-d₆, ppm) δ 9.65 (1H, s), 8.29 (1H, s), 7.57 (1H, s), 7.52 (1H, d), 7.33 (1H, t), 7.04 (1H, d), 4.62 (2H, d), 4.28 (2H, q), 4.02 (3H, s), 2.26 (3H, s), 1.30 (3H, t). |
| 2 | ¹H NMR (400MHz, DMSO-d₆, ppm) δ 9.46 (1H, s), 8.29 (1H, s), 7.56 (1H, s), 7.28 (1H, t), 7.12 (1H,d), 6.77 (1H, d), 4.60 (2H, d), 4.21 (2H, q), 4.01 (3H, s), 2.26 (3H, s), 1.28 (3H, t) |
| 3 | ¹H NMR (600 MHz, DMSO-d₆, ppm) δ 9.33 (s, 1H), 8.29 (s, 1H), 7.65 (s, 1H), 7.41 (d, 1H), 7.08 (d, 1H), 6.72 (d, 1H), 5.07 (quint, 1H), 4.20 (q, 2H), 4.00 (s, 3H), 2.26 (s, 3H), 1.48 (d, 3H), 1.26 (t, 3H). |
| 4 | ¹H NMR (400MHz, DMSO-d₆, ppm) δ 9.66 (1H, s), 8.29 (1H, s), 7.56 (1H, s), 7.52 (2H, d), 7.33 (1H, t), 7.08 (1H, d), 4.81 (1H, bs), 4.62 (2H, d), 4.25 (2H, t), 4.02 (3H, s), 3.69 (2H, bs), 2.26 (3H, s). |
| 5 | ¹H NMR (400MHz, DMSO-d₆, ppm) δ 9.70 (1H, s), 8.30 (1H, s), 7.57 (1H, s), 7.50 (1H, s), 7.37 (1H, t), 7.010 (1H, s), 5.13-5.09 (1H, m), 4.94 (1H, d), 4.68 (1H, t), 4.62 (2H, d), 4.24 (1H, dd), 4.11 (1H, dd), 4.01 (3H, s), 3.78 (1H, p), 3.42 (2H, t), 2.26 (3H, s). |
| 6 | ¹H NMR (400MHz, DMSO-d₆, ppm) δ 9.28 (1H, s), 8.30 (1H, s), 7.56 (1H, s), 7.50 (2H, d), 7.19 (1H, t), 7.10 (1H, t), 4.60 (2H, d), 4.01 (3H, s), 2.26 (3H, s). |
| 8 | ¹H NMR (400MHz, DMSO-d₆, ppm) δ 8.97 (1H, s), 8.28 (1H, s), 8.08 (1H, d), 8.05 (1H, d), 7.55 (1H, s), 7.12 (1H, t), 4.59 (2H, d), 4.32 (2H, q), 4.02 (3H, s), 2.26 (3H, s), 1.32 (3H, t). |
| 13 | ¹H NMR (400MHz, DMSO-d₆, ppm) δ 8.96 (1H, s), 8.28 (1H, s), 8.08 (1H, d), 8.03 (1H, d), 7.55 (1H, s), 7.09 (1H, t), 4.59 (2H, d), 4.12 (2H, d), 4.02 (3H, s), 2.26 (3H, s), 1.27-1.21 (1H, m), 0.57-0.52 (2H, m), 0.35-0.31 (2H, m). |
| 14 | ¹H NMR (400MHz, CDCl₃, ppm) δ 9.25 (1H, s), 8.86 (1H, bs), 7.79 (1H, s), 7.64 (1H, s), 6.79 (1H, s), 6.64 (1H, s), 4.75 (2H, bs), 4.65 (2H, q), 4.07 (3H, s), 2.33 (3H, s). |
| 15 | ¹H NMR (400MHz, CDCl₃, ppm) δ 9.05 (1H, s), 8.54 (1H, bs), 7.86 (1H, s), 7.78 (1H, s), 6.74 (1H, s), 6.52 (1H, s), 5.51 (2H, s), 4.76 (2H, s), 4.24 (2H, q), 3.42 (3H, s), 2.32 (3H, s), 1.31 (3H, t). |
| 16 | ¹H NMR (400MHz, DMSO-d₆, ppm) δ 9.54 (1H, s), 8.29 (1H, s), 7.56 (1H, s), 7.33 (1H, t), 7.17 (1H, d), 6.85 (1H, d), 4.76 (1H, t), 4.64 (1H, t), 4.61 (2H, d), 4.46 (1H, t), 4.39 (1H, t), 4.01 (3H, s), 2.26 (3H, s). |
| 17 | ¹H NMR (400MHz, DMSO-d₆, ppm) δ 9.56 (1H, s), 8.29 (1H, s), 7.56 (1H, s), 7.34 (1H, t), 7.20 (1H, d), 6.90 (1H, d), 6.34 (1H, tt), 4.61 (2H, d), 4.50 (2H, dt), 4.02 (3H, s), 2.26 (3H, s). |
| 18 | ¹H NMR (400MHz, DMSO-d₆, ppm) δ 9.62 (1H, s), 8.30 (1H, s), 7.57 (2H, d), 7.41 (1H, t), 7.14 (1H, d), 4.62 (2H, d), 4.28 (1H, q), 4.02 (3H, s), 2.27 (3H, s), 1.30 (3H, t). |
| 19 | ¹H NMR (400MHz, DMSO-d₆, ppm) δ 9.76 (1H, s), 8.29 (1H, s), 7.59 (1H, d), 7.57 (1H, s), 7.40 (1H, t), 7.17 (1H, d), 6.36 (1H, tt), 4.62 (2H, d), 4.56 (2H, dt), 4.02 (3H, s), 2.26 (3H, s). |
| 25 | ¹H NMR (400MHz, DMSO-d₆, ppm) δ 9.66 (1H, s), 8.28 (1H, s), 7.57 (1H, s), 7.53 (1H, s), 7.35 (1H, t), 7.07 (1H, d), 4.62 (2H, d), 4.37-4.34 (2H, m), 4.01 (3H, s), 3.65-3.63 (2H, m), 3.29 (3H, s), 2.26 (3H, s). |
| 27 | ¹H NMR (400MHz, DMSO-d₆, ppm) δ 9.49 (1H, s), 8.29 (1H, s), 7.56 (1H, s), 7.30 (1H, t), 7.14 (1H, d), 6.80 (1H, d), 4.60 (2H, d), 4.30-4.28 (2H, m), 4.01 (3H, s), 3.62-3.60 (2H, m), 3.28 (3H, s), 2.26 (3H, s). |
| 28 | ¹H NMR (400MHz, DMSO-d₆, ppm) δ 9.76 (1H, s), 8.29 (1H, s), 7.64 (1H, d), 7.57 (1H, s), 7.50 (1H, t), 7.26 (1H, d), 6.37 (1H, tt), 4.62 (2H, d), 4.55 (2H, dt), 4.02 (3H, s), 2.26 (3H, s). |
| 30 | ¹H NMR (400MHz, DMSO-d₆, ppm) δ 8.64 (1H, s), 8.33 (1H, s), 8.30 (1H, s), 7.86 (1H, t), 7.61 (1H, s), 4.64 (2H, d), 4.32 (2H, q), 4.02 (3H, s), 2.27 (3H, s), 2.11 (3H, s), 1.33 (3H, t) |
| 32 | ¹H NMR (400MHz, DMSO-d₆, ppm) δ 9.18 (1H, s), 8.28 (1H, s), 7.62 (1H, s), 7.61 (1H, s), 7.55 (1H, s), 7.17 (1H, t), 4.60 (2H, d), 4.02 (3H, s), 2.26 (3H, s). |
| 33 | ¹H NMR (400MHz, DMSO-d₆, ppm) δ 9.73 (1H, s), 8.31 (1H, s), 7.56 (2H, t), 7.39 (1H, t), 7.11 (1H, s), 4.62 (2H, d), 4.47-4.42 (1H, m), 4.20-4.15 (1H, m), 4.01 (3H, s), 2.26 (3H, s), 2.26-2.17 (1H, m), 1.72-1.70 (1H, m), 1.57-1.52 (1H, m). |
| 38 | ¹H NMR (400MHz, DMSO-d₆, ppm) δ 10.08 (1H, s), 8.31 (1H, s), 8.12 (1H, s), 7.59 (1H, s), 7.33 (1H, s), 4.67 (2H, d), 4.31 (2H, q), 4.02 (3H, s), 2.26 (3H, s), 1.29 (3H, t). |
| 40 | ¹H NMR (400MHz, DMSO-d₆, ppm) δ 9.69 (1H, s), 8.29 (1H, s), 7.56 (1H, s), 7.49 (1H, d), 7.40 (1H, t), 7.07 (1H, d), 5.13-5.09 (1H, m), 4.62 (2H, d), 4.02 (3H, s), 3.86-3.81 (2H, m), 3.52-3.46 (2H, m), 2.26 (3H, s), 2.01-1.96 (1H, m), 1.67-1.58 (1H, m). |
| 43 | ¹H NMR (400MHz, DMSO-d₆, ppm) δ 9.86 (1H, s), 8.47 (1H, d), 8.29 (1H, s), 8.03 (1H, d), 7.57-7.53 (1H, 3), 4.63 (2H, d), 4.01 (3H, s), 2.26 (3H, s). |
| 45 | ¹H NMR (400MHz, DMSO-d₆, ppm) δ 8.58 (1H, s), 8.32 (1H, s), 8.30 (1H, s), 7.99 (1H, d), 7.67 (1H, s), 5.14-5.07 (1H, m), 4.31 (2H, q), 4.02 (3H, s), 2.28 (3H, s), 2.10 (3H, s), 1.51 (3H, d), 1.32 (3H, t). |
| 47 | ¹H NMR (400MHz, DMSO-d₆, ppm) δ 9.60 (1H, s), 8.29 (1H, s), 7.55 (1H, s), 7.29 (2H, d), 6.82 (1H, s), 4.61 (2H, d), 4.39 (4H, t), 4.02 (3H, s), 2.26 (3H, s). |
| 48 | ¹H NMR (400MHz, DMSO-d₆, ppm) δ 9.21 (1H, s), 8.28 (1H, s), 7.54 (1H, s), 7.14 (1H, t), 6.94-6.20 (3H, m), 4.59 (2H, d), 4.45 (2H, dt), 4.32 (4H, t), 4.01 (3H, s), 2.26 (3H, s). |
| 49 | ¹H NMR (400MHz, DMSO-d₆, ppm) δ 8.84 (1H, s), 8.46 (1H, t), 8.42 (1H, s), 8.30 (1H, s), 7.61 (1H, s), 4.65 (2H, d), 4.39 (2H, q), 4.02 (3H, s), 2.27 (3H, s), 1.35 (3H, t). |
| 50 | ¹H NMR (400 MHz, DMSO-d₆, ppm) δ 8.59 (1H, s), 8.29 (1H, s), 7.98 (1H, s), 7.88 (1H, t), 7.60 (1H, s), 6.50-6.22 (1H, m), 4.63 (2H, d), 4.51 (2H, td), 4.01 (3H, s), 2.27 (3H, d), 2.05 (3H, s). |
| 52 | ¹H NMR (400 MHz, DMSO-d₆, ppm) δ 9.66 (1H, s), 8.38 (1H, s), 7.57 (1H, s), 7.51 (1H, d), 7.34 (1H, t), 7.03 (1H, s), 5.22-5.19 (1H, m), 4.62 (2H, d), 4.27 (2H, q), 4.04-3.99 (3H, m), 3.87-3.81 (1H, m), 2.47-2.34 (2H, m), 2.27 (3H, d), 1.30 (3H, t). |
| 53 | ¹H NMR (400 MHz, DMSO-d₆, ppm) δ 9.68 (1H, s), 8.31 (1H, s), 7.56 (1H, s), 7.51 (1H, s), 7.36 (1H, t), 7.04 (1H, s), 4.62 (2H, d), 4.44 (2H, t), 4.28 (2H, q), 3.77 (2H, t), 3.26 (3H, s), 2.26 (3H, s), 1.29 (3H, t). |
| 54 | ¹H NMR (400 MHz, DMSO-d₆, ppm) δ 9.62 (1H, s), 7.95 (1H, s), 7.51 (2H, d), 7.31 (1H, t), 7.04 (1H, s), 4.59 (2H, d), 4.26 (2H, q), 3.80 (3H, s), 2.33 (3H, s), 2.08-2.01 (1H, m), 1.29 (3H, t), 0.78-0.74 (4H, m). |
| 56 | ¹H NMR (400 MHz, DMSO-d₆, ppm) δ 9.69 (1H, s), 8.25 (1H, s), 7.56 (1H, s), 7.51 (1H, s), 7.37 (1H, t), 7.04 (1H, s), 5.07 (1H, d), 4.62 (2H, d), 4.28-4.09 (5H, m), 2.27 (3H, d), 1.29 (3H, t), 1.11 (3H, d). |
| 57 | ¹H NMR (400MHz, DMSO-d₆, ppm) δ 8.68 (1H, s), 8.34 (1H, s), 8.30 (1H, s), 7.87 (1H, t), 7.61 (1H, s), 4.65 (2H, d), 4.30 (1H, q), 4.02 (3H, s), 2.27 (3H, s), 2.11 (3H, s), 1.32 (3H, t). |
| 58 | ¹H NMR (400MHz, DMSO-d₆, ppm) δ 8.69 (1H, s), 8.36 (1H, s), 8.31 (1H, s), 8.26 (1H, t), 7.61 (1H,s), 4.64 (2H, d), 4.28 (2H, q), 4.02 (3H, s), 2.28 (3H, s), 1.51-1.44 (1H, m), 1.32 (3H, t), 1.12-1.07 (2H, m), 0.62-0.58 (2H, m). |
| 59 | ¹H NMR (400MHz, DMSO-d₆, ppm) δ 8.79 (1H, s), 8.44 (1H, s), 8.30 (1H, s), 7.94 (1H, t), 7.61 (1H, t), 6.40 (1H, tt), 4.66 (2H, d), 4.57 (2H, dt), 4.02 (3H, s), 2.27 (3H, s), 2.15 (3H, s). |
| 60 | ¹H NMR (500 MHz, DMSO-d₆, ppm) δ 9.54 (s, 1H), 8.14 (s, 1H), 7.29 (t, 1H), 7.19 (d, 1H), 6.89 (d, 1H), 6.75 (s, 1H), 6.35 (tt, 1H), 4.55-4.45 (m, 4H), 4.12 (t, 4H), 4.01 (s, 3H). |
| 61 | ¹H NMR (300 MHz, DMSO-d₆, ppm) δ 9.55 (s, 1H), 8.28 (s, 1H), 7.32 (t, 1H), 7.16-7.21 (m, 2H), 6.89 (d, 1H), 6.35 (tt, 1H), 4.57 (d, 2H), 4.49 (dt, 2H), 4.03 (s, 3H), 1.70-1.82 (m, 1H), 1.01-1.11 (m, 2H), 0.79-0.88 (m, 2H). |
| 62 | ¹H NMR (300 MHz, DMSO-d₆, ppm) δ 9.56 (s, 1H), 8.37 (s, 1H), 7.32 (t, 1H), 7.19 (d, 1H), 7.13 (s, 1H), 6.90 (d, 1H), 6.35 (tt, 1H), 4.43-4.58 (m, 4H), 4.02 (s, 3H), 3.56-3.66 (m, 4H), 2.87-2.97 (m, 2H). |
| 63 | ¹H NMR (400 MHz, DMSO-d₆, ppm) δ 9.55 (s, 1H), 8.17 (s, 1H), 7.30 (t, 1H), 7.19 (s, 1H), 6.96 (s, 1H), 6.89 (s, 1H), 6.35 (tt, 1H), 4.43-4.55 (m, 4H), 3.99 (s, 3H), 2.67 (s, 6H). |
| 64 | ¹H NMR (400 MHz, DMSO-d₆, ppm) δ 9.59 (s, 1H), 8.37 (s, 1H), 7.36 (t, 1H), 7.33 (s, 1H), 7.20 (d, 1H), 6.90 (d, 1H), 6.35 (tt, 1H), 4.60 (d, 2H), 4.49 (dt, 2H), 4.00 (s, 3H), 3.93 (s, 3H). |
| 65 | ¹H NMR (400 MHz, DMSO-d₆, ppm) δ 9.60 (s, 1H), 8.25 (s, 1H), 7.69 (s, 1H), 7.39 (t, 1H), 7.20 (d, 1H), 6.90 (d, 1H), 6.35 (tt, 1H), 4.66 (d, 2H), 4.49 (dt, 2H), 4.02 (s, 3H), 3.33 (s, 2H), 2.11 (s, 6H). |
| 66 | ¹H NMR (400 MHz, DMSO-d₆, ppm) δ 9.74 (s, 1H), 8.21 (s, 1H), 7.47 - 7.52 (m, 2H), 7.03 (s, 1H), 4.66 (d, 2H), 4.28 (q, 2H), 4.02 (s, 3H), 2.30 (s, 3H), 2.20 (s, 3H), 1.30 (t, 2H). |
| 67 | ¹H NMR (400 MHz, DMSO-d₆, ppm) δ 10.03 (1H, s), 8.30 (1H, s), 7.56 (1H, br s), 7.55 (2H, d), 7.14 (1H, s), 4.98 (2H, s), 4.61 (2H, d), 4.01 (3H, s), 2.99 (3H, s), 2.81 (3H, s), 2.26 (2H, s). |
| 68 | ¹H NMR (400 MHz, DMSO-d₆, ppm) δ 9.45 (1H, s), 8.05 (1H, s), 7.47 (1H, s), 7.27 (1H, t), 7.12 (1H, s), 6.77 (1H, s), 4.56 (2H, d), 4.21 (2H, q), 3.84 (3H, s), 2.33 (3H, s), 2.26 (3H, s), 1.28 (3H, t). |
| 70 | ¹H NMR (400MHz, DMSO-d₆, ppm) δ 9.69 (1H, s), 7.76 (1H, s), 7.45 (1H, t), 7.21 (1H, s), 7.08 (1H, s), 6.91 (1H, s), 6.34 (1H, tt), 4.65 (2H, d), 4.49 (2H, dt), 2.17 (3H, s), 2.12 (3H, s), 2.11 (3H, s). |
| 71 | 1H NMR (400MHz, DMSO, ppm) δ 8.61 (1H, s), 8.29 (1H, s), 8.14 (1H, t), 7.81 (1H, s), 7.58 (1H, s), 4.62 (2H, d), 4.35 (2H, q), 4.02 (3H, s), 2.30 (3H, s), 2.27 (3H, s), 1.31 (3H, t). |
| 72 | 1H NMR (400MHz, DMSO, ppm) δ 8.30 (1H, s), 8.22 (1H, s), 7.68 (1H, t), 7.61 (1H, s), 7.59 (1H, s), 4.62 (2H, d), 4.26 (2H, q), 4.03 (3H, s), 2.28 (3H, s), 2.27 (3H, s), 2.00 (3H, s), 1.30 (3H, t). |
| 73 | 1H NMR (400MHz, DMSO, ppm) δ 8.68 (1H, s), 8.34 (1H, s), 8.31 (1H, s), 7.91 (1H, t), 7.61 (1H, s), 4.82 (1H, t), 4.65 (2H, d), 4.28 (2H, t), 4.02 (3H, s), 3.72 (2H, q), 2.27 (3H, s), 2.13 (3H, s). |
| 74 | 1H NMR (400MHz, DMSO, ppm) δ 8.31 (1H, s), 8.29 (1H, s), 7.74 (1H, t), 7.71 (1H, s), 7.58 (1H, s), 6.36 (1H, tt), 4.62 (2H, d), 4.50 (2H, dt), 4.02 (3H, s), 2.28 (3H, s), 2.27 (3H, s), 2.03 (3H, s). |
| 75 | 1H NMR (400MHz, DMSO, ppm) δ 9.25 (1H, s), 8.30 (1H, s), 7.57 (1H, s), 7.71 (1H, t), 7.58 (1H, s), 6.34 (1H, tt), 4.60 (2H, d), 4.48 (2H, dt), 4.02 (3H, s), 2.30 (3H, s), 2.26 (3H, s). |
| 76 | 1H NMR (400MHz, DMSO, ppm) δ 9.97 (1H, s), 8.64 (1H, s), 8.30 (1H, s), 7.63 (1H, s), 7.41 (1H, s), 6.99 (1H, s), 4.81 (2H, d), 4.31 (2H, q), 4.02 (3H, s), 2.28 (3H, s), 1.31 (3H, t). |
| 77 | 1H NMR (400MHz, DMSO, ppm) δ 9.03 (1H, s), 8.25 (1H, s), 7.47 (1H, s), 6.53 (1H, s), 5.43 (2H,s), 4.46 (2H, q), 4.00 (3H, s), 2.35 (3H, s), 2.20 (3H, s), 1.37 (3H, t). |
| 78 | 1H NMR (400MHz, DMSO, ppm) δ 9.20 (1H, s), 8.30 (1H, s), 7.73 (1H, s), 7.51 (1H, s), 6.71 (1H, s), 4.66 (2H, d), 4.30 (2H, q), 4.02 (3H, s), 2.32 (3H,s), 2.28 (3H, s), 1.32 (3H, t). |
| 79 | 1H NMR (400MHz, DMSO, ppm) δ 9.27 (1H, s), 8.31 (1H, s), 7.84 (1H, s), 7.53 (1H, s), 6.82 (1H, s), 6.40 (1H, tt), 4.68 (2H, d), 4.548 (2H, dt), 4.02 (3H, s), 2.34 (3H,s), 2.28 (3H, s), 1.97 (3H, s). |
| 80 | 1H NMR (400MHz, DMSO, ppm) δ 9.59 (1H, s), 8.32 (1H, s), 8.06 (1H, s), 7.58 (1H, s), 7.38 (1H, s), 4.71 (2H, d), 4.36 (2H, q), 4.03 (3H, s), 2.28 (3H,s), 2.05 (3H, s), 1.36 (3H, t). |
| 81 | 1H NMR (400MHz, DMSO, ppm) δ 9.36 (1H, s), 7.24 (1H, d), 7.13 (1H, t), 7.10 (1H, d), 6.75 (1H, d), 4.41 (2H, d), 4.21 (2H, q), 3.94 (2H, t), 3.76 (2H, t), 2.49-2.42 (2H, m) 2.32 (3H, s), 1.26 (3H, t). |
| 82 | 1H NMR (400MHz, DMSO, ppm) δ 9.37 (1H, s), 7.32 (1H, d), 7.14 (1H, t), 7.11 (1H, d), 6.76 (1H, d), 4.45 (2H, d), 4.21 (2H, q), 3.78-3.74 (2H, m), 3.40 (2H, d), 2.55 (2H, dd) 2.27 (3H, s), 1.27 (3H, t), 1.12 (6H, s) |
| 83 | 1H NMR (400MHz, DMSO, ppm) δ 9.39 (1H, s), 7.26 (1H, d), 7.18 (1H, t), 7.11 (1H, d), 6.76 (1H, d), 4.59 (4H, t), 4.42 (2H, d), 4.21 (2H, q), 2.19 (3H, s), 1.27 (3H, t). |
| 84 | 1H NMR (400MHz, DMSO, ppm) δ 8.71 (1H, s), 8.18 (1H, s), 8.06 (1H, s), 6.39 (1H, tt), 4.59 (2H, dt), 3.97-3.94 (2H, m), 3.55-3.49 (2H, m), 2.48-2.44 (2H, m), 1.12 (3H, s), 1.10 (3H, s). |
| 87 | 1H NMR (400MHz, DMSO, ppm) δ 9.69 (1H, s), 7.51 (1H, s), 7.33 (2H, s), 7.03 (1H, s), 4.47 (2H, s), 4.27 (2H, q), 3.80-3.73 (2H, m), 3.42-3.39 (2H, m), 2.58-2.52 (2H, m), 2.27 (3H, s), 1.30 (3H, t), 1.13 (3H, s), 1.12 (3H, s). |
| 91 | 1H NMR (400MHz, DMSO-d6, ppm) δ 9.58 (1H, s), 7.29 (1H, t), 7.22 (1H, s), 7.18 (1H, s), 6.89 (1H, s), 6.49-6.20 (1H, m), 4.52-4.50 (4H, m), 4.05-4.03 (1H, m), 3.87-3.86 (2H, d), 3.73-3.58 (2H, m), 3.05 (3H, s), 2.34-2.27 (5H, m). |
| 93 | 1H NMR (400MHz, DMSO, ppm) δ 9.11 (1H, s), 7.32 (1H, s), 7.10 (1H, t), 6.73 (1H, s), 6.71 (1H, s), 4.44 (2H, d), 4.21 (2H, q), 3.78-3.72 (2H, m), 3.42-3.38 (2H, m), 2.57-2.52 (2H, m), 2.27 (3H, s), 2.28 (3H,s), 1.26 (3H, t), 1.13 (3H, s), 1.12 (3H, s). |
| 94 | 1H NMR (400MHz, DMSO, ppm) δ 8.53 (1H, s), 8.00 (1H, t), 7.81 (1H, s), 7.35 (1H, s), 4.47 (2H, d), 4.34 (2H, q), 3.81-3.73 (2H, m), 3.42-3.39 (2H, m), 2.58-2.52 (2H, m), 2.29 (3H, s), 2.28 (3H,s), 1.31 (3H, t), 1.14 (3H, s), 1.12 (3H, s). |
| 96 | 1H NMR (400MHz, DMSO, ppm) δ 8.11 (1H, s), 7.59 (1H, s), 7.53 (1H, t), 7.34 (1H, s), 4.45 (2H, d), 4.25 (2H, q), 3.78-3.74 (2H, m), 3.42-3.39 (2H, m), 2.58-2.54 (2H, m), 2.28 (3H, s), 2.25 (3H,s), 1.97 (3H,s), 1.28 (3H, t), 1.14 (3H, s), 1.12 (3H, s). |
| 97 | 1H NMR (400MHz, DMSO-d6, ppm) δ 9.69 (1H, s), 7.52 (1H, d), 7.42 (1H, s), 7.36 (1H, t), 7.04 (1H, d), 6.99 (1H, s), 4.51 (2H, d), 4.28 (2H, q), 3.65 (3H, s), 3.58-3.55 (2H, m), 2.69 (2H, t), 2.23 (3H, s), 2.06-2.00 (2H, m), 1.30 (3H, t). |
| 99 | 1H NMR (400 MHz, DMSO-d6) δ ppm 9.65 (1H, s), 8.29 (1H, s), 7.57 (1H, s), 7.51 (1H, d), 7.34 (1H, t), 7.04 (1H, br s), 4.62 (2H, d), 4.02 (3H, s), 2.26 (3H, s). |
| 100 | 1H NMR (400MHz, DMSO, ppm) δ 9.57 (1H, s), 7.50 (1H, s), 7.33 (1H, s), 7.22 (1H, t), 7.02 (1H, d), 4.47 (2H, d), 4.28 (2H, q), 4.11-4.07 (2H, m), 3.19-3.16 (2H, m), 2.95-2.91 (2H, m), 2.29 (3H, s), 1.30 (3H, t), 1.25 (3H, s), 1.23 (3H, s). |
| 101 | 1H NMR (400MHz, DMSO, ppm) δ 9.56 (1H, s), 7.50 (1H, s), 7.33 (1H, s), 7.21 (1H, t), 7.03 (1H, s), 4.47 (2H, d), 4.28 (2H, q), 4.11-4.07 (2H, m), 3.19-3.16 (2H, m), 2.95-2.91 (2H, m), 2.29 (3H, s), 1.30 (3H, t), 1.25 (3H, s), 1.23 (3H, s). |

### BIOLOGICAL EXAMPLES

### Example 3. In vitro assays

### 3.1. Ca²⁺ assay

Triggering of S1PR2 by administration of Sphingosine-1-phosphate leads to a transient increase in intracellular Ca²⁺. The Ca²⁺ flux assays are measuring the release of Ca²⁺ intracellularly by use of a Ca²⁺ sensitive fluorescent dye. The assay is firstly run in agonist mode (incubation of compounds alone) to ensure that the Ca²⁺ released measured is not caused by the test compound having an agonistic effect. Then the assay is continued in antagonist mode (Sphingosine-1-Phosphate added to incubated medium containing the test compounds).

### 3.1.1. S1P2 agonist assay

CHO cells stably overexpressing human GPCR sphingosine 1-phosphate receptor 2 (CHO-S1PR2 Perkin Elmer; ES-594-A) are seeded from a frozen stock in 384 wells sterile microplates (50 µL; 7,500 cells/well) and are incubated overnight at 37°C and 5% CO₂. The next day cells are washed twice with starvation medium (F-12 Ham's medium containing 0.1% BSA (Fatty acid free: FAF)) and left in 25µL starvation medium for 1 h at 37°C, 5% CO₂. After this starvation cells are incubated with 25µL buffer containing the Ca²⁺-sensitive fluorescent dye (0.5mg Fluo8 + 125mg Allura Red in 100 ml of 1% DMSO, in HBSS+20 mM Hepes + 5mM probenecid). The cells are incubated for an additional 1 hour after which 10 µL compounds, diluted in HBSS buffer with 20mM Hepes and 0.1% BSA (FAF), are added to the cells and intracellular Ca²⁺ changes are immediately measured by reading fluorescence during 3 min (FDSS/µCELL reader). The ratio of the maximal fluorescence over the background fluorescence before compound injection is used to determine compound response.

### 3.1.2. S1P2 antagonist assay

After readout of the agonist activity, the plates are incubated for 15 min at 37°C and 5% CO₂. Then, cells are stimulated with 10 µL Sphingosine-1-Phosphate (S1P) (Avanti Polar lipids-860492P) at its EC80 concentration. Intracellular Ca²⁺ changes are immediately measured by reading fluorescence during 3 min (FDSS/µCELL reader). The ratio of the maximal fluorescence over the background fluorescence before compound injection is used to determine compound response.

For EC₅₀ determination, a 10 point dilution series of compounds starting from 23.3µM and 20µM highest final concentration for agonist and antagonist respectively, 1/3 dilution was performed.

The obtained ratio's for agonist and antagonist readout were normalized versus vehicle and EC100 of SIP as controls for agonist mode and versus vehicle and EC₅₀ of SIP for antagonist mode. From these normalized data EC₅₀ of the compounds are derived.

**Table V. S1P2 antagonist EC₅₀ of illustrative compounds of the invention**

| **Cpd#** | **S1P2 EC₅₀ (nM)** |
|---|---|
| 1 | 9.5 |
| 2 | 8.5 |
| 3 | 26.0 |
| 4 | 348.0 |
| 5 | 6610.0 |
| 6 | 16.9 |
| 7 | 17.8 |
| 8 | 22.0 |
| 9 | 23.7 |
| 10 | 15.0 |
| 11 | 49.6 |
| 12 | 11.7 |
| 13 | 40.3 |
| 14 | 24.2 |
| 15 | 9.2 |
| 16 | 10.8 |
| 17 | 8.6 |
| 18 | 10.9 |
| 19 | 17.1 |
| 20 | 8.8 |
| 21 | 17.3 |
| 22 | 17.3 |
| 23 | 10.6 |
| 24 | 8.7 |
| 25 | 792.0 |
| 26 | 110.0 |
| 27 | 414.0 |
| 28 | 16.0 |
| 29 | 27.3 |
| 30 | 6.1 |
| 31 | 3140.0 |
| 32 | 11.1 |
| 33 | 37.1 |
| 34 | 65.8 |
| 35 | 74.5 |
| 36 | 791.0 |
| 37 | 5430.0 |
| 38 | 40.2 |
| 39 | 219.0 |
| 40 | 291.0 |
| 41 | 101.0 |
| 42 | 292.0 |
| 43 | 6030.0 |
| 44 | 4500.0 |
| 45 | 19.7 |
| 46 | 17.2 |
| 47 | 9.3 |
| 48 | 27.1 |
| 49 | 11.5 |
| 50 | 8.0 |
| 51 | 86.7 |
| 52 | 11.4 |
| 53 | 10.0 |
| 54 | 15.3 |
| 55 | 16.9 |
| 56 | 11.2 |
| 57 | 7.6 |
| 58 | 69.6 |
| 59 | 7.2 |
| 60 | 3280.0 |
| 61 | 14.0 |
| 62 | 3130.0 |
| 63 | 66.8 |
| 64 | 8.5 |
| 65 | 1620.0 |
| 66 | 4246 |
| 67 | 19800 |
| 68 | 25.428 |
| 69 | 945 |
| 70 | 903.8 |
| 71 | 9 |
| 72 | 9 |
| 73 | 84 |
| 74 | 22 |
| 75 | 59 |
| 76 | 17 |
| 77 | 19800 |
| 78 | 106 |
| 79 | 546 |
| 80 | 13 |
| 81 | 6 |
| 82 | 6 |
| 83 | 11 |
| 84 | 6511 |
| 85 | 487 |
| 86 | 1358 |
| 87 | 42 |
| 88 | 4507 |
| 89 | 34 |
| 90 | 452 |
| 91 | 529 |
| 92 | 188 |
| 93 | 1051 |
| 94 | 27 |
| 96 | 33 |
| 97 | 81 |
| 98 | 19800 |

### 3.1.3. S1PR1 and S1PR5 Selectivity assays

To evaluate the selectivity of the compounds for S1PR2 over S1PR1 and S1PR5, a similar assay set up as described above for the S1PR2 calcium flux assays was performed: CHO cells stably overexpressing human GPCR sphingosine 1-phosphate receptor 1 and Gq protein (Euroscreen, FAST-0197A) or overexpressing human GPCR sphingosine 1-phosphate receptor 5 and Gq protein were used (Perkin Elmer, ES-593-A). In case of the S1PR5 assay triggering was done with an S1PR5 specific agonist instead of with SIP (Hobson et al., 2015).

**S1P1 antagonist EC₅₀ of illustrative compounds of the invention**

| **Cpd#** | **S1P1 EC₅₀ (nM)** |
|---|---|
| 1 | 5971.3 |
| 2 | 4543 |
| 3 | 19800 |
| 4 | 19800 |
| 5 | 19800 |
| 7 | 19800 |
| 8 | 19800 |
| 9 | 19800 |
| 10 | 19800 |
| 15 | 6610 |
| 17 | 4936 |
| 18 | 6610 |
| 20 | 3866.3 |
| 22 | 6610 |
| 23 | 19800 |
| 24 | 19800 |
| 25 | 19800 |
| 26 | 19800 |
| 27 | 19800 |
| 28 | 6610 |
| 30 | 4478 |
| 34 | 2023 |
| 35 | 19800 |
| 37 | 19800 |
| 38 | 19800 |
| 39 | 6610 |
| 40 | 5153 |
| 41 | 19800 |
| 43 | 19800 |
| 44 | 19800 |
| 45 | 19800 |
| 46 | 6610 |
| 47 | 6610 |
| 48 | 5096 |
| 50 | 5921 |
| 52 | 5891 |
| 53 | 4023 |
| 54 | 2484.5 |
| 55 | 19800 |
| 57 | 6610 |
| 58 | 19800 |
| 59 | 6610 |
| 60 | 2800 |
| 61 | 918.633 |
| 62 | 344.733 |
| 63 | 2084.5 |
| 64 | 1623 |
| 65 | 6610 |
| 68 | 19800 |
| 69 | 19800 |
| 70 | 19800 |
| 71 | 6610 |
| 72 | 16503 |
| 73 | 19800 |
| 74 | 12997 |
| 75 | 19800 |
| 76 | 13205 |
| 78 | 19800 |
| 79 | 19800 |
| 80 | 19800 |
| 81 | 6610 |
| 82 | 6610 |
| 87 | 1398 |
| 89 | 3795 |
| 90 | 6610 |
| 91 | 19800 |
| 92 | 19800 |
| 95 | 6610 |
| 96 | 6610 |
| 100 | 6610 |

**Table VI. S1P5 antagonist EC₅₀ of illustrative compounds of the invention**

| **Cpd#** | **S1P5 EC₅₀ (nM)** |
|---|---|
| 1 | 6610 |
| 2 | 4599 |
| 3 | 6610 |
| 4 | 19800 |
| 5 | 19800 |
| 7 | 19800 |
| 8 | 19800 |
| 9 | 19800 |
| 10 | 19800 |
| 15 | 19800 |
| 17 | 6610 |
| 18 | 19800 |
| 20 | 2103.5 |
| 22 | 19800 |
| 23 | 19800 |
| 24 | 19800 |
| 25 | 19800 |
| 26 | 19800 |
| 27 | 19800 |
| 28 | 6610 |
| 30 | 3277 |
| 34 | 6610 |
| 35 | 19800 |
| 37 | 19800 |
| 38 | 19800 |
| 39 | 6610 |
| 40 | 6610 |
| 41 | 19800 |
| 43 | 19800 |
| 44 | 19800 |
| 45 | 6610 |
| 46 | 6610 |
| 47 | 19800 |
| 48 | 19800 |
| 50 | 3649 |
| 52 | 6615 |
| 53 | 6610 |
| 54 | 4579 |
| 55 | 6610 |
| 57 | 4620 |
| 58 | 19800 |
| 59 | 5497 |
| 60 | 6610 |
| 61 | 4044.3 |
| 62 | 11010 |
| 63 | 11007 |
| 64 | 4384.5 |
| 65 | 19800 |
| 69 | 19800 |
| 70 | 19800 |
| 71 | 6610 |
| 72 | 11007 |
| 74 | 19800 |
| 75 | 2888 |
| 76 | 6610 |
| 78 | 19800 |
| 79 | 19800 |
| 80 | 6610 |
| 81 | 3767.5 |
| 82 | 340.6 |
| 87 | 249.2 |
| 89 | 839.4 |
| 90 | 2092 |
| 91 | 19800 |
| 92 | 19800 |
| 93 | 6610 |
| 94 | 478.9 |
| 95 | 809.4 |
| 96 | 626.5 |
| 100 | 2477 |

### 3.2. S1PR2 Binding assay

The following assay can be used for determination of S1PR2 binding. The binding assay measures the potential to compete with radioactively labeled SIP for binding to the receptor.

The assay is performed in a 96 well plate where the following reagents are added. First 50 µL compound is added into the assay plate, followed by addition of 100 µL of a mixture consisting of membrane and Scintillation proximity Assay (SpA) beads [mixture consists of 20 µg/well membranes derived from stable cell line over expressing S1PR2, 0.5 mg/well Polyvinyltoluene-Wheat Germ-Agglutinin (PVT-WGA) beads (Perkin Elmer, RPNQ0001)]. All components are diluted in assay buffer containing 20mM Tris pH 7.5; 10mM MgCl₂; 100mM NaCl; 0.4% BSA FAF; 1 mM Na₃VO₄) and incubated for 15 min until addition to the assay plate. Subsequently, 50 ul of radioactively labeled SIP is added to the wells (Sphingosine, D-erythro-[3-3H] 1-phosphate; ARC; ART0778). After an incubation for 2h at room temperature, plates are centrifuged at 2000 rpm during 20 min. Plates are read on a Topcount reader (Perkin Elmer) immediately after centrifugation (readout time, 1 min/well).

### 3.3. Cell based assay: GTp-γS binding assay

The following assay can be used for determination of S1PR2 activation. The [³⁵S] GTPγS assay measures the level of G protein activation following agonist occupation of a GPCR, by determining the binding of the non-hydrolysable analog [³⁵S] GTPγS to Gα subunits.

The assay is performed in a 96 well plate where the following reagents are added. First 50 µL compound is added into the assay plate, followed by addition of 20 µL SIP at EC80 concentration (concentration which gives 80% of the activity of S1PR2). Then, 30 µL of a mixture consisting of membranes-GTPγS-SpA beads is added [mixture consists of 2.5 µg/well membranes derived from stable cell line over expressing S1PR2 (membranes are pre-incubated with 1 µM GDP for 15 min at 4°C), 0.1 nM [35S]GTPγS (Perkin Elmer, NEG030) and 0.5 mg/well PVT-WGA SpA beads (Perkin Elmer, RPNQ0001)]. All components are diluted in assay buffer containing 20mM Tris pH 7.5; 10mM MgCl₂; 100mM NaCl; 0.1% BSA FAF; 50ug/ml saponin. After an incubation for 4h at room temperature, plates are centrifuged at 2000 rpm during 20 min. Plates are read on a Topcount reader (Perkin Elmer) immediately after centrifugation (readout time, 1 min/well).

**Table VII. S1PR2 binding EC₅₀ for illustrative compounds of the invention**

| **Cpd#** | **S1PR2 EC (nM)** |
|---|---|
| 1 | 33.2 |
| 2 | 22.4 |
| 3 | 115.0 |
| 4 | 873.0 |
| 5 | 11100.0 |
| 6 | 50.2 |
| 7 | 330.0 |
| 8 | 306.0 |
| 9 | 532.0 |
| 10 | 281.0 |
| 11 | 716.0 |
| 12 | 590.0 |
| 13 | 1120.0 |
| 14 | 225.0 |
| 15 | 91.6 |
| 16 | 62.9 |
| 17 | 36.1 |
| 18 | 35.8 |
| 19 | 88.7 |
| 20 | 15.2 |
| 21 | 135.5 |
| 22 | 86.0 |
| 23 | 74.5 |
| 24 | 53.8 |
| 25 | 2030.0 |
| 26 | 770.0 |
| 27 | 641.0 |
| 28 | 89.7 |
| 29 | 275.0 |
| 30 | 26.2 |
| 31 | 1650.0 |
| 32 | 164.0 |
| 33 | 164.0 |
| 34 | 408.0 |
| 35 | 242.0 |
| 36 | 1800.0 |
| 37 | 9580.0 |
| 38 | 109.0 |
| 39 | 1040.0 |
| 40 | 1080.0 |
| 41 | 542.0 |
| 42 | 604.0 |
| 43 | 4650.0 |
| 44 | 4150.0 |
| 45 | 211.0 |
| 46 | 97.0 |
| 47 | 34.5 |
| 48 | 154.0 |
| 49 | #N/A |
| 50 | 24.1 |
| 51 | 473.0 |
| 52 | 70.2 |
| 53 | 52.4 |
| 54 | 151.0 |
| 55 | 338.0 |
| 56 | 129.0 |
| 57 | 24.4 |
| 58 | 223.0 |
| 59 | 38.5 |
| 60 | 1520.0 |
| 61 | 193.0 |
| 62 | 2370.0 |
| 63 | 298.0 |
| 64 | 48.7 |
| 65 | 1470.0 |
| 66 | #N/A |
| 67 | 28700 |
| 68 | 110.54 |
| 69 | 751.2 |
| 70 | 903.8 |
| 71 | 25 |
| 72 | 25 |
| 73 | 160 |
| 74 | 81 |
| 75 | 282 |
| 76 | 62 |
| 77 | 20000 |
| 78 | 863 |
| 79 | 2132 |
| 80 | 45 |
| 81 | 197 |
| 82 | 156 |
| 83 | 160 |
| 85 | 3564 |
| 87 | 331 |
| 89 | 422 |
| 90 | 1174 |
| 91 | 919 |
| 92 | 469 |
| 93 | 2172 |
| 94 | 509 |
| 96 | 302 |
| 97 | 274 |
| 98 | 16270 |

### 3.4. IL-8 production

SIP is able to induce cytokines such as IL-8 in a process that is S1PR2 dependent (O'Sullivan et al, 2014; Bruennert et al, 2015). This assay is designed to test inhibitory activity of compounds on SIP induced IL-8 on HFL-1 cells, a human fetal lung fibroblast cell line.

### 3.4.1. IL8 assay

Human Fetal Lung cells (HFL-1) are seeded in 96 well plates in growth medium (F12K + 10% heat inactivated FBS + 1% Pen/strep). After overnight incubation at 37°C, 5% CO₂ cells are refreshed with starvation medium without HSA (F12K + 1% FBS + 1% Pen/strep). On day three, compounds are added (10 point serial dilution, 30µM highest concentration, 1/3 dilution, 0.3% DMSO final) and plates are incubated for one hour at 37°C, 5% CO₂. Subsequently SIP at 1µM final concentration is added and plates are incubated for 16 to 24 hours at 37°C, 5% CO₂ after which the supernatant was collected. IL-8 levels in the supernatant are determined with the IL-8 ELISA of R&D systems.

**Table VIII. IL8 production assay for illustrative compounds of the invention**

| **Cpd#** | **IL8 EC₅₀ (nM)** |
|---|---|
| 1 | 2.2 |
| 2 | 1.5 |
| 3 | 22.0 |
| 4 | 234.0 |
| 5 | 1540.0 |
| 8 | 82.9 |
| 16 | 4.6 |
| 18 | 4.6 |
| 21 | 11.3 |
| 30 | 1.5 |
| 35 | 61.9 |
| 38 | 31.8 |
| 39 | 122.0 |
| 40 | 142.0 |
| 41 | 48.9 |
| 42 | 50.9 |
| 45 | 41.1 |
| 46 | 25.6 |
| 47 | 4.6 |
| 48 | 23.9 |
| 50 | 1.5 |
| 51 | 29.5 |
| 52 | 7.8 |
| 53 | 6.6 |
| 54 | 11.6 |
| 55 | 35.8 |
| 56 | 4.6 |
| 57 | 0.9 |
| 59 | 0.9 |
| 60 | 166.0 |
| 63 | 55.4 |
| 64 | 16.6 |
| 65 | 2632 |
| 66 | 996 |
| 67 | 2180 |
| 68 | 153.3 |
| 69 | 107.9 |
| 71 | 2 |
| 72 | 2 |
| 73 | 51 |
| 74 | 15 |
| 75 | 38 |
| 76 | 8 |
| 77 | 2741 |
| 78 | 1091 |
| 79 | 439 |
| 80 | 3 |
| 81 | 32 |
| 82 | 53.5 |
| 83 | 66 |
| 85 | 427 |
| 87 | 82 |
| 89 | 33 |
| 90 | 244 |
| 91 | 1246 |
| 92 | 432 |
| 93 | 753 |
| 94 | 102 |
| 96 | 44 |
| 97 | 62 |

### 3.4.2. IL8 assay in the presence of human serum albumin

To evaluate the influence of plasma protein binding of SIP as well as compound, the SIP induced IL-8 levels were evaluated in presence of 2% Human Serum Albumin (HSA, equivalent to 40% human serum). By adding 2% HSA, which affects both the activity of the compounds as well as the SIP potency, the physiological condition are reproduced and the shift in potency expected under in vivo serum conditions can be measured.

Human Fetal Lung cells (HFL-1) are seeded in 96 well plates in growth medium (F12K + 10% heat inactivated FBS + 1% Pen/strep). After overnight incubation at 37°C, 5% CO₂ cells are refreshed with starvation medium with HSA (F12K + 1% FBS + 1% Pen/strep+1.95% HSA). On day three, compounds are added (10 point serial dilution, 30µM highest concentration, 1/3 dilution, 0.3% DMSO final) and plates are incubated for 1 h at 37°C, 5% CO₂. Subsequently SIP at 5 µM final concentration is added and plates are incubated for 16 to 24 h at 37°C, 5% CO₂ after which the supernatant was collected. IL-8 levels in the supernatant are determined with the IL-8 ELISA of R&D systems.

**Table IX. IL8 production assay with HSA for illustrative compounds of the invention**

| **Cpd #** | **HSA IL8 EC₅₀ (nM)** |
|---|---|
| 1 | 61.5 |
| 2 | 25.1 |
| 3 | 478.0 |
| 4 | 652.0 |
| 5 | 9950.0 |
| 8 | 169.0 |
| 16 | 74.9 |
| 18 | 29.0 |
| 21 | 393.0 |
| 30 | 37.9 |
| 35 | 185.0 |
| 38 | 198.0 |
| 39 | 9950.0 |
| 40 | 3860.0 |
| 41 | 597.0 |
| 42 | 1240.0 |
| 45 | 84.2 |
| 46 | 9950.0 |
| 47 | 42.4 |
| 48 | 104.0 |
| 50 | 74.0 |
| 51 | 758.0 |
| 52 | 79.3 |
| 53 | 51.2 |
| 54 | 278.0 |
| 55 | 179.0 |
| 56 | 98.5 |
| 57 | 17.3 |
| 59 | 116.0 |
| 60 | 4340.0 |
| 63 | 829.0 |
| 64 | 119.0 |
| 65 | 10000 |
| 66 | 29900 |
| 67 | 9950 |
| 68 | 3457 |
| 69 | 9950 |
| 71 | 56 |
| 72 | 47 |
| 73 | 256 |
| 74 | 118 |
| 75 | 223 |
| 76 | 111 |
| 77 | 29900 |
| 78 | 1249 |
| 79 | 7036 |
| 80 | 239 |
| 81 | 10000 |
| 82 | 730.5 |
| 83 | 30000 |
| 85 | 9950 |
| 87 | 2172 |
| 89 | 1578 |
| 90 | 30000 |
| 91 | 2838 |
| 92 | 10000 |
| 93 | 2202 |
| 94 | 5953 |
| 96 | 2273 |
| 97 | 1795 |

### 3.5. In vivo assays

### 3.5.1. Prophylactic bleomycin induced pulmonary fibrosis 14-day mice model

The aim of the study is to test the efficacy of a test compound at three different doses in a 14-day model of bleomycin induced pulmonary fibrosis in mice.

### 3.5.1.1. Animals

This study is carried out on C5²7BL/6N male mice, supplied by Charles River, Italy, which are acclimatized for at least 5 days in an environment maintained at 22°C, at 55% relative humidity, with 15-20 air changes per hour under light cycles of 12 h. Mice pelleted food and water are provided ad libitum.

At least one day prior to start of experiment, all animals are allocated randomly into groups as indicated in the table below.

All animal related research is conducted in accordance with 2010/63/EU and National legislation regulating the use of laboratory animals in scientific research and for other purposes (Official Gazette 55/13).

**Table X. Study groups**

| **Groups** | **Purpose** | **n** | **Dose** | **Treatment schedule Days (Frequency)** | **Route** | **Vehicle** |
|---|---|---|---|---|---|---|
| **1 PBS+Vehicle** | control | 15 | - | D0-D14 (BID) | NA | NA |
| **2 BLM+Vehicle** | control | 15 | - | D0-D14 (BID) | PO | PEG/MC |
| **3 BLM+ Pirfenidone** | control | 15 | 50 mg/kg | D0-D14 (BID) | PO | 0.1% Natrosol |
| **4 BLM+ test compound** | Active | 15 | 1 mg/kg | D0-D14 (BID) | PO | PEG400/MC 0.5% 20/80 (v/v) |
| **5 BLM+ test compound** | Active | 15 | 3 mg/kg | D0-D14 (BID) | PO | PEG400/MC 0.5% 20/80 (v/v) |
| **6 BLM+ test compound** | Active | 15 | 10 mg/kg | D0-D14 (BID) | PO | PEG400/MC 0.5% 20/80 (v/v) |
| **7 BLM+ test compound satellite for PK** | Active | 10 | 10 mg/kg | D0-D7 (BID) | PO | PEG400/MC 0.5% 20/80 (v/v) |

### 3.5.1.2. Materials

The solvent for the test solutions is prepared by adding 0.5 g of hydroxyethylcellulose (Natrosol) into 500 mL Aqua distillate (0.1 %) under continuous stirring without heating for 5 h on a magnetic stirrer.

Anesthetic solution is prepared by adding 1 mL of Narketan (Narketan 10, Vetoquinol, Bern, Switzerland, 03605877535982) and 0.5 mL of Rompun (Rompun, 2%: Bayer, Leverkusen, Germany) into 9 mL saline. The resulting solution is administered at 10 mL/kg.

To prepare a solution for intranasal challenge (i.n.) challenge, 0.8 mg/mL stock solutions of bleomycin (Bleomycin sulphate, Enzo Life Sciences, Inc., USA; CAS No. 9041-93-4; Cat. No. BML-AP302-0010) are thawn and diluted in 330 µL of saline.

Prior to i.n administration, mice are anesthetized i.p. with the anesthetic solution described above.

Fresh pirfenidone formulation is prepared daily in 0.1% Natrosol formulations to a final concentration of 5 mg/mL. Before dosing, animals are weighed and the Pirfenidone amount administered is adjusted accordingly to individual weights corresponding to 10 mL/kg body weight, twice daily p.o., with 7.5 h interval between two administrations.

Finally, test compound solutions are prepared by dissolving the suitable amount of said test compound in PEG 400 (20% of the final volume) then MC 0.5% (80% of the final volume) to reach final concentrations of 1 mg/mL, 0.3 mg/mL and 0.1 mg/mL, thus yielding compound for a doses of 10 mg/kg, 3 mg/kg and 1 mg/kg. Prior to dosing, animals are weighed and the amount administered adjusted accordingly to individual weights.

The application volume of the test doses corresponds to 10 mL/kg body weight, and is the test compounds are administered p.o. twice daily, with 7.5 h interval between two administrations.

### 3.5.1.3. Study

Animals are examined clinically twice daily. List of clinical signs and parameters are indicated in human endpoints table. Animals are weighed daily starting from D0.

On day 14, two hours post dosing with pirfenidone or test compound, mice are sacrificed by anesthetic overdose.

The lungs are excised and weighed individually. For the all groups: the whole superior right lung lobe is placed into a Precellys tube containing silica beads and immediately snap frozen in liquid nitrogen and subjected to gene expression analysis.

All remaining lungs are placed into marked bottles containing 10% buffered formalin for further histopathological evaluation.

### 3.5.1.4. Sample analysis, data processing and statistical evaluation

Body weight data and lung weight data are processed using MS Excel. Statistical analysis and graphical presentation are performed using GraphPad Prism software (version 5.04).

One-way ANOVA or Mann-Whitney test are employed for lung weights.

Two-way ANOVA are employed for body weight changes.

Differences between groups will be considered statistically significant when p<0.05.

For histopathological evaluation, whole lungs (except sampled superior right lung) are embedded in paraffin and stained with Mallory's trichrome.

Pulmonary histological changes are assessed using Matsuse modification of Ashcroft score (Ashcroft et al., 1988; Matsuse et al., 1999). Statistical analysis and graphical presentation is performed using GraphPad Prism software (version 5.04). Mann-Whitney test is employed.

Differences between groups will be considered statistically significant when p<0.05.

| | **Ashcroft Score** |
|---|---|
| 1 | Normal lungs (no fibrosis) |
| 2 | Minimal fibrotic thickening of alveolar or bronchial walls (network of fine collagen fibrils) |
| 3 | Moderate fibrotic thickening of walls without obvious damage to lung architecture |
| 4 | Fibrosis with damage of pulmonary structure (coarse fibrous bands or small fibrous masses, intra-alveolar collagen fibrils) |
| 5 | Large fibrous area with svere distortion of lung structure |

### 3.5.1.5. PK analysis - Group 7

### 3.5.1.5.1 Protocol

Animals in group 7 (n=10) are included for PK study only and are not be subjected to clinical sign scoring.

These animals are induced with the disease at the start of treatment at day 0 and are sequentially sacrificed on day 7 at 1 h, 3 h, 6 h, 8 h, 24 h after the first administration of test compound.

A blood sample (50 µL) is collected from the tail vein into Li-heparin anticoagulant tubes for each time point and kept on ice until separation. Within maximum 30 min after collection, blood samples are centrifuged at 2000 g for 10 min at 4°C and the resulting plasma samples are aliquoted into polypropylene tubes (1x 25 µL). The samples are stored frozen at -20°C until analysis.

The lung tissue is collected at sacrifice after blood sampling for each animal, then weighed and placed into polypropylene tubes prior to freezing. The samples are stored frozen at -80°C until analysis.

### 3.5.1.5.2 Plasma concentration and pharmacokinetic analysis

Plasma and lung concentrations are measured via LC-MS/MS. Samples are prepared for LC-MS/MS analysis via protein precipitation. The plasma concentrations measured below the lower limit of quantification (LLOQ) are reported as below the limit of quantification (BLQ).

The test compound concentrations in plasma are expressed in ng/mL.

Mean plasma concentrations are calculated. For mean calculation, the concentrations below the LLOQ are set to zero. Therefore, mean values may be BLQ. Standard deviation (SD), standard error of the mean (SE) and coefficient of variation (CV, %) are tabulated when at least three plasma concentration values are above the LLOQ.

Non-compartmental analysis on individual plasma concentrations is performed using PhoenixTM WinNonlin® 6.3 (Pharsight Corporation) to determine at least, the following pharmacokinetic parameters:
- Maximum plasma concentration, Cmax (µg/mL) with the corresponding time, tmax (h),
- Area under the plasma concentration versus time curve up to the last quantifiable concentration AUC₀₋ₜ or up to 24 h AUC₀₋₂₄ₕ (µg.h/mL) (if compound is quantifiable up to 24h postdose), and/or up to infinity AUC_{0-∞}, (µg.h/mL) is calculated according to the linear up/log down trapezoidal rule. Partial AUC may be calculated if deemed necessary. Concentrations below the limit of quantification (BLQ) are set to zero. No AUC is calculated if there are less than three quantifiable time points. AUC_{0-∞} is considered if %AUCextra < 20%,
- Apparent terminal elimination half-life, t1/2 (h) is only reported if three or more time points, excluding tmax is used for linear regression, and if the adjusted R² > 0.80.
- Normalized AUC and Cmax dose.
- Mean pharmacokinetic parameters are calculated. Standard deviation (SD) and coefficient of variation (CV, %) are tabulated if at least three values are available.

### Example 4. ADME Models

### 4.1. Thermodynamic solubility

Thermodynamic solubility of a compound is determined in water, phosphate or citrate buffer with pH of choice or biologically relevant gastrointestinal media (FaSSIF, FeSSIF, SGF). Dry matter of the compound is added to the medium of choice and incubated for 24 h at room temperature. The concentration of compound in the supernatant is analyzed by LC/MS-MS and the signal is plotted against the linear standard curve of that compound.

2.5-3 mg dry matter of test compound is dissolved in water, phosphate or citrate buffer with pH of choice or biologically relevant gastrointestinal media (FaSSIF, FeSSIF, SGF) in a glass vial. After addition of a magnetic stirrer, the samples are stirred for 24 h at room temperature. The vials are then centrifuged shortly and the supernatant is filtered. Each sample is diluted by a factor of 100 and a 10 in DMSO. A final 100 fold dilution in 70/30 water/acetonitrile is used for LCMS-MS analysis.

A standard curve is made starting from a 10 mM stock in DMSO, freshly prepared from dry matter. From this 10 mM DMSO stock solution, intermediate working solutions of 200, 50 and 10 µg/mL in DMSO are made and used to prepare 40, 20, 10, 5, 1, 0.2, 0.1 and 0.04 µg/mL solutions in DMSO. Two quality control samples are made: one of 15 µg/mL and one of 0.5 µg/mL in DMSO, also starting from the DMSO working stock solutions.

The standard curve and quality controls are diluted by a factor of 100 in 70/30 water/acetonitrile and analyzed on LC/MS-MS. The peak areas of the standard curve are plotted in a graph and a linear or polynomial of the second order equation is used to calculate the unknown concentrations of the test compound.

Solubility values are reported in µM or µg/mL.

### 4.2. Microsomal stability

A 10 mM stock solution of compound in DMSO is 1,668 fold diluted in a 105 mM phosphate buffer pH 7.4. Of this compound dilution, 50 µL is transferred in two 96-well plates: one for time point 0 min (T0 plate) and one for time point 30 min (T30 plate) and pre-warmed at 37 °C.

In the time zero reference sample (T0 plate), 100 µL MeOH (1:1) is added to the wells. In each assay plate (T0 and T30 min), 50 µL of microsomal mix is then added.

Final reaction concentrations are: 3 µM compound, 0.5 mg/mL microsomes, 0.4 U/mL GDPDH, 3.3 mM MgCl₂, 3.3 mM glucose-6-phosphate and 1.3 mM NADP+.

The T30 plate is incubated at 37 °C, 300 rpm and after 30 min of incubation the reaction is stopped with MeOH (1:1). The samples are mixed, centrifuged and the supernatant is harvested for analysis on LC-MS/MS (API2000 from Applied Biosystems).

The samples are analyzed on LC-MS/MS with a flow rate of 0.5mL/min. Solvent A is 0.1% Formic Acid in water and solvent B is 0.1% Formic Acid in methanol. The sample is run under positive ion spray on a Pursuit 5 C18 2.0mm column (Varian). The solvent gradient has a total run time of 1.4 min and ranges from 10% B to 100% B. Peak area from the parent compound at time 0 is considered to be 100% remaining. The percentage remaining after 30 min incubation is calculated from time 0. The solubility of the compound in the final test concentration in buffer is inspected by microscope and results are also reported.

### 4.3. Hepatocyte stability.

Test compounds (1 µM initial concentration, n=2) are incubated in Williams' Medium E, containing 4 mM L-glutamine and 2 mM magnesium sulphate, with pooled cryopreserved hepatocytes (Celsis International) in suspension at cell densities of 0.25-0.5 million viable cells/mL. The incubations are performed at 37 °C in a shaking water bath with 100 µL samples taken from the incubation at 0, 10, 20, 45 and 90 min, and reactions terminated by addition of 100 µL of acetonitrile containing carbamazepine as analytical internal standard. Samples are centrifuged and the supernatant fractions analysed by LC-MS/MS. The instrument responses (i.e. peak heights) are referenced to the zero time-point samples (as 100%) in order to determine the percentage of compound remaining. Ln plots of the % remaining for each compound are used to determine the half-life for the hepatocyte incubations. Half-life values are calculated from the relationship: T1/2 (min) = -0.693/λ, where λ is the slope of the Ln concentration vs time curve. Standard compounds testosterone, midazolam, and 4-methylumbelliferone are included in the assay design.

### FINAL REMARKS

It will be appreciated by those skilled in the art that the foregoing descriptions are exemplary and explanatory in nature, and intended to illustrate the invention and its preferred embodiments.

It should be understood that factors such as the differential cell penetration capacity of the various compounds can contribute to discrepancies between the activity of the compounds in the *in vitro* biochemical and cellular assays.

At least some of the chemical names of compound of the invention as given and set forth in this application, may have been generated on an automated basis by use of a commercially available chemical naming software program, and have not been independently verified. Representative programs performing this function include the Lexichem naming tool sold by Open Eye Software, Inc. and the Autonom Software tool sold by MDL, Inc. In the instance where the indicated chemical name and the depicted structure differ, the depicted structure will control.

### REFERENCES

Adada, M., Canals, D., Hannun, Y.A., Obeid, L.M., 2013. Sphingosine-1-phosphate receptor 2. FEBS J. 280, 6354-6366. doi:10.1111/febs.12446
Ashcroft, T., Simpson, J.M., Timbrell, V., 1988. Simple method of estimating severity of pulmonary fibrosis on a numerical scale. J. Clin. Pathol. 41, 467-470.
Blankenbach, K.V., Schwalm, S., Pfeilschifter, J., Meyer Zu Heringdorf, D., 2016. Sphingosine-1-Phosphate Receptor-2 Antagonists: Therapeutic Potential and Potential Risks. Front. Pharmacol. 7, 167. doi:10.3389/fphar.2016.00167
Hobson, A.D., Harris, C.M., van der Kam, E.L., Turner, S.C., Abibi, A., Aguirre, A.L., Bousquet, P., Kebede, T., Konopacki, D.B., Gintant, G., Kim, Y., Larson, K., Maull, J.W., Moore, N.S., Shi, D., Shrestha, A., Tang, X., Zhang, P., Sarris, K.K., 2015. Discovery of A-971432, An Orally Bioavailable Selective Sphingosine-1-Phosphate Receptor 5 (S1P5) Agonist for the Potential Treatment of Neurodegenerative Disorders. J. Med. Chem. 58, 9154-9170. doi:10.1021/acs.jmedchem.5b00928
Matsuse, T., Teramoto, S., Katayama, H., Sudo, E., Ekimoto, H., Mitsuhashi, H., Uejima, Y., Fukuchi, Y., Ouchi, Y., 1999. ICAM-1 mediates lung leukocyte recruitment but not pulmonary fibrosis in a murine model of bleomycin-induced lung injury. Eur. Respir. J. 13, 71-77.
Milstien, S., Spiegel, S., 2006. Targeting sphingosine-1-phosphate: A novel avenue for cancer therapeutics. Cancer Cell 9, 148-150. doi:10.1016/j.ccr.2006.02.025
Nanthakumar, C.B., Hatley, R.J.D., Lemma, S., Gauldie, J., Marshall, R.P., Macdonald, S.J.F., 2015. Dissecting fibrosis: therapeutic insights from the small-molecule toolbox. Nat. Rev. Drug Discov. 14, 693-720. doi:10.1038/nrd4592
Sobel, K., Menyhart, K., Killer, N., Renault, B., Bauer, Y., Studer, R., Steiner, B., Bolli, M.H., Nayler, O., Gatfield, J., 2013. Sphingosine 1-Phosphate (S1P) Receptor Agonists Mediate Pro-fibrotic Responses in Normal Human Lung Fibroblasts via S1P2 and S1P3 Receptors and Smad-independent Signaling. J. Biol. Chem. 288, 14839-14851. doi:10.1074/jbc.M112.426726
Takabe, K., Paugh, S.W., Milstien, S., Spiegel, S., 2008. "Inside-Out" Signaling of Sphingosine-1-Phosphate: Therapeutic Targets. Pharmacol. Rev. 60, 181-195. doi:10.1124/pr.107.07113

## Claims

1. A compound according to Formula Ia: 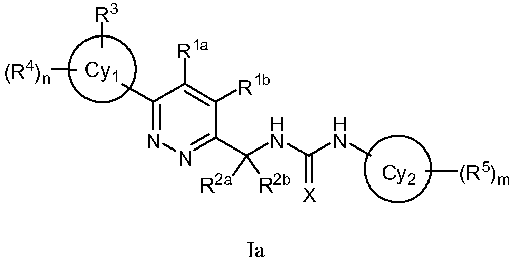 wherein
X is =O, or =N-CN;
R^{1a} is selected from:
- C₁₋₄ alkyl optionally substituted with one or more groups independently selected from
∘ OH,
∘ C₁₋₄ alkoxy optionally substituted with one or more independently selected OH, or C₁₋₄ alkoxy,
∘ -SO₂-C₁₋₄ alkyl,
∘ -O-C₃₋₇ monocyclic cycloalkyl, and
∘ -O-heterocycloalkyl wherein said heterocycloalkyl is a 4-7 membered monocyclic heterocycloalkyl comprising one, two, or three heteroatoms independently selected from N, O, and S;
- -_{NR}6a_{R}6b,
- C₁₋₄ alkoxy,
- C₃₋₇ monocyclic cycloalkyl,
- 4-7 membered monocyclic heterocycloalkyl comprising one, two, or three heteroatoms independently selected from N, O, and S, optionally substituted with one or more halo,
- -O-C₃₋₇ monocyclic cycloalkyl, and
- -O-heterocycloalkyl wherein said heterocycloalkyl is a 4-7 membered monocyclic heterocycloalkyl comprising one, two, or three heteroatoms independently selected from N, O, and S;
R^{1b} is H, or C₁₋₄ alkyl;
Cy₁ is a 5 membered monocyclic heteroaryl ring, comprising one, two, or three heteroatoms independently selected from N, O, or S, or
Cy₁ is 4-7 membered monocyclic heterocycloalkyl ring comprising one, two, or three heteroatoms independently selected from N, O, or S, or 4-7 membered monocyclic heterocycloalkyl ring comprising one, two, or three heteroatoms independently selected from N, O, or S, fused to a 5-6 membered heteroaryl ring comprising one, two, or three heteroatoms independently selected from N, O, or S, which heteroaryl may optionally substituted with one C₁₋₄ alkyl;
each R^{2a} and R^{2b} is independently selected from H, and C₁₋₄ alkyl optionally substituted with one or more independently selected -OH, or C₁₋₄ alkoxy;
R³ is selected from:
- C₁₋₄ alkyl optionally substituted with one or more independently selected:
∘ halo,
∘ -CN,
∘ -OH,
∘ -C₁₋₄ alkoxy, or
∘ -NR^{7a}R^{7b};
- C₁₋₄ alkoxy substituted with one or more halo,
- C₃₋₇ monocyclic cycloalkyl,
- 4-7-membered monocyclic heterocycloalkyl comprising one, two or three heteroatoms independently selected from N, O, and S,
- -CN,
- -S(O)₂-C₁₋₄ alkyl,
- -NR^{8a}R^{8b}, and
- -C(=O)NR^{8c}R^{8d};
each R⁴ is independently selected from:
- C₁₋₄ alkyl optionally substituted with one or more independently selected R¹² groups,
- C₃₋₇ monocyclic cycloalkyl, and
- 4-7 membered monocyclic heterocycloalkyl comprising one, two, or three heteroatoms independently selected from N, O, and S;
Cy₂ is phenyl or 5-6 membered monocyclic heteroaryl comprising one or two heteroatoms independently selected from N, O, and S;
each R⁵ is independently selected from:
- halo,
- -CN,
- -OH,
- C₁₋₄ alkyl optionally substituted with one or more independently selected R¹³groups,
- C₁₋₄ alkoxy optionally substituted with one or more independently selected R¹³groups,
- C₃₋₇ monocyclic cycloalkyl optionally substituted with one or more independently selected R¹⁴ groups,
- 4-11 membered monocyclic, or fused or spiro bicyclic heterocycloalkyl comprising one, two, or three heteroatoms independently selected from N, O, and S, optionally substituted with one or more independently selected R¹⁴ groups,
- -O-C₃₋₇ monocyclic cycloalkyl optionally substituted with one or more independently selected R¹⁴ groups,
- -O-heterocycloalkyl wherein said heterocycloalkyl is a 4-7 membered monocyclic heterocycloalkyl comprising one, two, or three heteroatoms independently selected from N, O, and S, optionally substituted with one or more independently selected R¹⁴ groups, and wherein if a N heteroatom is present, said N heteroatom, is further substituted with one -C(=O)-C₁₋₄ alkyl, -C(=O)-C₁₋₄ alkoxy, -SO₂-C₁₋₄ alkyl, -C(=O)-NH₂, -C(=O)NHC₁₋₄ alkyl, or -C(=O)N(C₁₋₄ alkyl)₂,
- -SO₂-C₁₋₄ alkyl,
- -SO₂NR^{15a}R^{15b},
- -C(=O)NR^{15c}R^{15d}, and
- -NR^{17a}R^{17b};
each R¹² is independently selected from:
- halo,
- OH,
- C₁₋₄ alkoxy,
- -SO₂-C₁₋₄ alkyl,
- C₃₋₇ monocyclic cycloalkyl optionally substituted with one or more independently selected -OH, halo, -CN, C₁₋₄ alkyl, C₁₋₄ alkoxy or =O,
- 4-7 membered monocyclic heterocycloalkyl, comprising one, two, or three heteroatoms independently selected from N, O, and S, optionally substituted with one or more independently selected -OH, halo, -CN, C₁₋₄ alkyl, C₁₋₄ alkoxy or =O,
- -NR^{9a}R^{9b}, and
- -CN;
each R¹³ is independently selected from:
- halo,
- -CN,
- -OH,
- C₁₋₄ alkoxy optionally substituted with one or more independently selected OH, C₁₋₄ alkoxy, or halo,
- -C(=O)NR^{16a}R^{16b},
- -NR^{16c}C(=O)-C₁₋₄ alkyl,
- -NR^{16d}C(=O)-C₁₋₄ alkoxy,
- -SO₂-C₁₋₄ alkyl
- -SO₂NR^{16e}R^{16f},
- -NR^{16g}SO₂-C₁₋₄ alkyl,
- C₃₋₇ monocyclic cycloalkyl optionally substituted with one or more independently selected halo, or C₁₋₄ alkyl optionally substituted with one or more halo, and
- 4-7 membered monocyclic heterocycloalkyl comprising one, two, or three heteroatoms independently selected from N, O, and S, optionally substituted with one or more independently selected halo, or C₁₋₄ alkyl optionally substituted with one or more halo, and wherein if a N heteroatom is present, said N heteroatom, is further substituted with one -C(=O)-C₁₋₄ alkyl, -C(=O)-C₁₋₄ alkoxy, -SO₂-C₁₋₄ alkyl, -C(=O)-NH₂, -C(=O)NHC₁₋₄ alkyl, or -C(=O)N(C₁₋₄ alkyl)₂;
each R¹⁴ is independently selected from:
- halo,
- -CN,
- -OH,
- C₁₋₄ alkoxy optionally substituted with one or more halo, and
- C₁₋₄ alkyl optionally substituted with one or more halo;
each R^{6a}, R^{6b}, R^{7a}, R^{7b}, R^{8a}, R^{8b}, R^{8c}, R^{8d}, R^{9a}, R^{9b}, R^{15a}, R^{15b}, R^{15c}, R^{15d}, R^{16a}, R^{16b}, R^{16e}, R^{16d}, R^{16e}, R^{16f}, and R^{16g} is independently selected from H and C₁₋₄ alkyl;
each R^{17a} and R^{17b} is independently selected from H and C₁₋₄ alkyl optionally substituted with one or more independently selected halo, OH, or C₁₋₄ alkoxy;
the subscript n is 0, 1, 2, or 3; and
the subscript m is 0, 1, 2, 3 or 4;
or a pharmaceutically acceptable salt thereof, or the solvate or the salt of a solvate thereof.

2. A compound or a pharmaceutically acceptable salt thereof according to claim 1, wherein the compound is according to Formula Ib: 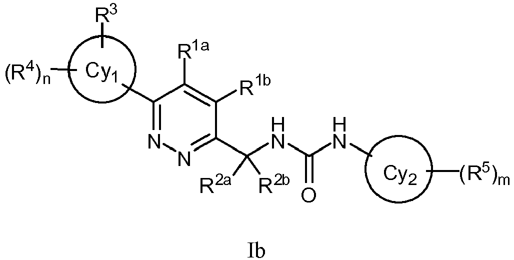 wherein
R^{1a} is selected from:
- C₁₋₄ alkyl optionally substituted with one or more groups independently selected from
∘ OH,
∘ C₁₋₄ alkoxy optionally substituted with one or more independently selected OH, or C₁₋₄ alkoxy,
∘ -SO₂-C₁₋₄ alkyl,
∘ -O-C₃₋₇ monocyclic cycloalkyl, and
∘ -O-heterocycloalkyl wherein said heterocycloalkyl is a 4-7 membered monocyclic heterocycloalkyl comprising one, two, or three heteroatoms independently selected from N, O, and S;
- -NR^{6a}R^{6b},
- C₁₋₄ alkoxy,
- C₃₋₇ monocyclic cycloalkyl,
- 4-7 membered monocyclic heterocycloalkyl comprising one, two, or three heteroatoms independently selected from N, O, and S, optionally substituted with one or more halo,
- -O-C₃₋₇ monocyclic cycloalkyl, and
- -O-heterocycloalkyl wherein said heterocycloalkyl is a 4-7 membered monocyclic heterocycloalkyl comprising one, two, or three heteroatoms independently selected from N, O, and S;
R^{1b} is H, or C₁₋₄ alkyl;
Cy₁ is a 5 membered monocyclic heteroaryl ring, comprising one, two, or three heteroatoms independently selected from N, O, or S;
each R^{2a} and R^{2b} is independently selected from H, and C₁₋₄ alkyl optionally substituted with one or more independently selected -OH, or C₁₋₄ alkoxy;
R³ is selected from:
- C₁₋₄ alkyl optionally substituted with one or more independently selected:
∘ halo,
∘ -CN,
∘ -C₁₋₄ alkoxy, or
∘ -NR^{7a}R^{7b};
- C₁₋₄ alkoxy substituted with one or more halo,
- C₃₋₇ monocyclic cycloalkyl,
- 4-7-membered monocyclic heterocycloalkyl comprising one, two or three heteroatoms independently selected from N, O, and S,
- -CN,
- -NR^{8a}R^{8b}, and
- -C(=O)NR^{8c}R^{8d};
each R⁴ is independently selected from:
- C₁₋₄ alkyl optionally substituted with one or more independently selected R¹² groups,
- C₃₋₇ monocyclic cycloalkyl, and
- 4-7 membered monocyclic heterocycloalkyl comprising one, two, or three heteroatoms independently selected from N, O, and S;
Cy₂ is phenyl or 5-6 membered monocyclic heteroaryl comprising one or two heteroatoms independently selected from N, O, and S;
each R⁵ is independently selected from:
- halo,
- -CN,
- -OH,
- C₁₋₄ alkyl optionally substituted with one or more independently selected R¹³ groups,
- C₁₋₄ alkoxy optionally substituted with one or more independently selected R¹³ groups,
- C₃₋₇ monocyclic cycloalkyl optionally substituted with one or more independently selected R¹⁴ groups,
- 4-11 membered monocyclic, or fused or spiro bicyclic heterocycloalkyl comprising one, two, or three heteroatoms independently selected from N, O, and S, optionally substituted with one or more independently selected R¹⁴ groups,
- -O-C₃₋₇ monocyclic cycloalkyl optionally substituted with one or more independently selected R¹⁴ groups,
- -O-heterocycloalkyl wherein said heterocycloalkyl is a 4-7 membered monocyclic heterocycloalkyl comprising one, two, or three heteroatoms independently selected from N, O, and S, optionally substituted with one or more independently selected R¹⁴ groups, and wherein if a N heteroatom is present, said N heteroatom, is further substituted with one -C(=O)-C₁₋₄ alkyl, -C(=O)-C₁₋₄ alkoxy, -SO₂-C₁₋₄ alkyl, -C(=O)-NH₂, -C(=O)NHC₁₋₄ alkyl, or -C(=O)N(C₁₋₄ alkyl)₂,
- -SO₂-C₁₋₄ alkyl,
- -SO₂NR^{15a}R^{15b},
- -C(=O)NR^{15c}R^{15d}, and
- -NR^{17a}R^{17b};
each R¹² is independently selected from:
- halo,
- OH,
- C₁₋₄ alkoxy,
- -SO₂-C₁₋₄ alkyl,
- C₃₋₇ monocyclic cycloalkyl optionally substituted with one or more independently selected -OH, halo, -CN, C₁₋₄ alkyl, C₁₋₄ alkoxy or =O,
- 4-7 membered monocyclic heterocycloalkyl, comprising one, two, or three heteroatoms independently selected from N, O, and S, optionally substituted with one or more independently selected -OH, halo, -CN, C₁₋₄ alkyl, C₁₋₄ alkoxy or =O,
- -NR^{9a}R^{9b}, and
- -CN;
each R¹³ is independently selected from:
- halo,
- -CN,
- -OH,
- C₁₋₄ alkoxy optionally substituted with one or more independently selected OH, C₁₋₄ alkoxy, or halo,
- -C(=O)NR^{16a}R^{16b},
- -NR^{16c}C(=O)-C₁₋₄ alkyl,
- -NR^{16d}C(=O)-C₁₋₄ alkoxy,
- -SO₂-C₁₋₄ alkyl
- -SO₂NR^{16e}R^{16f},
- -NR^{16g}SO₂-C₁₋₄ alkyl,
- C₃₋₇ monocyclic cycloalkyl optionally substituted with one or more independently selected halo, or C₁₋₄ alkyl optionally substituted with one or more halo, and
- 4-7 membered monocyclic heterocycloalkyl comprising one, two, or three heteroatoms independently selected from N, O, and S, optionally substituted with one or more independently selected halo, or C₁₋₄ alkyl optionally substituted with one or more halo, and wherein if a N heteroatom is present, said N heteroatom, is further substituted with one -C(=O)-C₁₋₄ alkyl, -C(=O)-C₁₋₄ alkoxy, -SO₂-C₁₋₄ alkyl, -C(=O)-NH₂, -C(=O)NHC₁₋₄ alkyl, or -C(=O)N(C₁₋₄ alkyl)₂;
each R¹⁴ is independently selected from:
- halo,
- -CN,
- -OH,
- C₁₋₄ alkoxy optionally substituted with one or more halo, and
- C₁₋₄ alkyl optionally substituted with one or more halo;
each R^{6a}, R^{6b}, R^{7a}, R^{7b}, R^{8a}, ^{R8b}, R^{8c}, R^{8d}, R^{9a}, R^{9b}, R^{15a}, R^{15b}, R^{15c}, R^{15d}, R^{16a}, R^{16b}, R^{16c}, R^{16d}, R^{16e}, R^{16f}, and R^{16g} is independently selected from H and C₁₋₄ alkyl;
each R^{17a} and R^{17b} is independently selected from H and C₁₋₄ alkyl optionally substituted with one or more independently selected halo, OH, or C₁₋₄ alkoxy;
the subscript n is 0, 1, 2, or 3; and
the subscript m is 0, 1, 2, 3 or 4;
or a pharmaceutically acceptable salt thereof, or the solvate or the salt of a solvate thereof.

3. A compound or a pharmaceutically acceptable salt thereof according to claim 1 or 2, wherein the compound is according to Formula IV:

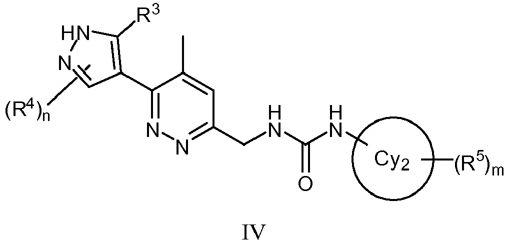

4. A compound or a pharmaceutically acceptable salt thereof according to claim 1, 2 or 3, wherein R³ is -CH₃, -CH₂CH₃, or -(CH₂)₂CH₃, each of which is substituted with one, two or three independently selected halo, -CN, -C₁₋₄ alkoxy, or -NR^{7a}R^{7b}.

5. A compound or a pharmaceutically acceptable salt thereof according to claim 1, 2 or 3, wherein R³ is C₃₋₇ monocyclic cycloalkyl.

6. A compound or a pharmaceutically acceptable salt thereof according to claim 1, 2 or 3, wherein the compound is according to any one of Formula Va-Vb:

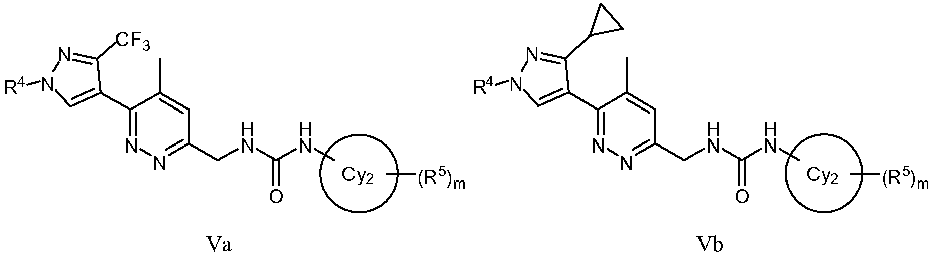

7. A compound or a pharmaceutically acceptable salt thereof according to any one of claims 1-6, wherein R⁴ is C₁₋₄ alkyl.

8. A compound or a pharmaceutically acceptable salt thereof according to any one of claims 1-7, wherein Cy₂ is pyridinyl.

9. A compound or a pharmaceutically acceptable salt thereof according to any one of claims 1-8, wherein the subscript m is 1, 2, 3, or 4.

10. A compound or a pharmaceutically acceptable salt thereof according to any one of claims 1-9, wherein R⁵ is C₁₋₄ alkyl substituted with one or more independently selected R¹³ groups.

11. A compound or a pharmaceutically acceptable salt thereof according to claim 10, wherein R¹³ is F, -CN, -OH, -OCH₃, -OCF₃, -OCH₂CH₃, -OCH₂CF₃, -OCH₂CH₂OH, or -OCH₂CH₂OCH₃.

12. A compound or a pharmaceutically acceptable salt thereof according to any one of claims 1-11, wherein R⁵ is C₁₋₄ alkoxy.

13. A pharmaceutical composition comprising a compound or a pharmaceutically acceptable salt thereof according to any one of claims 1-12, and a pharmaceutically acceptable carrier.

14. A pharmaceutical composition according to claim 13 comprising a further therapeutic agent.

15. A compound or a pharmaceutically acceptable salt thereof, according to any one of claims 1-12, or a pharmaceutical composition according to claim 13 or 14 for use in medicine.

16. A compound or a pharmaceutically acceptable salt thereof according to any one of claims 1-12, or a pharmaceutical composition according to claim 13 or 14 for use in the prophylaxis and/or treatment of fibrotic diseases, inflammatory diseases, autoimmune diseases, metabolic diseases, cardiovascular diseases, and/or proliferative diseases.

17. A pharmaceutical composition according to claim 14, wherein the further therapeutic agent is an agent for the prophylaxis and/or treatment of fibrotic diseases, inflammatory diseases, autoimmune diseases, metabolic diseases, cardiovascular diseases, and/or proliferative diseases.

## Patentansprüche

1. Verbindung gemäß Formel 1a: 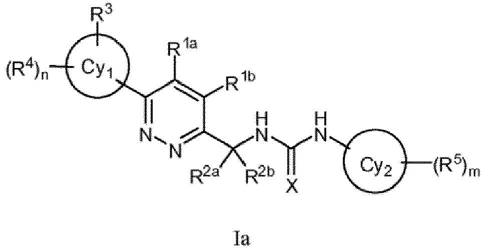 wobei
X=O oder =N-CN ist;
R^{1a} ausgewählt ist aus:
- C₁₋₄ Alkyl, optional substituiert mit einer oder mehreren Gruppen, die unabhängig voneinander ausgewählt sind aus
∘ OH,
∘ C₁₋₄ Alkoxy, optional substituiert mit einem oder mehreren, ausgewählt aus OH oder C₁₋₄Alkoxy,
∘ -SO₂-C₁₋₄Alkoxy,
∘ -O-C₃₋₇ monocyclischem Cycloalkyl, und
∘ Heterocycloalkyl, wobei das Heterozykloalkyl ein 4-7-gliedriges monozyklisches Heterocykloalkyl mit einem, zwei oder drei Heteroatomen ist, die unabhängig voneinander ausgewählt sind aus N, O und S;
- -NR^{6a}R^{6b},
- C₁₋₄Alkoxy,
- C₃₋₇ monocyclischem Cycloalkyl,
- 4-7-gliedrigem monozyklischem Heterocycloalkyl umfassend ein, zwei oder drei Heteroatome, die unabhängig voneinander ausgewählt sind aus N, O und S, optional substituiert mit einem oder mehreren Halogenen,
- -O-Heterocycloalkyl, wobei das Heterocycloalkyl ein 4-7-gliedriges monozyklisches Heterocycloalkyl ist, umfassend ein, zwei oder drei Heteroatome, die unabhängig voneinander ausgewählt sind aus N, O und S;
R^{1b} H oder C₁₋₄Alkyl ist;
Cy₁ ein 5-gliedriger monocyclischer Heteroarylring ist, umfassend ein, zwei oder drei Heteroatome, unabhängig voneinander ausgewählt aus N, O oder S, oder
Cy₁ ein 4-7-gliedriger monocyclischer Heterocycloalkylring ist, umfassend ein, zwei oder drei Heteroatome, unabhängig voneinander ausgewählt aus N, O oder S, oder ein 4-7-gliedriger monocyclischer Heterocycloalkylring ist, umfassend ein, zwei oder drei Heteroatome, unabhängig voneinander ausgewählt aus N, O oder S, kondensiert mit einem 5-6-gliedrigen Heteroarylring, umfassend ein, zwei oder drei Heteroatome, unabhängig voneinander ausgewählt aus N, O oder S, wobei das Heteroaryl optional mit einem C₁₋₄ Alkyl substituiert sein kann;
jedes R^{2a} und R^{2b} unabhängig voneinander ausgewählt ist aus H und C₁₋₄ Alkyl, optional substituiert mit einem oder mehreren unabhängig voneinander ausgewählten -OH oder C₁₋₄ Alkoxy;
R³ ausgewählt ist aus:
- C₁₋₄ Alkyl, optional substituiert mit einem oder mehreren unabhängig voneinander ausgewählten:
∘ Halogen,
∘ -CN,
∘ -OH,
∘ C₁₋₄ Alkoxy oder
∘ -NR^{7a}R^{7b};
- C₁₋₄ Alkoxy, substituiert mit einem oder mehreren Halogenatomen,
- C₃₋₇ monocyclischem Cycloalkyl,
- 4-7-gliedrigem monocyclischem Heterocycloalkyl, umfassend ein, zwei oder drei Heteroatome, unabhängig voneinander ausgewählt aus N, O und S,
- -CN,
- -S(O)₂-C₁₋₄ Alkyl,
- NR^{8a}R^{8b} und
- -C(=O)NR^{8c}R^{8d};
jedes R⁴ unabhängig voneinander ausgewählt aus:
- C₁₋₄ Alkyl, optional substituiert mit einer oder mehreren unabhängig voneinander ausgewählten R¹² -Gruppen,
- C₃₋₇ monocyclischem Cycloalkyl, und
- 4-7-gliedrigem monocyclischem Heterocycloalkyl, umfassend ein, zwei oder drei Heteroatome, die unabhängig voneinander ausgewählt sind aus N, O und S;
Cy₂ Phenyl oder 5-6-gliedriges monocyclisches Heteroaryl ist, umfassend ein oder zwei Heteroatome, unabhängig voneinander ausgewählt aus N, O und S; jedes R⁵ unabhängig voneinander ausgewählt ist aus:
- Halogen,
- -CN,
- -OH,
- C₁₋₄ Alkyl, optional substituiert mit einer oder mehreren unabhängig voneinander ausgewählten R¹³ -Gruppen,
- C₁₋₄ Alkoxy, optional substituiert mit einer oder mehreren unabhängig voneinander ausgewählten R¹³ -Gruppen,
- C₃₋₇ monocyclischem Cycloalkyl, optional substituiert mit einer oder mehreren unabhängig voneinander ausgewählten R¹⁴ -Gruppen,
- 4-11-gliedrigem monocyclischem oder kondensiertem oder spirobicyclischem Heterocycloalkyl, umfassend ein, zwei oder drei Heteroatome, die unabhängig voneinander ausgewählt sind aus N, O und S, optional substituiert mit einer oder mehreren unabhängig voneinander ausgewählten R¹⁴ -Gruppen,
- O-C₃₋₇ monocyclischem Cycloalkyl, optional substituiert mit einer oder mehreren unabhängig voneinander ausgewählten R¹⁴ -Gruppen,
- O-Heterocycloalkyl, wobei das Heterocycloalkyl ein 4-7-gliedriges monocyclisches Heterocycloalkyl ist, umfassend ein, zwei oder drei Heteroatome, die unabhängig voneinander ausgewählt sind aus N, O und S, optional substituiert mit einer oder mehreren unabhängig voneinander ausgewählten R¹⁴ -Gruppen, und wobei, falls ein N-Heteroatom vorhanden ist, das N-Heteroatom weiter substituiert ist mit einem -C(=O) C₁₋₄ Alkyl, -C(=O)-C₁₋₄ Alkoxy, -SO₂-C₁₋₄ Alkyl,
- C(=O)-NH₂, -C(=O)-NHC₁₋₄ Alkyl oder -C(=O)-N(C₁₋₄ Alkyl)₂,
- SO₂-C₁₋₄ Alkyl,
- SO₂NR^{15a}R^{15b},
- C(=O)NR^{15c}R^{15d}, und
- NR^{17a}R^{17b};
jedes R¹² unabhängig voneinander ausgewählt ist aus:
- Halogen,
- OH,
- C₁₋₄ Alkoxy,
- SO₂-C₁₋₄ Alkyl,
- C₃₋₇ monocyclischem Cycloalkyl, optional substituiert mit einem oder mehreren unabhängig voneinander ausgewählten -OH, Halogen, -CN, C₁₋₄ Alkyl, C₁₋₄ Alkoxy oder =O,
- 4-7-gliedrigem monocyclischem Heterocycloalkyl, umfassend ein, zwei oder drei Heteroatome, die unabhängig voneinander ausgewählt sind aus N, O und S, optional substituiert mit einem oder mehreren unabhängig voneinander ausgewählten -OH, Halogen, -CN, C₁₋₄ Alkyl, C₁₋₄ Alkoxy oder =O,
- -NR^{9a}R^{9b}, und
- -CN;
jedes R¹³ unabhängig voneinander ausgewählt ist aus:
- Halogen,
- -CN,
- -OH,
- C₁₋₄ Alkoxy, optional substituiert mit einem oder mehreren unabhängig voneinander ausgewählten OH, C₁₋₄ Alkoxy oder Halogen,
- -C(=O)NR^{16a}R^{16b},
- -NR^{16c}C(=O)-C₁₋₄ Alkyl,
- -NR^{16d}C(=O)-C₁₋₄ Alkoxy,
- -SO₂-C₁₋₄ Alkyl
- -SO₂NR^{16e}R^{16f},
- -NR^{16g}SO₂-C₁₋₄ Alkyl,
- C₃₋₇ monocyclischem Cycloalkyl, optional substituiert mit einem oder mehreren unabhängig voneinander ausgewählten Halogenatomen, oder C₁₋₄ Alkyl, optional substituiert mit einem oder mehreren Halogenatomen, und
- 4-7-gliedrigem monocyclischem Heterocycloalkyl, umfassend ein, zwei, oder drei Heteroatome, die unabhängig voneinander ausgewählt sind aus N, O und S, optional substituiert mit einem oder mehreren unabhängig voneinander ausgewählten Halogenatomen oder C₁₋₄ Alkyl, optional substituiert mit einem oder mehreren Halogenatomen, und wobei, falls ein N-Heteroatom enthalten ist, das N-Heteroatom ferner substituiert ist mit einem -C(=O)-C₁₋₄ Alkyl, -C(=O)-C₁₋₄ Alkoxy, -SO₂-C₁₋₄ Alkyl,
- C(=O)-NH₂, -C(=O)-NHC₁₋₄ Alkyl oder -C(=O)N(C₁₋₄ Alkyl)₂;
jedes R¹⁴ unabhängig voneinander ausgewählt ist aus:
- Halogen,
- -CN,
- -OH,
- C₁₋₄ Alkoxy, optional substituiert mit einem oder mehreren Halogenatomen,
- C₁₋₄ Alkyl, optional substituiert mit einem oder mehreren Halogenatomen,
jedes R^{6a}, R^{6b}, R^{7a}, R^{7b}, R^{8a}, R^{8b}, R^{8c}, R^{8d}, R^{9a}, R^{9b}, R^{15a}, R^{15b}, R^{15c}, R^{15d}, R^{16a}, R^{16b}, R^{16c}, R^{16d}, R^{16e}, R^{16f} und R^{16g} unabhängig voneinander ausgewählt ist aus H und C₁₋₄ Alkyl;
jedes R^{17a} und R^{17b} unabhängig voneinander ausgewählt ist aus H und C₁₋₄ Alkyl, optional substituiert mit einem oder mehreren unabhängig voneinander ausgewählten Halogenatomen, OH oder C₁₋₄ -Alkoxy;
der Index n 0, 1, 2 oder 3 ist; und
der Index m 0, 1, 2, 3 oder 4 ist;
oder ein pharmazeutisch annehmbares Salz dieser oder das Solvat oder das Salz eines Solvats dieser.

2. Verbindung oder pharmazeutisch annehmbares Salz dieser nach Anspruch 1, wobei die Verbindung gemäß Formel Ib ist: 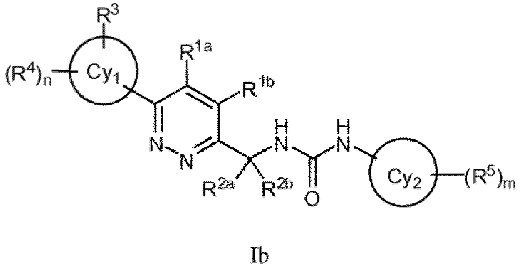 wobei
R^{1a} ausgewählt ist aus:
- C₁₋₄ Alkyl, optional substituiert mit einer oder mehreren Gruppen, die unabhängig voneinander ausgewählt sind aus
∘ OH,
∘ C₁₋₄ Alkoxy, optional substituiert mit einem oder mehreren, ausgewählt aus OH oder C₁₋₄Alkoxy,
∘ -SO₂-C₁₋₄Alkyl,
∘ -O-C₃₋₇ monocyclischem Cycloalkyl, und
∘ -O-Heterocycloalkyl, wobei das Heterozykloalkyl ein 4-7-gliedriges monozyklisches Heterocycloalkyl mit einem, zwei oder drei Heteroatomen ist, die unabhängig voneinander ausgewählt sind aus N, O und S;
- -NR^{6a}R^{6b},
- C₁₋₄Alkoxy,
- C₃₋₇ monocyclischem Cycloalkyl,
- 4-7-gliedrigem monozyklischem Heterocycloalkyl umfassend ein, zwei oder drei Heteroatome, die unabhängig voneinander ausgewählt sind aus N, O und S, optional substituiert mit einem oder mehreren Halogenen,
- -O-C₃₋₇ monocyclischem Cycloalkyl, und
- -O-Heterocycloalkyl, wobei das Heterocykloalkyl ein 4-7-gliedriges monozyklisches Heterozycloalkyl ist, umfassend ein, zwei oder drei Heteroatome, die unabhängig voneinander ausgewählt sind aus N, O und S;
R^{1b} H oder C₁₋₄Alkyl ist;
Cy₁ ein 5-gliedriger monocyclischer Heteroarylring ist, umfassend ein, zwei oder drei Heteroatome, die unabhängig voneinander ausgewählt sind aus N, O oder S, oder
jedes R^{2a} und R^{2b} unabhängig voneinander ausgewählt ist aus H und C₁₋₄ Alkyl, optional substituiert mit einem oder mehreren unabhängig voneinander ausgewählten -OH oder C₁₋₄ Alkoxy;
R³ ausgewählt ist aus:
- C₁₋₄ Alkyl, optional substituiert mit einem oder mehreren unabhängig voneinander ausgewählten:
∘ Halogen,
∘ -CN,
∘ -OH,
∘ C₁₋₄ Alkoxy oder
∘ -NR^{7a}R^{7b};
- C₁₋₄ Alkoxy, substituiert mit einem oder mehreren Halogenatomen,
- C₃₋₇ monocyclischem Cycloalkyl,
- 4-7-gliedrigem monozyklischem Heterocycloalkyl, umfassend ein, zwei oder drei Heteroatome, die unabhängig voneinander ausgewählt sind aus N, O und S,
- -CN,
- NR^{8a}R^{8b} und
- -C(=O)NR^{8c}R^{8d};
jedes R⁴ unabhängig voneinander ausgewählt aus:
- C₁₋₄ Alkyl, optional substituiert mit einer oder mehreren unabhängig voneinander ausgewählten R¹² -Gruppen,
- C₃₋₇ monocyclischem Cycloalkyl, und
- 4-7-gliedrigem monozyklischem Heterocycloalkyl, umfassend ein, zwei oder drei Heteroatome, die unabhängig voneinander ausgewählt sind aus N, O und S;
Cy₂ Phenyl oder 5-6-gliedriges monocyclisches Heteroaryl ist, umfassend ein oder zwei Heteroatome, die unabhängig voneinander ausgewählt sind aus N, O und S;
jedes R⁵ unabhängig voneinander ausgewählt ist aus:
- Halogen,
- -CN,
- -OH,
- C₁₋₄ Alkyl, optional substituiert mit einer oder mehreren unabhängig voneinander ausgewählten R¹³ -Gruppen,
- C₁₋₄ Alkoxy, optional substituiert mit einer oder mehreren unabhängig voneinander ausgewählten R¹³ -Gruppen,
- C₃₋₇ monocyclischem Cycloalkyl, optional substituiert mit einer oder mehreren unabhängig voneinander ausgewählten R¹⁴ -Gruppen,
- 4-11-gliedrigem monocyclischem oder kondensiertem oder spirobicyclischem Heterocycloalkyl, umfassend ein, zwei oder drei Heteroatome, die unabhängig voneinander ausgewählt sind aus N, O und S, optional substituiert mit einer oder mehreren unabhängig voneinander ausgewählten R¹⁴ -Gruppen,
- O-C₃₋₇ monocyclischem Cycloalkyl, optional substituiert mit einer oder mehreren unabhängig voneinander ausgewählten R¹⁴ -Gruppen,
- O-Heterocycloalkyl, wobei das Heterocycloalkyl ein 4-7-gliedriges monocyclisches Heterocycloalkyl ist, umfassend ein, zwei oder drei Heteroatome, die unabhängig voneinander ausgewählt sind aus N, O und S, optional substituiert mit einer oder mehreren unabhängig voneinander ausgewählten R¹⁴ -Gruppen, und wobei, falls ein N-Heteroatom vorhanden ist, das N-Heteroatom weiter substituiert ist mit einem -C(=O)-C₁₋₄ Alkyl, -C(=O)-C₁₋₄ Alkoxy, -SO₂-C₁₋₄ Alkyl,
- C(=O)-NH₂, -C(=O)-NHC₁₋₄ Alkyl oder -C(=O)N(C₁₋₄ Alkyl)₂,
- SO₂-C₁₋₄ Alkyl,
- SO₂NR^{15a}R^{15b},
- C(=O)NR^{15c}R^{15d} und
- NR^{17a}R^{17b};
jedes R¹² unabhängig voneinander ausgewählt ist aus:
- Halogen,
- OH,
- C₁₋₄ Alkoxy,
- SO₂-C₁₋₄ Alkyl,
- C₃₋₇ monocyclischem Cycloalkyl, optional substituiert mit einem oder mehreren unabhängig voneinander ausgewählten -OH, Halogen, -CN, C₁₋₄ Alkyl, C₁₋₄ Alkoxy oder =O,
- 4-7-gliedrigem monocyclischem Heterocycloalkyl, umfassend ein, zwei oder drei Heteroatome, die unabhängig voneinander ausgewählt sind aus N, O und S, optional substituiert mit einem oder mehreren unabhängig voneinander ausgewählten -OH, Halogen, -CN, C₁₋₄ Alkyl, C₁₋₄ Alkoxy oder =O,
- -NR^{9a}R^{9b}, und
- -CN;
jedes R¹³ unabhängig voneinander ausgewählt ist aus:
Halogen,
- -CN,
- -OH,
- C₁₋₄ Alkoxy, optional substituiert mit einem oder mehreren unabhängig voneinander ausgewählten OH, C₁₋₄ Alkoxy oder Halogen,
- -C(=O)NR^{16a}R^{16b},
- -NR^{16c}C(=O)-C₁₋₄ Alkyl,
- -NR^{16d}C(=O)-C₁₋₄ Alkoxy,
- -SO₂-C₁₋₄ Alkyl
- -SO₂NR^{16e}R^{16f},
- -NR^{16g}SO₂-C₁₋₄ Alkyl,
- C₃₋₇ monocyclischem Cycloalkyl, optional substituiert mit einem oder mehreren unabhängig voneinander ausgewählten Halogenatomen, oder C₁₋₄ Alkyl, optional substituiert mit einem oder mehreren Halogenatomen, und
- 4-7-gliedrigem monocyclischem Heterocycloalkyl, umfassend ein, zwei, oder drei Heteroatome, die unabhängig voneinander ausgewählt sind aus N, O und S, optional substituiert mit einem oder mehreren unabhängig voneinander ausgewählten Halogenatomen oder C₁₋₄ Alkyl, optional substituiert mit einem oder mehreren Halogenatomen, und wobei, falls ein N-Heteroatom enthalten ist, das N-Heteroatom ferner substituiert ist mit einem -C(=O)-C₁₋₄ Alkyl, -C(=O)-C₁₋₄ Alkoxy, -SO₂-C₁₋₄ Alkyl, -C(=O)-NH₂, -C(=O)-NHC₁₋₄ Alkyl oder -C(=O)N(C₁₋₄ Alkyl)₂;
jedes R¹⁴ unabhängig voneinander ausgewählt ist aus:
- Halogen,
- -CN,
- -OH,
- C₁₋₄ Alkoxy, optional substituiert mit einem oder mehreren Halogenatomen,
- C₁₋₄ Alkyl, optional substituiert mit einem oder mehreren Halogenatomen,
jedes R^{6a}, R^{6b}, R^{7a}, R^{7b}, R^{8a}, R^{8b}, R^{8c}, R^{8d}, R^{9a}, R^{9b}, R^{15a}, R^{15b}, R^{15c}, R^{15d}, R^{16a}, R^{16b}, R^{16c}, R^{16d}, R^{16e}, R^{16f} und R^{16g} unabhängig voneinander ausgewählt ist aus H und C₁₋₄ Alkyl;
jedes R^{17a} und R^{17b} unabhängig voneinander ausgewählt ist aus H und C₁₋₄ Alkyl, optional substituiert mit einem oder mehreren unabhängig voneinander ausgewählten Halogenatomen, OH oder C₁₋₄ Alkoxy;
der Index n 0, 1, 2 oder 3 ist; und
der Index m 0, 1, 2, 3 oder 4 ist;
oder ein pharmazeutisch annehmbares Salz dieser oder das Solvat oder das Salz eines Solvats dieser.

3. Verbindung oder pharmazeutisch annehmbares Salz dieser nach Anspruch 1 oder 2, wobei die Verbindung gemäß Formel IV ist:

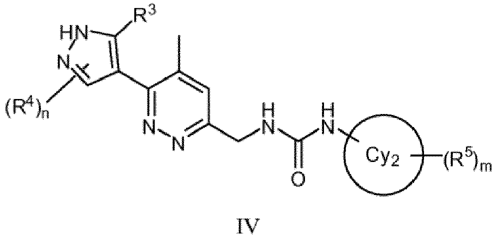

4. Verbindung oder pharmazeutisch annehmbares Salz dieser nach Anspruch 1, 2 oder 3, wobei R³ -CH₃, -CH₂CH₃ oder -(CH₂)₂CH₃ ist, von denen jedes mit unabhängig voneinander ausgewählten Halogenatomen, -CN, -C₁₋₄ Alkoxy oder -NR^{7a}R^{7b} substituiert ist.

5. Verbindung oder pharmazeutisch annehmbares Salz dieser nach Anspruch 1, 2 oder 3, wobei R³C₃₋₇ monocyclisches Cycloalkyl ist.

6. Verbindung oder pharmazeutisch annehmbares Salz dieser nach Anspruch 1, 2 oder 3, wobei die Verbindung gemäß einer der Formeln Va-Vb ist:

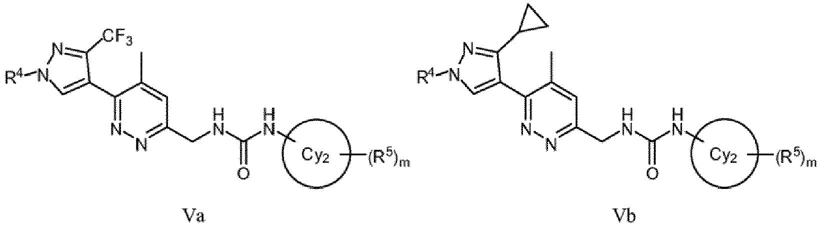

7. Verbindung oder pharmazeutisch annehmbares Salz dieser nach einem der Ansprüche 1-6, wobei R⁴C₁₋₄ Alkyl ist.

8. Verbindung oder pharmazeutisch annehmbares Salz dieser nach einem der Ansprüche 1-7, wobei Cy₂ Pyridinyl ist.

9. Verbindung oder pharmazeutisch annehmbares Salz dieser nach einem der Ansprüche 1-8, wobei der Index m 1, 2, 3 oder 4 ist.

10. Verbindung oder pharmazeutisch annehmbares Salz dieser nach einem der Ansprüche 1-9, wobei R⁵ C₁₋₄ Alkyl ist, substituiert mit einer oder mehreren unabhängig voneinander ausgewählten R¹³ -Gruppen.

11. Verbindung oder pharmazeutisch annehmbares Salz dieser nach Anspruch 10, wobei R¹³ F, -CN, -OH, -OCH₃, -OCF₃, -OCH₂CH₃, -OCH₂CF₃, -OCH₂CH₂OH oder -OCH₂CH₂OCH₃ ist.

12. Verbindung oder pharmazeutisch annehmbares Salz dieser nach einem der Ansprüche 1-11, wobei R⁵ C₁₋₄ Alkoxy ist.

13. Pharmazeutische Zusammensetzung, umfassend eine Verbindung oder ein pharmazeutisch annehmbares Salz dieser nach einem der Ansprüche 1-12 und einen pharmazeutisch annehmbaren Trägerstoff.

14. Pharmazeutische Zusammensetzung nach Anspruch 13, umfassend einen weiteren therapeutischen Wirkstoff.

15. Verbindung oder pharmazeutisch annehmbares Salz dieser nach einem der Ansprüche 1-12 oder pharmazeutische Zusammensetzung nach Anspruch 13 oder 14 zur Anwendung in der Medizin.

16. Verbindung oder pharmazeutisch annehmbares Salz dieser nach einem der Ansprüche 1-12 oder pharmazeutische Zusammensetzung nach Anspruch 13 oder 14 zur Anwendung in der Prophylaxe und/oder Behandlung von fibrotischen Erkrankungen, entzündlichen Erkrankungen, Autoimmunerkrankungen, Stoffwechselerkrankungen. Herz-Kreislauf-Erkrankungen und/oder proliferativen Erkrankungen.

17. Pharmazeutische Zusammensetzung nach Anspruch 14, wobei der weitere therapeutische Wirkstoff ein Mittel zur Prophylaxe und/oder Behandlung von fibrotischen Erkrankungen, entzündlichen Erkrankungen, Autoimmunerkrankungen, Stoffwechselerkrankungen, Herz-Kreislauferkrankungen und/oder proliferativen Erkrankungen ist.

## Revendications

1. Composé de Formule la : 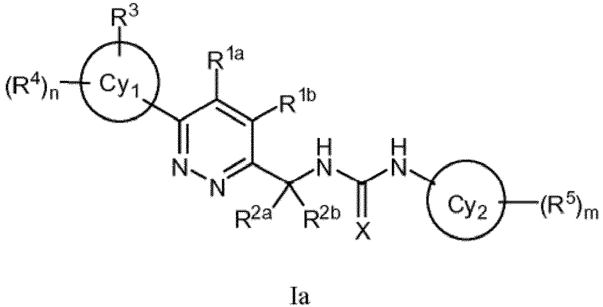 dans laquelle
X est =O, ou =N-CN ;
R^{1a} est sélectionné parmi :
- un groupe alkyle en C₁ à C₄ éventuellement substitué par un ou plusieurs groupes sélectionnés indépendamment parmi
∘ OH,
∘ un groupe alcoxy en C₁ à C₄ éventuellement substitué par un ou plusieurs groupes OH, ou alcoxy en C₁ à C₄ sélectionnés indépendamment,
∘ -SO₂-alkyle en C₁ à C₄,
∘ -O-cycloalkyle monocyclique en C₃ à C₇, et
∘ -O-hétérocycloalkyle dans laquelle ledit groupe hétérocycloalkyle est un groupe hétérocycloalkyle monocyclique de 4 à 7 chaînons comprenant un, deux, ou trois hétéroatomes sélectionnés indépendamment parmi N, O et S ;
- -NR^{6a}R^{6b},
- alcoxy en C₁ à C₄,
- cycloalkyle monocyclique en C₃ à C₇,
- hétérocycloalkyle monocyclique de 4 à 7 chaînons comprenant un, deux, ou trois hétéroatomes sélectionnés indépendamment parmi N, O et S, éventuellement substitués par un ou plusieurs groupes halogéno,
- -O-cycloalkyle monocyclique en C₃ à C₇, et
- -O-hétérocycloalkyle dans laquelle ledit groupe hétérocycloalkyle est un groupe hétérocycloalkyle monocyclique de 4 à 7 chaînons comprenant un, deux ou trois hétéroatomes sélectionnés indépendamment parmi N, O, et S ;
R^{1b} est H, ou alkyle en C₁ à C₄ ;
Cy₁ est un cycle hétéroaryle monocyclique de 5 chaînons, comprenant un, deux, ou trois hétéroatomes sélectionnés indépendamment parmi N, O, ou S, ou
Cy₁ est un cycle hétérocycloalkyle monocyclique de 4 à 7 chaînons comprenant un, deux, ou trois hétéroatomes sélectionnés indépendamment parmi N, O, ou S, ou un cycle hétérocycloalkyle monocyclique de 4 à 7 chaînons comprenant un, deux, ou trois hétéroatomes sélectionnés indépendamment parmi N, O ou S, fusionnés à un cycle hétéroaryle de 5 à 6 chaînons comprenant un, deux, ou trois hétéroatomes sélectionnés indépendamment parmi N, O, ou S, lequel cycle hétéroaryle peut éventuellement être substitué par un groupe alkyle en C₁ à C₄ ;
chaque R^{2a} et R^{2b} est sélectionné indépendamment parmi H, et un groupe alkyle en C₁ à C₄ éventuellement substitué par un ou plusieurs groupes -OH, ou alcoxy en C₁ à C₄, sélectionnés indépendamment ;
R³ est sélectionné parmi :
- un groupe alkyle en C₁ à C₄ éventuellement substitué par un ou plusieurs groupes sélectionnés indépendamment :
∘ halogéno
∘ -CN,
∘ -OH,
∘ -alcoxy en C₁ à C₄, ou
∘ -NR^{7a}R^{7b},
- alcoxy en C₁ à C₄ substitué par un ou plusieurs groupes halogéno,
- cycloalkyle monocyclique en C₃ à C₇,
- hétérocycloalkyle monocyclique de 4 à 7 chaînons comprenant un, deux ou trois hétéroatomes sélectionnés indépendamment parmi N, O, et S,
- -CN,
- -S(O)₂-alkyle en C₁ à C₄,
- -NR^{8a}R^{8b}, et
- -C(=O)NR^{8c}R^{8d} ;
chaque R⁴ est sélectionné indépendamment parmi :
- un groupe alkyle en C₁ à C₄ éventuellement substitué par un ou plusieurs groupes R¹² sélectionnés indépendamment,
- cycloalkyle monocyclique en C₃ à C₇, et
- hétérocycloalkyle monocyclique de 4 à 7 chaînons comprenant un, deux ou trois hétéroatomes sélectionnés indépendamment parmi N, O, et S ;
Cy₂ est un groupe phényle ou hétéroaryle monocyclique de 5 à 6 chaînons comprenant un ou deux hétéroatomes sélectionnés indépendamment parmi N, O, et S ;
chaque R⁵ est sélectionné indépendamment parmi :
- un groupe halogéno,
- -CN,
- -OH,
- alkyle en C₁ à C₄ éventuellement substitué par un ou plusieurs groupes R¹³ sélectionnés indépendamment,
- alcoxy en C₁ à C₄ éventuellement substitué par un ou plusieurs groupes R¹³ sélectionnés indépendamment,
- cycloalkyle monocyclique en C₃ à C₇ éventuellement substitué par un ou plusieurs groupes R¹⁴ sélectionnés indépendamment,
- hétérocycloalkyle monocyclique de 4 à 11 chaînons, ou bicyclique fusionné ou spiro comprenant un, deux, ou trois hétéroatomes sélectionnés indépendamment parmi N, O, et S, éventuellement substitués par un ou plusieurs groupes R¹⁴ sélectionnés indépendamment,
- O-cycloalkyle monocyclique en C₃ à C₇ éventuellement substitué par un ou plusieurs groupes R¹⁴ sélectionnés indépendamment,
- -O-hétérocycloalkyle dans laquelle ledit groupe hétérocycloalkyle est un groupe hétérocycloalkyle monocyclique de 4 à 7 chaînons comprenant un, deux, ou trois hétéroatomes sélectionnés indépendamment parmi N, O, et S, éventuellement substitués par un ou plusieurs groupes R¹⁴ sélectionnés indépendamment, et dans laquelle si un hétéroatome de N est présent, ledit hétéroatome de N est en outre substitué par un groupe --C(=O)-alkyle en C₁ à C₄, -C(=O)-alcoxy en C₁ à C₄, -SO₂-alkyle en C₁ à C₄, -C(=O)-NH₂, -C(=O)NH-alkyle en C₁ à C₄, ou -C(=O)N(alkyle en C₁ à C₄)₂,
- -SO₂-alkyle en C₁ à C₄,
- -SO₂NR^{15a}R^{15b},
- -C(=O)NR^{15c}R^{15d}, et
- ₋NR^{17a}R^{17b};
chaque R¹² est sélectionné indépendamment parmi :
- un groupe halogéno,
- OH,
- alcoxy en C₁ à C₄,
- -SO₂-alkyle en C₁ à C₄,
- cycloalkyle monocyclique en C₃ à C₇ éventuellement substitué par un ou plusieurs groupes -OH, halogéno, -CN, alkyle en C₁ à C₄, alcoxy en C₁ à C₄ ou =O sélectionnés indépendamment,
- hétérocycloalkyle monocyclique de 4 à 7 chaînons, comprenant un, deux, ou trois hétéroatomes sélectionnés indépendamment parmi N, O, et S, éventuellement substitué par un ou plusieurs groupes -OH, halogéno, -CN, alkyle en C₁ à C₄, alcoxy en C₁ à C₄ ou =O sélectionnés indépendamment,
- -NR^{9a}R^{9b}, et
- -CN ;
chaque R¹³ est sélectionné indépendamment parmi :
- un groupe halogéno,
- -CN,
- -OH,
- alcoxy en C₁ à C₄ éventuellement substitué par un ou plusieurs groupes OH, alcoxy en C₁ à C₄, ou halogéno sélectionnés indépendamment,
- -C(=O)NR^{16a}R^{16b},
- -NR^{16c}C(=O)-alkyle en C₁ à C₄,
- -NR^{16d}C(=O)-alcoxy en C₁ à C₄,
- -SO₂-alkyle en C₁ à C₄
- -SO₂NR^{16e}R^{16f},
- -NR^{16g}SO₂-alkyle en C₁ à C₄,
- cycloalkyle monocyclique en C₃ à C₇ éventuellement substitué par un ou plusieurs groupes halogéno ou alkyle en C₁ à C₄ sélectionnés indépendamment, éventuellement substitué par un ou plusieurs groupes halogéno, et
- hétérocycloalkyle monocyclique de 4 à 7 chaînons comprenant un, deux, ou trois hétéroatomes sélectionnés indépendamment parmi N, O, et S, éventuellement substitué par un ou plusieurs groupes halogéno, ou alkyle en C₁ à C₄ sélectionné indépendamment, éventuellement substitué par un ou plusieurs groupes halogéno, et dans laquelle si un hétéroatome de N est présent, ledit hétéroatome de N est en outre substitué par un groupe -C(=O)-alkyle en C₁ à C₄, -C(=O)-alcoxy en C₁ à C₄, -SO₂-alkyle en C₁ à C₄, -C(=O)-NH₂, -C(=O)NH-alkyle en C₁ à C₄, ou -C(=O)N(alkyle en C₁ à C₄)₂ ;
chaque R¹⁴ est sélectionné indépendamment parmi :
- un groupe halogéno,
- -CN,
- -OH,
- alcoxy en C₁ à C₄ éventuellement substitué par un ou plusieurs groupes halogéno, et
- alkyle en C₁ à C₄ éventuellement substitué par un ou plusieurs groupes halogéno ;
chaque R^{6a}, R^{6b}, R^{7a}, R^{7b}, R^{8a}, R^{8b}, R^{8c}, R^{8d}, R^{9a}, R^{9b}, R^{15a}, R^{15b}, R^{15c}, R^{15d}, R^{16a}, R^{16b}, R^{16c}, R^{16d}, R^{16e}, R^{16f}, et R^{16g} est sélectionné indépendamment parmi H et un groupe alkyle en C₁ à C₄;
chaque R^{17a} et R^{17b} est sélectionné indépendamment parmi H et un groupe alkyle en C₁ à C₄ éventuellement substitué par un ou plusieurs groupes halogéno, OH, ou alcoxy en C₁ à C₄ sélectionnés indépendamment ;
l'indice n est 0, 1, 2, ou 3 ; et
l'indice m est 0, 1, 2, 3 ou 4 ;
ou sel de celui-ci, ou le solvate ou sel d'un solvate de celui-ci, pharmaceutiquement acceptable.

2. Composé ou sel pharmaceutiquement acceptable de celui-ci selon la revendication 1, dans lequel le composé est selon la Formule Ib : 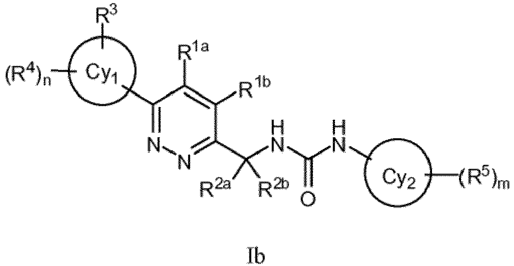 dans laquelle
R^{Ia} est sélectionné parmi :
- un groupe alkyle en C₁ à C₄ éventuellement substitué par un ou plusieurs groupes sélectionnés indépendamment parmi
∘ OH,
∘ un groupe alcoxy en C₁ à C₄ éventuellement substitué par un ou plusieurs groupes OH, ou alcoxy en C₁ à C₄ sélectionnés indépendamment,
∘ -SO₂-alkyle en C₁ à C₄,
∘ -O-cycloalkyle monocyclique en C₃ à C₇, et
∘ -O-hétérocycloalkyle dans laquelle ledit groupe hétérocycloalkyle est un groupe hétérocycloalkyle monocyclique de 4 à 7 chaînons comprenant un, deux, ou trois hétéroatomes sélectionnés indépendamment parmi N, O, et S ;
- -NR^{6a}R^{6b},
- alcoxy en C₁ à C₄,
- cycloalkyle monocyclique en C₃ à C₇,
- hétérocycloalkyle monocyclique de 4 à 7 chaînons comprenant un, deux, ou trois hétéroatomes sélectionnés indépendamment parmi N, O et S, substitué éventuellement par un ou plusieurs groupes halogéno,
- -O-cycloalkyle monocyclique en C₃ à C₇, et
- -O-hétérocycloalkyle dans laquelle ledit groupe hétérocycloalkyle est un groupe hétérocycloalkyle monocyclique de 4 à 7 chaînons comprenant un, deux, ou trois hétéroatomes sélectionnés indépendamment parmi N, O, et S ;
R^{1b} est H, ou un groupe alkyle en C₁ à C₄ ;
Cy₁ est un cycle hétéroaryle monocyclique de 5 chaînons, comprenant un, deux, ou trois hétéroatomes sélectionnés indépendamment parmi N, O, ou S ;
chaque R^{2a} et R^{2b} est sélectionné indépendamment parmi H, et un groupe alkyle en C₁ à C₄ éventuellement substitué par un ou plusieurs groupes -OH, ou alcoxy en C₁ à C₄, sélectionnés indépendamment ;
R³ est sélectionné parmi :
- un groupe alkyle en C₁ à C₄ éventuellement substitué par un ou plusieurs groupes sélectionnés indépendamment :
∘ un groupe halogéno
∘ -CN,
∘ -alcoxy en C₁ à C₄, ou
∘ -NR^{7a}R^{7b};
- alcoxy en C₁ à C₄ substitué par un ou plusieurs groupes halogéno,
- cycloalkyle monocyclique en C₃ à C₇,
- hétérocycloalkyle monocyclique de 4 à 7 chaînons comprenant un, deux ou trois hétéroatomes sélectionnés indépendamment parmi N, O, et S,
- -CN,
- -NR^{8a}R^{8b}, et
- -C(=O)NR^{8c}R^{8d};
chaque R⁴ est sélectionné indépendamment parmi :
- un groupe alkyle en C₁ à C₄ éventuellement substitué par un ou plusieurs groupes R¹² sélectionnés indépendamment,
- cycloalkyle monocyclique en C₃ à C₇, et
- hétérocycloalkyle monocyclique de 4 à 7 chaînons comprenant un, deux, ou trois hétéroatomes sélectionnés indépendamment parmi N, O, et S ;
Cy₂ est un groupe phényle ou hétéroaryle monocyclique de 5 à 6 chaînons comprenant un ou deux hétéroatomes sélectionnés indépendamment parmi N, O, et S ;
chaque R⁵ est sélectionné indépendamment parmi :
- un groupe halogéno,
- -CN,
- -OH,
- alkyle en C₁ à C₄ éventuellement substitué par un ou plusieurs groupes R¹³ sélectionnés indépendamment,
- alcoxy en C₁ à C₄ éventuellement substitué par un ou plusieurs groupes R¹³ sélectionnés indépendamment,
- cycloalkyle monocyclique en C₃ à C₇ éventuellement substitué par un ou plusieurs groupes R¹⁴ sélectionnés indépendamment,
- hétérocycloalkyle monocyclique de 4 à 11 chaînons, ou bicyclique fusionné ou spiro comprenant un, deux, ou trois hétéroatomes sélectionnés indépendamment parmi N, O, et S, éventuellement substitué par un ou plusieurs groupes R¹⁴ sélectionnés indépendamment,
- O-cycloalkyle monocyclique en C₃ à C₇ éventuellement substitué par un ou plusieurs groupes R¹⁴ sélectionnés indépendamment,
- -O-hétérocycloalkyle dans laquelle ledit groupe hétérocycloalkyle est un groupe hétérocycloalkyle monocyclique de 4 à 7 chaînons comprenant un, deux, ou trois hétéroatomes sélectionnés indépendamment parmi N, O, et S, éventuellement substitué par un ou plusieurs groupes R¹⁴ sélectionnés indépendamment, et dans laquelle si un hétéroatome de N est présent, ledit hétéroatome de N est en outre substitué par un groupe -C(=O)-alkyle en C₁ à C₄, -C(=O)-alcoxy en C₁ à C₄, -SO₂-alkyle en C₁ à C₄, -C(=O)-NH₂, -C(=O)NH-alkyle en C₁ à C₄, ou -C(=O)N(alkyle en C₁ à C₄)₂,
- -SO₂-alkyle en C₁ à C₄,
- -SO₂NR^{15a}R^{15b},
- -C(=O)NR^{15c}R^{15d}, et
- -NR^{17a}R^{17b};
chaque R¹² est sélectionné indépendamment parmi :
- un groupe halogéno,
- OH,
- alcoxy en C₁ à C₄,
- -SO₂-alkyle en C₁ à C₄,
- cycloalkyle monocyclique en C₃ à C₇ éventuellement substitué par un ou plusieurs groupes -OH, halogéno, -CN, alkyle en C₁ à C₄, alcoxy en C₁ à C₄ ou =O sélectionnés indépendamment,
- hétérocycloalkyle monocyclique de 4 à 7 chaînons, comprenant un, deux, ou trois hétéroatomes sélectionnés indépendamment parmi N, O, et S, éventuellement substitué par un ou plusieurs groupes -OH, halogéno, -CN, alkyle en C₁ à C₄, alcoxy en C₁ à C₄ ou =O sélectionnés indépendamment,
- -NR^{9a}R^{9b}, et
- -CN ;
chaque R¹³ est sélectionné indépendamment parmi :
- un groupe halogéno,
- -CN,
- -OH,
- alcoxy en C₁ à C₄ éventuellement substitué par un ou plusieurs groupes OH, alcoxy en C₁ à C₄, ou halogéno sélectionnés indépendamment,
- -C(=O)NR^{16a}R^{16b},
- -NR^{16c}C(=O)-alkyle en C₁ à C₄,
- -NR¹⁶¹C(=O)-alcoxy en C₁ à C₄,
- -SO₂-alkyle en C₁ à C₄
- -SO₂NR^{16e}R^{16f},
- -NR^{16g}SO₂-alkyle en C₁ à C₄,
- cycloalkyle monocyclique en C₃ à C₇ éventuellement substitué par un ou plusieurs groupes halogéno ou alkyle en C₁ à C₄ sélectionnés indépendamment, éventuellement substitués par un ou plusieurs groupes halogéno, et
- hétérocycloalkyle monocyclique de 4 à 7 chaînons comprenant un, deux, ou trois hétéroatomes sélectionnés indépendamment parmi N, O, et S, éventuellement substitué par un ou plusieurs groupes halogéno, ou alkyle en C₁ à C₄ sélectionnés indépendamment, éventuellement substitué par un ou plusieurs groupes halogéno, et dans laquelle si un hétéroatome de N est présent, ledit hétéroatome de N est en outre substitué par un groupe -C(=O)-alkyle en C₁ à C₄, -C(=O)-alcoxy en C₁ à C₄, -SO₂-alkyle en C₁ à C₄, -C(=O)-NH₂, -C(=O)NH-alkyle en C₁ à C₄, ou -C(=O)N(alkyle en C₁ à C₄)₂;
chaque R¹⁴ est sélectionné indépendamment parmi :
- un groupe halogéno,
- -CN,
- -OH,
- alcoxy en C₁ à C₄ éventuellement substitué par un ou plusieurs groupes halogéno, et
- alkyle en C₁ à C₄ éventuellement substitué par un ou plusieurs groupes halogéno ;
chaque R^{6a}, R^{6b}, R^{7a},R^{7b},R^{8a},R^{8b},R^{8c},R^{8d},R^{9a},R^{9b},R^{15a},R^{15b},R¹⁵C,R^{15d},R^{16a},R^{16b},R^{16c}, R^{16d},R^{16e},R^{16f}, et R^{16g} est sélectionné indépendamment parmi H et un groupe alkyle en C₁ à C₄ ;
chaque R^{17a} et R^{17b} est sélectionné indépendamment parmi H et un groupe alkyle en C₁ à C₄ éventuellement substitué par un ou plusieurs groupes halogéno, OH, ou alcoxy en C₁ à C₄ sélectionnés indépendamment ;
l'indice n est 0, 1, 2, ou 3 ; et
l'indice m est 0, 1, 2, 3 ou 4 ;
ou sel de celui-ci, ou le solvate ou sel d'un solvate de celui-ci, pharmaceutiquement acceptables.

3. Composé ou sel pharmaceutiquement acceptable de celui-ci selon la revendication 1 ou 2, dans lequel le composé est selon la Formule IV :

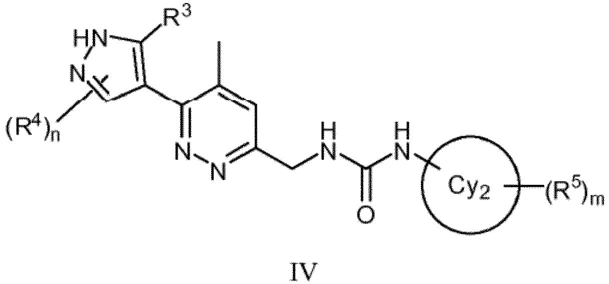

4. Composé ou sel pharmaceutiquement acceptable de celui-ci selon la revendication 1, 2, ou 3, dans lequel R³ est -CH₃, -CH₂CH₃, ou -(CH₂)₂CH₃, chacun d'eux étant substitué par un, deux ou trois groupes halogéno, -CN, -alcoxy en C₁ à C₄, ou -NR^{7a}R^{7b} sélectionnés indépendamment.

5. Composé ou sel pharmaceutiquement acceptable de celui-ci selon la revendication 1, 2 ou 3, dans lequel R³ est un groupe cycloalkyle monocyclique en C₃ à C₇.

6. Composé ou sel pharmaceutiquement acceptable de celui-ci selon la revendication 1, 2 ou 3, dans lequel le composé est selon l'une quelconque des Formules Va à Vb :

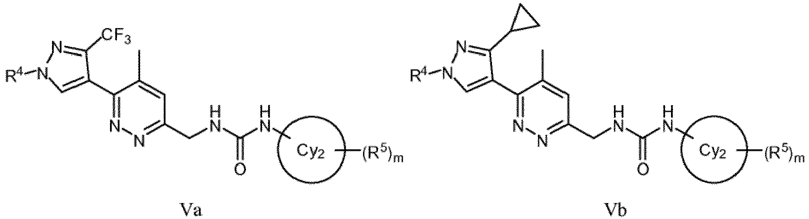

7. Composé ou sel pharmaceutiquement acceptable de celui-ci selon l'une quelconque des revendications 1 à 6, dans lequel R⁴ est un groupe alkyle en C₁ à C₄.

8. Composé ou sel pharmaceutiquement acceptable de celui-ci selon l'une quelconque des revendications 1 à 7, dans lequel Cy₂ est un groupe pyridinyle.

9. Composé ou sel pharmaceutiquement acceptable de celui-ci selon l'une quelconque des revendications 1 à 8, dans lequel l'indice m est 1, 2, 3, ou 4.

10. Composé ou sel pharmaceutiquement acceptable de celui-ci selon l'une quelconque des revendications 1 à 9, dans lequel R⁵ est un groupe alkyle en C₁ à C₄ substitué par un ou plusieurs groupes R¹³ sélectionnés indépendamment.

11. Composé ou sel pharmaceutiquement acceptable de celui-ci selon la revendication 10, dans lequel R¹³ est F, -CN, -OH, -OCH₃, -OCF₃, -OCH₂CH₃, -OCH₂CF₃, -OCH₂CH₂0H, ou -OCH₂CH₂OCH₃.

12. Composé ou sel pharmaceutiquement acceptable de celui-ci selon l'une quelconque des revendications 1 à 11, dans lequel R⁵ est un groupe alcoxy en C₁ à C₄.

13. Composition pharmaceutique comprenant un composé ou un sel pharmaceutiquement acceptable de celui-ci selon l'une quelconque des revendications 1 à 12, et un véhicule pharmaceutiquement acceptable.

14. Composition pharmaceutique selon la revendication 13 comprenant un autre agent thérapeutique.

15. Composé ou sel pharmaceutiquement acceptable de celui-ci, selon l'une quelconque des revendications 1 à 12, ou composition pharmaceutique selon la revendication 13 ou 14 pour une utilisation en médecine.

16. Composé ou sel pharmaceutiquement acceptable de celui-ci, selon l'une quelconque des revendications 1 à 12, ou composition pharmaceutique selon la revendication 13 ou 14 pour une utilisation en prophylaxie et/ou traitement de maladies fibrotiques, de maladies inflammatoires, de maladies auto-immunes, de maladies métaboliques, de maladies cardiovasculaires, et/ou de maladies prolifératives.

17. Composition pharmaceutique selon la revendication 14, dans laquelle l'autre agent thérapeutique est un agent pour la prophylaxie et/ou le traitement de maladies fibrotiques, de maladies inflammatoires, de maladies auto-immunes, de maladies métaboliques, de maladies cardiovasculaires, et/ou de maladies prolifératives.
